# EUROPEAN PATENT SPECIFICATION

(11) **EP 2 844 642 B1**
(45) Date of publication and mention of the grant of the patent: **13.11.2019**
(21) Application number: 13788352.6
(22) Date of filing: 15.03.2013
(51) Int. Cl.: C07D 239/48, C07D 403/12, C07F 9/6561, C07F 9/6584, C07F 9/6512, C07F 9/6521, A61K 31/505, A61K 31/506, A61P 35/00

(54) **COMPOUNDS FOR INHIBITING CELL PROLIFERATION IN EGFR-DRIVEN CANCERS**
VERFAHREN ZUR HEMMUNG DER ZELLPROLIFERATION BEI EFGR-VERMITTELTEM KREBS
COMPOSÉS POUR INHIBER LA PROLIFÉRATION CELLULAIRE DANS LES CANCERS INDUITS PAR L'EGFR

(30) Priority: 05.05.2012 US 201261643228 P; 07.11.2012 US 201261723779 P; 08.11.2012 US 201261724283 P
(43) Date of publication of application: 11.03.2015
(73) Proprietor: ARIAD PHARMACEUTICALS, INC., Cambridge, MA 02139-4234 (US); Zhu, Xiaotian, Newton, MA 02465 (US); Wang, Yihan, Newton, MA 02460 (US); Shakespeare, William C., Southborough, MA 01772 (US); Huang, Wei-Sheng, Acton, MA 01720 (US); Dalgarno, David C., Brookline, MA 02446 (US)
(72) Inventor: ZHU, Xiaotian, Newton, MA 02465 (US); WANG, Yihan, Newton, MA 02460 (US); SHAKESPEARE, William, C., Southborough, MA 01772 (US); HUANG, Wei-Sheng, Acton, MA 01720 (US); DALGARNO, David, C., Brookline, MA 02446 (US)
(74) Representative: D Young & Co LLP
(86) International application number: PCT/US2013/032713
(87) International publication number: WO 2013/169401

(56) References cited:
- WO-A1-2009/143389
- WO-A1-2011/090760
- WO-A1-2012/051587
- US-A1- 2011 230 494
- US-A1- 2012 094 999
- US-A1- 2012 316 135

## Description

### Background of the Invention

This invention relates to compounds, pharmaceutical compositions and compounds for use in the treatment of EGFR-driven cancers.

In human clinical studies with non-small cell lung cancer (NSCLC) patients, the kinase inhibitors, erlotinib and gefitinib have been found to be effective, but in only a subset of patients. It was later determined that the responsive patients had certain mutations in the gene for epidermal growth factor receptor (EGFR). The mutant forms of EGFR are enzymatically active without the need for ligand stimulation. They are also particularly sensitive to kinase inhibitors like erlotinib and gefitinib, which competitively bind to the ATP binding site of the EGFR kinase domain. Those mutations have been cataloged and described at length in the scientific literature. They include small deletions or point mutations in the kinase domain as has previously been written about extensively. See e.g., Sharma, Nat. Rev. Cancer 7:169 (2007) (exon 19 mutations characterized by in-frame deletions of amino-acids 747 account for 45% of mutations, exon 21 mutations resulting in L858R substitutions account for 40-45% of mutations, and the remaining 10% of mutations involve exon 18 and 20); Sordella et al., Science 305:1163 (2004); and Mulloy et al., Cancer Res. 67:2325 (2007).

Unfortunately, additional mutations in the EGFR gene, e.g., the T790M mutation, render drugs like erlotinib and gefitinib less effective. Those mutations are associated with resistance to the drugs and to relapse in patients with cancer cells having the T790M mutation.

New therapies are needed for the treatment of EGFR-driven cancers in which mutations confer resistance to front line tyrosine kinase inhibitor ("TKI") therapies. In particular, new therapies for inhibiting cells expressing such gefitinib-resistant or erlotinib-resistant EGFR genes could be of profound benefit.

### Summary of the Invention

This invention relates to the discovery of a class of compounds that inhibit EGFR and medically significant mutant forms thereof. Those mutants include mutant EGFR proteins that are enzymatically active in the absence of protein ligand and mutants such as the T790M EGFR mutant that are resistant to the EGFR inhibitors erlotinib and gefitinib. Compounds of this invention possessing desirable selectivity for the intended biological targets and advantageous pharmaceutical properties make them of interest for treating cancers characterized by the expression of EGFR or an EGFR mutant, especially in cases that are resistant or refractory to erlotinib or gefitinib.

This invention provides a compound as defined in claim 1.

For reference only, also described herein is a compound of Formula (I): 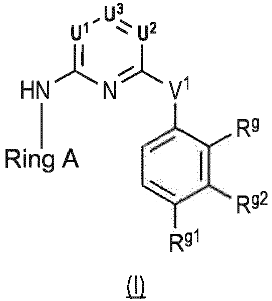 or a pharmaceutically acceptable salt thereof, wherein
U¹ and U² are both N and U³ is C-R^{e}; or U³ is N, one of U¹ and U² is N, and the other is C-R^{d}; or U³ is C-R^{e}, one of U¹ and U² is N, and the other is C-R^{d};
**V¹** is O, S, NR^{V}, CO, CH₂, or CF₂;
**R^{V}** is H, alkyl, alkenyl, alkynyl, cycloalkyl, cycloalkenyl, cycloalkynyl, or aryl;
**R^{d}** is H, halo, CN, alkyl, cycloalkyl, alkoxy, haloalkyl, alkenyl, haloalkenyl or halocycloalkyl;
**R^{e}** is H or NH₂; or, **R^{d}** and **R^{e},** together with the ring atom to which each is attached, form a 5- or 6-membered ring containing one, two or three heteroatoms, independently selected from N, S and O, wherein the 5- or 6-membered ring so formed is substituted with R^{h} which is C₁₋₄ alkyl or halo;
**R^{g}** is H, F, -P(O)(R^{3A})(R^{3B}), -S(O)N(R^{3C})(R^{3D}), -S(O)₂R^{3E}, -C(O)N(R^{3F})(R^{3G}), -OC(O)N(R^{3F})(R^{3G}), -NR^{3H}C(O)OR^{3I} or a 5- or 6- membered heterocyclic ring comprising 1, 2, 3 or 4 N atoms;
each R^{3A}, R^{3B}, R^{3C}, R^{3D}, R^{3E}, R^{3F}, R3G, R3H, and R^{3I} is independently selected from H, alkyl, alkenyl, alkynyl, cycloalkyl, cycloalkenyl, cycloalkynyl, and heteroalkyl, or R^{3A} and R^{3B}, or R^{3C} and R^{3D}, or R^{3F} and R^{3G}, together with the atom to which each is attached, form an unsubstituted or substituted 5- or 6-membered heterocyclic ring;
**R^{g2}** is H, F, **W¹,** -P(O)(R^{3A})(R^{3B}), -S(O)N(R^{3C})(R^{3D}), -S(O)₂R^{3E}, -C(O)N(R^{3F})(R^{3G}), -OC(O)N(R^{3F})(R^{3G}), -NR^{3H}C(O)OR^{3I}, C₁₋₆ alkoxy or C₁₋₄ alkyl;
or, **R^{g2}** and **R^{g}** together with the atom to which each is attached form an unsubstituted or substituted 5- to 7-membered heterocyclic ring comprising 1 - 3 heteroatoms independently selected from P, N , O and S;
**R^{g1}** is H, F, -OR², -P(O)(R^{3A})(R^{3B}), -S(O)N(R^{3C})(R^{3D}), -S(O)₂R^{3E}, -C(O)N(R^{3F})(R^{3G}), -OC(O)N(R^{3F})(R^{3G}), -NR^{3H}C(O)OR^{3I}, or substituted or unsubstituted 5- or 6- membered heterocyclic ring;
**Ring A** is: 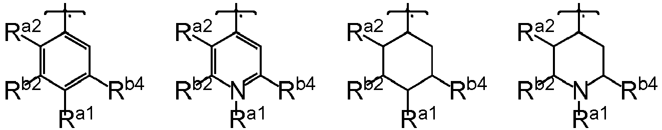 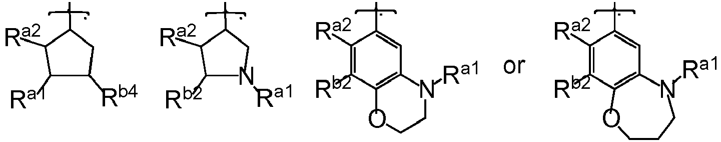
   **R^{b2}** is H, F, or an optionally substituted 5- or 6- membered heterocyclic ring containing 1, 2 or 3 N or O atoms;
   **R^{b4}** is H, F, **W¹,** C₁₋₆ alkoxy, C₃₋₆ alkenyloxy, C₃₋₆ cycloalkoxy, -OC(O)N(R^{5A})(R^{5B}), -NR^{5C}C(O)OR^{5D}, or substituted or unsubstituted 5- or 6- membered heterocyclic ring comprising 1, 2 or 3 N or O atoms;
   each **R^{5A}, R^{5B}, R^{5C}** and **R^{5D}** is independently H, alkyl, alkenyl, alkynyl or heteroalkyl, or R^{5A} and R^{5B}, together with the atom to which each is attached, form a substituted or unsubstituted 5- or 6-membered heterocyclic ring;
   **R^{a1}** is H, halo, **W¹,** -CN, -NO₂, -R¹, -OR², -O-NR¹R², -NR¹R², -NR¹-NR¹R², -NR¹-OR², -C(O)YR², -OC(O)YR², -NR¹C(O)YR², -SC(O)YR², -NR¹C(=S)YR², -OC(=S)YR², -C(=S)YR², -YC(=NR¹)YR², -YC(=N-OR¹)YR², -YC(=N-NR¹R²)YR², -Y**P**(=O)(YR¹)(YR²), -S(O)ᵣR², -SO₂NR¹R²,
   -NR¹SO₂NR¹R² or 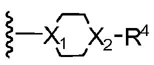 and **X₁** and **X₂** are each independently CH or N;
   or **R^{a1}** and **R^{b4},** together with the atom to which each is attached, form an optionally substituted 5- or 6- membered heterocyclic ring comprising 1, 2 or 3 heteroatoms independently selected from N and O;
   **R^{a2}** is H, halo, C₁₋₆ alkyl, C₂₋₆ alkenyl, C₃₋₆ cycloalkyl, C₁₋₆ alkoxy, C₂₋₆ alkenyloxy, C₃₋₆ cycloalkyloxy or 4- to 7-membered heterocyclyl, wherein the alkyl, alkenyl, cycloalkyl, alkoxy, C₂₋₆ alkenyloxy, cycloalkyloxy and heterocyclyl are unsubstituted or substituted with one or more halo, amino, C₁₋₆ alkylamino, or di-C₁₋₆ alkylamino groups;
   **Y** is independently a bond, -O-, -S- or -NR¹-;
   **R¹** and **R²** are independently H or R¹⁵;
   **R⁴** is alkyl, alkenyl, alkynyl, cycloalkyl, cycloalkenyl, cycloalkynyl, aryl, heteroalkyl, heterocyclic and heteroaryl;
   **W¹** is -NR⁷C(O)C(R¹¹)=CR⁹R¹⁰, -C(O)C(R¹¹)=CR⁹R¹⁰, -CH₂P(O)C(R¹¹)=CR⁹R¹⁰, -OP(O)C(R⁸)=CR⁹R¹⁰, -NR⁷S(O)₂C(R⁹)(R¹⁰)(**R^{X}**), -NR⁷S(O)₂C(R¹¹)=CR⁹R¹⁰, -NR⁷C(O)C≡C-**R¹⁴**, -NR⁷C(O)C(R⁹)(R¹⁰)(**R^{X}**), 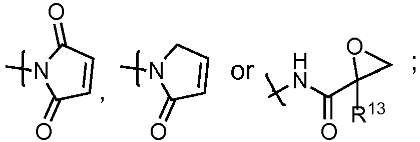
   **R^{X}** is halo;
   **R⁷** is H, alkyl or heteroalkyl, wherein the the alkyl and heteroalkyl groups are independently optionally substituted with an amino, alkylamino or dialkylamino group;
   **R⁸** is C₁₋₆ alkyl;
   **R⁹** and **R¹⁰** are independently H, halo, -C(O)**R¹⁶**, alkyl, alkoxy, alkenyl, alkynyl, cycloalkyl, cycloalkenyl, cycloalkynyl, aryl, heteroalkyl, heterocyclyl or heteroaryl, wherein **R⁹** and **R¹⁰,** if not H, are optionally substituted with one or more halo, amino, alkylamino, dialkylamino, alkoxy, cycloalkyl, heterocyclyl or heteroaryl groups, wherein said group, if not halo, is optionally substituted with one or more halo, C₁₋₄ alkyl, alkoxyl, halo(C₁₋₄)alkyl or C₃₋₇ cycloalkyl groups;
   **R¹¹** is H, halo, -C(O)-OR¹², alkyl, alkenyl, alkynyl, cycloalkyl, cycloalkenyl, cycloalkynyl, aryl, heteroalkyl, heterocyclic, or heteroaryl, wherein **R¹¹,** if not H, is optionally substituted with one or more halo, amino, alkoxyl, cycloalkyl, heterocyclic or heteroaryl groups, wherein said group, if not halo, is optionally substituted with one or more halo or alkyl, alkoxyl, cycloalkyl or heterocyclyl groups, wherein the alkyl, alkoxyl, cycloalkyl and heterocyclyl group is optionally substituted with one or more alkyl, halo or hydroxyl substituents;
   or **R⁹** and **R¹¹,** taken together with the atom to which each is attached, form a cycloalkenyl or heterocyclic ring;
   **R¹²** is alkyl, alkenyl, alkynyl, cycloalkyl, cycloalkenyl, cycloalkynyl, aryl, heteroalkyl, heterocyclic, or heteroaryl;
   **R13** is H or C₁₋₄ alkyl;
   **R¹⁴** is **R^{T}** or **R^{W};**
   **R¹⁵** is alkyl, alkenyl, alkynyl, cycloalkyl, cycloalkenyl, cycloalkynyl, aryl, heteroalkyl, 4- to 7-membered heterocyclyl, or heteroaryl, wherein each R¹⁵ is optionally substituted with one or more halo, cycloalkyl, heterocyclic or heteroaryl groups, wherein said cycloalkyl, heterocyclic or heteroaryl groups(s) are independently optionally substituted with one or more halo, alkyl, haloalkyl, hydroxyalkyl, amino, dialkylamino or cycloalkyl groups;
   **R¹⁶** is OH, -O-alkyl, cycloalkyl, heterocyclyl, -NH₂, -NH-alkyl, or -N-dialkyl wherein the alkyl, cycloalkyl or heterocyclyl moiety is optionally substituted with halo, amino, alkylamino, dialkylamino, alkyl or hydroxyl;
   **R^{T}** is H or -CH₃;
   **R^{W}** is halo; substituted methyl; or an optionaly substituted group selected from (C₂₋₆)alkyl, (C₁₋₆)heteroalkyl, heterocyclyl, aryl and heteroaryl; wherein the substituents on the optionally substituted (C₂₋₆)alkyl, (C₁₋₆)heteroalkyl, heterocyclyl, aryl and heteroaryl groups are selected from halo, haloalkyl, alkoxy, heterocyclyl, substituted heterocyclyl, amino, alkylamino, and dialkylamino, and in the case of an optionally substituted heterocyclyl, the optional substituents may further be selected from hydroxyl, alkyl, haloalkyl, hyroxyalkyl, alkoxyalkyl, amino, alkylamino and dialkylamino;
   wherein
   (a) the compound is not one of the following two compounds: 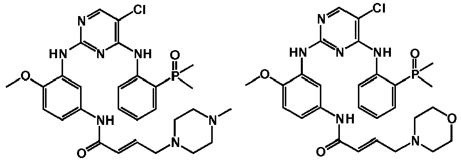
   (b) at least one of **R^{a1}, R^{g2},** and **R^{b4}** is **W¹;**
   (c) the compound comprises at least one -P(O)(R^{3A})(R^{3B}); and
   (d) the compound further has one or more of the following features:
      **R^{d}** is halo(C3-5)cycloalkyl;
      **R^{a2}** is halo; substituted alkyl, substituted alkoxy, or optionally substituted cycloalkyl, wherein substituents on the alkyl, alkoxy or cylcoalkyl groups are selected from halo, amino and dialkylamino groups;
      **R^{a1}** is an optionally substituted 4-membered heterocycle;
      **R^{a1}** is alkyl, alkenyl, alkynyl, cycloalkyl, cycloalkenyl or heteroalkyl, and is substituted with one or more groups selected from halo, OH, heterocyclyl, substituted heterocyclyl, heteroaryl, and substituted heteroaryl;
      **R^{a1}** is heterocyclyl or heterocyclyl-O-, wherein **R^{a1}** is substituted with one or more groups selected from -OH, halo, 4-membered heterocyclyl, substituted 4-membered heterocyclyl and R¹⁸, wherein R¹⁸ is alkyl, alkenyl, alkynyl, cycloalkyl, cycloalkenyl or heteroalkyl, and R¹⁸ is optionally substituted;
      **R^{a1}** and **R^{b4},** together with the atom to which each is attached, form an optionally substituted 5-membered heterocyclic ring;
      **R^{a2}** is an optionally substituted 4- to 7-membered membered heterocycle;
      **R^{b4}** is -NR⁷C(O)C(R¹¹)=CR⁹R¹⁰ or -NR⁷C(O)C-C≡C-**R**^{W};
      **R^{g1}** is -OR²;
      **R⁹** or **R¹⁰** is cycloalkyl, -CO₂H, -CO₂-alkyl, -C(O)-heterocyclyl, -C(O)NH2, -C(O)NH-alkyl or -C(O)N-dialkyl wherein an alkyl, cycloalkyl or heterocyclyl substituent or portion of a substituent is optionally substituted with amino, alkylamino, dialkylamino, alkyl or hydroxyl;
      one or more of R⁹, R¹⁰ and R¹¹ is halo, haloalkyl, alkyl, alkoxy, heteroalkyl, alkenyl, alkynyl, cycloalkyl, cycloalkenyl, cycloalkynyl, aryl, heterocyclyl, heterocyclylalkyl, heteroaryl, heteroarylalkyl or arylalkyl wherein alkyl, heterocylic, heteroaryl or aryl substituent, or an alkyl, heterocylic, heteroaryl or aryl portion of a substituent, is optionally substituted with one or more groups selected from halo, haloalkyl, hydroxyl, hydroxyalkyl, amino, alkylamino, dialkylamino, alkyl, alkenyl, SO₂alkyl, oxo, heterocyclyl and heterocycle substituted with one or more alkyl, amino alkylamino, dialkylamino, hydroxyl, hydroxyalkyl, SO₂alkyl substituents; and,
      **R⁹** and **R¹¹,** taken together with the atom to which each is attached, form a cycloalkenyl or heterocyclic ring.

### DETAILED DESCRIPTION

The invention features a compound for use in a method of treating an EGFR-driven cancer in a subject.

In a related aspect, the invention features a compound for use in a method of treating an EGFR-driven cancer in a subject, the method including (a) providing a subject having an EGFR-driven cancer characterized by the presence of a mutation in epidermal growth factor receptor kinase (EGFR), and (b) administering to the subject a therapeutically effective amount of a compound of the invention, or a pharmaceutically acceptable salt thereof. In certain embodiments, EGFR-driven cancer is characterized by the presence of one or more mutations selected from: (i) L858R, (ii) T790M, (iii) both L858R and T790M, (iv) delE746_A750, and (v) both delE746_A750 and T790M.

In the above uses, the EGFR-driven cancer can be a non-small cell lung cancer (NSCLS); glioblastoma; pancreatic cancer; head and neck cancer (e.g., squamous cell carcinoma); breast cancer; colorectal cancer; epithelial cancer; ovarian cancer; prostate cancer; an adenocarcinoma, or any EGFR-driven cancer described herein.

In certain embodiments of the above uses, the method further includes administering to the subject a first kinase inhibitor selected from erlotinib, gefitinib, and pharmaceutically acceptable salts thereof, within 6 days (e.g., within 2 weeks, 1 week, 6 days, 5 days, 4 days, 3 days, 2 days, 1 day, or simultaneously) of administering the compound of the invention, wherein each of the compound of the invention and the first kinase inhibitor are administered in an amount that together is sufficient to treat the EGFR-driven cancer.

Described herein is a method of inhibiting the proliferation of a cell expressing an EGFR mutant, the method including contacting the cell with a compound of the invention, or a pharmaceutically acceptable salt thereof, in an amount sufficient to inhibit the proliferation. For example, the EGFR mutant can be characterized by the presence of one or more mutations in epidermal growth factor receptor kinase (EGFR) selected from: (i) L858R, (ii) T790M, (iii) both L858R and T790M, (iv) delE746_A750, (v) both delE746_A750 and T790M, and any other EGFR mutations described herein. In certain embodiments, the cell is a cancer cell (e.g., a cell from a non-small cell lung cancer (NSCLS); glioblastoma; pancreatic cancer; head and neck cancer (e.g., squamous cell carcinoma); breast cancer; colorectal cancer; epithelial cancer; ovarian cancer; prostate cancer; an adenocarcinoma, or any other EGFR expressing cancer described herein).

The invention further features a compound for use in a method of treating an EGFR-driven cancer refractory to a first kinase inhibitor selected from erlotinib, gefitinib, and pharmaceutically acceptable salts thereof, in a subject by administering to the subject a compound of the invention, or a pharmaceutically acceptable salt thereof, in an amount sufficient to treat the cancer.

The compound can be either in its free base form, or a pharmaceutically acceptable salt.

The response criteria for the compounds of the invention can be graded according to the response evaluation criteria in solid tumors (RECIST) guidelines (see Eur. J. Cancer 45:228 (2009)) that define when cancer patients improve ("respond"), stay the same ("stabilize"), or worsen ("progression") during treatments. A complete response is characterized by: (i) disappearance of all target lesions; and (ii) any pathological lymph nodes (whether target or non-target) must have reduction in short axis to <10 mm. A partial response is characterized by: (i) at least a 30% decrease in the sum of diameters of target lesions, taking as reference the baseline sum diameters. A progressive disease is characterized by (i) at least a 5%, 10%, or 20% increase in the sum of diameters of target lesions, taking as reference the smallest sum on study (this includes the baseline sum if that is the smallest on study); or (ii) the appearance of one or more new lesions.

The term "administration" or "administering" refers to a method of giving a dosage of a pharmaceutical composition to a mammal, where the method is, e.g., oral, intravenous, intraperitoneal, intraarterial, or intramuscular. The preferred method of administration can vary depending on various factors, e.g., the components of the pharmaceutical composition, site of the potential or actual disease and severity of disease.

By "EGFR-driven cancer" is meant a cancer characterized by inappropriately high expression of an EGFR gene or by a mutation in an EGFR gene that alters the biological activity of an EGFR nucleic acid molecule or polypeptide. EGFR-driven cancers can arise in any tissue, including brain, blood, connective tissue, liver, mouth, muscle, spleen, stomach, testis, and trachea. EGFR-driven cancers can include non-small cell lung cancer (NSCLS), including one or more of squamous cell carcinoma, adenocarcinoma, adenocarcinoma, bronchioloalveolar carcinoma (BAC), BAC with focal invasion, adenocarcinoma with BAC features, and large cell carcinoma; neural tumors, such as glioblastomas; pancreatic cancer; head and neck cancers (e.g., squamous cell carcinoma); breast cancer; colorectal cancer; epithelial cancer, including squamous cell carcinoma; ovarian cancer; prostate cancer; adenocarcinomas; and including cancers which are EGFR mediated.

An "EGFR mutant" or "mutant" includes one or more deletions, substitutions, or additions in the amino acid or nucleotide sequences of EGFR protein, or EGFR coding sequence. The EGFR mutant can also include one or more deletions, substitutions, or additions, or a fragment thereof, as long as the mutant retains or increases tyrosine kinase activity, compared to wild type EGFR. In particular EGFR mutations, kinase or phosphorylation activity can be increased (e.g., by at least 5%, 10%, 15%, 20%, 30%, 40%, 50%, 60%, 70%, 80%, 90%, or even 100%), as compared to wild type EGFR. Particular EGFR mutants are described herein, where mutations are provided relative to the position of an amino acid in human EGFR, as described in the sequence provided in NCBI GenBank Reference Sequence: NP_005219.2.

As used herein, the term "inhibiting the proliferation of a cell expressing an EGFR mutant" refers to measurably slowing, stopping, or reversing the growth rate of the EGFR-expressing cells in vitro or in vivo. Desirably, a slowing of the growth rate is by at least 10%, 20%, 30%, 50%, or even 70%, as determined using a suitable assay for determination of cell growth rates (e.g., a cell growth assay described herein). The EGFR mutant can be any EGFR mutant described herein.

As used herein, the term "refractory" refers to a cancer which is progressive in response to a given particular therapy. The cancer can be refractory either from the initial administration of the therapy; or become refractory over time in response to the therapy.

The term "sequence identity" is meant the shared identity between two or more nucleic acid sequence, or two or more amino acid sequences, expressed in the terms of the identity between the sequences. Sequence identity can be measured in terms of percentage identity; the higher the percentage, the more identical the sequences are. Homologs or orthologs of nucleic acid or amino acid sequences possess a relatively high degree of sequence identity when aligned using standard methods. Methods of alignment of sequences for comparison are well known in the art. Various programs and alignment algorithms are described in: Smith and Watermann, Adv. Appl. Math. 2:482 (1981); Needleman and Wunsch, J. Mol. Biol. 48:443 (1970); Pearson and Lipman, Proc. Natl. Acad. Sci. U.S.A. 85:2444 (1988); Corpet et al., Nuc. Acid Res. 16:10881 (1988); Huang et al., Computer Appls. in the Biosciences 8:155 (1992); and Pearson et al., Meth. Mol. Biol. 24:307 (1994). Altschul et al. (J. Mol. Biol. 215:403 (1990)) presents a detailed consideration of sequence alignment methods and homology calculations. The NCBI Basic Local Alignment Search Tool (BLAST) (Altschul et al., J. Mol. Biol. 215:403 (1990)) is available from several sources, including the National Center for Biological Information (NCBI) website, for use in connection with the sequence analysis programs blastp, blastn, blastx, tblastn, and tblastx. Additional information can be found at the NCBI website. BLASTN is used to compare nucleic acid sequences, while BLASTP is used to compare amino acid sequences. To compare two nucleic acid sequences, the option can be set as follows: -i is set to a file containing the first nucleic acid sequence to be compared; -j is set to a file containing the second nucleic acid sequence to be compared; -p is set to blastn; -o is set to any desired file name; -q is set to -1; -r is set to 2; and all other options are left at their default setting. Once aligned, the number of matches is determined by counting the number of positions where an identical nucleotide or amino acid residue is present in both sequences. The percent sequence identity is determined by dividing the number of matches either by the length of the sequence set forth in the identified sequence, or by an articulated length (such as 30, 35, 40, 45, 50, 60, 70, 80, 90, 100, 150, 200, 250, 300, 350, or 400 consecutive nucleotides or amino acid residues from a sequence set forth in an identified sequence), followed by multiplying the resulting value by 100. One indication that two nucleic acid molecules are closely related is that the two molecules hybridize to each other under stringent conditions. Stringent conditions are sequence-dependent and are different under different environmental parameters. Nucleic acid molecules that hybridize under stringent conditions to an EGFR gene sequence typically hybridize to a probe based on either an entire EGFR gene or selected portions of the gene (e.g., the kinase domain or a segment of the gene that contains the mutated codons described herein), under conditions described above.

As used herein, the term "treating" refers to administering a pharmaceutical composition for prophylactic and/or therapeutic purposes. To "prevent disease" refers to prophylactic treatment of a subject who is not yet ill, but who is susceptible to, or otherwise at risk of, a particular disease. To "treat disease" or use for "therapeutic treatment" refers to administering treatment to a subject already suffering from a disease to improve or stabilize the subject's condition. Thus, in the claims and embodiments, treating is the administration to a subject either for therapeutic or prophylactic purposes.

Where optional substitution of compounds of the invention is indicated, but one or more optional substituents is not specified, the substituent may be selected from those disclosed herein as generally appropriate in the given context, e.g. on an alkyl carbon, an aryl carbon, etc., and specifically include substitution exemplified in the examples. Other parameters of functional groups are also disclosed in detail below.

The term "alkyl" refers to linear, branched, cyclic, and polycyclic non aromatic hydrocarbon groups, which may be substituted or unsubstituted. Unless otherwise specified, "alkyl" groups contain one to eight, and typically one to six carbon atoms. Examples of alkyl include, without limitation, methyl, ethyl, n-propyl, isopropyl, cyclopropyl, butyl, isobutyl, sec-butyl, tert-butyl, cyclobutyl, pentyl, isopentyl tert-pentyl, cyclopentyl, hexyl, isohexyl, cyclohexyl, and n-heptyl, among others. Exemplary substituted alkyl groups include, without limitation, haloalkyl groups (e.g., fluoromethyl, difluoromethyl, trifluoromethyl, 2-fluoroethyl, 3-fluoropropyl), hydroxymethyl, 2-hydroxyethyl, 3-hydroxypropyl, benzyl, substituted benzyl, and phenethyl, among others. Note that as used herein, "alkyl" encompasses cyclic alkyl groups, which if not otherwise specified, contain 3 - 8 carbon atoms.

The term "alkoxy" refers to a subset of alkyl in which an alkyl group as defined above with the indicated number of carbons attached through an oxygen bridge, -O-alkyl, wherein the alkyl group contains 1 to 8 carbons atoms and is substituted or unsubstituted. Exemplary alkoxy groups include, without limitation, methoxy, ethoxy, n-propoxy, i-propoxy, t-butoxy, n-butoxy, s-pentoxy, -OCF₃, and-O-cyclopropyl.

The term "haloalkyl" refers to a subset of alkyl in which an alkyl group as defined above having one or more hydrogen atoms of the alkyl substituted with a halogen atom. Exemplary haloalkyl groups include, without limitation, fluoromethyl, trifluoromethyl, trichloromethyl, pentafluoroethyl and the like.

The term "alkenyl" refers to a branched or unbranched hydrocarbon group containing one or more double bonds and having from 2 to 8 carbon atoms. An alkenyl may optionally include monocyclic or polycyclic rings, in which each ring desirably has from three to six members. The alkenyl group may be substituted or unsubstituted. Alkenyl groups include, without limitation, vinyl, allyl, 2-cyclopropyl-1-ethenyl, 1-propenyl, 1-butenyl, 2-butenyl, 3-butenyl, 2-methyl-1-propenyl, and 2-methyl-2-propenyl.

The term "alkynyl" refers to a branched or unbranched hydrocarbon group containing one or more triple bonds and having from 2 to 8 carbon atoms. The alkynyl group may be substituted or unsubstituted. Alkynyls include, without limitation, ethynyl, 1-propynyl, 2-propynyl, 1-butynyl, 2-butynyl, and 3-butynyl.

The term "cycloalkyl" refers to cyclic or polycyclic hydrocarbon groups of from 3 to 13 carbon atoms, any of which is saturated. Cycloalkyl groups may be substituted or unsubstituted. Exemplary cycloalkyl groups include, without limitation, cyclopropyl, norbornyl, [2.2.2]bicyclooctane, and [4.4.0]bicyclodecane, and the like, which, as in the case of other alkyl moieties, may optionally be substituted.

The term "cycloalkenyl" refers to cyclic or polycyclic hydrocarbon groups of from 3 to 13 carbon atoms, preferably from 5 to 8 carbon atoms, containing one or more double bonds. Cycloalkenyl groups may be substituted or unsubstituted. Exemplary cycloalkenyl groups include, without limitation, cyclopentenyl, cyclohexenyl, and cyclooctenyl.

The term "cycloalkynyl" refers to cyclic or polycyclic hydrocarbon groups of from 5 to 13 carbon atoms containing one or more triple bonds. Cycloalkynyl groups may be substituted or unsubstituted.

The term "heteroalkyl" means a branched or unbranched alkyl, alkenyl, or alkynyl group having from 1 to 14 carbon atoms in addition to 1, 2, 3 or 4 heteroatoms independently selected from the group consisting of N, O, S, and P. Heteroalkyls include, without limitation, tertiary amines, secondary amines, ethers, thioethers, amides, thioamides, carbamates, thiocarbamates, hydrazones, imines, phosphodiesters, phosphoramidates, sulfonamides, and disulfides. A heteroalkyl may optionally include monocyclic, bicyclic, or tricyclic rings, in which each ring desirably has three to six members. The heteroalkyl group may be substituted or unsubstituted. Examples of heteroalkyls include, without limitation, polyethers, such as methoxymethyl and ethoxyethyl.

As used herein, "heterocyclic ring" and "heterocyclyl" refer to non-aromatic ring systems having five to fourteen ring atoms in which one or more ring carbons, preferably one to four, are each replaced by a heteroatom such as N, O, S, or P, which may be used alone or as part of a larger moiety as in "heterocyclyl-alkyl" (a heterocyclyl-substituted C₁₋₆ alkyl), "heterocyclyl-alkoxy" (a heterocyclyl-substituted C₁₋₆ alkoxy), or "heterocycloxy-alkyl" (a heterocycloxy-substituted C₁₋₆ alkyl), and includes aralkyl, aralkoxy, and aryloxyalkyl groups. Heterocyclic rings may be substituted or unsubstituted and may include one, two, or three fused or unfused ring systems. Desirably, the heterocyclic ring is a 5- to 7-membered monocyclic or 7- to 14-membered bicyclic heterocyclic ring consisting of 2 to 6 carbon atoms and 1, 2, 3, or 4 heteroatoms independently selected from N, O, and S and including any bicyclic group in which any of the above-defined heterocyclic rings is fused to a benzene ring. Exemplary heterocyclic rings include, without limitation, 3-1H-benzimidazol-2-one, (1-substituted)-2-oxo-benzimidazol-3-yl, 2-tetrahydrofuranyl, 3-tetrahydrofuranyl, 2-tetrahydrothiophenyl, 3-tetrahydrothiophenyl, 2-morpholinyl, 3-morpholinyl, 4-morpholinyl, 2-thiomorpholinyl, 3-thiomorpholinyl, 4-thiomorpholinyl, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxanyl, benzopyrrolidinyl, benzopiperidinyl, benzoxolanyl, benzothiolanyl, and benzothianyl. A heterocyclyl group can include two or more of the ring systems listed above. Heterocyclic rings include those in which a non-aromatic heteroatom-containing ring is fused to one or more aromatic or non-aromatic rings, such as in an indolinyl, chromanyl, phenanthridinyl, or tetrahydroquinolinyl, where the radical or point of attachment is on the non-aromatic heteroatom-containing ring.

The term "aryl" used alone or as part of a larger moiety as in "aralkyl" (an aryl-substituted C₁₋₆ alkyl), "aralkoxy" (an aryl-substituted C₁₋₆ alkoxy), or "aryloxyalkyl" (an aryloxy-substituted C₁₋₆ alkyl), refers to aromatic monocyclic or polycyclic ring groups having six to fourteen ring atoms, such as phenyl, 1-naphthyl, 2-naphthyl, 1-anthracyl, and 2-anthracyl and includes aralkyl, aralkoxy, and aryloxyalkyl groups. An "aryl" ring may be substituted or unsubstituted. The term "aryl" includes fused polycyclic aromatic ring systems in which an aromatic ring is fused to one or more rings. Non-limiting examples of aryl groups include phenyl, hydroxyphenyl, halophenyl, alkoxyphenyl, dialkoxyphenyl, trialkoxyphenyl, alkylenedioxyphenyl, naphthyl, phenanthryl, anthryl, phenanthro, 1-naphthyl, 2-naphthyl, 1-anthracyl, and 2-anthracyl. Also included within the scope of the term "aryl", as it is used herein, is a group in which an aromatic ring is fused to one or more non-aromatic rings, such as in a indanyl, phenanthridinyl, or tetrahydronaphthyl, where the radical or point of attachment is on the aromatic ring.

The term "heteroaryl" as used herein refers to stable heterocyclic, and polyheterocyclic aromatic moieties having 5 - 14 ring atoms. Heteroaryl groups may be substituted or unsubstituted and include both monocyclic and polycyclic ring systems. Examples of typical heteroaryl rings include 5-membered monocyclic rings, such as thienyl, pyrrolyl, imidazolyl, pyrazolyl, furyl, isothiazolyl, furazanyl, isoxazolyl, and thiazolyl; 6-membered monocyclic rings, such as pyridyl, pyrazinyl, pyrimidinyl, pyridazinyl, and triazinyl; and polycyclic heterocyclic rings, such as benzo[b]thienyl, naphtho[2,3-b]thienyl, thianthrenyl, isobenzofuranyl, chromenyl, xanthenyl, phenoxathienyl, indolizinyl, isoindolyl, indolyl, indazolyl, purinyl, isoquinolyl, quinolyl, phthalazinyl, naphthyridinyl, quinoxalinyl, quinazolinyl, benzothiazole, benzimidazole, tetrahydroquinoline cinnolinyl, pteridinyl, carbazolyl, beta-carbolinyl, phenanthridinyl, acridinyl, perimidinyl, phenanthrolinyl, phenazinyl, isothiazolyl, phenothiazinyl, and phenoxazinyl (see e.g. Katritzky, Handbook of Heterocyclic Chemistry). Exemplary heteroaryl rings include, without limitation, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 1-pyrrolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, 3-thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, indolyl, isoindolyl, acridinyl, and benzoisoxazolyl. Heteroaryl groups further include a group in which a heteroaromatic ring is fused to one or more aromatic or nonaromatic rings where the radical or point of attachment is on the heteroaromatic ring, such as tetrahydroquinoline, tetrahydroisoquinoline, and pyrido[3,4-d]pyrimidinyl, imidazo[1,2-a]pyrimidyl, imidazo[1,2-a]pyrazinyl, imidazo[1,2-a]pyiridinyl, imidazo[1,2-c]pyrimidyl, pyrazolo[1,5-a][1,3,5]triazinyl, pyrazolo[1,5-c]pyrimidyl, imidazo[1,2-b]pyridazinyl, imidazo[1,5-a]pyrimidyl, pyrazolo[1,5-b][1,2,4]triazine, quinolyl, isoquinolyl, quinoxalyl, imidazotriazinyl, pyrrolo[2,3-d]pyrimidyl, triazolopyrimidyl, and pyridopyrazinyl.

An aryl group or heteroaryl group may contain one or more substituents. Exemplary substituents for aryl or heteroaryl group include halogen (F, Cl, Br or I), alkyl, alkenyl, alkynyl, heteroalkyl, -NO₂, -CN, -R^{A}, -OR^{B}, -S(O)ᵣR^{B}, (wherein r is 0, 1 or 2), -SO₂NR^{A}R^{B}, -NR^{A}R^{B}, -O-NR^{A}R^{B}, -NR^{A}-NR^{A}R^{B}, -(CO)YR^{B}, -O(CO)YR^{B}, -NR^{A}(CO)YR^{B}, -S(CO)YR^{B}, -NR^{A}C(=S)YR^{B},-OC(=S)YR^{B}, -C(=S)YR^{B}, -YC(=NR^{A})YR^{B}, -YC(=N-OR^{A})YR^{B}, -YC(=N-NR^{A}R^{B})YR^{B}, -COCOR^{B},-COMCOR^{B} (where M is a C₁₋₆ alkyl group), -YP(O)(YR^{G})(YR^{G}), -P(O)(R^{C})₂, -Si(R^{C})₃,-NR^{A}SO₂R^{B}, and -NR^{A}SO₂NR^{A}R^{B}, wherein each occurrence of Y is, independently, -O-, -S-, -NR^{A}-, or a chemical bond (i.e., -(CO)YR^{B} thus encompasses -C(=O)R^{B}, -C(=O)OR^{B}, and -C(=O)NR^{A}R^{B}).

R^{C} is selected from alkyl, alkenyl, alkynyl, cycloalkyl, cycloalkenyl, cycloalkynyl, aryl, heteroaryl, and heterocyclyl. At each occurrence, each of R^{A} and R^{B} is, independently, selected from hydrogen, alkyl, alkenyl, alkynyl, cycloalkyl, cycloalkenyl, cycloalkynyl, aryl, heteroaryl, and heterocyclyl.

Each of R^{A}, R^{B} and R^{C} optionally bears one or more substituents selected from amino, alkylamino, dialkylamino, aminocarbonyl, halogen, alkyl, aryl, heteroalkyl, heteroaryl, carbocycle, heterocycle, alkylaminocarbonyl, dialkylaminocarbonyl, alkylaminocarbonyloxy, dialkylaminocarbonyloxy, nitro, cyano, carboxy, alkoxycarbonyl, alkylcarbonyl, alkoxy, haloalkoxy groups, hydroxy, protected hydroxyl groups (e.g., -O-X, where X is acyl, phenyl, substituted phenyl, benzyl, substituted benzyl, phenethyl, or substituted phenethyl), -M-heteroaryl, -M-heterocycle, -M-aryl, -M-OR^{B}, -M-SR^{B}, -M-NR^{A}R^{B}, -M-OC(O)NR^{A}R^{B}, -M-C(=NR^{B})NR^{A}R^{B}, -M-C(=NR^{A})OR^{B}, -M-P(=O)(R^{C})₂, Si(R^{C})₃, -M-NR^{A}C(O)R^{B}, -M-NR^{A}C(O)OR^{B}, -M-C(O)R^{B}, -M-C(=S)R^{B}, -M-C(=S)NR^{A}R^{B}, -M-C(O)NR^{A}R^{B}, -M-C(O)NR^{B}-M-NR^{A}R^{B}, -M-NR^{B}C(NR^{A})NR^{A}R^{B}, -M-NR^{A}C(S)NR^{A}R^{B} -M-S(O)₂R^{A}, -M-C(O)R^{A}, -M-OC(O)R^{A}, -MC(O)SR^{B}, -M-S(O)₂NR^{A}R^{B}, -C(O)-M-C(O)R^{B}, -MCO₂R^{B}, -MC(=O)NR^{A}R^{B}, -M-C(=NH)NR^{A}R^{B}, and -M-OC(=NH)NR^{A}R^{B}, wherein M is a C₁₋₆ alkyl group. Non-limiting illustrations of a substituted R^{A}, R^{B} or R^{C} group include haloalkyl and trihaloalkyl, alkoxyalkyl, halophenyl, chloromethyl, trichloromethyl, trifluoromethyl, methoxyethyl, alkoxyphenyl, halophenyl, -CH₂-aryl, -CH₂-heterocycle, -CH₂C(O)NH₂,-C(O)CH₂N(CH₃)₂, -CH₂CH₂OH, -CH₂OC(O)NH₂, -CH₂CH₂NH₂, -CH₂CH₂CH₂NEt₂, -CH₂OCH₃, -C(O)NH₂, -CH₂CH₂-heterocycle, -C(=S)CH₃, -C(=S)NH₂, -C(=NH)NH₂, -C(=NH)OEt, -C(O)NH-cyclopropyl, -C(O)NHCH₂CH₂-heterocycle, -C(O)NHCH₂CH₂OCH₃, -C(O)CH₂CH₂NHCH₃, -CH₂CH₂F, -C(O)CH₂-heterocycle, -CH₂C(O)NHCH₃, -CH₂CH₂P(=O)(CH₃)₂, and -Si(CH₃)₃.

An alkyl, alkenyl, alkynyl, alkoxy, haloalkyl, heteroalkyl, cycloalkyl, cycloalkenyl, cycloalkynyl, or heterocyclic group may contain one or more substituents selected from those listed above for aryl and heteroaryl groups, in addition to =O, =S, =NH, =NNR^{A}R^{B}, =NNHC(O)R^{B}, =NNHCO₂R^{B}, or =NNHSO₂R^{B}, wherein R^{A} and R^{B} are as defined above.

In drawings of chemical structures in this document, atoms not indicated by label or line are understood to be H.

Other features and advantages of the invention will be apparent from the following detailed description and the claims.

### EGFR Mutants

The EGFR-driven cancers which can be treated using the compounds and compositions of the invention include, for example, EGFR mutants including one or more deletions, substitutions, or additions in the amino acid or nucleotide sequences of EGFR, or fragments thereof.

Mutations in EGFR can occur in any part of the EGFR sequence. Generally, EGFR mutants arise from mutations in the kinase domain (i.e., exons 18-24 in the EGFR sequence) or in the extracellular domain (i.e., exons 2-16 in the EGFR sequence). For example, mutations typically occur in the kinase domain, including one or more of a point mutation in exon 18 (e.g., L688P, V689M, P694L/S, N700D, L703V, E709K/Q/A/G/V, I715S, L718P, G719C/A/S/R, or S720P/F), a deletion in exon 19 that may or may not include an insertion (e.g., delG719, delE746_E749, delE746_A750, delE746_A750insRP, delE746_A750insQP, delE746_T751, delE746_T751insA/I/V, delE746_T751insVA, delE746_S752, delE746_S752insA/V/D, delE746_P53insLS, delL747_E749, delL747_A750, delL747_A750insP, delL747_T751, delL747_T751insP/S/Q, delL747_T751insPl, delL747_S752, delL747_S752insQ, delL747_P753, delL747_P753insS/Q, delL747_L754insSR, delE749_A750, delE749_A750insRP, delE749_T751, delT751_I759, delT751_I759insS/N, or delS752_I759), a duplication in exon 19 (e.g., K739_I44dupKIPVAI), a point mutation in exon 19 (e.g., L730F, W731Stop, P733L, G735S, V742A, E746V/K, A750P, 1751I, S752Y, P753S, A754P, or D761Y), an in-frame insertion in exon 20 (e.g., D761_E762insEAFQ, A767_S768insTLA, V769_D770insY, V769_D770insCV, V769_D770insASV, D770_N771insD/G, D770_N771insNPG, D770_N771insSVQ, P772_H773insNN, P772_H773insYNP, or V774_C775insHV), a deletion in exon 20 that may or may not include an insertion (e.g., delM766_A767, delM766_A767insAl, delA767_V769, delD770, or delP772_H773insNP), a duplication in exon 20 (e.g., S768_D770dupSVD, A767_V769dupASV, or H773dupH), a point mutation in exon 20 (e.g., D761N, A763V, V765A/M, S768I, V769L/M, S768I, P772R, N771T, H773R/Y/L, V774M, R776G/H/C, G779S/F, T783A, T784F, L792P, L798H/F, T790M, R803W, K806E, or L814P), or a point mutation in exon 21 (e.g., G810S, N826S, L833V, H835L, L838V, A839T, K846R, T847I, H850N, V851I/A, I853T, L858M/R, A859T, L861Q/R, G863D, A864T, E866K, or G873E). In lung cancer, activation mutants are typical, and 90% deletion of 746-750 (ELREA) and L858R result in sustained phosphorylation of EGFR without ligand stimulation. In particular, drug resistance in 50% of lung cancers arises from the T790M point mutation.

For example, in glioblastoma, mutations typically, but not exclusively, occur in the extracellular domain, including EGFR variant I (EGFRvl) lacking the extracellular domain and resembling the v-erbB oncoprotein; EGFRvII lacking 83 amino acids from domain IV; and EGFRvIII lacking amino acids 30-297 from domains I and II, which is the most common amplification and is reported in 30-50% of glioblastomas and 5% of squamous cell carcinoma. Other mutations for glioblastoma include one or more of point mutations in exon 2 (e.g., D46N or G63R), exon 3 (e.g., R108K in domain I), exon 7 (e.g., T263P or A289D/T/V in domain II), exon 8 (e.g., R324L or E330K), exon 15 (e.g., P596L or G598V in domain IV), or exon 21 (L861Q in the kinase domain).

EGFR mutants also include those with a combination of two or more mutations, as described herein. Exemplary combinations include S768I and G719A; S768I and V769L; H773R and W731Stop; R776G and L858R; R776H and L861Q; T790M and L858R; T790M and delE746_A750; R803W and delE746_T751insVA; delL747_E749 and A750P; delL747_S752 and E746V; delL747_S752 and P753S; P772_H773insYNP and H773Y; P772_H773insNP and H773Y; and D770_N771insG and N771T. Other exemplary combinations include any including T790M (e.g., T790M and L858R or T790M and delE746_A750, with or without concomitant inhibition of single mutants L858R and delE746_A750).

EGFR mutants can be either activation mutants or resistant mutants. Activation mutants include those with substitutions that increase drug sensitivity (e.g., G719C/S/A, delE746_A750, or L858R). Resistant mutants include those with substitutions that increase drug resistance (e.g., T790M or any combination including T790M).

EGFR-driven cancers include those having any mutant described herein. For example, EGFRvIII is commonly found in glioblastoma and has also been reported in breast, ovarian, prostate, and lung carcinomas. Exemplary EGFR-driven cancers: glioblastoma, lung cancer (e.g., squamous cell carcinoma, non-small cell lung cancer, adenocarcinoma, bronchioloalveolar carcinoma (BAC), BAC with focal invasion, adenocarcinoma with BAC features, and large cell carcinoma), pancreatic cancer, head and neck cancers (e.g., squamous cell carcinoma), breast cancer, colorectal cancer, epithelial cancer (e.g., squamous cell carcinoma), ovarian cancer, and prostate cancer.

In particular, the invention described herein would benefit patient populations having higher risk for TKI-resistant mutations. About 8,000 to 16,000 new cases per year can be estimated based on: incidence of non-small cell lung cancer (about 160,000 new cases in the U.S.), the response to erlonitinib in the general population (about 10%, resulting in a sensitive population of 16,000), the presence of activation mutations (10-20% in white and 30-40% in Asian population, resulting in a sensitive population of 16,000-32,000), acquisition of secondary resistance (most if not all patients, resulting in a sensitive population of 16,000-32,000), and percentage of patients carrying the T790M point mutations (about 50%, resulting in a sensitive population of 8,000-16,000). Patients having TKI-resistant mutations include those patients having cancers resistant to one or more of erlotinib, gefitinib, CL-387,785, BIBW 2992 (CAS Reg. No. 439081-18-2), CI-1033, neratinib (HKI-272), MP-412 (AV-412), PF-299804, AEE78, and XL64.

In particular, the inventions relates to treatment of EGFR-driven cancers having the T790M point mutation. Generally, reversible inhibitors (e.g., CI-1033, neratinib (HKI-272), and PF-299804) are less potent in cell lines having the T790M mutation and do not inhibit T790M at clinically achievable concentrations. Since the ATP Km of T790M and WT are similar, concentrations that inhibit the mutant will inhibit the WT and result in gastrointestinal and cutaneous events.

An EGFR mutant also includes other amino acid and nucleotide sequences of EGFR with one or more deletions, substitutions, or additions, such as point mutations, that retain or increase tyrosine kinase or phosphorylation activity. Where the mutant is a protein or polypeptide, preferable substitutions are conservative substitutions, which are substitutions between amino acids similar in properties such as structural, electric, polar, or hydrophobic properties. For example, the substitution can be conducted between basic amino acids (e.g., Lys, Arg, and His), or between acidic amino acids (e.g., Asp and Glu), or between amino acids having non-charged polar side chains (e.g., Gly, Asn, Gin, Ser, Thr, Tyr, and Cys), or between amino acids having hydrophobic side chains (e.g., Ala, Val, Leu, Ile, Pro, Phe, and Met), or between amino acids having branched side chains (e.g., Thr, Val, Leu, and Ile), or between amino acids having aromatic side chains (e.g., Tyr, Trp, Phe, and His).

Where the mutant is a nucleic acid, the DNA encoding an EGFR mutant protein may comprise a nucleotide sequence capable of hybridizing to a complement sequence of the nucleotide sequence encoding an EGFR mutant, as defined herein, under stringent conditions. As used herein, the stringent conditions include low, medium or high stringent conditions. An example of the stringent conditions includes hybridization at approximately 42-55°C in approximately 2-6 x SSC, followed by wash at approximately 50-65°C in approximately 0.1-1 x SSC containing approximately 0.1-0.2% SDS, where 1 x SSC is a solution containing 0.15 M NaCl and 0.015 M Na citrate, pH 7.0. Wash can be performed once or more. In general, stringent conditions may be set at a temperature approximately 5°C lower than a melting temperature (Tm) of a specific nucleotide sequence at defined ionic strength and pH.

The amino acid and nucleotide sequences of EGFR and DNAs encoding them are available from known databases such as NCBI GenBank (USA), EMBL (Europe), etc. For example, GenBank accession numbers for EGFR [Homo sapiens] include MIM131550, AAl28420, NM_005228, NP_005219.2, and GeneID: 1956.

### Characterization of EGFR-driven Cancers

The compositions and compounds of the invention can be used to treat subjects having an EGFR-driven cancer (i.e., cancers characterized by EGFR mutant expression or overexpression). EGFR mutant expression or overexpression can be determined in a diagnostic or prognostic assay by evaluating levels of EGFR mutants in biological sample, or secreted by the cell (e.g., via an immunohistochemistry assay using anti-EGFR antibodies or anti-p-EGFR antibodies; FACS analysis, etc.). Alternatively, or additionally, one can measure levels of EGFR mutant-encoding nucleic acid or mRNA in the cell, e.g., via fluorescent in situ hybridization using a nucleic acid based probe corresponding to an EGFR mutant-encoding nucleic acid or the complement thereof; (FISH; see WO98/45479, published October, 1998), Southern blotting, Northern blotting, or polymerase chain reaction (PCR) techniques, such as real time quantitative PCR (RT-PCR). One can also study EGFR mutant expression by measuring shed antigen in a biological sample, such as serum, e.g., using antibody-based assays (see also, e.g., U.S. Patent No. 4,933,294, issued June 12, 1990; WO91/05264, published April 18, 1991; U.S. Patent 5,401,638 .issued March 28, 1995; and Sias et al., J. Immunol. Methods 132:73 (1990)). Aside from the above assays, various *in vivo* assays are available to the skilled practitioner. For example, one can expose cells within the body of the mammal to an antibody which is optionally labeled with a detectable label, e.g., a radioactive isotope, and binding of the antibody to cells in the mammal can be evaluated, e.g., by external scanning for radioactivity or by analyzing a biopsy taken from a mammal previously exposed to the antibody.

Examples of biological properties that can be measured in isolated cells include mRNA expression, protein expression, and DNA quantification. Additionally, the DNA of cells isolated by the methods of the invention can be sequenced, or certain sequence characteristics (e.g., polymorphisms and chromosomal abnormalities) can be identified using standard techniques, e.g., FISH or PCR. The chemical components of cells, and other analytes, may also be assayed after isolation. Cells may also be assayed without lysis, e.g., using extracellular or intracellular stains or by other observation, e.g., morphology or growth characteristics in various media.

While any hybridization technique can be used to detect the gene rearrangements, one preferred technique is fluorescent in situ hybridization (FISH). FISH is a cytogenetic technique which can be used to detect and localize the presence or absence of specific DNA or RNA sequences on chromosomes. FISH incorporates the use of fluorescently labeled nucleic acid probes which bind only to those parts of the chromosome with which they show a high degree of sequence similarity. Fluorescence microscopy can be used to find out where the fluorescent probe bound to the chromosome. The basic steps of FISH are outlined below. Exemplary FISH probes include Vysis EGFR SpectrumOrange/ CEP SpectrumGreen Probe (Abbott, Downers Grove, IL), which hybridizes to band 7p12; and ZytoLight SPEC EGFR/CEN 7 Dual Color Probe (ZytoVision), which hybridizes to the alpha-satellite sequences of the centromere of chromosome 7.

For FISH, a probe is constructed that is long enough to hybridize specifically to its target (and not to similar sequences in the genome), but not too large to impede the hybridization process. Probes are generally labeled with fluorophores, with targets for antibodies, with biotin, or any combination thereof. This can be done in various ways, for example using random priming, nick translation, and PCR using tagged nucleotides.

Generally, a sample or aliquot of a population of cells is used for FISH analysis. For example, in one method of preparation, cells are trypsinized to disperse into single cells, cytospun onto glass slides, and then fixed with paraformaldehyde before storing in 70% ethanol. For preparation of the chromosomes for FISH, the chromosomes are firmly attached to a substrate, usually glass. After preparation, the probe is applied to the chromosome RNA and starts to hybridize. In several wash steps, all unhybridized or partially hybridized probes are washed away. If signal amplification is necessary to exceed the detection threshold of the microscope (which depends on many factors such as probe labeling efficiency, the kind of probe, and the fluorescent dye), fluorescent tagged antibodies or strepavidin are bound to the tag molecules, thus amplifying the fluorescence.

An epifluorescence microscope can be used for observation of the hybridized sequences. The white light of the source lamp is filtered so that only the relevant wavelengths for excitation of the fluorescent molecules arrive onto the sample. Emission of the fluorochromes happens, in general, at larger wavelengths, which allows one to distinguish between excitation and emission light by mean of another optical filter. With a more sophisticated filter set, it is possible to distinguish between several excitation and emission bands, and thus between several fluorochromes, which allows observation of many different probes on the same strand.

Depending on the probes used, FISH can have resolution ranging from huge chromosomes or tiny (-100 kilobase) sequences. The probes can be quantified simply by counting dots or comparing color.

Allele-specific quantitative real time-PCR may also be used to identify a nucleic acid encoding a mutant EGFR protein (see, for e.g., Diagnostic Innovations DxS BCR-ABL T3151 Mutation Test Kit, and Singer et al., Methods in Molec. Biol. 181:145 (2001)). This technique utilizes Taq DNA polymerase, which is extremely effective at distinguishing between a match and a mismatch at the 3'-end of the primer (when the 3'-base is mismatched, no efficient amplification occurs). Using this technique, the 3'-end of the primer may be designed to specifically hybridize to a nucleic acid sequence that corresponds to a codon that encodes a mutant amino acid in an EGFR mutant, as described herein. In this way, the specific mutated sequences can be selectively amplified in a patient sample. This technique further utilizes a Scorpion probe molecule, which is a bifunctional molecule containing a PCR primer, a fluorophore, and a quencher. The fluorophore in the probe interacts with a quencher, which reduces fluorescence. During a PCR reaction, when the Scorpion probe binds to the amplicon, the fluorophore and quencher in the Scorpion probe become separated, which leads to an increase in fluorescence from the reaction tube. Any of the primers described herein may be used in allele-specific quantitative real time PCR.

A biological sample can be analyzed to detect a mutation in an EGFR gene, or expression levels of an EGFR gene, by methods that are known in the art. For example, methods such as direct nucleic acid sequencing, altered hybridization, aberrant electrophoretic gel migration, binding or cleavage mediated by mismatch binding proteins, single-strand conformational polymorphism (SSCP) analysis, or restriction fragment length polymorphism (RFLP) analysis of PCR products derived from a patient sample can be used to detect a mutation in an EGFR gene; ELISA can be used to measure levels of EGFR polypeptide; and PCR can be used to measure the level of an EGFR nucleic acid molecule.

Any of these techniques may be used to facilitate detection of a mutation in a candidate gene, and each is well known in the art; examples of particular techniques are described, without limitation, in Orita et al. (Proc. Natl. Acad. Sci. USA 86:2766 (1989)) and Sheffield et al. (Proc. Natl. Acad. Sci. USA 86:232 (1989)). Furthermore, expression of the candidate gene in a biological sample (e.g., a biopsy) may be monitored by standard Northern blot analysis or may be aided by PCR (see, e.g., Ausubel et al., Current Protocols in Molecular Biology, John Wiley & Sons, New York, NY (1995); PCR Technology: Principles and Applications for DNA Amplification, H.A. Ehrlich, Ed., Stockton Press, NY; Yap et al., Nucl. Acids. Res. 19:4294 (1991)).

One skilled in the art may identify in a nucleic acid or protein sequence a residue (e.g., amino acid or nucleotide) or codon that corresponds to a residue or codon in wild-type EGFR or EGFR mutants using a number of sequence alignment software programs (e.g., NCBI BLAST website). Such software programs may allow for gaps in the alignment of the compared sequences. Using such software, one skilled in the art may identify a nucleotide, amino acid, or amino acid that corresponding to a specific nucleotide, amino acid, or codon in wild-type EGFR or EGFR mutants.

Levels of EGFR expression (e.g., DNA, mRNA, or protein) in a biological sample can be determined by using any of a number of standard techniques that are well known in the art or described herein. Exemplary biological samples include plasma, blood, sputum, pleural effusion, bronchoalveolar lavage, or biopsy, such as a lung biopsy and lymph node biopsy. For example, EGFR expression in a biological sample (e.g., a blood or tissue sample) from a patient can be monitored by standard northern blot analysis or by quantitative PCR (see, e.g., Ausubel et al., supra; PCR Technology: Principles and Applications for DNA Amplification, H.A. Ehrlich, Ed., Stockton Press, NY; Yap et al., Nucl. Acids. Res. 19:4294 (1991)).

### Synthesis

Compounds of the invention can be prepared using methods and materials analogous to those described in the art, e.g., as disclosed in detail in International patent applications WO 2004/080980, WO 2005/016894, WO 2006/021454, WO 2006/021457, WO 2009/143389, and WO 2009/126515. For instance, compounds of the invention in which R^{e} is H and R^{d} is H, Cl, CF₃, or CH₃, can be synthesized from 2,4-dichloropyrimidine, 2,4,5-trichloropyrimidine, 2,4-dichloro-5-(trifluoromethyl)pyrimidine, or 2,4-dichloro-5-methylpyrimidine, respectively, as described in PCT Publication No. WO/2009/143389.

Compounds of the invention in which R^{d} and R^{e}, together with the pyrimidine ring atoms to which they are attached, form a 5- or 6-membered ring containing one or two heteroatoms can be synthesized as described in PCT Publication No. WO2009/126515.

Compounds of the invention in which U¹ and U² are N can be synthesized, for example, using methods analogous to those described in Scheme A1.

Further details are provided in the Examples.

### Formulation

Compounds of the invention can be formulated into a pharmaceutical composition that comprises a compound of the invention (as an active pharmaceutical ingredient) or a pharmaceutically acceptable salt thereof and a pharmaceutically acceptable excipient. As such, the present disclosure provides a pharmaceutical composition comprising a compound of the invention or a pharmaceutically acceptable salt thereof and a pharmaceutically acceptable excipient.

Pharmaceutically acceptable compositions containing a compound of the invention suitable for administration may be formulated using conventional materials and methods, a wide variety of which are well known. Suitable dosage forms include those in solution, suspension or emulsion form, and solid oral dosage forms such as capsules, tablets, gel caps, caplets, etc.. Methods well known in the art for making formulations, including the foregoing unit dosage forms, are found, for example, in "Remington: The Science and Practice of Pharmacy" (20th ed., ed. A.R. Gennaro, 2000, Lippincott Williams & Wilkins).

Compounds of the invention can be formulated for any route of administration (e.g., orally, rectally, parenterally, intracisternally, intravaginally, intraperitoneally, topically (as by transdermal patch, powders, ointments, or drops), sublingually, bucally, as an oral or nasal spray) effective for use in the methods of the invention. For use in the methods of the invention, compounds of the invention are preferably formulated in dosage unit form for ease of administration and uniformity of dosage. For example, a compound of the invention can be formulated for as a capsule for oral administration containing nominally 10 mg, 50 mg, 100 mg, 150 mg, 250 mg, 500 mg, or any dosage amounts described herein as the free base or acid addition salt of the compound (e.g., the hydrochloride salt), The unit dosage forms of the invention can include a compound of the invention, or a salt thereof, formulated with excipients, fillers, flow enhancers, lubricants, and/or disintegrants as needed. For example, a unit dosage form can include colloidal silicon dioxide (a flow enhancer), lactose anhydrous (a filler), magnesium stearate (a lubricant), microcrystalline cellulose (a filler), and/or sodium starch glycolate (a disintegrant). The compound of the invention and the inactive ingredients can be formulated utilizing, for example, conventional blending, and encapsulation processes. Alternatively, compounds of the invention are formulated as described in PCT Publication Nos. WO2009/143389 and WO2009/126515.

### Therapy

Compounds of the invention can be useful for treating EGFR-driven cancers. In particular, the compounds can be useful for treating EGFR-driven cancers that express EGFR mutants and for treating EGFR-driven cancers that are refractory to TKI therapies (e.g., erlotinib or gefitinib).

Such cancers can include, among others, non-small cell lung cancer (NSCLS), including one or more of squamous cell carcinoma, adenocarcinoma, adenocarcinoma, bronchioloalveolar carcinoma (BAC), BAC with focal invasion, adenocarcinoma with BAC features, and large cell carcinoma; neural tumors, such as glioblastomas; pancreatic cancer; head and neck cancers (e.g., squamous cell carcinoma); breast cancer; colorectal cancer; epithelial cancer, including squamous cell carcinoma; ovarian cancer; prostate cancer; adenocarcinomas; and including cancers which are EGFR mediated.

The present invention is based upon the discovery that compounds of the invention can be used to treat EGFR-driven cancers, EGFR-driven cancers that express EGFR mutants, and for treating EGFR-driven cancers that are refractory to TKI therapy, such as erlotinib or gefitinib. Compounds of the invention can also be used in a maintenance role to prevent recurrence of cancer in patients in need of such a treatment.

The effective systemic dose of a compound of the invention will typically be in the range of an average daily dose of from 10 mg to 2,000 mg of the compound per kg of patient body weight, administered in single or multiple doses. Generally, a compound of the invention may be administered to patients in need of such treatment in a daily dose range of about 50 to about 2,000 mg per patient. Administration may be once or multiple times daily, weekly (or at some other multiple-day interval) or on an intermittent schedule. For example, the compound may be administered one or more times per day on a weekly basis (e.g. every Monday) indefinitely or for a period of weeks, e.g. 4 - 10 weeks. Alternatively, it may be administered daily for a period of days (e.g. 2 - 10 days) followed by a period of days (e.g. 1 - 30 days) without administration of the compound, with that cycle repeated indefinitely or for a given number of repetitions, e.g. 4 - 10 cycles. As an example, a compound of the invention may be administered daily for 5 days, then discontinued for 9 days, then administered daily for another 5 day period, then discontinued for 9 days, and so on, repeating the cycle indefinitely, or for a total of 4 -10 times.

When a TKI (e.g., erlotinib or gefitinib) is used in combination with a compound of the invention, each component of the combination therapy may be administered at their monotherapy dosing levels and schedules. For example, erlotinib has been administered orally for the treatment of NSCLC at 150 mg daily and of pancreatic cancer at 100 mg daily. In another example, gefitinib has been administered orally for the treatment of NSCLC at 250 mg daily.

The effective systemic dose of a compound of the invention will typically be in the range of an average daily dose of from 10 mg to 2,000 mg of the compound per kg of patient body weight, administered in single or multiple doses. Generally, a compound of the invention may be administered to patients in need of such treatment in a daily dose range of about 50 to about 2,000 mg per patient. Administration may be once or multiple times daily, weekly (or at some other multiple-day interval) or on an intermittent schedule. For example, the compound may be administered one or more times per day on a weekly basis (e.g. every Monday) indefinitely or for a period of weeks, e.g. 4 - 10 weeks. Alternatively, it may be administered daily for a period of days (e.g. 2 - 10 days) followed by a period of days (e.g. 1 - 30 days) without administration of the compound, with that cycle repeated indefinitely or for a given number of repititions, e.g. 4 - 10 cycles. As an example, a compound of the invention may be administered daily for 5 days, then discontinued for 9 days, then administered daily for another 5 day period, then discontinued for 9 days, and so on, repeating the cycle indefinitely, or for a total of 4 - 10 times.

Alternatively, a TKI (e.g., erlotinib or gefitinib) is used in combination with a compound of the invention with a reduced dosing level in one or both components.

The following examples are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

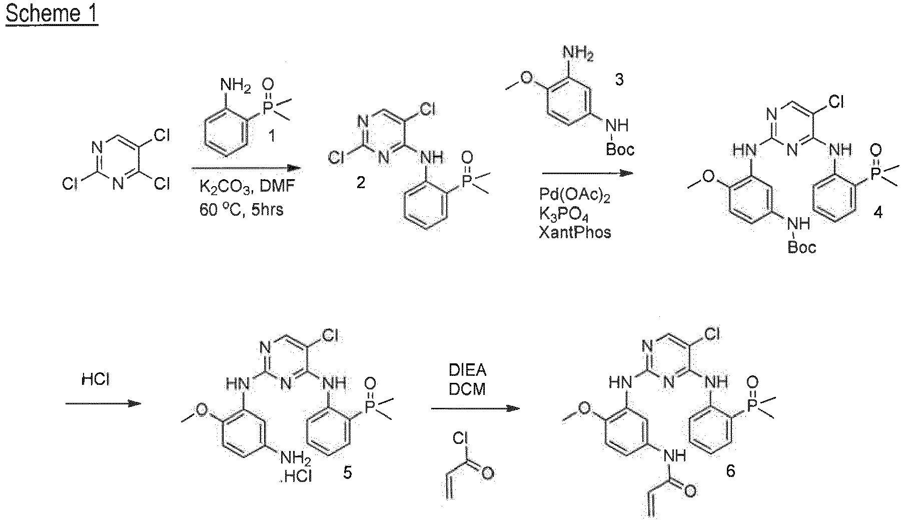

### Step 1: synthesis of compound 1

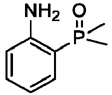

To a solution of 2-iodoaniline (1.0 eq) and dimethylphosphine oxide (1.1 eq) in DMF were added potassium phosphate (1.1 eq) and palladium acetate/Xantphos (catalytic). The reaction was stirred at 150 °C for 3 hours and cooled to room temperature. The solvent was evaporated and the residue was worked up with DCM/water. The crude product was purified with a column (EtOAc/MeOH 10:1) to give **1** as a brown solid (80% yield).

### Step 2: Synthesis of 2:

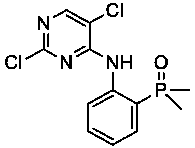

2,4,5-Trichloropyrimidine (1.57 eq), **1** (1.0 eq), and potassium carbonate (3.14 eq) in DMF were stirred at 60 °C for 5 hours and then cooled to room temperature. The mixture was filtered and the filtrate was concentrated. The residue was purified by column chromatography (ISCO machine) (DCM/MeOH 20:1) to give **2** as a yellow solid (61% yield).

### Step 2a: Synthesis of 8 and 3:

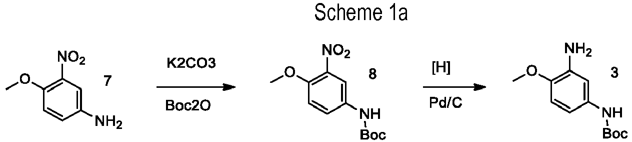

A suspension of 10 g (59.5 mmol, 1.0 eq) of 4-methoxy-3-nitroaniline in 65 mL of dioxane and 65 mL of water was adjusted to pH 12 with 40% NaOH, and then Boc₂O (26 g, 119.1 mmol, 2.0 eq) was added in 3 portions under ice bath. The reaction was stirred at room temperature overnight. After standing, the mixture was filtered to give a yellow solid, 15 g, in 95% yield.

Compound **8** (6 g, 22.4 mmol) was dissolved in 55 mL of ethyl acetate and Pd/C (10%, wet, 0.5 g) was added. The reduction was shaken at room temperature under H₂ (30 psi) for 1 hr and filtered. The filtrate was evaporated to a tan solid, 5.4 g, in a quantitative yield.

### Step 3: Synthesis of 4:

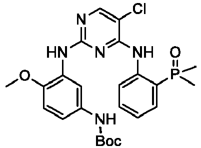

A suspension of 2(1.27g, 4.0 mmol), 3-Boc-amino-5-methoxyaniline(965mg, 4.0mmol), palladium acetate(133mg, 0.59mmol), XantPhos(352mg, 0.61mmol) and potassium phosphate(1.4g, 6.6mmol) in anhydrous DMF(35 mL) was heated at 120 °C overnight. After the reaction was cooling to room temperature, ethyl acetate was added to dilute the reaction and the content was filtered through celite. Solvent was removed under vacuum. The residue was purified by flash column chromatography on silica gel to give pure product 1.3g (yield 62%) as a tan solid.

### Step 4: Synthesis of 5:

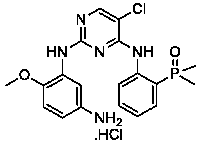

HCl/dioxane(4N, 4 mL) was added to a solution of compound **4** (440 mg, 0.85mmol) in MeOH(4 mL). The reaction mixture was stirred at room temp overnight. White precipitate was filtered and dried to give the title product as a white solid(339mg, Yield 81%).

### Step 5: Synthesis of 6:

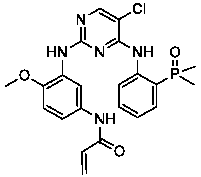

Compound **5** (100mg, 0.15 mmol) was dissolved in CH₂Cl₂ (1.6 mL) and DIEA(174 µL, 1mmol). The solution was cooled in an ice bath. Aryloyl chloride(13ul, 0.165 mmol) was added dropwise. The content was stirred at room temp for an hour. Solvent was removed *in vacuo* and the residue was purified by a prep-TLC plate(7.5%MeOH/DCM) to give final product as a tan solid (24mg, yield 34%).

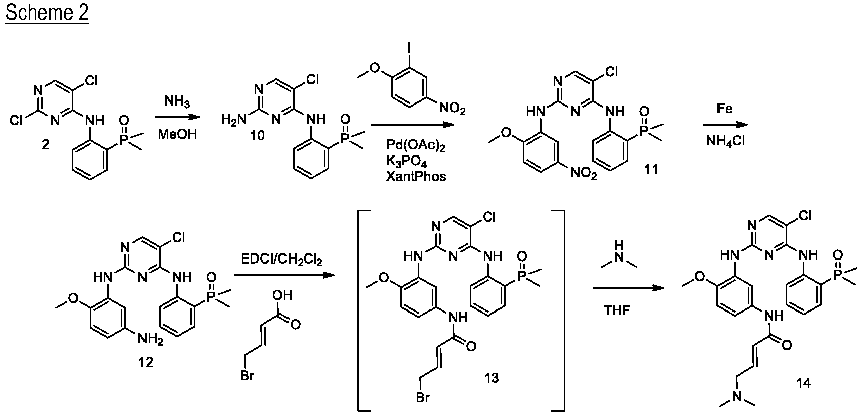

### Step 1: Synthesis of 10:

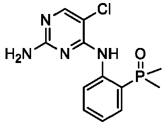

Compound **2** (2g, 6.3mmol) was dissolved in NH₃/MeOH(7N, 20 mL) in a sealed tube and the content was heated at 100 °C for 3 days. The volatile was evaporated and the residue partitioned between EtOAc/H2O, the organic layer was separated and aqueous was extracted with EtOAc(2X). Combined organic dried(Na₂SO₄). After concentration, the residue was column purified on Silica gel( 10% MeOH/DCM) to give the product as a white solid(1.1g, yield 57%).

### Step 2: Synthesis of 11

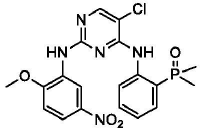

Compound **10** (908mg, 3.0mmol) and 2-iodo-4-nitroanisole (963mg, 4.15mmol) were dissolved in 25 mL anhydrous DMF in a sealed tube and was added Pd(OAc)₂ (86mg, 0.38mmol), XantPhos (227mg, 0.30mmol), K₃PO₄ (921 mg, 4.3 mmol). The content was heated at 120 °C overnight. After cooling to the room temp, EtOAc was added and the mixture was filtered through celite, washed with more EtOAc. Combined filtrate was conc in vacuo, and the residue was purified by CombiFlash(MeOH/DCM). The product (476 mg, 35.5%) was obtained as a tan solid.

### Step 3: Synthesis of 12

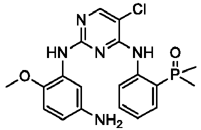

Compound **11** (476mg, 1.1mmol) was dissolved in THF(18 mL)/H₂O (13 mL) and was added Fe(300mg) and NH₄Cl(300 mL). The mixture was heated at 65 °C overnight. The liquid was decanted and the solid residue was washed with more THF. Combined solution was conc to give a residue. The residue was dissolved in DCM and filtered through cotton. The solution again is conc to give crude product 480mg.

### Step 4: Synthesis of 14

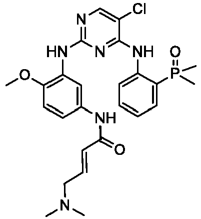

Compound **12** (125.4mg, 0.3 mmol), 4-bromocrotonoic acid (49mg, 0.3 mmol) was dissolved in 3 mL dry DCM. EDCI(63 mg, 0.33 mmol) was then added. The mixture was stirred at room temperature for 2 hrs. Volatiles were removed in vacuo and THF (3 mL) was added, followed by dimethylamine/H₂O solution (40%, 0.5 mL, 4.0mmol). The mixture was stirred at room temperature for another 2 hrs. Solvent was removed in vacuo and the residue was purified by prep-TLC (10 % MeOH/DCM) to give the final product as a tan solid(48mg, yield 30.2%).

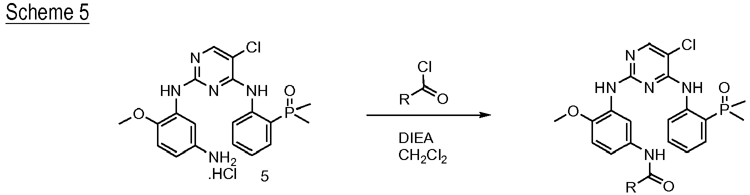

General procedures: compound **5** (100mg, 0.15 mmol) was dissolved in CH₂Cl₂ (1.6 mL) at 0 °C and DIEA (174 µL, 1mmol) was added. Acid chloride (13µl, 0.165 mmol, 1.1 eq) was added dropwise. The content was stirred at room temp for an hour. Solvent was removed *in vacuo* and the residue was purified by a prep-TLC plate(10%MeOH/DCM), the corresponding bands were collected to give final products.

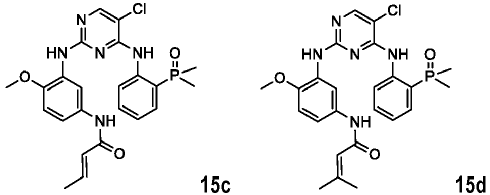

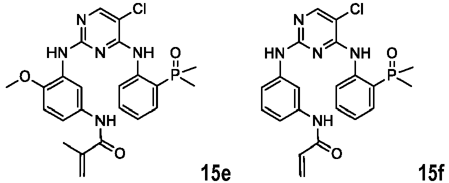

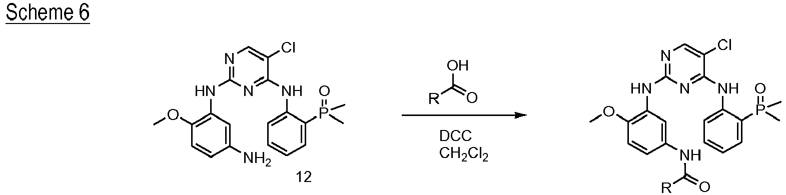

General procedures: compound **12** (84mg, 0.20 mmol), respective acid (0.27mmol, 1.35eq.) were dissolved in dry DMF (2.0 mL). DCC (56mg, 0.27mmol, 1.35eq) was added. The content was stirred at room temp overnight. Solvent was removed in vacuo and the residue was purified by a prep-TLC plate (6.5% MeOH/DCM), the corresponding bands were collected to give final products.

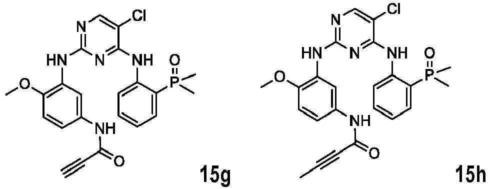

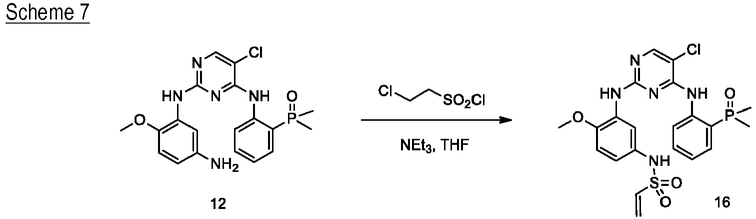

Compound **12** (200mg, 0.48 mmol), and NEt₃ (344 µL) was dissolved in 5 mL THF at room temperature, 2-chloroethylenesulfonyl chloride (115 µL, 1.1mmol) was added and the content was stirred at room temperature for 1 hr. The volatile was removed *in vacuo* and the residue was purified on prep-TLC(2X) (7.5 % MeOH/DCM). The product was a tan solid (56mg, yield 23%).

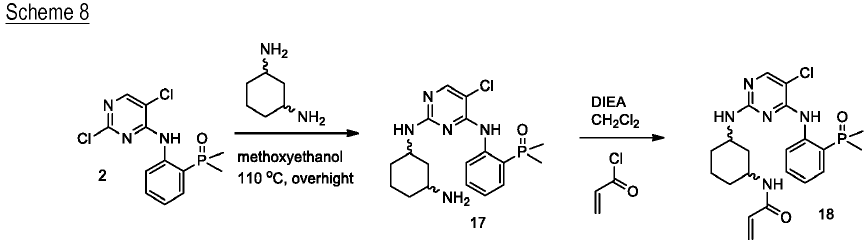

### Step 1: Synthesis of 17

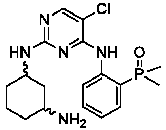

Compound 2 (158mg, 0.50 mmol), 1,3-diaminocyclohexane (57.1mg, 0.5mmol) was dissolved in methoxyethanol (1.6 mL) in a sealed tube and HCl/EtOH (200 µl) was added. The content was heated at 110 °C overnight. Solvent was removed *in vacuo* and the residue was purified by 2 prep-TLC plate (360 mL DCM/24 mL MeOH/12 mL 7N NH₃.MeOH) to give final product as a light colored solid (134 mg, yield 68 %).

### Step 2: Synthesis of 18

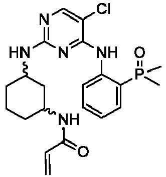

Compound **17** (70mg, 0.177 mmol) was dissolved in CH₂Cl₂ (1.6 mL) at 0 °C and DIEA (174 µL, 1mmol) was added. Aryloyl chloride (15µl, 0.185 mmol) was added dropwise. The content was stirred at room temp for an hour. Solvent was removed *in vacuo* and the residue was purified by a prep-TLC plate (10%MeOH/ DCM) to give two final product as tan solids (*trans* 29.4 mg, yield 43.8 %; *cis* 14.1 mg, 21.4%).

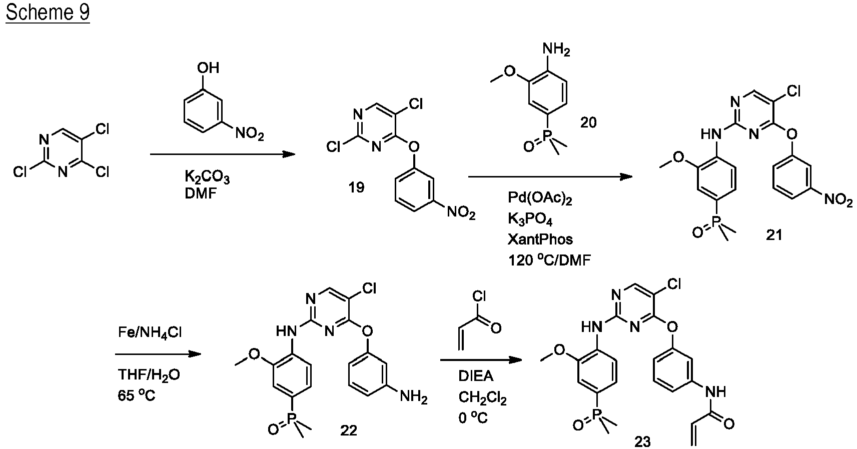

### Step 1: Synthesis of 21

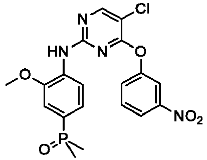

Compound **19** (154mg, 0.54mmol) and compound **20** (107mg, 0.54mmol) were dissolved in 4 mL anhydrous DMF in a sealed tube and was added Pd(OAc)₂ (14mg, 0.062mmol), XantPhos (37mg, 0.064mmol), and K₃PO₄ (150mg, 0.71 mmol). The content was heated at 120 °C overnight. After cooling to the room temp, EtOAc was added and the mixture was filtered through celite, washed with more EtOAc. Combined filtrate was conc *in vacuo,* and the residue was purified by CombiFlash (MeOH/DCM). The product **21** (140 mg, yield 58%) was obtained as an orange solid.

### Step 2: Synthesis of 22

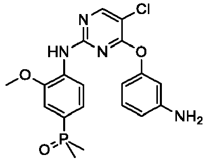

Compound **21** (140mg, 0.31mmol) was dissolved in THF/water (3 mL/3 mL) mixture and Fe (75mg,), NH₄Cl (75 mg,) was added. The content was heated at 65 °C overnight. After cooling to room temp., the mixture was filtered through cotton. The filtrate was concentrated and the residue was dissolved in 7.5% MeOH/DCM and was filtered through cotton again. After solvent was removed in *vacuo,* the crude product was obtained as a tan solid (126mg, yield 96%).

### Step 3: Synthesis of 23

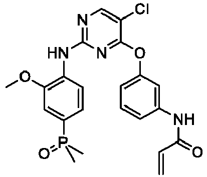

Compound **22** (163mg, 0.15 mmol) was dissolved in CH₂Cl₂(1.6 mL) at 0 °C and DIEA (174 µL, 1mmol) was added. Aryloyl chloride (13µl, 0.165 mmol) was added dropwise. The content was stirred at room temp for an hour. Solvent was removed *in vacuo* and the residue was purified by a prep-TLC plate (7.5%MeOH/DCM) to give final product as a tan solid (40 mg, yield 56 %).

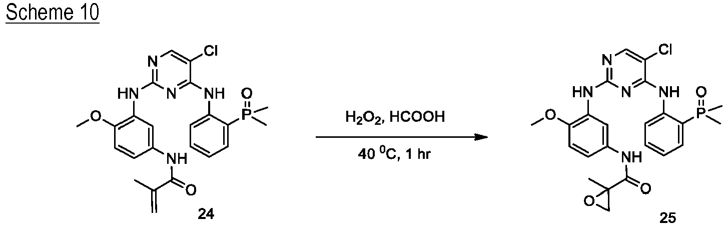

Procedure: compound **24** (49mg, 0.1mmol) was dissolved in 0.75 mL HCOOH and H₂O₂ (37%, 0.4 mL) was added. The content was heated at 40 °C for 1 hr. The volatiles were removed by N₂ blow and the residue was purified by prep-TLC (7.5% MeOH/DCM) to give the product as a tan solid (13.7 mg, yield 27%).

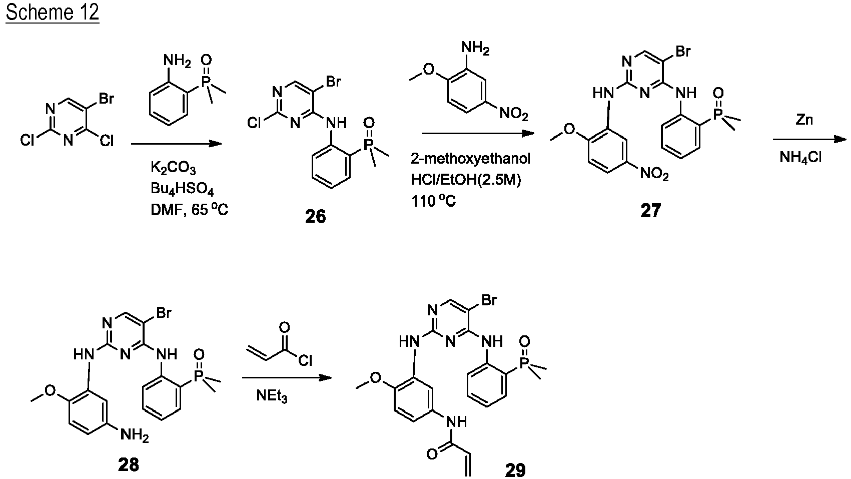

### Step 1: Synthesis of 26

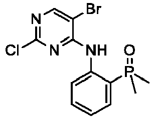

A suspension of 5-bromo- 2,4-dichloropyrimidine (2.8 g, 12.3 mmol, 1.0 eq), 2-dimethylphosphonylbenzeneamine (2.08 g, 12.3 mmol, 1.0 eq), K₂CO₃ (2.04 g, 14.8 mmol, 1.2 eq), and nBu₄HSO₄ (417 mg, 1.23 mmol, 0.1 eq) in 50 mL of DMF was stirred at 65 °C for 7 hours and cooled to room temperature. After a filtration, the filtrate was evaporated to an oil, which was chromatographed (DCM/MeOH 20:1) to give a yellow solid, 2.9 g, in 66% yield.

### Step 2: Synthesis of 27

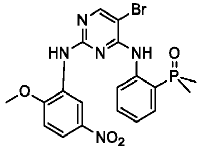

A mixture of **7** (1.42 g, 3.938 mmol, 1.0 eq), 2-methoxy-5-nitroaniline (927 mg, 5.513 mmol, 1.4 eq), and 2.5 M HCl/EtOH (6 mL) in 35 mL of 2-methoxyethanol was sealed and stirred at 110 °C for 5 hrs and cooled to room temperature. The mixture was worked up with sat. Na2CO3/DCM and purified with isco (MeOH/DCM 1:20) to give a yellow foam on oil pump, 520 mg, in 27% yield.

### Step 3: Synthesis of 28

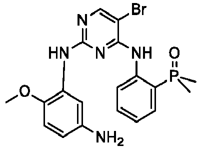

A mixture of the **27** (250 mg, 0.5 mmol), Zn (150 mg) and NH₄Cl (150 mg) in 2 mL of THF/H2O (5:1) was stirred at room temperature for 1.5 hours and filtered. The filtrate was worked up with saturated Na₂CO₃ and DCM. The crude product was purified with preparation plates to afford a yellow solid, 143 mg, in 61% yield.

### Step 4: Synthesis of 29

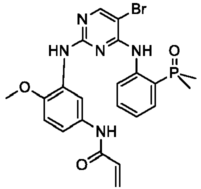

The aniline (280 mg, 0.606 mmol) was dissolved in 8 mL of DCM and 0.3 mL of triethylamine was added. The mixture was cooled to -35 °C and acryloyl chloride (54.8 mg, 49 µl, 0.606 mmol, 1.0 eq) was added in portions. The reaction was stirred around -30 °C for 15 min and quenched with saturated Na₂CO₃. The mixture was worked up with sat. Na₂CO₃/DCM and purified with preparation plates to give a light brown solid, 205 mg, in 66% yield.

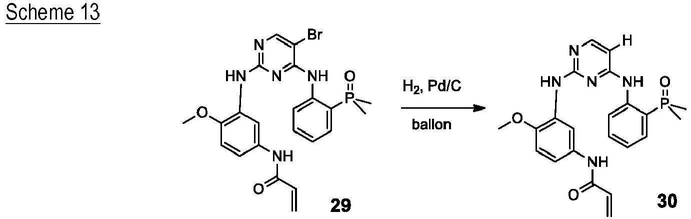

Procedure: 35 mg (0.0678 mmol) of **29** was dissolved in 1.5 mL of EtOH and Pd/C (10%, wet, 5 mg) was added. The mixture was stirred under H₂ balloon at room temperature overnight. The mixture was filtered and purified with a preparation plate to afford a white solid **30,** 8.9 mg, in 30% yield.

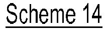

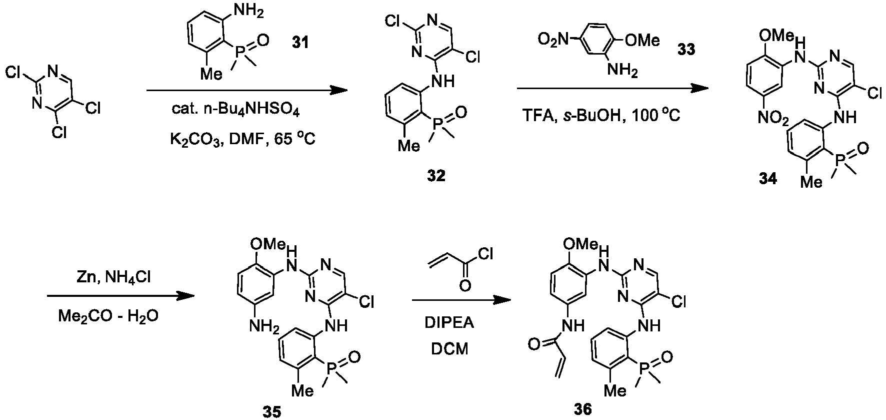

Step 1: Compound **31** was prepared according to the procedure described for the synthesis of compound **1** in Example 1, using 2-iodo-3-methylaniline instead of 2-iodoaniline as the starting material. A suspension of **31** (0.53 mmol), 2,4,5-trichloropyrimidine (1.0 eq), potassium carbonate (1.2 eq), and tetrabutylammonium hydrogensulfate (0.1 eq) in DMF was stirred at 65 °C for 18 hrs. Upon cooling the reaction mixture was filtered and the filtrate was concentrated. The residue was taken up into a mixture of EtOAc and water. After extraction with EtOAc (3x), the combined organic phases were concentrated to give essentially pure material which was used directly in next step reaction.

Step 2: A solution of **32** (0.82 mmol), 2-methoxy-5-nitroaniline **33** (1eq) and TFA (3 eq) in 2-BuOH (3 mL) was heated at 100 °C for 18 hrs. Upon cooling EtOAc and aq. NaHCO₃ were added to the reaction mixture. Extraction (3x) and concentration of combined extracts gave a solid which was purified on silica gel column (ISCO machine) with 10% MeOH in CH₂Cl₂ as the eluents, furnishing **34** as a brownish solid (55%).

Step 3: To a suspension of **34** (0.46 mmol) and zinc powder (6 eq) in acetone (9 mL) and water (1 mL) was added ammonium chloride (10 eq) at 0 °C. After the mixture was stirred at room temperature for 30 min, HPLC indicated a complete conversion. Acetone was removed on rotavap and the residue was suspended in DCM and water. Filtration was carried out and the filtrate was extracted with DCM. Concentration of combined organic layers gave crude aniline **35,** which was used in the next step without purification.

Step 4: To a solution of aniline **35** (0.43 mmol) and N, N-diisopropylethylamine (1.1 eq) in DCM (2 mL) was added acryloyl chloride (1.05 eq) at 0 °C. After the mixture was stirred at room temperature overnight, the volatile components were removed on rotavap. The residue was purified on silica gel column with 3% MeOH in DCM as eluents, furnishing amide **36** as beige solid (48 mg, 21%).

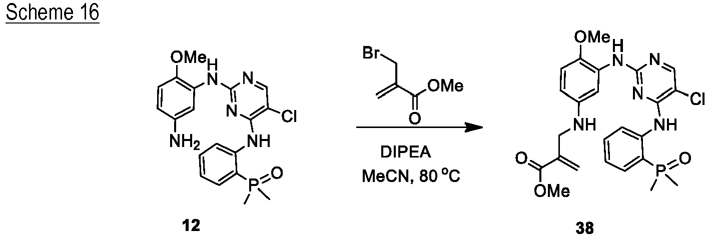

A solution of **12** (125 mg, 0.3 mmol), methyl 2-(bromomethyl)acrylate (1.3 eq) and N, N-diisopropylethylamine (1.3 eq) in MeCN (5 mL) was heated at 80 °C for 2 hrs. LC-MS indicated both mono- and bis-alkylation products were formed in almost equal amount, with small percentage of tris-alkylation products. The mixture was subjected to a prep-HPLC (reverse phase) purification and then a pre-TLC purification (normal phase silica gel, 10% MeOH in DCM as eluents), furnishing the title compound as a tan solid (15 mg, 10%).

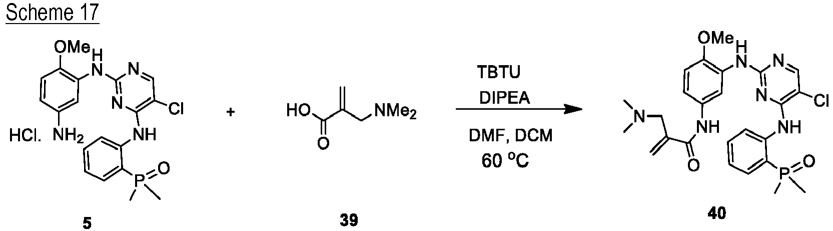

2-(Dimethylaminomethyl)acrylic acid **39** was prepared according to a literature procedure (Synth. Comm. 1995, 25, 641). To a solution of **39** (65 mg, 0.5 mmol), coupling reagent TBTU (1.2 eq) and N, N-diisopropylethylamine (3.0 eq) in DMF (5 mL) and DCM (20 mL) was added **5** (1 eq). After the mixture was stirred at room temperature overnight, the volatile components were removed on rotavap and the residue was purified by reverse phase prep-HPLC, furnishing the title compound as a tan solid (23 mg, 9%).

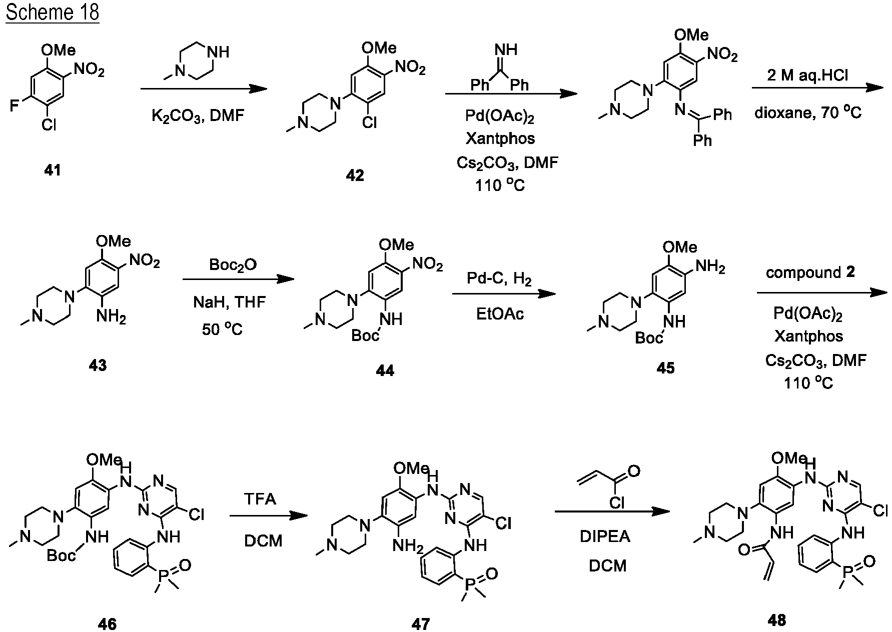

Step 1: the starting material **41** was prepared from 3-fluoro-4-chlorophenol via nitration and subsequent O-methylation, according to a published procedure (US Patent Publication No. 20080300242). The suspension of **41** (1.0 g, 4.86 mmol), 1-methylpiperzine (1 eq) and K₂CO₃ (1 eq) in DMF (20 mL) was heated at 80 °C for 4 hrs. DMF was removed and the residue was partitioned between DCM and water. Extraction and concentration followed by silica gel column chromatograph (10% MeOH in DCM as eluents) furnished **42** (1.26 g, 91%).

Steps 2 and 3: A degassed suspension of **42** (0.96 g, 3.4 mmol), benzophenone imine (1.5 eq), palladium acetate (0.1 eq), xantphos (0.2 eq) and cesium carbonate (1.6 eq) in DMF (20 mL) was heated at 110 °C overnight. Upon cooling the reaction mixture was filtered and the filtrate was concentrated. The solid residue was dissolved in dioxane and 2M aq. HCI (1:1, 40 mL) and then heated at 70 °C for 2 hrs. Upon removing dioxane on rotavap, the water layer was washed with DCM and then basified with aq. NaHCO₃. Extraction and concentration followed by silica gel column chromatograph (10% MeOH in DCM as eluents) furnished **43** (0.41 g, 45%).

Step 4: To a solution of **43** (0.38 g, 1.42 mmol) in THF (15 mL) was added NaH (2 eq.) under N₂ at 0 °C in multiple portions. After bubbles of H₂ were no longer observed, Boc₂O (4 eq.) was added. The resulting reaction mixture was heated at 50 °C and then refluxed for 2 hrs. The reaction was quenched with MeOH. Usual workup followed by silica gel column chromatograph (5% MeOH in DCM as eluents) furnished **44** (0.46 g, 88%).

Step 5: With EtOAc as the solvent **44** (0.46 g) was hydrogenated under 50 psi to afford **45** (0.42 g, 99%). Upon removing the solvent, the crude material was used directly in the next step.

Step 6: A degassed suspension of **45** (0.42 g, 3.4 mmol), **2** (1.5 eq), palladium acetate (0.1 eq), xantphos (0.2 eq) and cesium carbonate (1.3 eq) in DMF (10 mL) was heated at 110 °C for 48 hrs. Usual workup followed by silica gel column chromatograph (5% MeOH in DCM as eluents) furnished **46** (0.49 g, 64%).

Step 7: To a solution of **46** in DCM was added excessive TFA. After the mixture was stirred at room temperature for 2 hrs, the volatile components were removed on rotavap. The residue was dissolved in EtOAC and the solution was basified with aq. NaHCO₃. Extraction and concentration gave **47** as tan solid.

Step 8: Crude **47** (100 mg) was converted to **48** by using the procedure described in Example 14, step 4. The final product was purified by reverse phase prep-HPLC (10.4 mg, 9%).

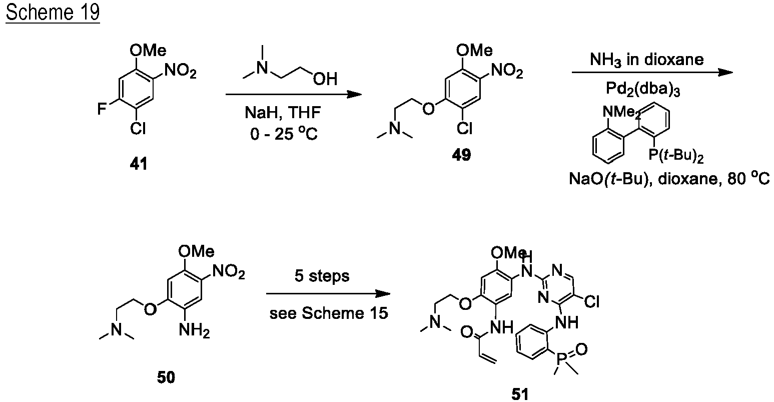

Step 1: Under N₂, to a suspension of **41** (0.5 g, 2.43 mmol) and NaH (1.5 eq) in THF (10 mL) was added dropwise a solution of 2-(dimethylamino)ethanol (1.1 eq) in THF (2 mL) at 0 °C. The resulting mixture was stirred at room temperature for 3 hrs. Usual workup followed by silica gel column chromatograph (10% MeOH in DCM as eluents) furnished **49** (0.53 g, 79%).

Step 2: To a degassed suspension of **49** (0.275 g, 1.0 mmol), Pd₂(dba)₃ (0.1 eq), 2-(di-t-butylphosphino)-N, N-dimethylbiphenylamine (0.1 eq) and sodium t-butoxide (1.4 eq) in dioxane (10 mL) was added a solution of NH₃ in dioxane (0.5 M in a N₂-sealed bottle, 10 mL). The resulting mixture was heated at 80 °C for 3 hrs. Usual workup followed by silica gel column chromatograph (15% MeOH in DCM as eluents) furnished **50** (0.15 g, 55%).

Steps 3 to 7: The poly-substituted aniline **50** was converted to the title compound **51** according to the procedure described in Example 18 by substituting **50** for **43.**

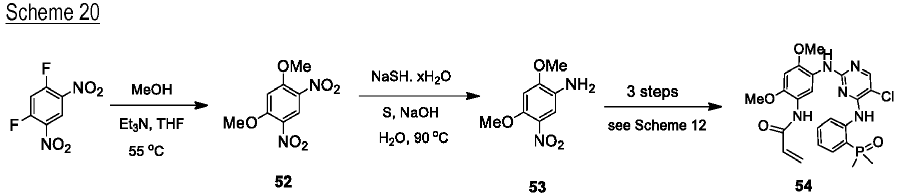

2,4-Dimethoxy-5-nitroaniline **53** was prepared from 1,5-difluoro-2,4-dinitrobenzene via double S_{N}Ar substitution to generate **52** and subsequent mono-reduction of nitro groups, according to a published procedure (J. Org. Chem. 2005, 70, 10660). This was converted to the title compound **54** as for Example 14 by substituting **53** for **33** and **2** for **32.**

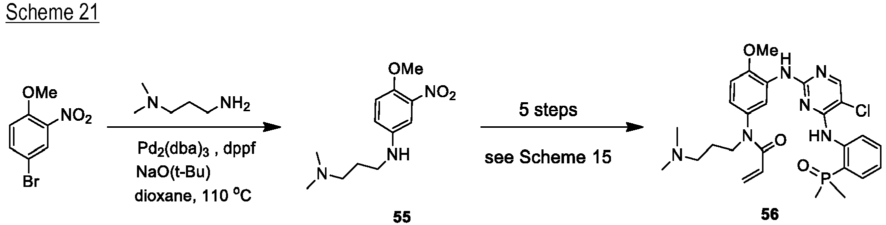

Step 1: A degassed suspension of 2-nitro-4-bromoanisole (2.32 g, 10 mmol), N,N-dimethyl-1,3-propanediamine (1.1 eq), Pd₂(dba)₃ (0.02 eq), dppf (0.04 eq) and sodium t-butoxide (1.5 eq) in dioxane (20 mL) was heated at 110 °C overnight. Upon cooling the reaction was quenched with water. The volatile components were removed on rotavap and the residue was partitioned between EtOAc and water. Extraction and concentration followed by silica gel column chromatograph (15% MeOH in DCM as eluents) furnished **55** (0.66 g, 26%).

Steps 2 to 6: The secondary amine **55** was converted to the title compound **56** as for Example 18 by substituting **55** for **43.**

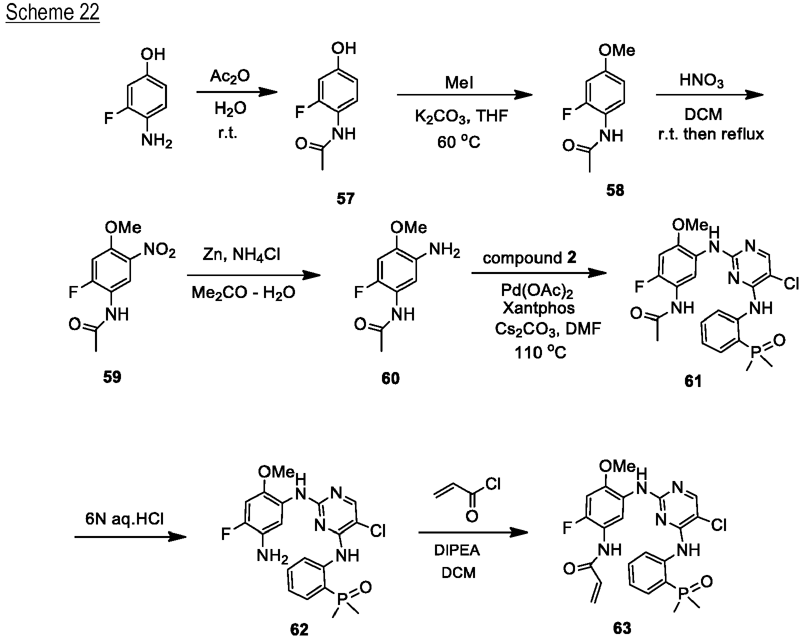

Step 1: Acetic anhydride (7.8 mL, 82.6 mmol) was added dropwise with vigorously stirring to a suspension of 3-fluoro-4-aminophenol (10 g, 78.6 mmol) in water (20 mL). Insoluble amide started to precipitate as a white solid in a few minutes. After the reaction mixture was stirred for 10 more minutes, the white solid was collected via filtration and washed with cold water. Silica gel column chromatograph (5% MeOH in DCM as eluent) gave pure **57** (8.75 g, 66%).

Step 2: To a suspension of **57** (8.75 g, 51.73 mmol) and K₂CO₃ (1.1 eq) in THF (30 mL) was added methyl iodide (1.2 eq). The mixture was heated in a sealed tube at 60 °C overnight. Filtration and concentration followed by silica gel column chromatograph (5% MeOH in DCM as eluent) furnished pure **58** (7.17 g, 89%).

Step 3: Nitric acid (70%, 3.83 mL) was added dropwise to a solution of **58** (7 g) in DCM (70 mL) with vigorously stirring. After stirred at room temperature for 1 hr, the reaction mixture was refluxed for 3 hrs. DCM was removed on rotavap and the residue was washed with cold water and then subjected to a silica gel column chromatograph purification (5% MeOH in DCM as eluent) to afford **59** (3.26 g, 37%).

Step 4: Poly-substituted nitrobenzene **59** was reduced to corresponding aniline **60** according to the procedure described in Example 14, step 3.

Step 5: Poly-substituted aniline **60** (200 mg, 1 mmol) was coupled with precursor **2** (1.5 eq) to afford **61** (320 mg, 67%) via the procedure described in Example 18, step 6.

Step 6: N-arylacetamide **61** (320 mg) was heated at reflux in 6N HCI for 30 min. After basification, extraction and concentration aryl amine **62** was obtained (290 mg, 98%).

Step 7: Crude **62** (150 mg) was converted to **63** by using the procedure described in Example 14, step 4. The final product was purified by reverse phase prep-HPLC (41 mg, 24%).

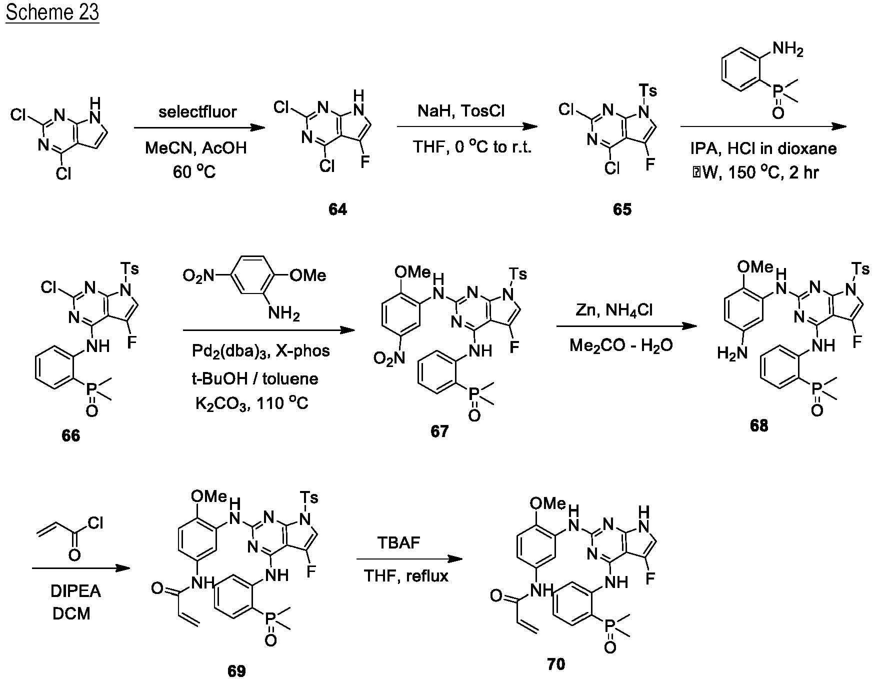

Step 1: 2,4-Dchloro-7H-pyrrolo[2,3-d]pyrimidine (5.0 g, 27 mmol) was suspended in MeCN (300 mL) and AcOH (60 mL); to this was added selectfluor (1-chloromethyl-4-fluoro-1,4-diazoniabicyclo[2.2.2]octane bis(tetrafluoroborate), 1.4 eq, 13.2 g) in one portion. The reaction mixture was stirred at 60 °C overnight. HPLC monitoring indicated complete conversion. After the solvents were evaporated to the volume of ∼100 mL, toluene (20 mL) was added and the suspension was filtered. The filtrate was evaporated to dryness and re-evaporated with toluene (2 × 20 mL). The residue was then purified via a short silica pad (washing with 1/1 DCM/EtOAc) and column chromatography (ISCO machine, EtOAc/DCM, EtOAc on a 0-100% gradient) to give the crude product. Upon standing the pure product was seen to precipitate from the column fractions. These were filtered and the mother liquors were combined to precipitate a 2^{nd} crop. Total 1.23 g of the product **64** was obtained (22% yield, ∼ 90% pure, Cl-isomer was the main impurity).

Step 2: A solution of **64** (1.22 g, 6 mmol) in THF (10 mL) was slowly added to a suspension of NaH (1 eq) in THF (10 mL) at 0 °C. After the mixture was stirred for 10 min, a solution of tosyl chloride (1 eq) in THF (5 mL) was slowly added. Stirring was continued for 30 min at 0 °C and then at room temperature overnight. The reaction was shown to be complete via HPLC monitoring and was quenched via the addition of aq. NH₄Cl (1 - 2 mL). The reaction mixture was then filtered through celite and the filtrate was evaporated. The crude product was purified via column chromatography (ISCO machine, EtOAc/Heptane, EtOAc on a 0-100% gradient) to give **65** (1.22 g, 56%).

Step 3: In a microwave vessel (20 mL) were placed **65** (600 mg, 1.7 mmol), 1 (282 mg, 1.7 mmol) and isopropanol (10 mL). After HCI (1.3 mL, 4 M in dioxane) was added, the resulting mixture was stirred in the microwave reactor at 150 °C for 2 hrs. The solvents were evaporated and the residue was purified via silica column chromatography (ISCO machine, EtOAc/Heptane, 0-100% EtOAc to elute impurities and then MeOH/DCM, 0-20% MeOH) to give pure **66** (900 mg, 54%).

Step 4: Intermediate **66** (493 mg, 1 mmol), 2-methoxy-5-nitroaniline **33** (168 mg, 1 mmol), K₂CO₃ (1.4 mmol), Pd₂dba₃ (5 mol%) and X-Phos (10 mol%) were weighed into a 100 mL round bottom flask and placed under N₂. The solvents toluene (10 mL) and tert-butanol (2 mL) were added as a mixture and the stirring solution was evacuated and backfilled with N₂ three times. The resulting mixture was then stirred overnight at 110 °C. HPLC showed the reaction to be complete and the solvents were evaporated. The residue was purified by silica column chromatography on ISCO machine (5% MeOH in DCM as eluents) to give coupling product **67** (570 mg, 91%).

Step 5: Poly-substituted nitrobenzene **67** was reduced to corresponding aniline **68** according to the procedure described in Example 14, step 3.

Step 6: Crude aniline **68** (123 mg) was converted to **69** by using the procedure described in Example 14, step 4. The product was purified by silica column chromatography on ISCO machine (5% MeOH in DCM as eluents). Yield: 69 mg, 51%.

Step 7: A solution of **69** (60 mg) and TBAF (1 M in THF, 0.3 mL) in THF (10 mL) was refluxed for 5 hrs. HPLC indicated a complete reaction. After the solvent was evaporated, the residue was purified by silica column chromatography on ISCO machine (5% MeOH in DCM as eluents). The product was co-eluted with TBAF; water wash furnished pure product (10 mg, 21%).

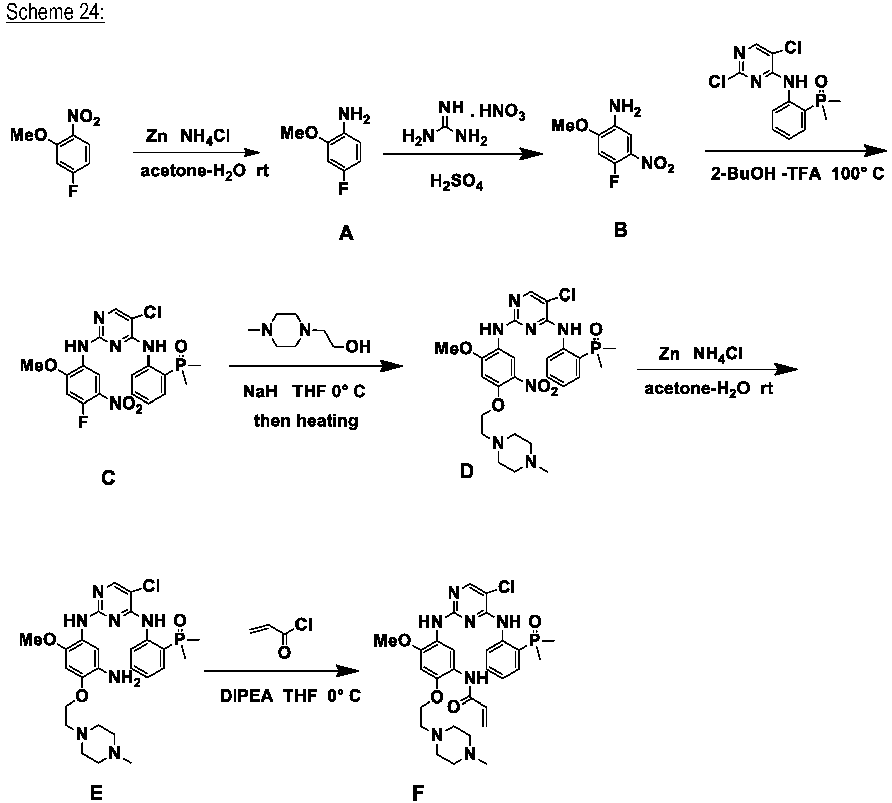

Step 1: To a suspension of 5-fluoro-2-nitroanisole (50 mmol, 8.5 g) and zinc powder (3.5 eq, 11.4 g) in acetone (45 mL) and water (5 mL) was added ammonium chloride (11 eq, 29.3 g) at 0 °C in multiple portions. After the mixture was stirred at r.t. overnight, HPLC indicated a complete conversion. Acetone was removed on rotavap and the residue was suspended in DCM and water. Filtration was carried out and the filtrate was extracted with DCM. Concentration of combined organic layers gave crude aniline (∼ 7.0 g), which was used in the next step without purification.

Step 2: To a suspension of 4-fluoro-2-methoxyaniline (5.1 g, 36.1 mmol) in concentrated sulfuric acid (55mL) was added guanidine nitrate (4.38g, 36.1mmol) in portion wise under ice cooling over 15 min. The mixture was stirred at the same temperature for additional 15 min. The reaction was then poured into a saturated cold NaHCO₃ solution and the precipitated solid were collected by filtration. The residue was taken up in EtOAc and dried over anhydrous Na₂SO₄. The solvent was stripped off to afford the **B** (4.72g).

Step 3: The above compound **B** (0.1g, 0.53mmol) and **4-(ortho dimethyl phosphinyl anilino)-5-chloro**-**2-chloro pyrimidine** (0.17g, 0.53mmol) were dissolved in a mixture of 2-butanol (1.2mL) & trifluoroacetic acid (0.25 mL) and were heated to 100° C in a seal tube for overnight. The reaction mixture was then cooled to rt and poured into a saturated NaHCO₃ solution while stirring to afford an orange solid which was filtered, washed with Et₂O to remove final traces of water. The product was dried to afford **C** (0.19g) which was directly used in the next step.

Step 4: NaH (0.039g, 0.96 mmol, 60% dispersion in oil) was taken up in a dry capped microwave vial. To this, **1-(2-hydroxyethyl)-4-methylpiperazine** (0.023g, 0.16mmol) dissolved in dry tetrahydrofuran ( 1.6mL) was added dropwise. The mixture was stirred at rt for 20 min. Intermediate **C** (0.075g, 0.16mmol) was then added in one portion to this suspension and the mixture was heated to 67° C in the closed seal tube for 25 min. The mixture was allowed to reach at rt and quenched with a few drops of methanol. Solvent was removed under vacuum and the resultant crude was subjected to FCC eluting with DCM- MeOH (95/5) to furnish the desired product **D** (0.081g).

Step 5: Compound **D** (0.078g, 0.13mmol) was dissolved in a mixture of acetone (1.3mL) and water (0.3mL). To this, zinc nano powder (0.07g, 1.3mmol) was added immediately followed by addition of NH₄Cl (0.16g, 2.6mmol) in small portions. The mixture was vigorously stirred at r.t for 30 min. Anhydrous Na2SO4 was then added to this stirring mixture and the resultant crude was filtered, solvents were evaporated and the residue were taken up in DCM and directly loaded on the silica gel cartridge and eluted with DCM- MeOH-NH₃ (90/10) to furnish the desired product **E** (0.044g).

Step 6: To a solution of **E** (0.044g, 0.078mmol) in dry tetrahydrofuran (0.52mL) was added DIPEA (0.027mL, 0.156 mmol) at 0° C under stirring. This was followed by the addition of acryloyl chloride (0.007g, 0.078mmol). The reaction was stirred at that temperature for additional 1h. Solvent was stripped off under vacuum and the crude was purified by FCC using DCM- MeOH-NH₃ (90/10) to furnish a gum which was further triturated with Et₂O to furnish a solid material F (0.02g).

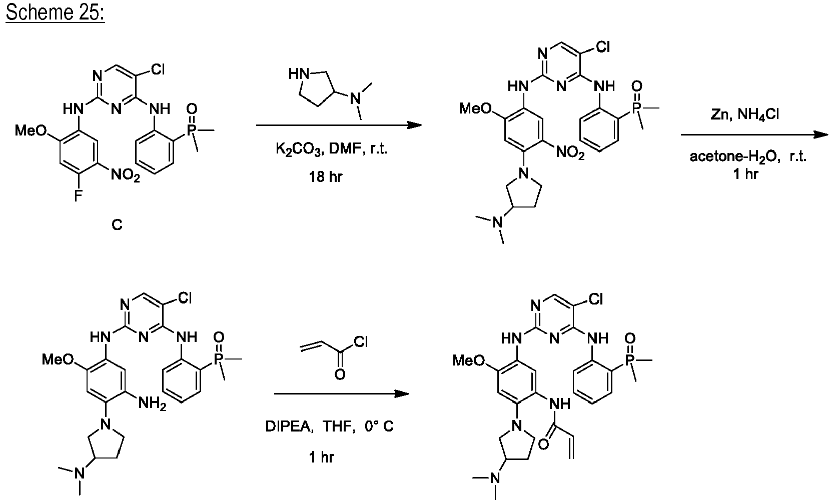

Step 1: The suspension of advanced intermediate C, (3-dimethylamino)pyrrolidine (1 eq) and K2CO3 (2eq) in DMF was stirred at r.t. overnight. The solid components were filtered off and the filtrate was concentrated on rotavap and then on vacuum pump. The residue was essentially pure by HPLC analysis and was used directly in the next step.

Steps 2 and 3: these were carried out according to the procedure outlined in Scheme 24, steps 5 and 6.

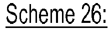

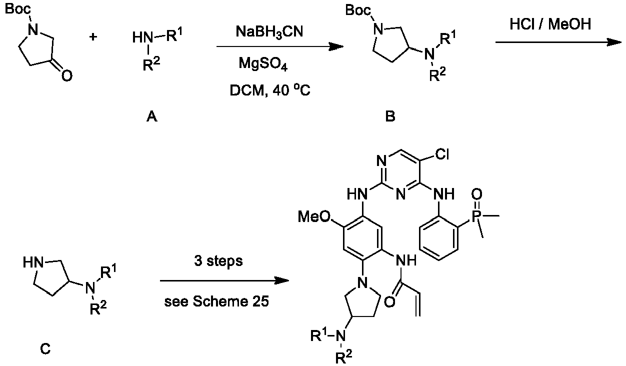

The suspension of N-Boc-pyrrolidin-3-one, secondary amines, sodium cyanoborohydride and magnesium sulfate in DCM was stirred at 40 oC overnight. After the solid components were removed by filtration, 2.5N HCI in MeOH was added to the filtrate and the resulting solution was stirred at r.t. for 30 min. Volatile components were removed on rotavap; the residue was partitioned between DCM and aq. NaHCO3. Combined organic phases were concentrated and the residue was purified by silica gel column chromatography to furnish (3-dialkylamino)pyrrolidine C, which was converted into final compounds by substituting C for (3-dimethylamino)pyrrolidine.

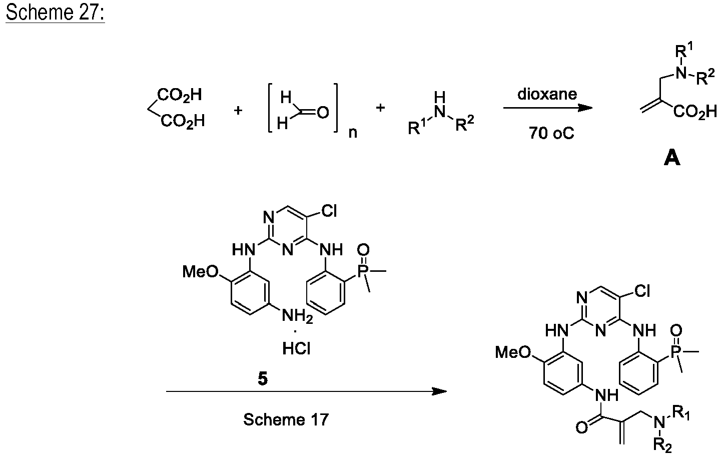

Secondary amines were converted into 2-(dialkylaminomethyl)acrylic acid **A** according to a literature method (Synth. Comm. 1995, 25, 641). These were converted into final compounds according to the procedure outlined in Scheme 17 by substituting A with 39.

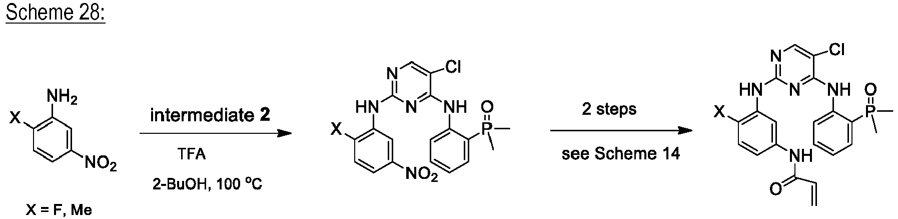

2-Fluoro-5-nitroaniline or 2-methyl-5-nitroaniline was converted into desired compound according to the procedure outlined in Scheme 28.

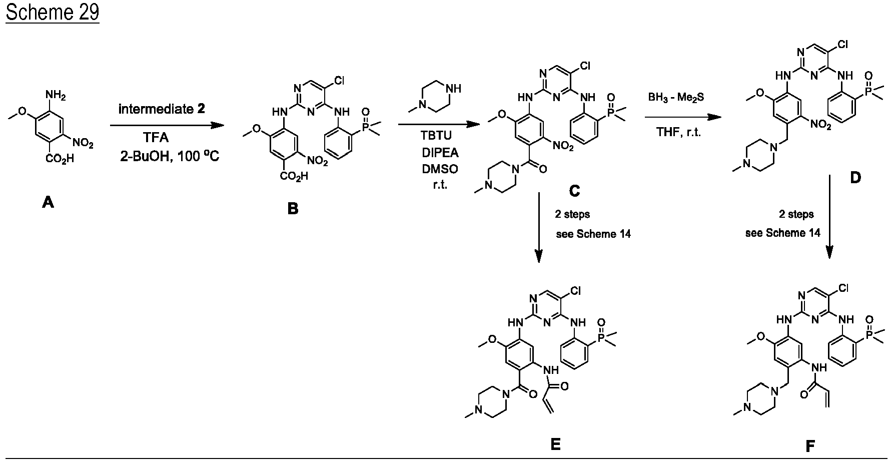

Step 1: the starting material A underwent a S_{N}Ar reaction with intermediate 2 according to the procedure described in step 1 of Example 14.

Step 2: To a solution of compound **B** (246 mg, 0.5 mmol), coupling reagent TBTU (1.2 eq) and N, N-diisopropylethylamine (3.0 eq) in DMSO (10 mL) was added piperazine (1 eq). After the mixture was stirred at room temperature overnight, water and DCM was added to facilitate extraction. Combined extracts were washed with water to remove DMSO. After drying over Na2SO4 and concentration on rotavap the residue was purified by silica gel column chromatography with 5% MEOH in DCM as the eluents, furnishing compound **C** as a tan solid (154 mg, 54%).

Step 3: A solution of compound **C** (570 mg) and BH3-Me2S (2.0 M in THF, 5 eq, 0.6 mL) in THF (5 mL) was stirred at r.t. overnight. 6N aq. HCI (15 mL) was added and the resulting solution was stirred at r.t. for 5 hr. Solid K2CO3 was carefully added to basify the reaction mixture. After extractions with DCM the combined organic phases were dried, concentrated and purified by silica gel column chromatography with 5% MeOH in DCM as the eluents, furnishing compound **D** as a tan solid (300 mg, 54%). Steps 4 and 5: Compounds **C** and **D** were converted into final compounds **E** and **F** respectively according to the procedure described in Example 14.

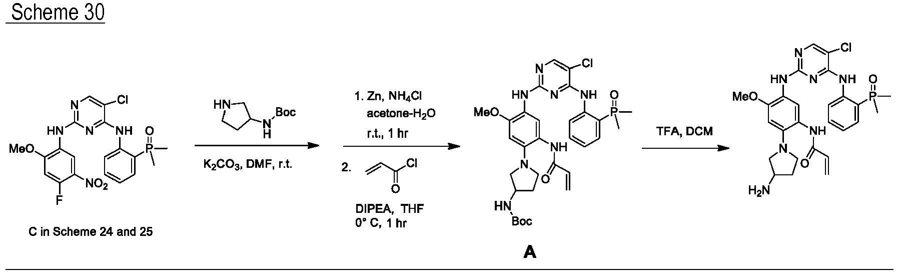

Compound A was synthesized according to the procedure outlined in Scheme 25 by substituting 3-(tert-butoxycarbonylamino)pyrrolidine for 3-(dimethylamino)pyrrolidine. Standard de-protection of Boc with TFA-DCM followed by basification and silica gel column chromatography (5% MeOH in DCM as the eluents) furnished title compound.

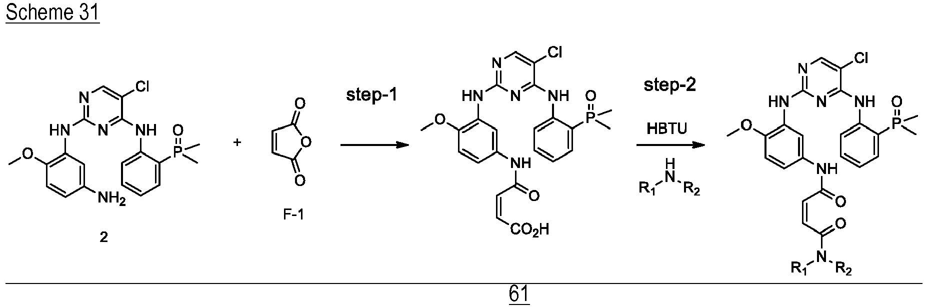

### Step-1: Synthesis of 61:

To a mixture of compound **2** (420 mg) in THF (2.0 mL) was added maleic anhydride (1.0 eq) at room temperature. The reaction was stirred at room temperature for 1 hour. The product was obtained by filtration and then washed with DCM (2.0 mL) to yield the product, 61, as a yellow solid (435 mg, yield 84%).

### Step-2: Synthesis of Examples 62, 63 and 64

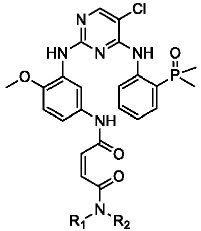

General procedures: The compound of Example 61 (77 mg, 0.15 mmol), prepared as in Step-1 (immediately above), was dissolved with the respective amine (0.15mmol, 1.0eq.) in dry DMF (2.0 mL). HBTU (80mg) was added and followed by Et₃N (30 uL). The contents were stirred at room temp for 1h. Solvent was removed in vacuo and the residue was purified by a prep-TLC plate (6.5% MeOH/DCM). The corresponding bands were collected to give the final products:

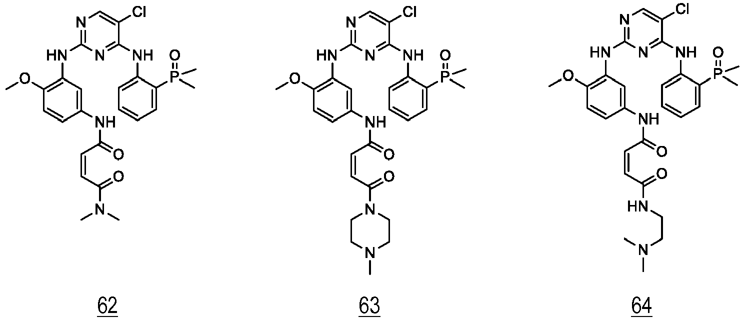

### Synthesis of Example 75

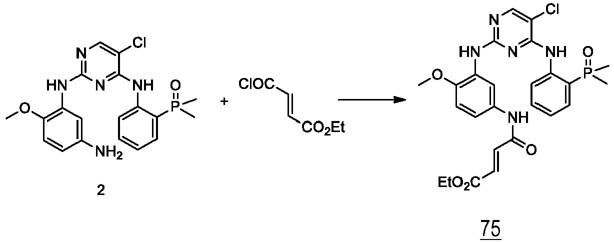

A solution of compound **2** (84 mg) in DCM (3.0 mL) and Et₃N (0.1 mL) was treated with Ethyl fumaroyl chloride (1.05eq). The contents were stirred at room temp for an hour. Solvent was removed *in vacuo* and the residue was purified by a prep-TLC plate (7.5%MeOH/DCM) to give the product as a yellow solid (34 mg, yield 31%).

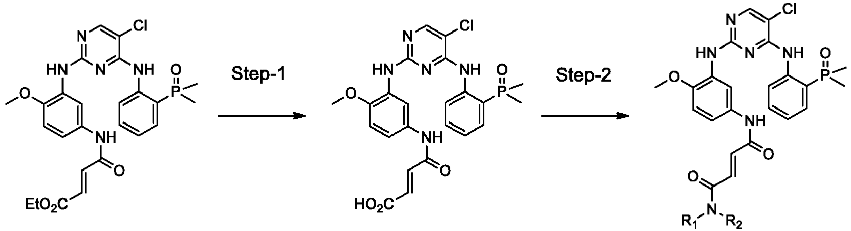

### Step-1

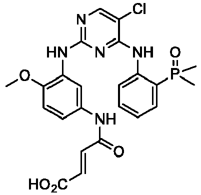

Example 75 (360 mg) in MeOH (1.5mL) and water (0.5mL) was treated with K₂CO₃ (1.0g) at 50°C for 2h. The reaction mixture was cooled down to room temperature. The organic solution was transferred to a new vial and diluted with DCM (3.0mL). The pH was adjusted to 6-7 by adding hydrochloric acid. The resulting organic layer was evaporated and the residue was washed with MeOH to give product as a yellow solid (304 mg, yield 89%)

### Step-2

Compounds for Examples 60, 59 and 58 were made according to the general procedure for Example 62, using the appropriate amine:

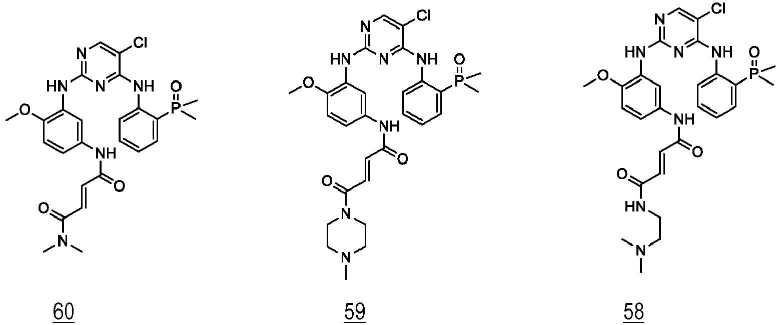

### Scheme 32 : Example 65

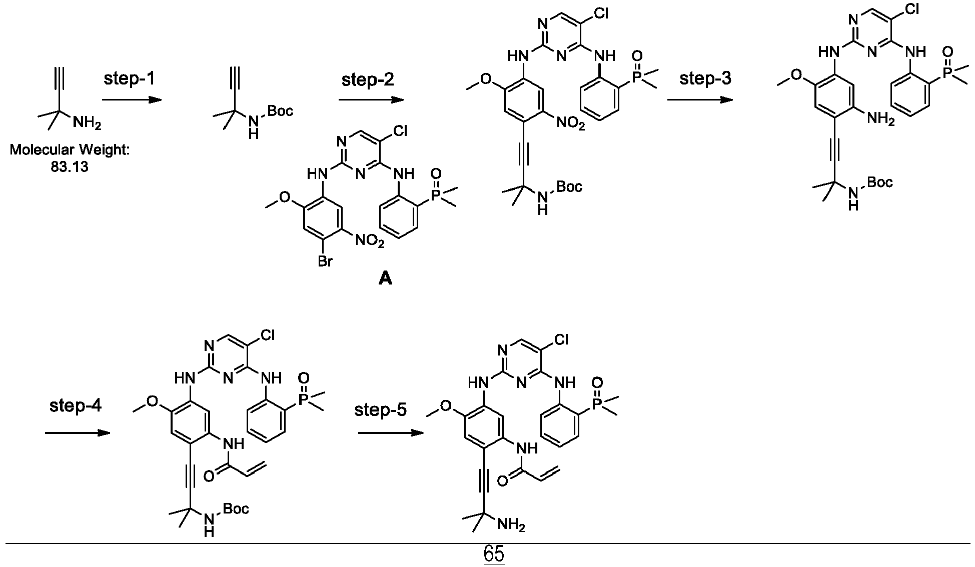

### Step-1

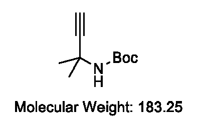

A mixture of 1,1-dimethylpropargylamine (4.2g) and Boc₂O (11g) without solvent was warmed up to 50°C for 30 min. The resulting solid was treated with n-hexane (10 mL) and the product was collected by filtration to give white solid (7.6 g, yield 82%)

### Step-2

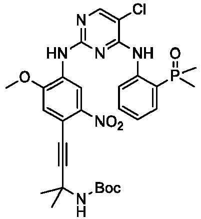

To a mixture of compound **A** (527 mg) and step-1 product (250 mg) in DMF (5.0 mL), was added (Ph₃P)₂PdCl₂ (0.1 g), Cul (0.05 g) and Et₃N (0.2 mL). The mixture was stirred at 80°C for 4h. The reaction mixture was concentrated and the residue was purified by a prep-TLC plate (8%MeOH/DCM) to give final product as a yellow solid (430 mg, yield 68%).

### Step-3

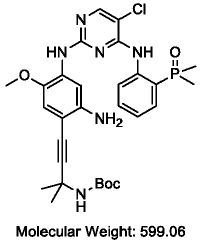

Water was added dropwise to a mixture containing the product of step-2 (240 mg) in acetone (1.0 mL), Zn dust (0.3 g) and NH₄Cl (0.15 g). The mixture was stirring at room temperature for 15 min and then diluted with DCM (5 mL). After filtration, the organic solution was evaporated and the residue was used for next step.

### Step-4

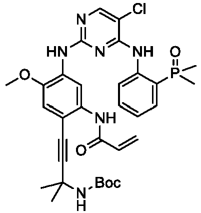

The residue from Step-3 was dissolved in THF (3.0 mL) and Et₃N (0.05 mL), and the resulting solution was treated with acroyl chloride. The reaction was monitored by HPLC until disappearance of starting material. The reaction was then quenched with aq.NaHCO₃ and extracted with DCM (2 x 5.0 mL). The organic solution was concentrated and the residue was purified by a prep-TLC plate (10%MeOH/DCM) to give product as a yellow solid (112 mg, yield 45% for two steps).

### Step-5

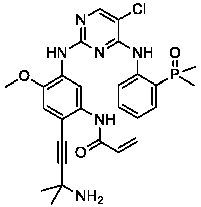

A solution of the product of Step-4 (80 mg) in MeOH (1.0 mL) and DCM (0.5 mL) was treated with TFA (0.5 mL) at 0°C. The mixture warmed up to room temperature for 1h. Solvent was evaporated, the residue was neutralized with aq. NaHCO3, and the neutralized material was subjected to prep-TLC plate (20%MeOH/DCM) to give the desired product, 65, as a light yellow solid (42 mg, yield 62%).

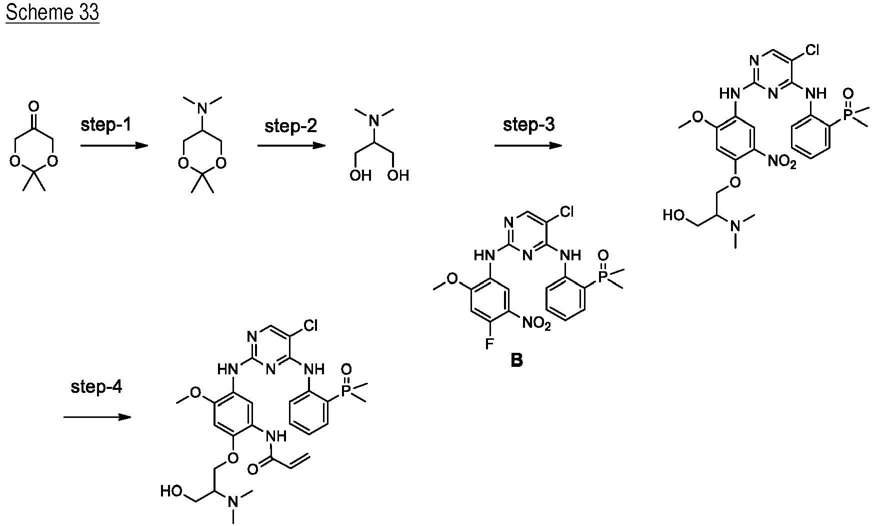

### Step-1

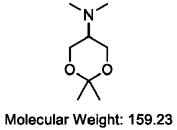

To a mixture of 2,2-dimethyl-1,3-dioxan-5-one (2.6 g), dimethyl amine HCl salt (1.8 g) in DCM (50 mL) was added NaHB(OAc)₃ (6.0 g) and Et₃N (3.0 mL). The reaction mixture was stirred at room temperature overnight and then diluted with aq.NaHCO3. The organic layer was dried and evaporated to give colorless oil (2.5 g, yield 79%).

### Step-2

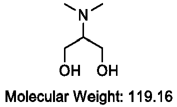

A solution of step-1 product (2.4 g) in MeOH (5.0 mL) was treated with HCl (10 mL, 2N). The mixture was heated to reflux for 15min. Solvent was evaporated and the residue was dissolved in DCM (6.0 mL). The organic solution was dried, filtered and evaporated to give colorless oil (1.5g, yield 77%).

### Step-3

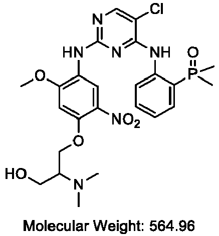

The nitro compound was synthesized by reacting compound **B** and the product of step-2 according the general procedure.

### Step-4

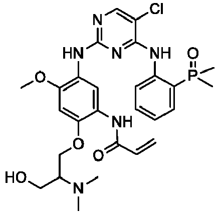

Reduction of the nitro group and formation of the corresponding acrylamide was carried out according to the procedure in Scheme 32, step-3 and step-4.

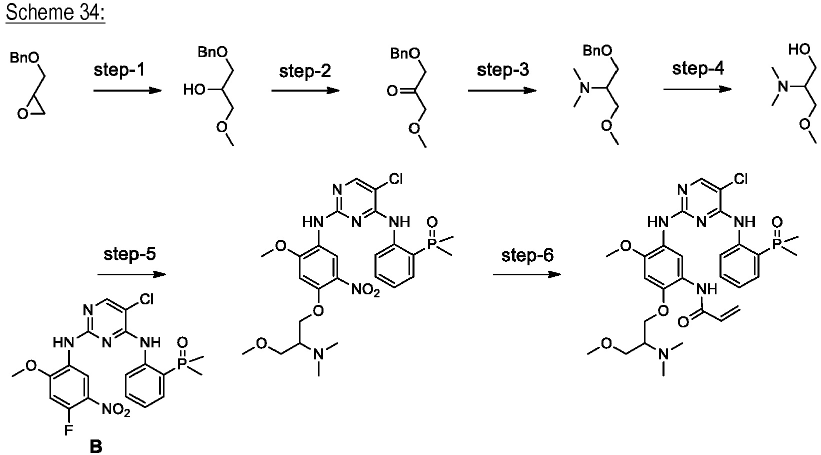

Step 1: To a solution of benzyl glycidyl ether (5.0 g) in MeOH (5.0 mL) was added NaOMe (5.0 mL, 25% in methanol). The mixture was warmed up to 50°C for 1h and then heated to reflux for 5min. The mixture was treated with wet NaHCO₃ and filtered. Solvent was evaporated and the residue was dissolved in DCM (20 mL). The solution was dried and evaporated to give yellowish oil (4.8 g, yield 80%).

Step 2: A solution of step 1 product (3.0 g) in DCM (100 mL) was treated with PDC (6.0 g) and molecular sieves (4.0 g). The mixture was stirred at room temperature for 4h and diluted with Et₂O (100mL). The mixture was filtered through a Celite pad and solvent evaporated to give yellow oil (1.7 g, yield 57%).

Step 3: The compound was synthesized according to the following procedure: To a mixture of 2,2-dimethyl-1,3-dioxan-5-one (2.6 g), dimethyl amine HCl salt (1.8 g) in DCM (50 mL) was added NaHB(OAc)₃ (6.0 g) and Et₃N (3.0 mL). The reaction mixture was stirred at room temperature overnight and then diluted with aq.NaHCO3. The organic layer was dried and evaporated to give colorless oil (2.5 g, yield 79%).

Step 4: A solution of step-3 product (1.5 g) in MeOH (10 mL) was charged with Pd-C (0.5 g, 10% wet) and hydrogenated under a hydrogen balloon at room temperature overnight. The catalyst was filtered off and the solvent was evaporated to give the product (0.64 g, yield 71%) as colorless oil.

Step 5: The compound was synthesized according the general procedure from compound **B** and step-4 product as a yellow solid.

Step 6: Example 37 was synthesized according to a similar procedure to the following: To a mixture of tert-butyl (4-(4-((5-chloro-4-((2-(dimethylphosphoryl)phenyl) amino)pyrimidin-2-yl)amino)-5-methoxy-2-nitrophenyl)-2-methylbut-3-yn-2-yl)carbamate (240 mg) in acetone (1.0 mL) and Zn dust (0.3 g), NH₄Cl (0.15 g), was added drops of water. The mixture was stirring at room temperature for 15 min and then diluted with DCM (5 mL). After filtration, the organic solution was evaporated and the residue was used for next step. The residue was dissolved in THF (3.0 mL) and Et₃N (0.05 mL) and solution was treated with acroyl chloride. The reaction was monitored by HPLC until start material disappear. The reaction was quenched with aq.NaHCO₃ and extracted with DCM (2 x 5.0 mL). The organic solution was concentrated and the residue was purified by a prep-TLC plate (10%MeOH/DCM) to give the desired product.

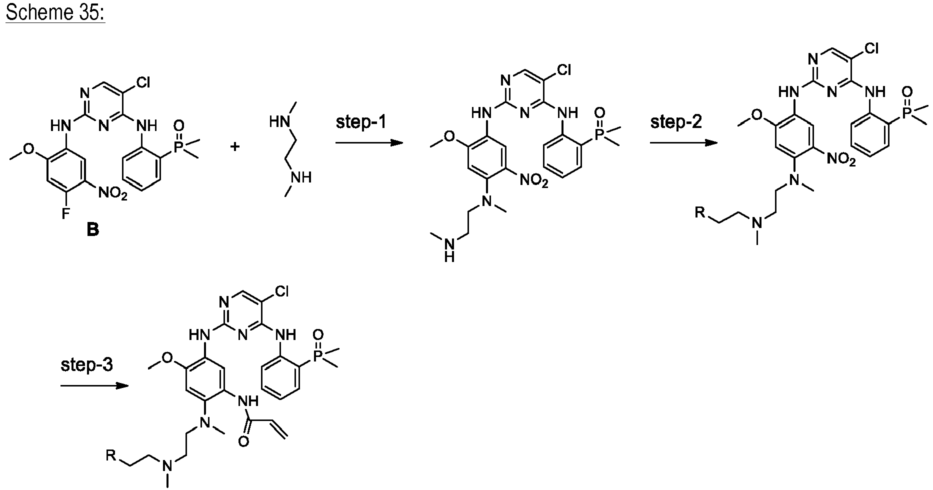

Step 1: To a solution of N,N'-dimethylethylenediamine (300 mg) in DMF (2.0 mL) was added K₂CO₃ (1.0 g) and compound **B** (466 mg). The mixture was heated at 80°C for 3h. Solvent was evaporated and the residue was extracted with DCM and then purified by a prep-TLC plate (10%MeOH/DCM with 1% NH₃ in methanol) to give product as a yellow solid (400 mg, yield 75%).

Step 2: A solution of step 1 product (1eq) in DMF (3.0 mL) was treated with NaHCO₃ (0.5 g) and the respective bromide (2.5eq) at 50°C for 5h. Solvent was evaporated and the products were purified by a prep-TLC plate (8%MeOH/DCM) to give product as yellow solids.

Step 3: Reduction of the nitro group and formation of the corresponding acrylamide was carried out according to the procedure in Scheme 32, step-3 and step-4.

The methoxy- and fluoro- ompounds below were synthesized by this method: 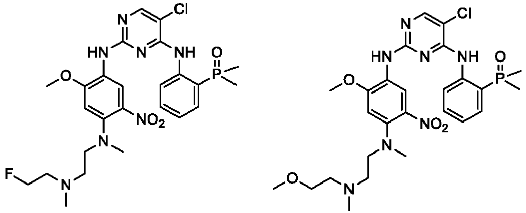 and were converted to the corresponding acrylamides. See Example 67.

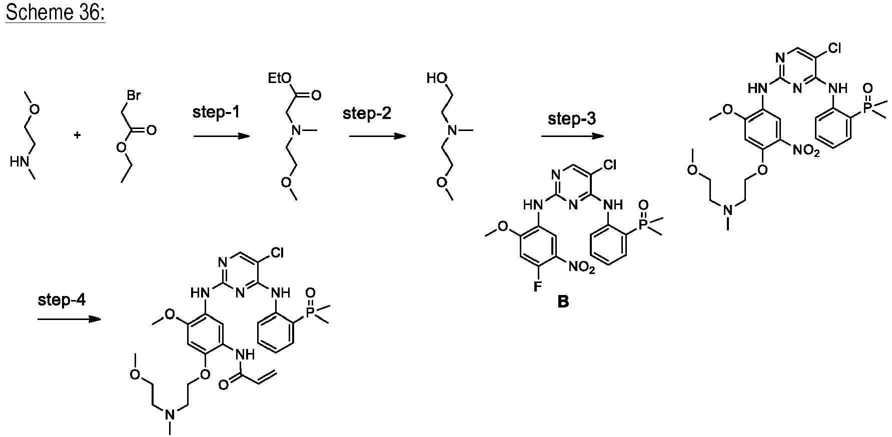

Step 1: A mixture of N-(2-methoxyethyl)methylamine (1.8 g) and ethyl bromoacetate (3.4 g) in acetonitrile (20 mL) was treated with K₂CO₃ (4.0 g) and Nal (20 mmol). The mixture was refluxed overnight. Solvent was evaporated and the residue was extracted with DCM and then purified on Silica gel column (0-8% MeOH/DCM) to give the product as colorless oil (3.2 g, yield 93%).

Step 2: To a solution of step-1 product (3.5 g) in THF (20 mL) was added LAH (800 mg) portion wise. The resulting mixture was stirred at room temperature overnight and then quenched with EtOAc and water. After filtration, the organic solution was evaporated to give colorless oil (2.0 g, yield 75%). Step 3: The step 3 product compound was synthesized according the general procedure from compound **B** (400 mg) and step-4 product to give the title compound as a yellow solid (200 mg, yield 40%).

Step 4: Example 39 was synthesized according to a similar procedure to that used in Step 6 of Example 37.

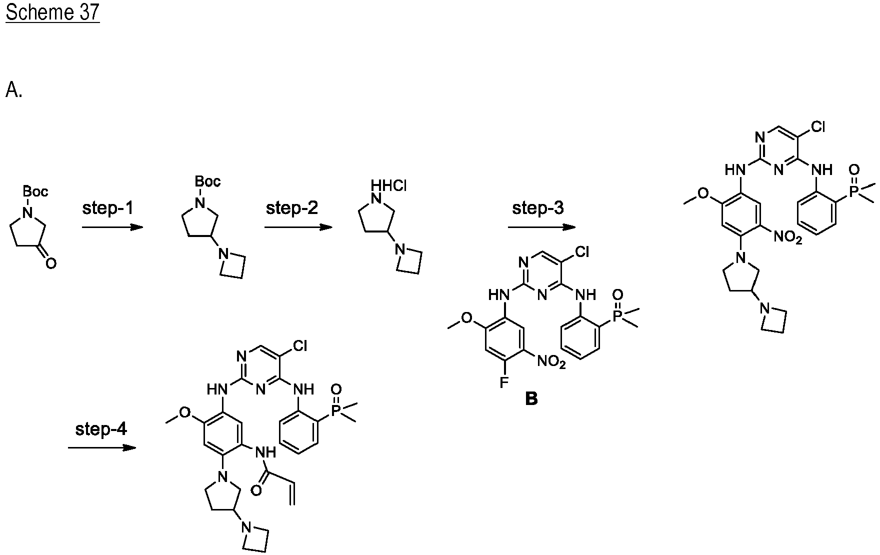

### Step-1

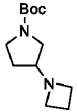

**Molecular Weight: 226.32**

To a mixture of 1-Boc-3-pyrrolidine (1.85 g), azetidine (0.65 g) in DCM (10 mL) was added NaHB(OAc)₃ (3.5 g) and molecular sieves (3.0 g). The reaction mixture was stirred at room temperature for 3h and diluted with aq.NaHCO₃. The organic layer was dried and evaporated. The residue was column purified on Silica gel (5% MeOH/DCM) to give the product as a white solid (1.6 g, yield 71%).

### Step-2

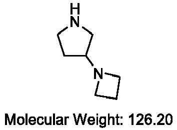

To a solution of step-1 product (230 mg) in MeOH (1.0 mL) was added HCl in dioane (3.0 mL, 4.0M solution) at room temperature. The mixture was stirred for 1h and solvent evaporated to give white solid for next step.

### Step-3

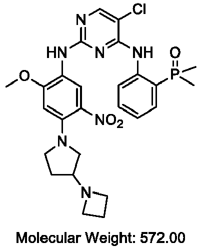

To a mixture of compound **B** (450 mg), product of step-2 in DMF (3.0 mL) was added molecular sieves (1.0 g) and K₂CO₃ (1.0 g). The reaction mixture was heated at 60°C for 30min. Solvent was evaporated and the residue was purified by a prep-TLC plate (10%MeOH/DCM) to give product as a yellow solid (380 mg, yield 69%).

### Step-4

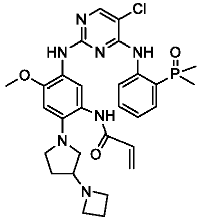

Step-4 was carried out using the procedures of Scheme 32 step-3 and step-4 to yield the desired product (Example 68) as a yellow solid (44 mg)

### B.

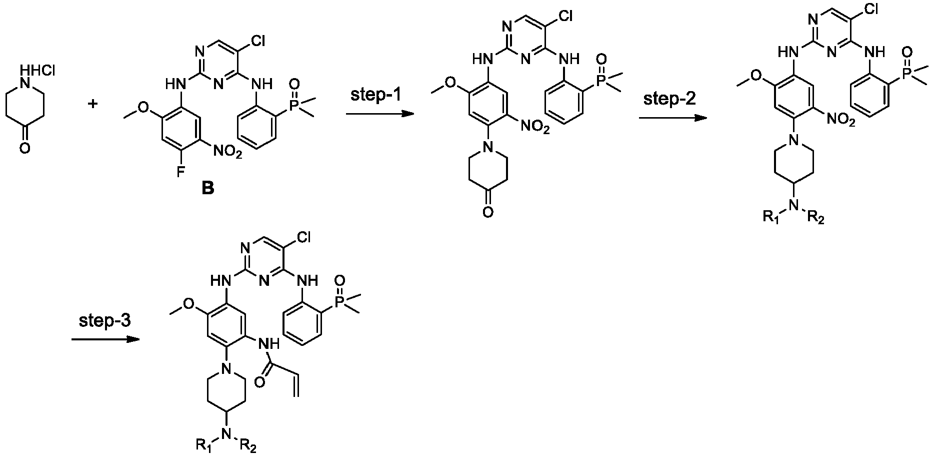

### Step-1

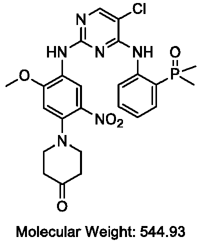

To a mixture of compound **B** (3.0 g) and 4-piperidinone HCl salt in DMF (5.0 mL) was added K₂CO₃ (2.5 g). The mixture was heated at 70°C for 3h and solvent was evaporated.

The residue was extracted with DCM and purified by CombiFlash(MeOH/DCM). The product (1.6 g, yield 46%) was obtained as a red solid.

### Step-2

General procedure of reductive amination: To a mixture of step-1 product (275 mg), respective amines (1.5 eq) in DCM was added NaHB(OAc)₃ (2eq) and molecular sieves (0.3g). The resulting mixture was shaked at room temperature for 4h and treated with wet NaHCO₃. The organic solution was separated and purified by prep-TLC plates (7-15%MeOH/DCM) to give products.

### Compounds synthesized by this method:

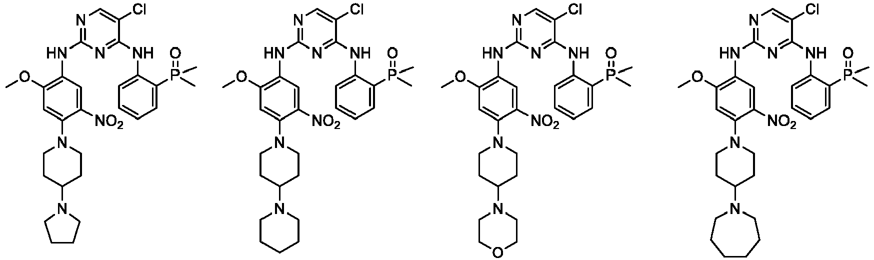

### Step-3

Reduction of the nitro group and formation of the corresponding acrylamide was carried out according to the procedure in Scheme 32, step-3 and step-4.

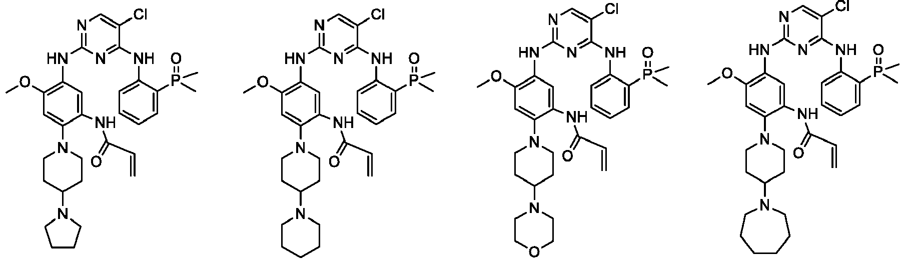

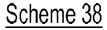

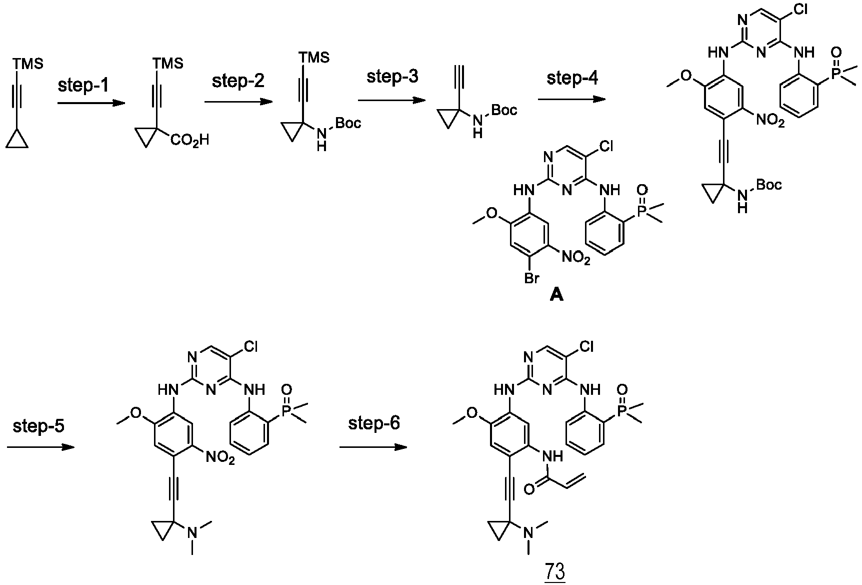

### Step-1

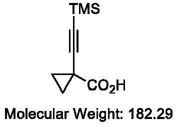

To a solution of cyclopropyl(trimethylsilyl)acetylene (13.8 g) in ether (200 mL) was added n-BuLi (40 mL, 2.5M in hexane). The reaction mixture was stirred at room temperature overnight. The dark-yellow solution was cooled to -78°C and a large excess of powdered dry ice was added in portions. The reaction mixture was allowed to warmed up to room temperature and poured into ice-cooled water. The layer was separated, and the aqueous layer was washed with Et₂O, acidified by adding 12N aq.HCl solution and extracted with Et₂O. The organic layer was dried and concentrated to give the title compound as a white solid (8.5 g, yield 47%).

### Step-2

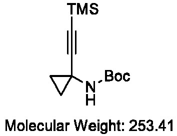

To a solution of step-1 product (1.8 g) and DPPA (2.79 g) in t-BuOH (30 mL) was added Et₃N (2.02 g). The mixture was refluxed for 20h. Solvent was evaporated and the residue was column purified on Silica gel (20% Et₂O)/heptane) to give the product as a white solid (1.3 g, yield 52%).

### Step-3

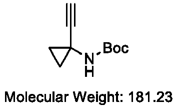

A solution of step-2 product (1.2 g) in THF (10 mL) was treated with n-Bu₄NF (6.0 ml, 1.0M solution in THF). The mixture was stirred at room temperature for 1.5h and solvent was evaporated. The residue was column purified on Silica gel (20% Et₂O)/heptane) to give the product as a white solid (0.75 g, yield 87%).

### Step-4

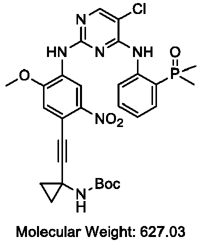

The product of Step-3 was then reacted with Compound **A** using the procedure of Scheme 32, step-2.

### Step-5

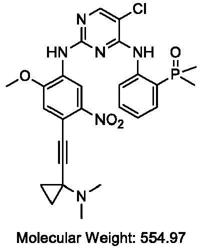

To a solution of step-4 product (250 mg) in DCM was added HCI in dioxane (1.5 mL, 4.0M solution) at - 78°C. The mixture was warmed up slowly to room temperature. Solvent was evaporated and the residue was treated with aq.K₂CO₃ and then purified by a prep-TLC plate (10%MeOH/DCM) to give yellow solid (150 mg).

The yellow solid was dissolved in DCM (3.0 mL) and treated with formyl aldehyde (5 drops, 40% aq. solution) and MgSO4 (0.5 g, anhydrous) for 30min. To the mixture was added NaBH(OAc)₃ (2eq). The reaction mixture was stirred for 1h and diluted with aq. NaHCO₃. The mixture was extracted with DCM and product was and purified by prep-TLC plates (10%MeOH/DCM) to give yellow solid (50 mg, yield 23%).

### Step-6

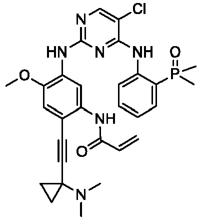

Reduction of the nitro group and formation of the corresponding acrylamide was carried out according to the procedure in Scheme 32, step-3 and step-4, to produce the final product pictured above (Example 73).

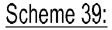

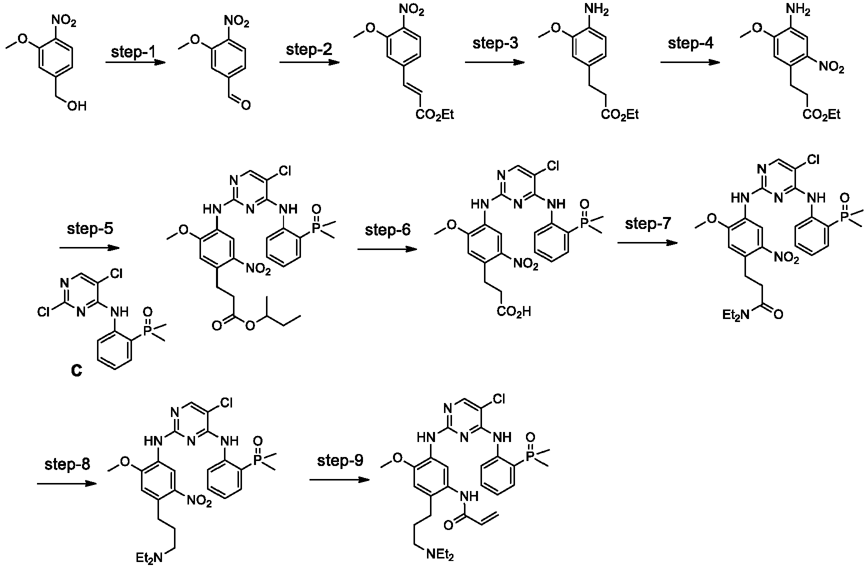

Step 1: To a solution of 3-methoxy-4-nitrobenzyl alcohol (5g) in DCM (100 mL) was added PDC (1.5 eq) and molecular sieves (6.0 g). The mixture was stirred at room temperature for 2h and diluted with Et₂O (100 mL). The mixture was filtered through a Celite pad and solvent was evaporated. The residue was washed with a small amount MeOH to give off white solid (3.7 g, yield 74%).

Step 2: To a solution of step-1 product (0.91 g) in DCM (10 mL) was added (Ethoxycarbonylmethlene)triphenylphosphorane (2.0 g). The mixture was stirred at room temperature for 30 min. Solvent was evaporated and the residue was column purified on Silica gel (20% Et₂O)/heptane) to give a yellowish solid (1.1 g, yield 87%).

Step 3: A solution of step-2 product (0.52 g) in MeOH (10 mL) was charged with Pd-C (0.5 g, 10% wet) and hydrogenated under a hydrogen balloon at room temperature overnight. The catalyst was filtered off and solvent was evaporated to give yellow oil (0.45 g, yield 97%).

Step 4: A vial was charged with H₂SO₄ (2.0 mL) and cooled to 0°C. Step-3 product (0.4 g) was carefully introduced. Guanidine nitrate (1eq) was added. The mixture was stirred at 0°C for 2h and at room temperature for 1h. The mixture was treated with excess wet NaHCO3 and extracted with DCM (10 mL). The product was and purified by prep-TLC plates (8%MeOH/DCM) to give orange solid (0.34 g, yield 71%).

Step 5: A solution of compound **C** (320 mg), step-4 product (268 mg) and TFA (0.3 mL) in 2-BuOH (2 mL) was heated at 100°C for 18 hrs. Upon cooling EtOAc and aq. NaHCO₃ were added to the reaction mixture. Extraction (3×) and concentration of combined extracts gave a solid which purified by prep-TLC plates (15%MeOH/DCM) to give orange solid (410 mg, yield 71%).

Step 6: To a suspension of step-5 product (400 mg) in MeOH (2.0 mL) was added K₂CO₃ (1.0 g) and water (0.5 mL). The reaction vial was capped and heated at 60°C for 15min. The mixture was cooled down to room temperature and the top layer was transferred to a new vial and diluted with water. The pH was adjusted to 5-6 by adding aq HCI (2N) and the product was collected by filtration as a yellow solid (310 mg, yield 86%).

Step7: To a mixture of step-6 product (260 mg) and Et₂NH (1.1 mmol) in DMF (2.0 mL) was added HBTU (1.3 mmol) and Et₃N (0.14 mL). The mixture was stirred at room temperature for 2h and diluted with DCM (5.0 mL). The mixture was washed with aq. K₂CO₃ and evaporated. The residue was purified by prep-TLC plates (15%MeOH/DCM) to give an orange solid (250 mg, yield 87%).

Step 8: To a solution of step-7 product (250 mg) in THF (1.0 mL) was added BH₃Me₂S (4.0 mL, 2.0M solution in THF). The mixture was stirred at 60°C for 2h and solvent was evaporated. The residue was dissolved in MeOH (2.0 ml) and treated with wet K₂CO₃ in a capped vial at 70°C for 1h. The organic solution was evaporated and the residue was purified by prep-TLC plates (25%MeOH/DCM) to give an orange solid (170 mg, yield 70%).

Step 9: Reduction of the nitro group and formation of the corresponding acrylamide was carried out according to the procedure in Scheme 32, step-3 and step-4, to produce the final product.

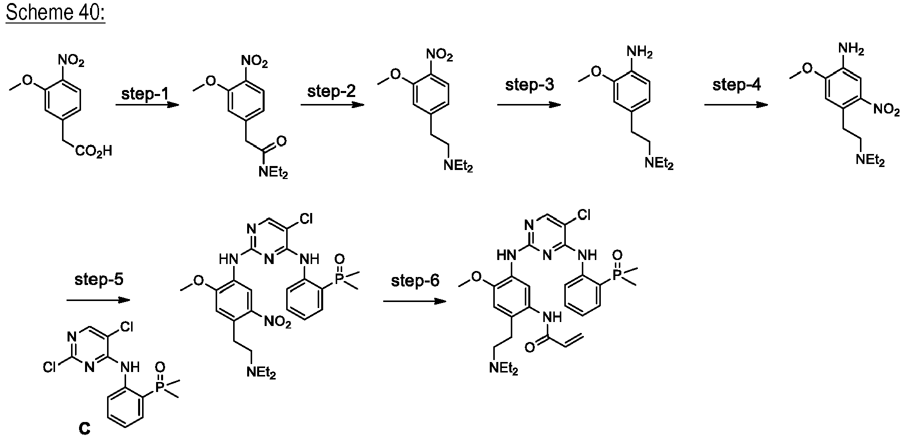

Step 1: N,N-diethyl-2-(3-methoxy-4-nitrophenyl)acetamide was made in accordance with the methods disclosed herein.

Step 2: To a solution of N,N-diethyl-2-(3-methoxy-4-nitrophenyl)acetamide (1.0 g) in THF (5.0 mL) was added BH₃Me₂S (20. mL, 2.0M solution in THF). The mixture was stirred at 60C for 2h and solvent was evaporated. The residue was dissolved in MeOH (10 ml) and treated with wet K₂CO₃ in a capped vial at 70°C for 1h. The organic solution was evaporated and the residue was purified on Silica gel column (5% MeOH/DCM) to give the product as an orange oil (0.62 g, yield 65%)

Step 3: A solution of step-2 product (600 mg) in MeOH (10 mL) was charged with Pd-C (0.5 g) and hydrogenated under a hydrogen balloon at room temperature for 3h. The catalyst was filtered off and the solvent was evaporated to give a yellow oil (430 mg, yield 81%)

Step 4: The product of step 4 was synthesized according the procedure of step 4 of Scheme 39 as an orange oil.

Step 5: The product of step 4 was synthesized according the procedure of step 5 of Scheme 13 to afford an orange solid.

Step 6: Reduction of the nitro group and formation of the corresponding acrylamide was carried out according to the procedure in Scheme 32, step-3 and step-4, to produce the final product.

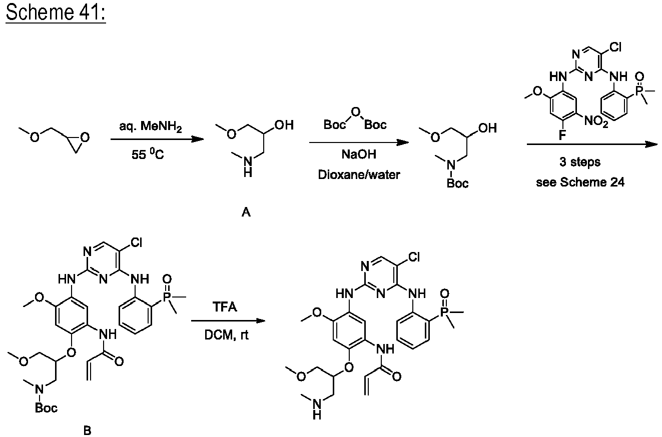

Step 1: A mixture of glycidyl methyl ether and aq. methylamine (1.5 eq) was heated at 55°C for 2hr. Removal of volatile components gave intermediate A which was used directly in next step.

Step 2: Intermediate A (400 mg, 3.34 mmol, 1.0 eq) in 4 ml of dioxane and 4 ml of water was adjusted to pH 12 with 4M NaOH, and cooled to 0°C. O(Boc)2 (806 mg, 3.69 mmol, 1.1 eq) was added in 2 portions and the mixture was stirred at R.T. overnight. The mixture was evaporated to reduce volume by one half and then treated with sat. NaHCO3 (3 ml). This was extracted with EtOAc (3x25 ml), dried, evaporated, and chromatographed (EtOAc/Heptane 1:2 to 1:1) to give an oil, 703 mg, in 96% yield. Steps 3-5: the chemistry was carried out following the procedures described in Scheme 24.

Step 6: to a solution of intermediate B (304 mg) in DCM (5mL) was added TFA (1.8 mL). The mixture was stirred at rt for 1 h then quenched with saturated sodium bicarbonate solution until pH=8.0. The organics were extracted with DCM (3x) and the combined organics were dried over anhydrous sodium sulfate, and the solvents were removed under reduced pressure to give the desired product as a pale brownish solid (262mg, 95%).

### Example 128

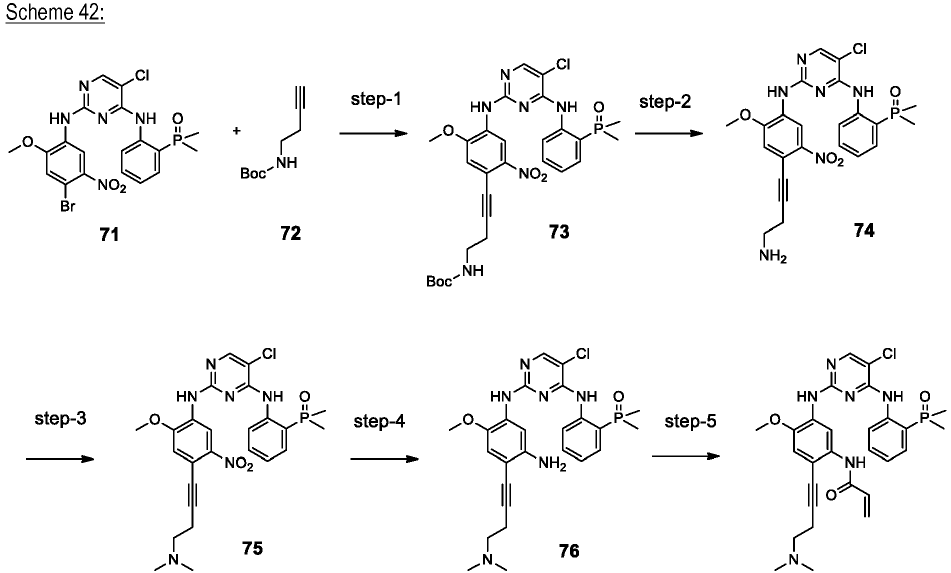

Step-1: to a solution of compound **71** (527 mg, 1.0 mmol) and tert-butyl (3-butynyl)carbamate (400 mg) in DMF (4.0 mL), was added (Ph₃P)₂PdCl₂ (0.1 g), Cul (0.05 g) and Et₃N (0.2 mL). This degased mixture was stirred at 80°C for 3h. The reaction mixture was concentrated and the residue was purified by a prep-TLC plate (8%MeOH/DCM) to give compound **73** as a yellow solid (280 mg, yield 46%). Step-2: compound **73** (280 mg, 0.46 mmol) was dissolved in DCM (2.0 mL) and treated with HCI in dioxane (1.0 mL, 4.0 M) at room temperature for 20 min. Solvent was evaporated and the residue was treated with aq. sodium bicarbonate and extracted with DCM. Combined organic layers were dried and evaporated to give compound **74** (220 mg, 93%) as yellow solid.

Step-3: to a suspension of compound **74** (220 mg, 0.43 mmol) and MgSO₄ (0.5g) in DCM (3.0 mL) was added 4 drops of formaldehyde solution (36.5%). After the resulting mixture was stirring for 20min NaHB(OAc)₃ (250 mg) was added. The mixture was heated at 50°C for 30min and then quenched with aq. NaHCO₃. The product was purified by prep. TLC (DCM:MeOH=100:15) to give compound **75** (180 mg, 78%) as yellow solid.

Step-4: to a suspension of compound **75** (180 mg) and Zn dust (0.3 g) in acetone (1.0 mL) was added NH₄Cl (0.15 g) and drops of water. The mixture was stirring at room temperature for 15 min and then diluted with DCM (5 mL). After filtration, the organic solution was evaporated and the residue (140 mg) was used for next step.

Step-5: to a solution of compound **76** (∼140 mg) in DCM (3.0 mL) was added acrylic acid (0.076 mL), EDC (200 mg), Et₃N (0.2 mL). The mixture was stirring at room temperature for 3h. Solvent was evaporated and the residue was purified by prep TLC (DCM : MeOH = 100:15) to give title compound (40mg, 26%) as light yellow solid.

### Example 129

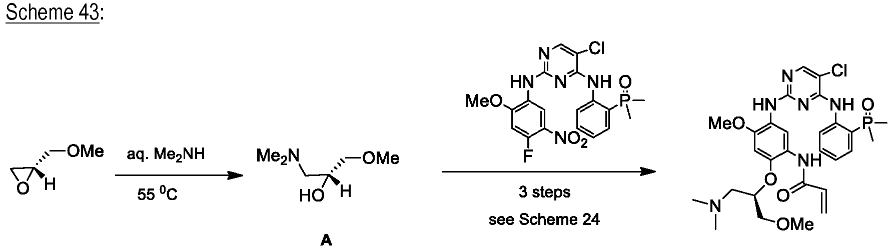

Using the procedure described in step 1 of Scheme 41 R-(-)-glycidyl methyl ether was converted into amino alcohol A. Title compound was obtained from transformation of A via a 3-step chemical sequence, as described in Scheme 24.

### Example 130

Title compound was prepared in a manner similar to Example 129 using S-(+)-glycidyl methyl ether as starting material.

### Example 131

Title compound was prepared in accordance with the method described in Scheme 12 by substituting 2,4-dichloro-5-methoxypyrimidine for 5-bromo-2,4-dichloropyrimidine.

### Example 132

Title compound was prepared in accordance with the method described in Scheme 5 by reacting intermediate **5** with 2-chloroacryloyl chloride.

### Example 133

Title compound was prepared in accordance with the method described in Scheme 12 by substituting 2,4,5-trichloropyrimidine for 5-bromo-2,4-dichloropyrimidine and 2-methoxy-4-methyl-5-nitroaniline for 2-methoxy-5-nitroaniline.

### Example 134

Title compound was prepared in accordance with the method described in Scheme 12 by substituting 2,4,5-trichloropyrimidine for 5-bromo-2,4-dichloropyrimidine and 2-(trifluoromethoxy)- 5-nitroaniline for 2-methoxy-5-nitroaniline.

### Example 135

Title compound was prepared in accordance with the method described in Scheme 12 by substituting 2,4,5-trichloropyrimidine for 5-bromo-2,4-dichloropyrimidine and 2-ethyl-5-nitroaniline for 2-methoxy-5-nitroaniline.

### Synthesis of triazine compound with CF₃ at R^{a2}:

Title compound was prepared in accordance with the method described in Scheme 12 by substituting 2,4-dichloro-1,3,5-triazine for 5-bromo-2,4-dichloropyrimidine and 2-(trifluoromethoxy)-5-nitroaniline for 2-methoxy-5-nitroaniline.

### Example 136

Title compound was prepared in accordance with the method described in Scheme 12 by substituting 2,4,5-trichloropyrimidine for 5-bromo-2,4-dichloropyrimidine and 2-chloro-5-nitroaniline for 2-methoxy-5-nitroaniline.

### Example 138

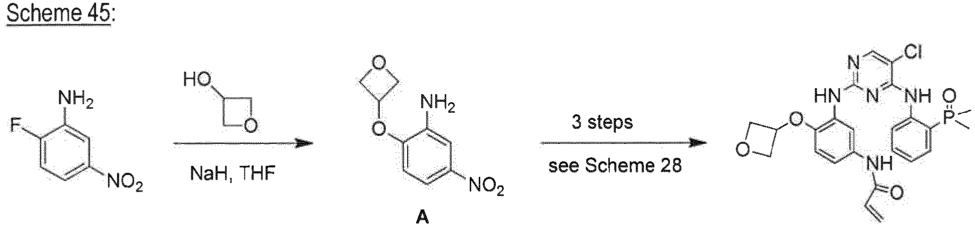

To a suspension of NaH (0,67 g, 5 eq) in THF (30 mL) was added dropwise a solution of oxetane-3-ol (0.24 g, 1 eq) in THF (5 mL) under N2 at r.t. After the mixture was stirred at r.t. for 1hr, 2-fluoro-5-nitroaniline (0.51 g, 1 eq) was added. The resulting mixture was heated at 70 oC for 3hr and then quenched with water. Concentration removed THF and the residue was taken up in DCM. Extraction, concentration and purification on silica gel column (0-100% EtOAc in heptane) furnished intermediate A as a yellow oil (52%).

Intermediate A was converted into desired final compound via a 3-step sequence outlined in Scheme 28.

### Example 139

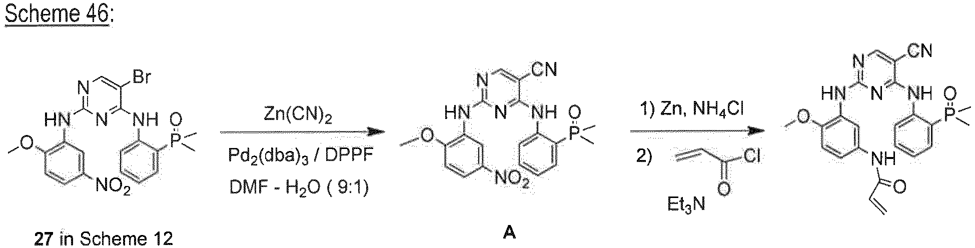

Zinc cyanide (70 mg, 0.65 eq), intermediate **27** (490 mg, 1 eq), Pd2(dba)3 (46 mg, 0.05 eq), DPPF (69 mg, .13 eq) was placed in a microwave tube containing DMF-H2O (3 mL). This mixture was flushed with Ar and then heated at 130 oC for 1.5 hr. The reaction mixture was evaporated and the residue was purified on silica gel column (0 - 5% MeOH in DCM) furnished intermediate **A** as a yellow solid (32%). Intermediate **A** was converted into desired final compound via a 2-step sequence outlined in Scheme 12.

### Example 142

Title compound was prepared in accordance with the method described in Scheme 43 by substituting (±)-glycidyl ethyl ether for R-(-)-glycidyl methyl ether.

### Examples 143 - 146

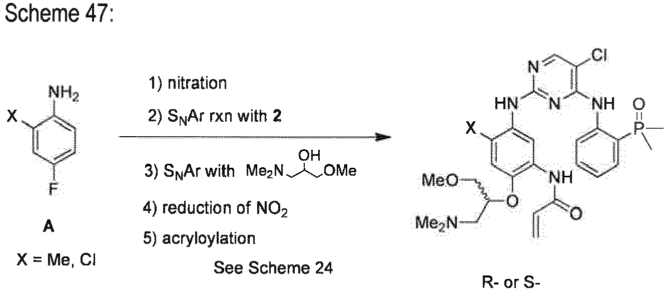

The title compounds were prepared in accordance with the method described in Scheme 24 by substituting aniline **A** for 2-methoxy-4-fluoroaniline and optically pure 1-(dimethylamino)-3-methoxy-2-propanol for 1-(2-hydroxyethyl)-4-methylpiperazine. 1-(dimethylamino)-3-methoxy-2-propanol was synthesized via a procedure described in Scheme 43.

### Examples 151, 152, and 156

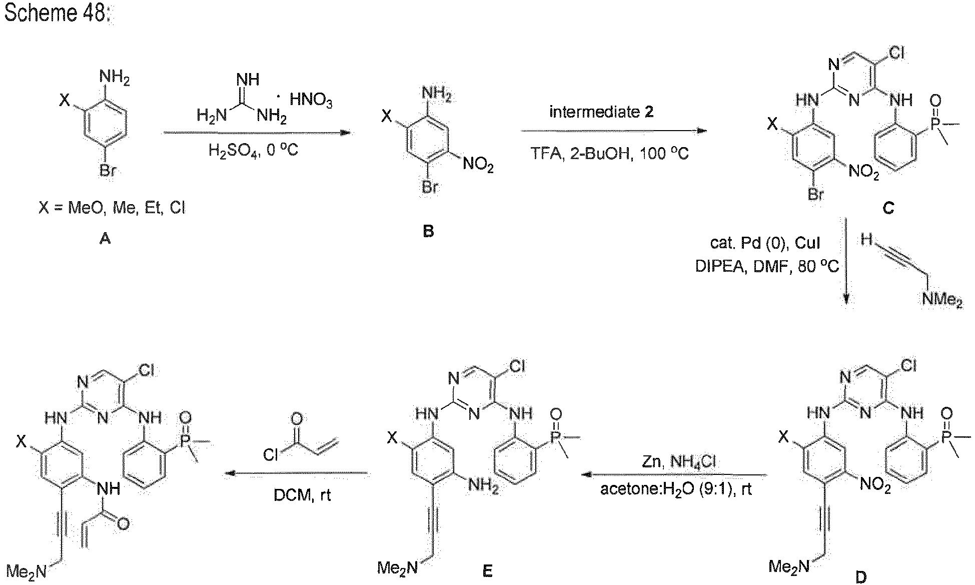

Steps 1-2: the chemistry was carried out according to the procedure described for steps 2 and 3 in Scheme 24.

Step 3: Into a Schlenk flask was loaded intermediate **C** (260 mg), PdCl2(PPh3)2 (5 mol%) and Cul (10 mol%). This flask was capped with a rubber septum and then degassed under vacuum for 1 min before N2 refill. Anhydrous DMF (4 mL) was added followed by the addition of DIPEA (0.1 mL) and 1-(dimethylamino)-3-butyne (0.1 mL, note this alkyne was purged with dry N2 gas immediately before use). The flask was sealed and the mixture was stirred at 80 oC for 16 h. Upon cooling, the reaction mixture was taken up in EtOAc and water. Extraction and concentration of combined organic layers afforded crude D which was used directly in the next step.

Steps 4-5: the chemistry was carried out according to the procedure described for steps 3 and 4 in Scheme 12.

### Example 153

Title compound was prepared in accordance with the method described in Scheme 48 by substituting 2-(isopropylsulfonyl)aniline for 2-(dimethylphosphoryl)aniline.

### Examples 154 and 155

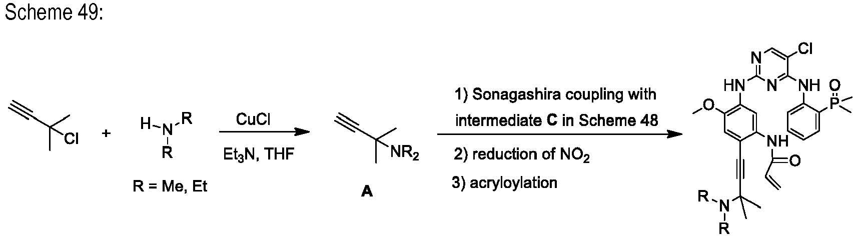

Step 1: To a stirred mixture of diethylamine (10.3 ml, 99.6 mmol), 3-chloro-3-methylbut-1-yne (10.2 g, 99.6 mmol), triethylamine (16.7 mL, 119 mmol), and THF (100 mL) at 0°C was added copper (I) chloride (0.986 g, 9.96 mmol). The resulting suspension was allowed to warm to room temperature, and stirring continued for 4 hours. The reaction mixture was partitioned between diethyl ether (250 mL) and a saturated aqueous solution of NaHCO₃ (100 mL). The phases were separated, and the aqueous phase was re-extracted with diethyl ether (100 mL). The combined organic phases were dried (Na₂SO₄), filtered, and concentrated. The brown oil was distilled (105-110°C) at atmospheric pressure under N₂. The product **A** was obtained as an oil (1.85 g, 9%).

Steps 2-4: intermediate **A** was converted into desired final compound in accordance with the method described in Scheme 48 by substituting **A** for N,N-dimethylpropargylamine.

### Example 160

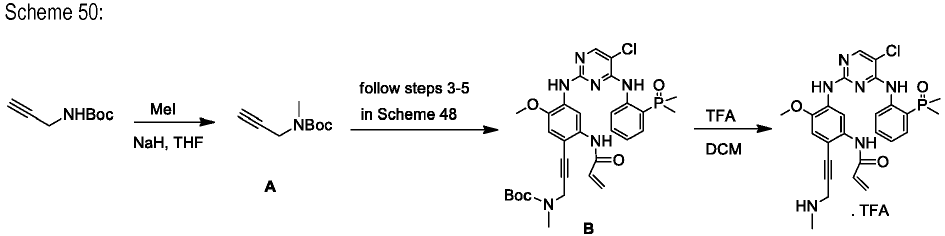

Step 1: to a solution of (N-Boc)propargylamine (3.1 g) in DMF (20 mL) was slowly added NaH (1.1 eq) at 0oC under N2. After the mixture was stirred at r.t. for 1 hr, methyl iodide (1.1 eq) was added at 0oC. Ice bath was removed and the flask was warmed up naturally to r.t. Water was added to quench the reaction. After removal of solvents the residue was taken up in EtOAc and water. Extraction and concentration of combined organic phases gave essentially pure **A** (95%).

Steps 2-4: Intermediate A was converted into compound **B** in accordance with the method described in steps 3-5 of Scheme 48 by substituting **A** for N,N-dimethylpropargylamine.

Step 5: to a solution of compound **B** (189 mg) in DCM (3 mL) was added TFA (1 mL). The mixture was stirred at rt for 1 h. The volatile components were removed under reduced pressure to give a cloudy residue. Trituration with MTBE followed by filtration and wash with MTBE gave the desired product as a off-white powder (92 mg, 57%).

### Example 164

Title compound was prepared in accordance with the method described in Scheme 12 by substituting 2,4,5-trichloropyrimidine for 5-bromo-2,4-dichloropyrimidine and 3-amino-4-(dimethylphosphoryl)-pyridine **A** for 2-(dimethylphosphoryl)aniline **1.** Compound **A** was prepared in accordance with the method described in step 1 of Scheme 1 for the synthesis of intermediate **1** using 3-amino-4-iodopyridine instead of 2-iodoaniline as starting material.

### Examples 157, 158, 159, 161,162, 165, 166, 167, 168

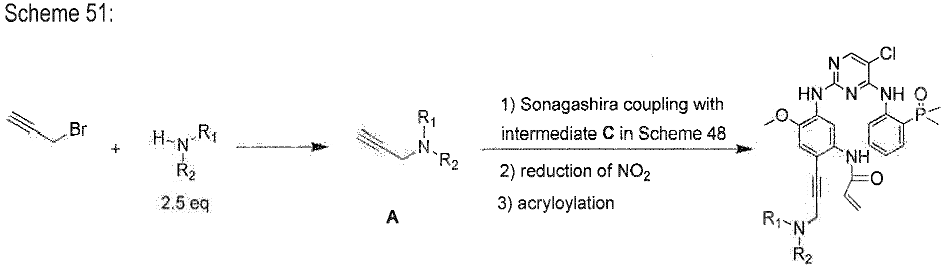

Step 1: a solution of appropriate secondary amine and propargyl bromide (supplied as a solution in toluene) in ether was stirred at 0°C then r.t, for 3 hr. The precipitate was filtered off and the filtrate was carefully distilled to furnish product **A.** Contamination of **A** with small amount of toluene was unavoidable in some cases but this did not affect next step reaction. Certain compounds **A** with relatively high boiling points were purified by silica gel column chromatography (5% MeOH in DCM) to remove residual secondary amine which was found to directly displace the bromide in the next step. Steps 2-4: Crude or pure **A** was converted into desired final compound in accordance with the method described in Scheme 48 by substituting **A** for N,N-dimethylpropargylamine.

### Compounds of the invention.

The compounds depicted below were synthesized using methods described herein or by methods analogous thereto and can be useful for treating EGFR-driven cancers.

Examples 1 - 21 were made in accordance with the methods shown in Scheme 24 by substituting appropriate alcohols for 1-(2-hydroxyethyl)-4-methylpiperazine.

### Example 1

### N-(5-((5-chloro-4-((2-(dimethylphosphoryl)phenyl)amino)pyrimidin-2-yl)amino)-4-methoxy-2-(2-(pyrrolidin-1-yl)ethoxy)phenyl)acrylamide

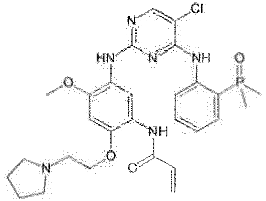

### Example 2

### N-(5-((5-chloro-4-((2-(dimethylphosphoryl)phenyl)amino)pyrimidin-2-yl)amino)-4-methoxy-2-(2-(4-methylpiperazin-1-yl)ethoxy)phenyl)acrylamide

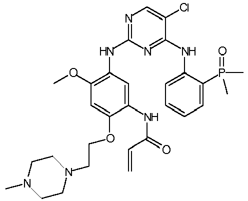

### Example 3

### N-(5-((5-chloro-4-((2-(dimethylphosphoryl)phenyl)amino)pyrimidin-2-yl)amino)-4-methoxy-2-((1-methylpiperidin-4-yl)methoxy)phenyl)acrylamide

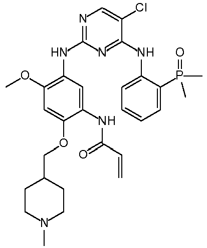

### Example 4

### N-(5-((5-chloro-4-((2-(dimethylphosphoryl)phenyl)amino)pyrimidin-2-yl)amino)-4-methoxy-2-(2-morpholinoethoxy)phenyl)acrylamide

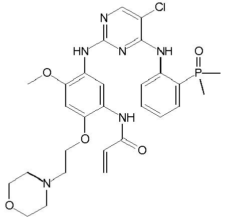

### Example 5

### N-(5-((5-chloro-4-((2-(dimethylphosphoryl)phenyl)amino)pyrimidin-2-yl)amino)-4-methoxy-2-(3-morpholinopropoxy)phenyl)acrylamide

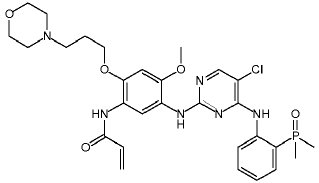

### Example 6

### N-(5-((5-chloro-4-((2-(dimethylphosphoryl)phenyl)amino)pyrimidin-2-yl)amino)-4-methoxy-2-((tetrahydro-2H-pyran-4-yl)methoxy)phenyl)acrylamide

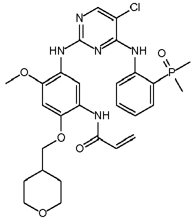

### Example 7

### N-(5-((5-chloro-4-((2-(dimethylphosphoryl)phenyl)amino)pyrimidin-2-yl)amino)-4-methoxy-2-((1-methylpiperidin-2-yl)methoxy)phenyl)acrylamide

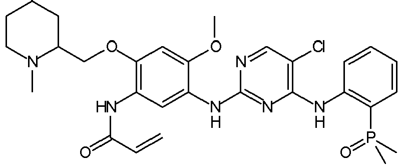

### Example 8

### N-(5-((5-chloro-4-((2-(dimethylphosphoryl)phenyl)amino)pyrimidin-2-yl)amino)-4-methoxy-2-(2-(piperidin-1-yl)ethoxy)phenyl)acrylamide

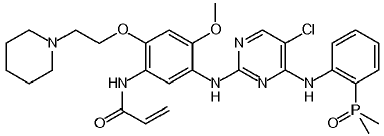

### Example 9

### N-(5-((5-chloro-4-((2-(dimethylphosphoryl)phenyl)amino)pyrimidin-2-yl)amino)-4-methoxy-2-(oxetan-3-yloxy)phenyl)acrylamide

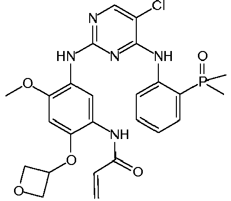

### Example 10

### N-(5-((5-chloro-4-((2-(dimethylphosphoryl)phenyl)amino)pyrimidin-2-yl)amino)-4-methoxy-2-(pyridin-3-ylmethoxy)phenyl)acrylamide

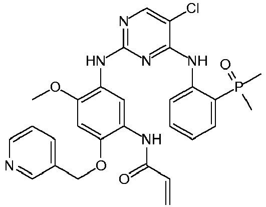

### Example 11

### N-(5-((5-chloro-4-((2-(dimethylphosphoryl)phenyl)amino)pyrimidin-2-yl)amino)-4-methoxy-2-(pyridin-4-ylmethoxy)phenyl)acrylamide

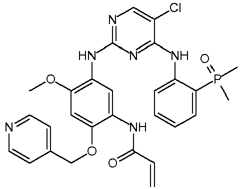

### Example 12

### N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide

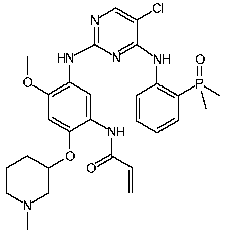

### Example 13

### N-(5-((5-chloro-4-((2-(dimethylphosphoryl)phenyl)amino)pyrimidin-2-yl)amino)-4-methoxy-2-((1-methylpyrrolidin-3-yl)oxy)phenyl)acrylamide

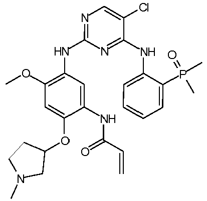

### Example 14

### rel-(R)-N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide

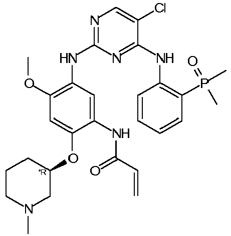

### Example 15

### N-(2-((1-(azetidin-1-yl)-3-methoxypropan-2-yl)oxy)-5-((5-chloro-4-((2-(dimethylphosphoryl)phenyl)amino)pyrimidin-2-yl)amino)-4-methoxyphenyl)acrylamide

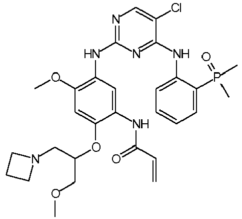

### Example 16

### rel-(R)-N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide

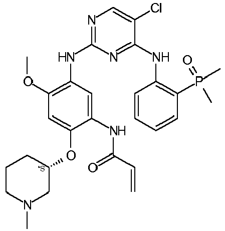

### Example 17

### N-(5-((5-chloro-4-((2-(dimethylphosphoryl)phenyl)amino)pyrimidin-2-yl)amino)-4-methoxy-2-((1-methylazetidin-3-yl)oxy)phenyl)acrylamide

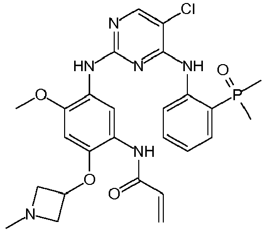

### Example 18

### N-(5-((5-chloro-4-((2-(dimethylphosphoryl)phenyl)amino)pyrimidin-2-yl)amino)-4-methoxy-2-((1-methylpyrrolidin-2-yl)methoxy)phenyl)acrylamide

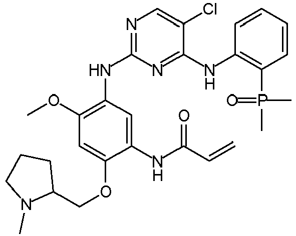

### Example 19

### N-(5-((5-chloro-4-((2-(dimethylphosphoryl)phenyl)amino)pyrimidin-2-yl)amino)-4-methoxy-2-((4-methyl-1,4-oxazepan-6-yl)oxy)phenyl)acrylamide

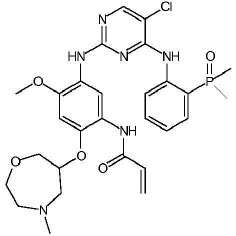

### Example 20

### N-(5-((5-chloro-4-((2-(dimethylphosphoryl)phenyl)amino)pyrimidin-2-yl)amino)-4-methoxy-2-((1-methyl pyrrolidin-3-yl)methoxy)phenyl)acrylamide

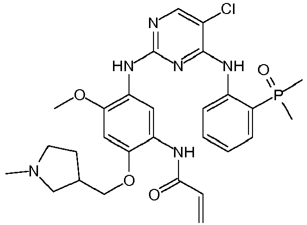

### Example 21

### N-(5-((5-chloro-4-((2-(dimethylphosphoryl)phenyl)amino)pyrimidin-2-yl)amino)-2-((2-(dimethylamino)cyclohexyl)oxy)-4-methoxyphenyl)acrylamide

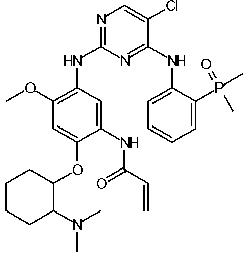

Examples 22 - 32 were made in accordance with the methods shown in Scheme 25.

### Example 22

### N-(5-((5-chloro-4-((2-(dimethylphosphoryl)phenyl)amino)pyrimidin-2-yl)amino)-4-methoxy-2-(4-(4-methylpiperazin-1-yl)piperidin-1-yl)phenyl)acrylamide

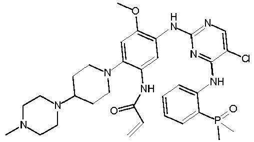

### Example 23

### N-(5-((5-chloro-4-((2-(dimethylphosphoryl)phenyl)amino)pyrimidin-2-yl)amino)-2-(4-(dimethylamino)piperidin-1-yl)-4-methoxyphenyl)acrylamide

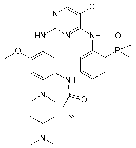

### Example 24

### N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide

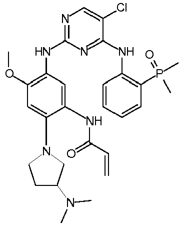

### Example 25

### N-(5-((5-chloro-4-((2-(dimethylphosphoryl)phenyl)amino)pyrimidin-2-yl)amino)-4-methoxy-2-(4-methyl-1,4-diazepan-1-yl)phenyl)acrylamide

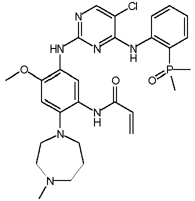

### Example 26

### rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide

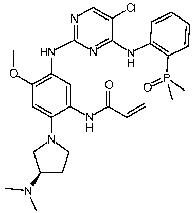

### Example 27

### N-(5-((5-chloro-4-((2-(dimethylphosphoryl)phenyl)amino)pyrimidin-2-yl)amino)-4-methoxy-2-(4-(1-methylpiperidin-4-yl)piperazin-1-yl)phenyl)acrylamide

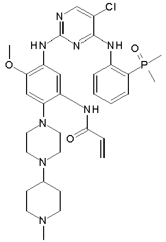

### Example 28

### N-(5-((5-chloro-4-((2-(dimethylphosphoryl)phenyl)amino)pyrimidin-2-yl)amino)-4-methoxy-2-(3-morpholinopyrrolidin-1-yl)phenyl)acrylamide

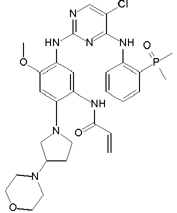

### Example 29

### rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide

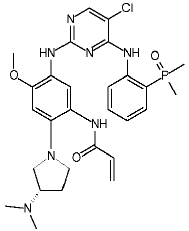

### Example 30

### N-(5-((5-chloro-4-((2-(dimethylphosphoryl)phenyl)amino)pyrimidin-2-yl)amino)-2-(3-(dimethylamino)piperidin-1-yl)-4-methoxyphenyl)acrylamide

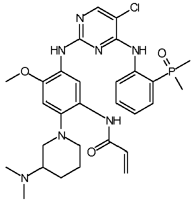

### Example 31

### N-(5-((5-chloro-4-((2-(dimethylphosphoryl)phenyl)amino)pyrimidin-2-yl)amino)-4-methoxy-2-(3-(pyrrolidin-1-yl)azetidin-1-yl)phenyl)acrylamide

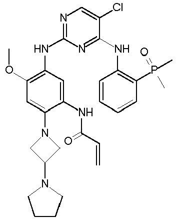

### Example 32

### N-(5-((5-chloro-4-((2-(dimethylphosphoryl)phenyl)amino)pyrimidin-2-yl)amino)-2-(3-hydroxypyrrolidin-1-yl)-4-methoxyphenyl)acrylamide

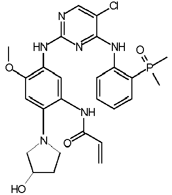

Examples 33 - 41 were made in accordance with the methods shown in Scheme 26 by substituting secondary amines for (3-dimethylamino)pyrrolidine.

### Example 33

### N-(5-((5-chloro-4-((2-(dimethylphosphoryl)phenyl)amino)pyrimidin-2-yl)amino)-4-methoxy-2-(3-(piperidin-1-yl)pyrrolidin-1-yl)phenyl)acrylamide

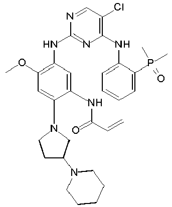

### Example 34

### N-(5-((5-chloro-4-((2-(dimethylphosphoryl)phenyl)amino)pyrimidin-2-yl)amino)-2-(3-(diethylamino)pyrrolidin-1-yl)-4-methoxyphenyl)acrylamide

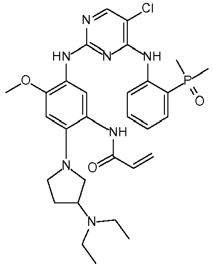

### Example 35

### N-(5-((5-chloro-4-((2-(dimethylphosphoryl)phenyl)amino)pyrimidin-2-yl)amino)-2-(3-hydroxy-[1,3'-bipyrrolidin]-1'-yl)-4-methoxyphenyl)acrylamide

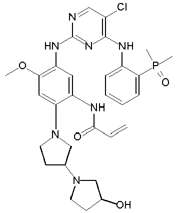

### Example 36

### N-(2-([1,3'-bipyrrolidin]-1'-yl)-5-((5-chloro-4-((2-(dimethylphosphoryl)phenyl)amino)pyrimidin-2-yl)amino)-4-methoxyphenyl)acrylamide

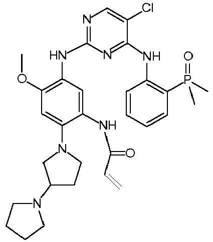

### Example 37

### N-(5-((5-chloro-4-((2-(dimethylphosphoryl)phenyl)amino)pyrimidin-2-yl)amino)-4-methoxy-2-(3-(4-methylpiperazin-1-yl)pyrrolidin-1-yl)phenyl)acrylamide

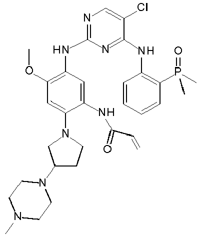

### Example 38

### N-(5-((5-chloro-4-((2-(dimethylphosphoryl)phenyl)amino)pyrimidin-2-yl)amino)-2-(3-((2-(diethylamino)ethyl)(methyl)amino)pyrrolidin-1-yl)-4-methoxyphenyl)acrylamide

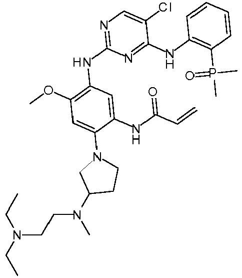

### Example 39

### N-(5-((5-chloro-4-((2-(dimethylphosphoryl)phenyl)amino)pyrimidin-2-yl)amino)-2-(3-(4-hydroxypiperidin-1-yl)pyrrolidin-1-yl)-4-methoxyphenyl)acrylamide

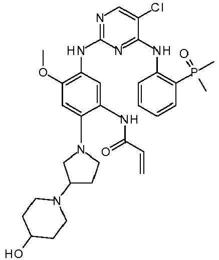

### Example 40

### N-(5-((5-chloro-4-((2-(dimethylphosphoryl)phenyl)amino)pyrimidin-2-yl)amino)-2-(3-(4-(2-hydroxyethyl)piperazin-1-yl)pyrrolidin-1-yl)-4-methoxyphenyl)acrylamide

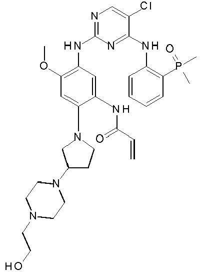

### Example 41

### N-(5-((5-chloro-4-((2-(dimethylphosphoryl)phenyl)amino)pyrimidin-2-yl)amino)-4-methoxy-2-(3-((2-methoxyethyl)(methyl)amino)pyrrolidin-1-yl)phenyl)acrylamide

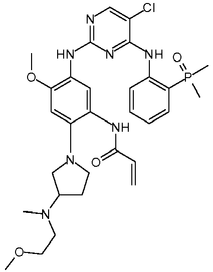

Examples 42 -53 were made in accordance with the methods shown in Scheme 27.

### Example 42

### N-(3-((5-chloro-4-((2-(dimethylphosphoryl)phenyl)amino)pyrimidin-2-yl)amino)-4-methoxyphenyl)-2-(pyrrolidin-1-ylmethyl)acrylamide

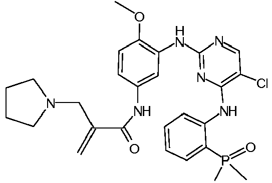

### Example 43

### N-(3-((5-chloro-4-((2-(dimethylphosphoryl)phenyl)amino)pyrimidin-2-yl)amino)-4-methoxyphenyl)-2-(piperidin-1-ylmethyl)acrylamide

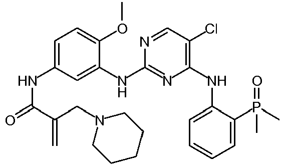

### Example 44

### N-(3-((5-chloro-4-((2-(dimethylphosphoryl)phenyl)amino)pyrimidin-2-yl)amino)-4-methoxyphenyl)-2-((4-(methylsulfonyl)piperazin-1-yl)methyl)acrylamide

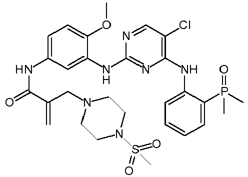

### Example 45

### N-(3-((5-chloro-4-((2-(dimethylphosphoryl)phenyl)amino)pyrimidin-2-yl)amino)-4-methoxyphenyl)-2-((4-(4-methylpiperazin-1-yl)piperidin-1-yl)methyl)acrylamide

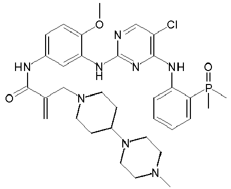

### Example 46

### N-(3-((5-chloro-4-((2-(dimethylphosphoryl)phenyl)amino)pyrimidin-2-yl)amino)-4-methoxyphenyl)-2-((1,1-dioxidothiomorpholino)methyl)acrylamide

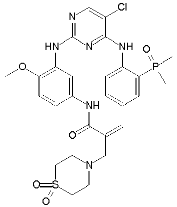

### Example 47

### N-(3-((5-chloro-4-((2-(dimethylphosphoryl)phenyl)amino)pyrimidin-2-yl)amino)-4-methoxyphenyl)-2-((3-oxopiperazin-1-yl)methyl)acrylamide

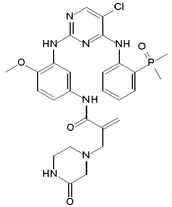

### Example 48

### N-(3-((5-chloro-4-((2-(dimethylphosphoryl)phenyl)amino)pyrimidin-2-yl)amino)-4-methoxyphenyl)-2-(morpholinomethyl)acrylamide

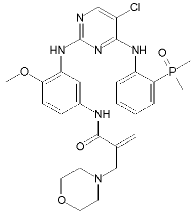

### Example 49

### N-(3-((5-chloro-4-((2-(dimethylphosphoryl)phenyl)amino)pyrimidin-2-yl)amino)-4-methoxyphenyl)-2-((4-methylpiperazin-1-yl)methyl)acrylamide

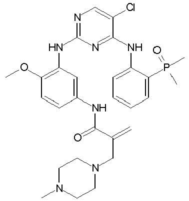

### Example 50

### N-(3-((5-chloro-4-((2-(dimethylphosphoryl)phenyl)amino)pyrimidin-2-yl)amino)-4-methoxyphenyl)-2-((4-isopropylpiperazin-1-yl)methyl)acrylamide

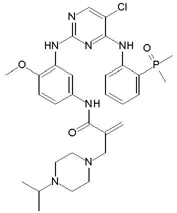

### Example 51

### N-(3-((5-chloro-4-((2-(dimethylphosphoryl)phenyl)amino)pyrimidin-2-yl)amino)-4-methoxyphenyl)-2-((4-morpholinopiperidin-1-yl)methyl)acrylamide

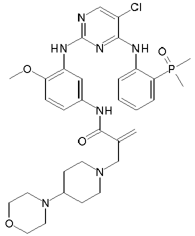

### Example 52

### N-(3-((5-chloro-4-((2-(dimethylphosphoryl)phenyl)amino)pyrimidin-2-yl)amino)-4-methoxyphenyl)-2-((4-(2-hydroxyethyl)piperazin-1-yl)methyl)acrylamide

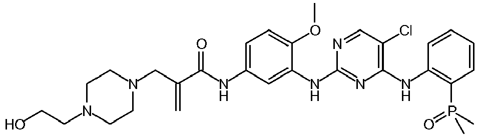

### Example 53

### N-(3-((5-chloro-4-((2-(dimethylphosphoryl)phenyl)amino)pyrimidin-2-yl)amino)-4-methoxyphenyl)-2-((3-hydroxypyrrolidin-1-yl)methyl)acrylamide

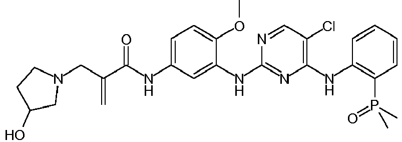

Example 54 was made in accordance with the methods shown in Scheme 28.

### Example 54

### N-(3-((5-chloro-4-((2-(dimethylphosphoryl)phenyl)amino)pyrimidin-2-yl)amino)-4-fluorophenyl)acrylamide

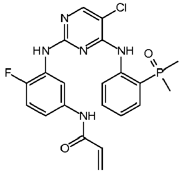

Examples 55 - 57 were made in accordance with the methods shown in Scheme 29.

### Example 55

### N-(5-((5-chloro-4-((2-(dimethylphosphoryl)phenyl)amino)pyrimidin-2-yl)amino)-4-methoxy-2-((4-methylpiperazin-1-yl)methyl)phenyl)acrylamide

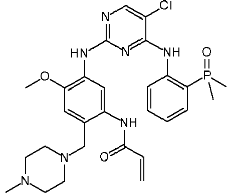

### Example 56

### N-(5-((5-chloro-4-((2-(dimethylphosphoryl)phenyl)amino)pyrimidin-2-yl)amino)-4-methoxy-2-(4-methylpiperazine-1-carbonyl)phenyl)acrylamide

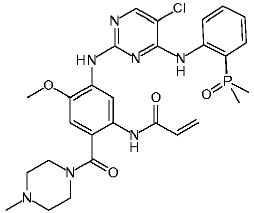

Example 57 was made in accordance with the methods shown in Scheme 30.

### Example 57

### N-(2-(3-aminopyrrolidin-1-yl)-5-((5-chloro-4-((2-(dimethylphosphoryl)phenyl)amino)pyrimidin-2-yl)amino)-4-methoxyphenyl)acrylamide

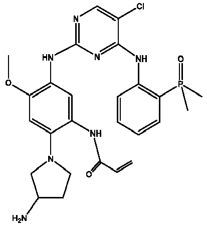

Examples 58 - 64 were made in accordance with the methods shown in Scheme 31.

### Example 58

### N1-(3-((5-chloro-4-((2-(dimethylphosphoryl)phenyl)amino)pyrimidin-2-yl)amino)-4-methoxyphenyl)-N4-(2-(dimethylamino)ethyl)fumaramide

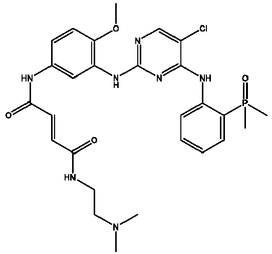

### Example 59

### (E)-N-(3-((5-chloro-4-((2-(dimethylphosphoryl)phenyl)amino)pyrimidin-2-yl)amino)-4-methoxyphenyl)-4-(4-methylpiperazin-1-yl)-4-oxobut-2-enamide

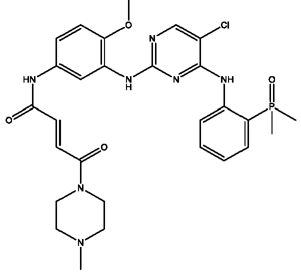

### Example 60

### N1-(3-((5-chloro-4-((2-(dimethylphosphoryl)phenyl)amino)pyrimidin-2-yl)amino)-4-methoxyphenyl)-N4,N4-dimethylfumaramide

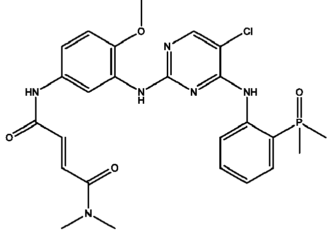

### Example 61

### (Z)-4-((3-((5-chloro-4-((2-(dimethylphosphoryl)phenyl)amino)pyrimidin-2-yl)amino)-4-methoxyphenyl)amino)-4-oxobut-2-enoic acid

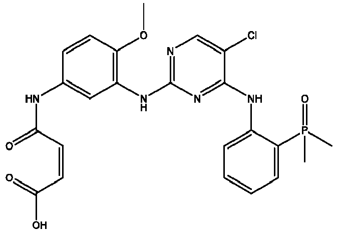

### Example 62

### N1-(3-((5-chloro-4-((2-(dimethylphosphoryl)phenyl)amino)pyrimidin-2-yl)amino)-4-methoxyphenyl)-N4,N4-dimethylmaleamide

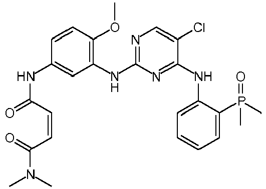

### Example 63

### (Z)-N-(3-((5-chloro-4-((2-(dimethylphosphoryl)phenyl)amino)pyrimidin-2-yl)amino)-4-methoxyphenyl)-4-(4-methylpiperazin-1-yl)-4-oxobut-2-enamide

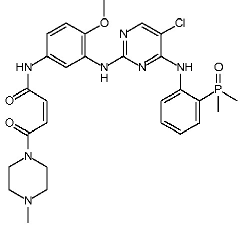

### Example 64

### N1-(3-((5-chloro-4-((2-(dimethylphosphoryl)phenyl)amino)pyrimidin-2-yl)amino)-4-methoxyphenyl)-N4-(2-(dimethylamino)ethyl)maleamide

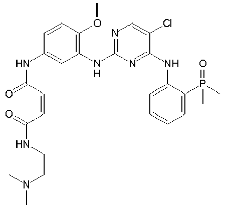

Example 65 was made in accordance with the methods shown in Scheme 32.

### Example 65

### N-(2-(3-amino-3-methylbut-1-yn-1-yl)-5-((5-chloro-4-((2-(dimethylphosphoryl)phenyl)amino)pyrimidin-2-yl)amino)-4-methoxyphenyl)acrylamide

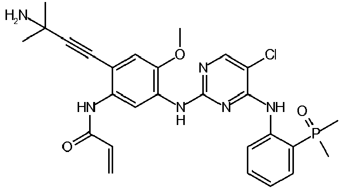

Example 66 was made in accordance with the methods shown in Scheme 33.

### Example 66

### N-(5-((5-chloro-4-((2-(dimethylphosphoryl)phenyl)amino)pyrimidin-2-yl)amino)-2-(2-(dimethylamino)-3-hydroxypropoxy)-4-methoxyphenyl)acrylamide

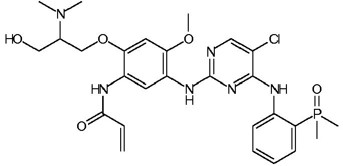

Example 67 was made in accordance with the methods shown in Scheme 35.

### Example 67

### N-(5-((5-chloro-4-((2-(dimethylphosphoryl)phenyl)amino)pyrimidin-2-yl)amino)-2-((2-((2-fluoroethyl)(methyl)amino)ethyl)(methyl)amino)-4-methoxyphenyl)acrylamide

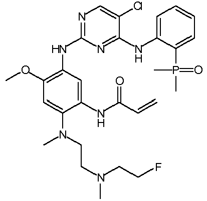

Examples 68 - 73 were made in accordance with the methods shown in Scheme 37.

### Example 68

### N-(2-(3-(azetidin-1-yl)pyrrolidin-1-yl)-5-((5-chloro-4-((2-(dimethylphosphoryl)phenyl)amino)pyrimidin-2-yl)amino)-4-methoxyphenyl)acrylamide

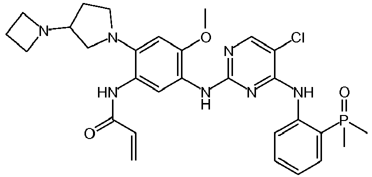

### Example 69

### N-(2-([1,4'-bipiperidin]-1'-yl)-5-((5-chloro-4-((2-(dimethylphosphoryl)phenyl)amino)pyrimidin-2-yl)amino)-4-methoxyphenyl)acrylamide

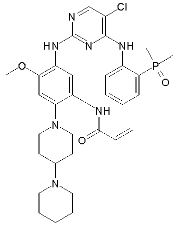

### Example 70

### N-(5-((5-chloro-4-((2-(dimethylphosphoryl)phenyl)amino)pyrimidin-2-yl)amino)-4-methoxy-2-(4-morpholinopiperidin-1-yl)phenyl)acrylamide

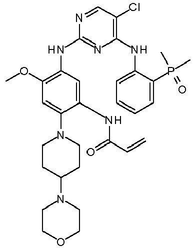

### Example 71

### N-(5-((5-chloro-4-((2-(dimethylphosphoryl)phenyl)amino)pyrimidin-2-yl)amino)-4-methoxy-2-(4-(4-methyl-1,4-diazepan-1-yl)piperidin-1-yl)phenyl)acrylamide

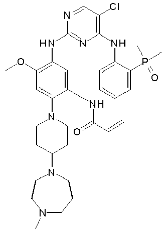

### Example 72

### N-(5-((5-chloro-4-((2-(dimethylphosphoryl)phenyl)amino)pyrimidin-2-yl)amino)-4-methoxy-2-(4-(pyrrolidin-1-yl)piperidin-1-yl)phenyl)acrylamide

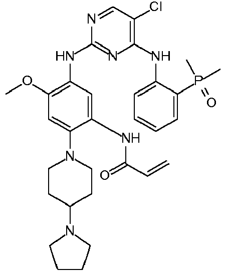

Example 73 was made in accordance with the methods shown in Scheme 38.

### Example 73

### N-(5-((5-chloro-4-((2-(dimethylphosphoryl)phenyl)amino)pyrimidin-2-yl)amino)-2-((1-(dimethylamino)cyclopropyl)ethynyl)-4-methoxyphenyl)acrylamide

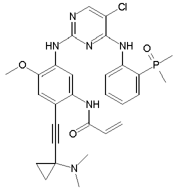

Example 74 was made in accordance with the methods shown in Scheme 40.

### Example 74

### N-(5-((5-chloro-4-((2-(dimethylphosphoryl)phenyl)amino)pyrimidin-2-yl)amino)-4-methoxy-2-(2-(4-methylpiperazin-1-yl)ethyl)phenyl)acrylamide

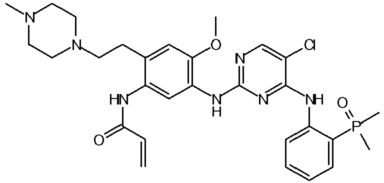

Example 75 was made in accordance with the methods shown in Scheme 41.

### Example 75

### (E)-ethyl 4-((3-((5-chloro-4-((2-(dimethylphosphoryl)phenyl)amino)pyrimidin-2-yl)amino)-4-methoxyphenyl)amino)-4-oxobut-2-enoate

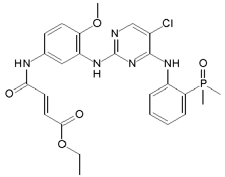

Example 76 was made in accordance with the methods shown in Scheme 42.

### Example 76

### (E)-tert-butyl (1-(3-((3-((5-chloro-4-((2-(dimethylphosphoryl)phenyl)amino)pyrimidin-2-yl)amino)-4-methoxyphenyl)amino)-3-oxoprop-1-en-1-yl)cyclopropyl)carbamate

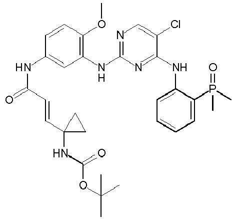

### Example 77

### N-(3-((5-chloro-4-((2-(dimethylphosphoryl)phenyl)amino)pyrimidin-2-yl)amino)-4-methoxyphenyl)-2-fluoroacrylamide

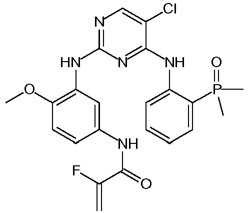

### Example 78

### (E)-3-chloro-N-(3-((5-chloro-4-((2-(dimethylphosphoryl)phenyl)amino)pyrimidin-2-yl)amino)-4-methoxyphenyl)acrylamide

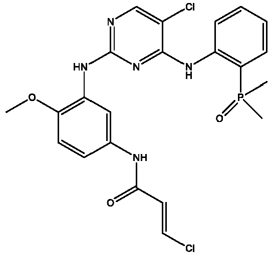

### Example 79

### N-(3-((5-chloro-4-((2-(dimethylphosphoryl)phenyl)amino)pyrimidin-2-yl)amino)-4-methoxyphenyl)cyclohex-1-enecarboxamide

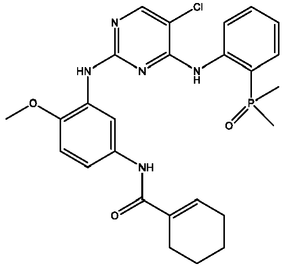

### Example 80

### N-(5-((5-chloro-4-((2-(dimethylphosphoryl)phenyl)amino)pyrimidin-2-yl)amino)-4-methoxy-2-(2-(pyrrolidin-1-yl)ethoxy)phenyl)but-2-ynamide

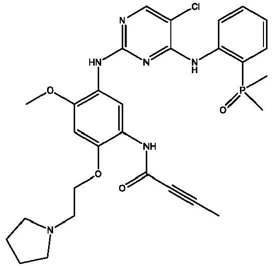

### Example 81

### N-(3-((5-chloro-4-((2-(dimethylphosphoryl)phenyl)amino)pyrimidin-2-yl)amino)-4-methoxyphenyl)-4-(dimethylamino)but-2-ynamide

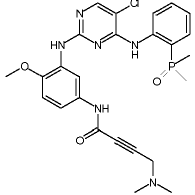

### Example 82

### N-(3-((5-chloro-4-((2-(dimethylphosphoryl)phenyl)amino)pyrimidin-2-yl)amino)-4-methoxyphenyl)-2-(trifluoromethyl)acrylamide

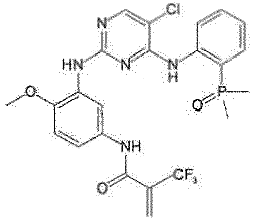

### Example 84

### N-(3-((5-chloro-4-((2-(dimethylphosphoryl)phenyl)amino)pyrimidin-2-yl)amino)-4-methoxyphenyl)-1-methyl-1,2,5,6-tetrahydropyridine-3-carboxamide

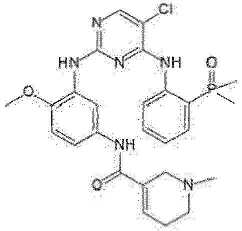

### Example 85

### (E)-3-chloro-N-(5-((5-chloro-4-((2-(dimethylphosphoryl)phenyl)amino)pyrimidin-2-yl)amino)-2-fluoro-4-methoxyphenyl)acrylamide

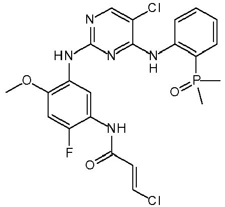

### Example 86

### N-(3-((5-chloro-4-((2-(dimethylphosphoryl)-4-(2-methoxyethoxy)phenyl)amino)pyrimidin-2-yl)amino)-4-methoxyphenyl)acrylamide

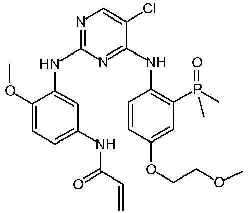

### Example 87

### N-(5-((5-chloro-4-((2-(dimethylphosphoryl)phenyl)amino)pyrimidin-2-yl)amino)-2-fluoro-4-methoxyphenyl)-4-(dimethylamino)but-2-ynamide

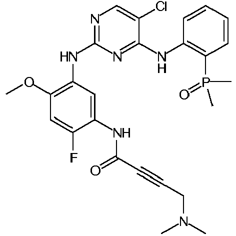

### Example 88

### N-(5-((5-chloro-4-((2-(dimethylphosphoryl)phenyl)amino)pyrimidin-2-yl)amino)-2-fluoro-4-methoxyphenyl)-5-(piperidin-1-yl)pent-2-ynamide

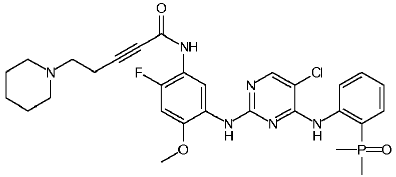

### Example 89

### N-(5-((5-chloro-4-((2-(dimethylphosphoryl)phenyl)amino)pyrimidin-2-yl)amino)-2-fluoro-4-methoxyphenyl)-4-(pyrrolidin-1-yl)but-2-ynamide

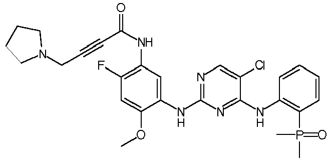

### Example 90

### N-(5-((5-chloro-4-((2-(dimethylphosphoryl)phenyl)amino)pyrimidin-2-yl)amino)-2-fluoro-4-methoxyphenyl)-4-methyl-4-(pyrrolidin-1-yl)pent-2-ynamide

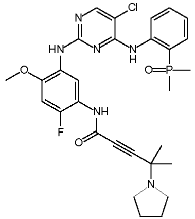

### Example 91

### N-(3-((5-chloro-4-((2-(dimethylphosphoryl)phenyl)amino)pyrimidin-2-yl)amino)-4-methoxyphenyl)-4-methyl-4-(pyrrolidin-1-yl)pent-2-ynamide

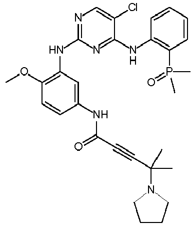

### Example 92

### (E)-N-(3-((5-chloro-4-((2-(dimethylphosphoryl)phenyl)amino)pyrimidin-2-yl)amino)-4-methoxyphenyl)-4-(piperidin-1-yl)but-2-enamide

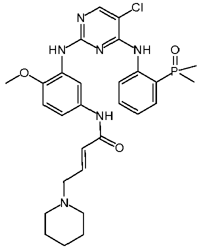

### Example 93

### N-(3-((5-chloro-4-((2-(dimethylphosphoryl)phenyl)amino)pyrimidin-2-yl)amino)-4-methoxyphenyl)-3-phenylpropiolamide

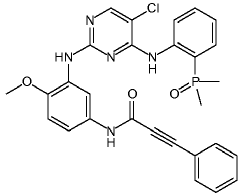

### Example 94

### (E)-3-(1-aminocyclopropyl)-N-(3-((5-chloro-4-((2-(dimethylphosphoryl)phenyl)amino)pyrimidin-2-yl)amino)-4-methoxyphenyl)acrylamide

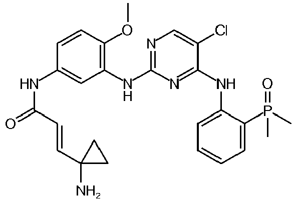

### Example 95

### (E)-N-(3-((5-chloro-4-((2-(dimethylphosphoryl)phenyl)amino)pyrimidin-2-yl)amino)-4-methoxyphenyl)-4-(pyrrolidin-1-yl)but-2-enamide

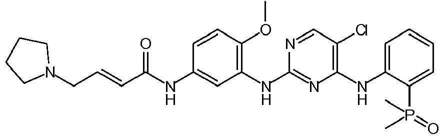

### Example 96

### N-(5-((5-chloro-4-((2-(dimethylphosphoryl)phenyl)amino)pyrimidin-2-yl)amino)-2-(3-(diethylamino)prop-1-yn-1-yl)-4-methoxyphenyl)acrylamide

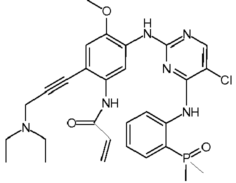

### Example 97

### N-(3-((5-chloro-4-((2-(dimethylphosphoryl)phenyl)amino)pyrimidin-2-yl)amino)-4-methoxyphenyl)-4-methoxybut-2-ynamide

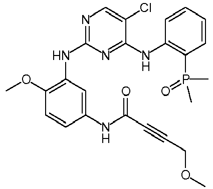

### Example 98

### N-(5-((5-chloro-4-((2-(dimethylphosphoryl)-4-(2-(pyrrolidin-1-yl)ethoxy)phenyl)amino)pyrimidin-2-yl)amino)-2-fluoro-4-methoxyphenyl)acrylamide

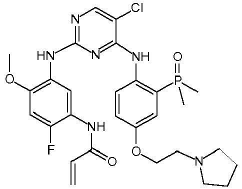

### Example 99

### N-(3-((5-chloro-4-((2-(dimethylphosphoryl)-4-((tetrahydrofuran-3-yl)oxy)phenyl)amino)pyrimidin-2-yl)amino)-4-methoxyphenyl)acrylamide

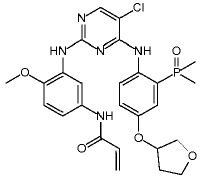

### Example 100

### N-(3-((5-chloro-4-((2-(dimethylphosphoryl)-4-((tetrahydro-2H-pyran-4-yl)methoxy)phenyl)amino)pyrimidin-2-yl)amino)-4-methoxyphenyl)acrylamide

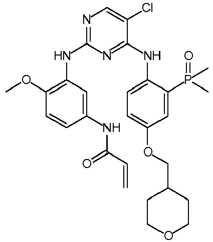

### Example 101

### N-(5-((5-chloro-4-((2-(dimethylphosphoryl)phenyl)amino)pyrimidin-2-yl)amino)-4-methoxy-2-morpholinophenyl)-5-(piperidin-1-yl)pent-2-ynamide

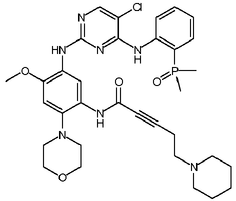

### Example 102

### N-(5-((5-chloro-4-((2-(dimethylphosphoryl)phenyl)amino)pyrimidin-2-yl)amino)-4-methoxy-2-((tetrahydro-2H-pyran-4-yl)methoxy)phenyl)but-2-ynamide

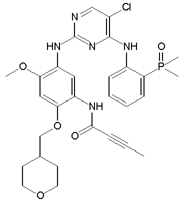

### Example 103

### N-(5-((5-chloro-4-((2-(dimethylphosphoryl)phenyl)amino)pyrimidin-2-yl)amino)-4-methoxy-2-(oxetan-3-yloxy)phenyl)but-2-ynamide

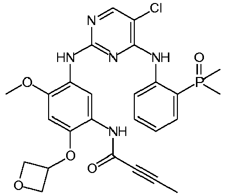

### Example 104

### N-(5-((5-chloro-4-((2-(dimethylphosphoryl)phenyl)amino)pyrimidin-2-yl)amino)-4-methoxy-2-morpholinophenyl)-4-(dimethylamino)but-2-ynamide

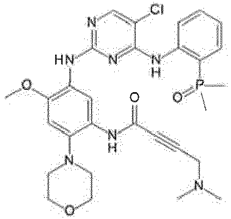

### Example 105

### N-(5-((5-chloro-4-((2-(dimethylphosphoryl)phenyl)amino)pyrimidin-2-yl)amino)-4-methoxy-2-morpholinophenyl)-4-methoxybut-2-ynamide

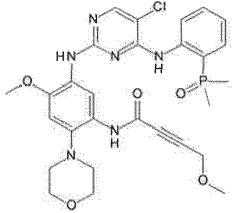

### Example 106

### N-(5-((5-chloro-4-((2-(dimethylphosphoryl)phenyl)amino)pyrimidin-2-yl)amino)-4-methoxy-2-(4-(4-methylpiperazin-1-yl)piperidin-1-yl)phenyl)but-2-ynamide

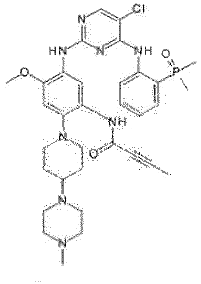

### Example 108

### N-(3-((5-chloro-4-((2-(dimethylphosphoryl)-4-(2-(piperidin-1-yl)ethoxy)phenyl)amino)pyrimidin-2-yl)amino)-4-methoxyphenyl)acrylamide

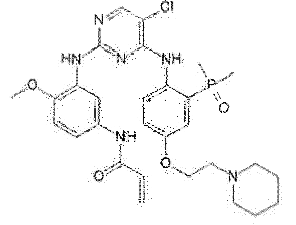

### Example 109

### N1-(3-((5-chloro-4-((2-(dimethylphosphoryl)phenyl)amino)pyrimidin-2-yl)amino)-4-methoxyphenyl)-N4-methylfumaramide

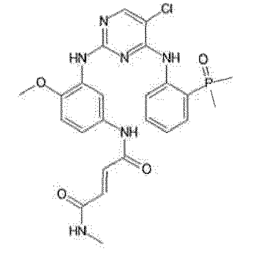

### Example 110

### N1-(3-((5-chloro-4-((2-(dimethylphosphoryl)phenyl)amino)pyrimidin-2-yl)amino)-4-methoxyphenyl)-N4-methylmaleamide

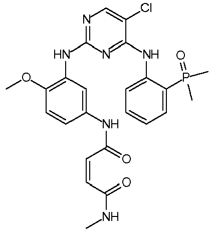

### Example 111

### N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)-4-methoxybut-2-ynamide

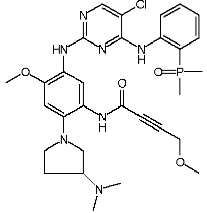

### Example 112

### N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)but-2-ynamide

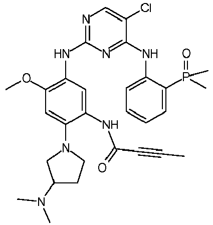

### Example 113

### rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)methacrylamide

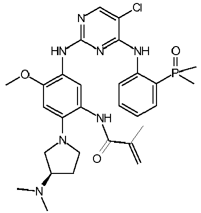

### Example 114

### N-(5-((5-chloro-4-((2-(dimethylphosphoryl)phenyl)amino)pyrimidin-2-yl)amino)-4-methoxy-2-morpholinophenyl)-5-(dimethylamino)pent-2-ynamide

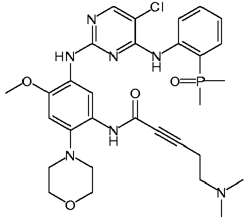

### Example 115

### N-(5-((5-chloro-4-((2-(dimethylphosphoryl)phenyl)amino)pyrimidin-2-yl)amino)-4-methoxy-2-morpholinophenyl)-5-methoxypent-2-ynamide

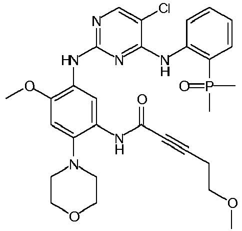

### Example 116

### N-(5-((5-chloro-4-((2-(dimethylphosphoryl)phenyl)amino)pyrimidin-2-yl)amino)-4-methoxy-2-(3-methyl-3-(pyrrolidin-1-yl)but-1-yn-1-yl)phenyl)acrylamide

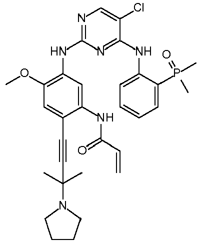

### Example 117

### N-(3-((5-chloro-4-((2-(dimethylphosphoryl)phenyl)amino)pyrimidin-2-yl)amino)-4-(2-(dimethylamino)ethoxy)phenyl)acrylamide

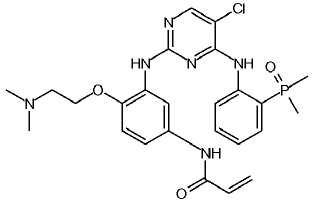

### Example 118

### N-(5-((5-chloro-4-((2-(dimethylphosphoryl)phenyl)amino)pyrimidin-2-yl)amino)-4-methoxy-2-morpholinophenyl)-5-(4-methylpiperazin-1-yl)pent-2-ynamide

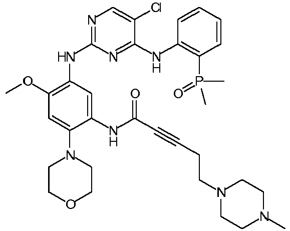

### Example 119

### N-(5-((5-chloro-4-((2-(dimethylphosphoryl)phenyl)amino)pyrimidin-2-yl)amino)-4-methoxy-2-(pyrrolidin-1-yl)phenyl)-5-(4-methylpiperazin-1-yl)pent-2-ynamide

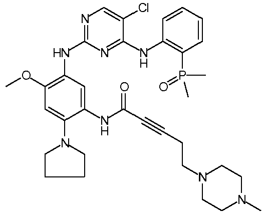

### Example 120

### N-(5-((5-cyclopropyl-4-((2-(dimethylphosphoryl)phenyl)amino)pyrimidin-2-yl)amino)-2-((2-(diethylamino)ethyl)(methyl)amino)-4-methoxyphenyl)acrylamide

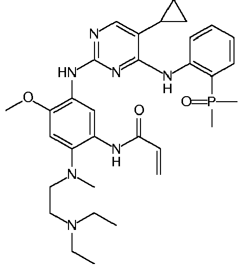

### Example 121

### N-(5-((5-chloro-4-((2-(dimethylphosphoryl)phenyl)amino)pyrimidin-2-yl)amino)-2-((1-(diethylamino)-3-fluoropropan-2-yl)oxy)-4-methoxyphenyl)acrylamide

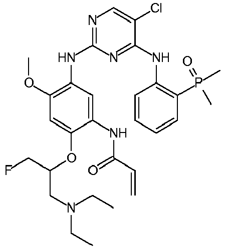

### Example 122

### N-(5-((5-chloro-4-((2-(dimethylphosphoryl)phenyl)amino)pyrimidin-2-yl)amino)-4-methoxy-2-morpholinophenyl)-5-(2-(methoxymethyl)pyrrolidin-1-yl)pent-2-ynamide

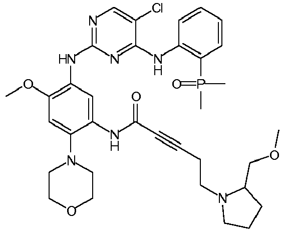

### Example 123

### N-(5-((5-chloro-4-((2-(dimethylphosphoryl)phenyl)amino)pyrimidin-2-yl)amino)-4-methoxy-2-((1-(2-methoxyethyl)piperidin-3-yl)oxy)phenyl)acrylamide

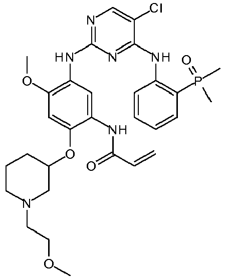

### Example 124

### N-(5-((5-chloro-4-((2-(dimethylphosphoryl)phenyl)amino)pyrimidin-2-yl)amino)-2-(3-(dimethylamino)prop-1-yn-1-yl)-4-methoxyphenyl)acrylamide

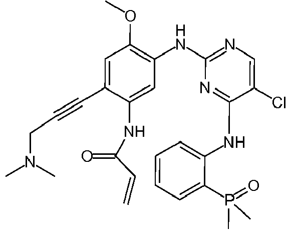

### Example 125

### N-(3-((5-chloro-4-((4-(2-(dimethylamino)ethoxy)-2-(dimethylphosphoryl)phenyl)amino)pyrimidin-2-yl)amino)-4-methoxyphenyl)acrylamide

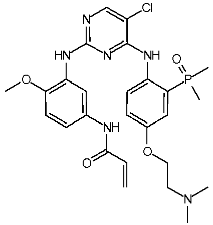

### Example 126

### N-(3-((5-chloro-4-((4-(3-(dimethylamino)propoxy)-2-(dimethylphosphoryl)phenyl)amino)pyrimidin-2-yl)amino)-4-methoxyphenyl)acrylamide

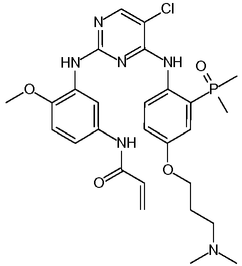

### Example 127

### 1-(6-((5-chloro-4-((2-(dimethylphosphoryl)phenyl)amino)pyrimidin-2-yl)amino)-5-methoxyindolin-1-yl)prop-2-en-1-one

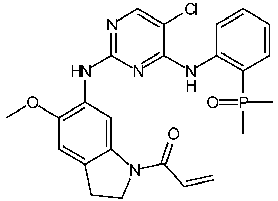

### Example 128

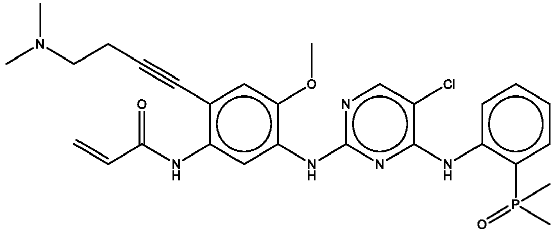

### N-(5-((5-chloro-4-((2-(dimethylphosphoryl)phenyl)amino)pyrimidin-2-yl)amino)-2-(4-(dimethylamino)but-1-yn-1-yl)-4-methoxyphenyl)acrylamide

### Example 129

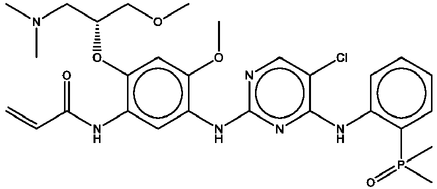

### (R)-N-(5-((5-chloro-4-((2-(dimethylphosphoryl)phenyl)amino)pyrimidin-2-yl)amino)-2-((1-(dimethylamino)-3-methoxypropan-2-yl)oxy)-4-methoxyphenyl)acrylamide

### Example 130

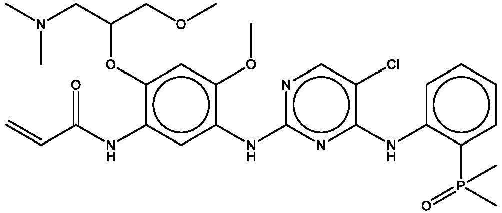

### N-(5-((5-chloro-4-((2-(dimethylphosphoryl)phenyl)amino)pyrimidin-2-yl)amino)-2-((1-(dimethylamino)-3-methoxypropan-2-yl)oxy)-4-methoxyphenyl)acrylamide

### Example 131

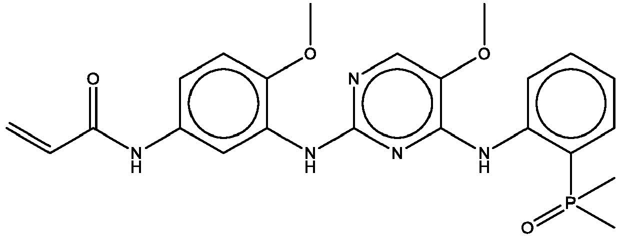

### N-(3-((4-((2-(dimethylphosphoryl)phenyl)amino)-5-methoxypyrimidin-2-yl)amino)-4-methoxyphenyl)acrylamide

### Example 132

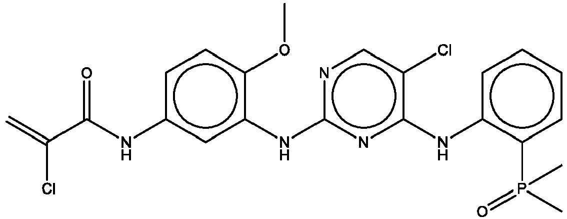

### 2-chloro-N-(3-((5-chloro-4-((2-(dimethylphosphoryl)phenyl)amino)pyrimidin-2-yl)amino)-4-methoxyphenyl)acrylamide

### Example 133

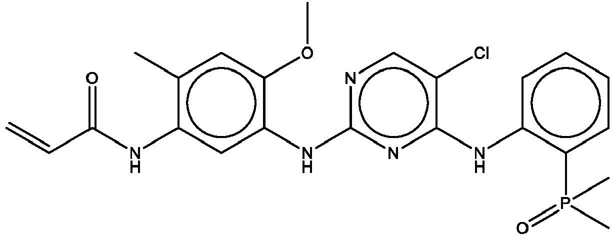

### N-(5-((5-chloro-4-((2-(dimethylphosphoryl)phenyl)amino)pyrimidin-2-yl)amino)-4-methoxy-2-methylphenyl)acrylamide

### Example 134

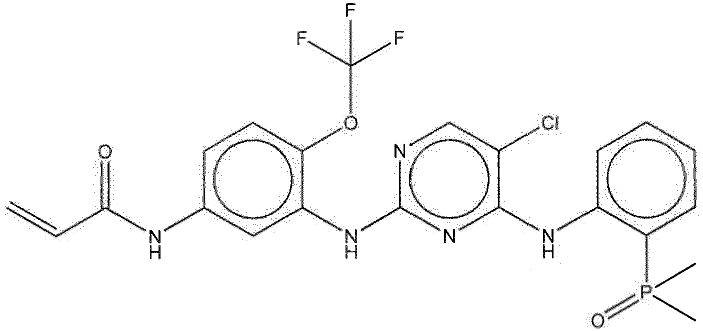

### N-(3-((5-chloro-4-((2-(dimethylphosphoryl)phenyl)amino)pyrimidin-2-yl)amino)-4-(trifluoromethoxy)phenyl)acrylamide

### Example 135

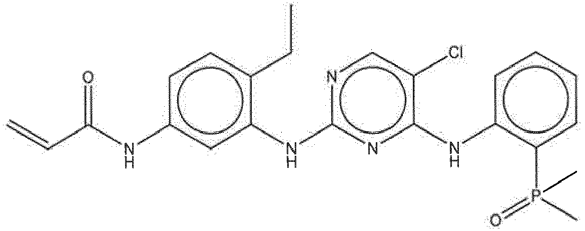

### N-(3-((5-chloro-4-((2-(dimethylphosphoryl)phenyl)amino)pyrimidin-2-yl)amino)-4-ethylphenyl)acrylamide

### Example 136

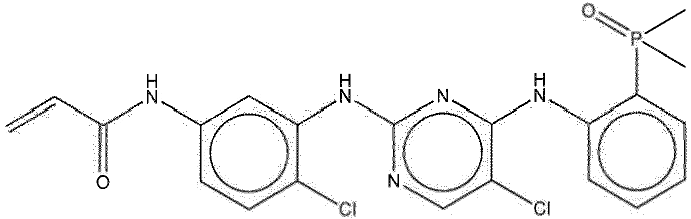

### N-(4-chloro-3-((5-chloro-4-((2-(dimethylphosphoryl)phenyl)amino)pyrimidin-2-yl)amino)phenyl)acrylamide

### Example 138

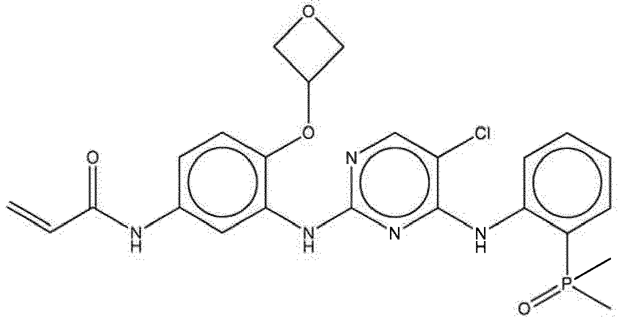

### N-(3-((5-chloro-4-((2-(dimethylphosphoryl)phenyl)amino)pyrimidin-2-yl)amino)-4-(oxetan-3-yloxy)phenyl)acrylamide

### Example 139

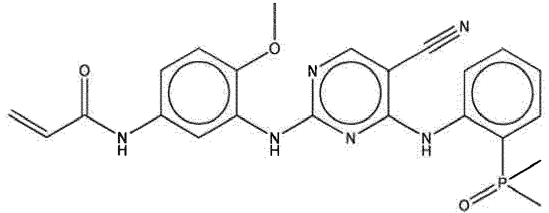

### N-(3-((5-cyano-4-((2-(dimethylphosphoryl)phenyl)amino)pyrimidin-2-yl)amino)-4-methoxyphenyl)acrylamide

### Example 142

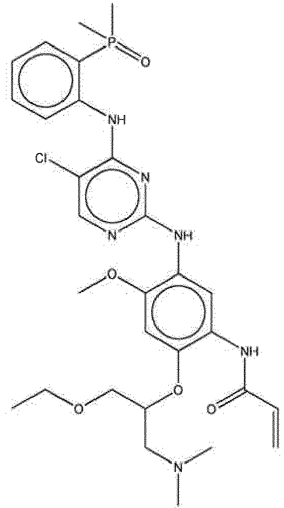

### N-(5-((5-chloro-4-((2-(dimethylphosphoryl)phenyl)amino)pyrimidin-2-yl)amino)-2-((1-(dimethylamino)-3-ethoxypropan-2-yl)oxy)-4-methoxyphenyl)acrylamide

### Example 143

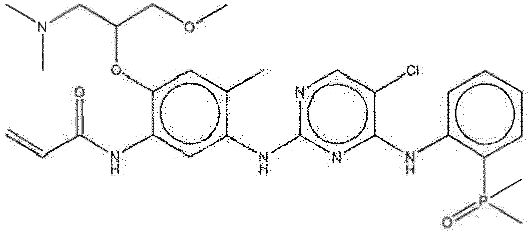

### N-(5-((5-cliloro-4-((2-(dimethylphosphoryl)phenyl)amino)pyrimidin-2-yl)amino)-2-((1-(dimethylamino)-3-methoxypropan-2-yl)oxy)-4-methylphenyl)acrylamide

### Example 144

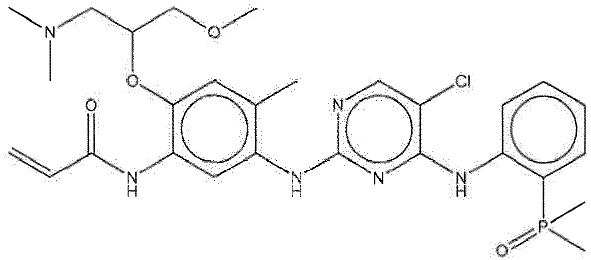

### N-(5-((5-chloro-4-((2-(dimethylphosphoryl)phenyl)amino)pyrimidin-2-yl)amino)-2-((1-(dimethylamino)-3-methoxypropan-2-yl)oxy)-4-methylphenyl)acrylamide

### Example 145

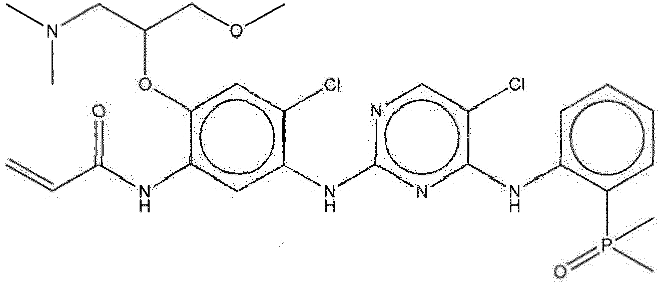

### N-(4-chloro-5-((5-chloro-4-((2-(dimethylphosphoryl)phenyl)amino)pyrimidin-2-yl)amino)-2-((1-(dimethylamino)-3-methoxypropan-2-yl)oxy)phenyl)acrylamide

### Example 146

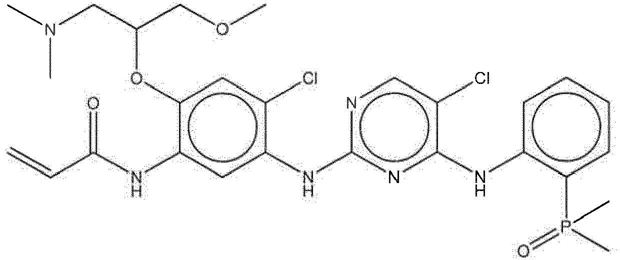

### N-(4-chloro-5-((5-chloro-4-((2-(dimethylphosphoryl)phenyl)amino)pyrimidin-2-yl)amino)-2-((1-(dimethylamino)-3-methoxypropan-2-yl)oxy)phenyl)acrylamide

### Example 151

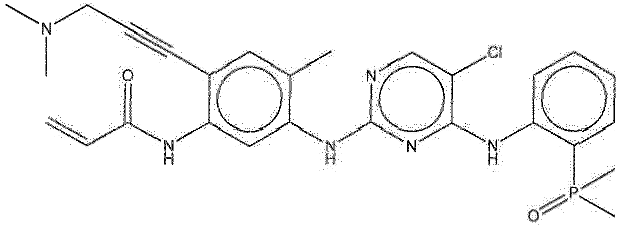

### N-(5-((5-chloro-4-((2-(dimethylphosphoryl)phenyl)amino)pyrimidin-2-yl)amino)-2-(3-(dimethylamino)prop-1-yn-1-yl)-4-methylphenyl)acrylamide

### Example 152

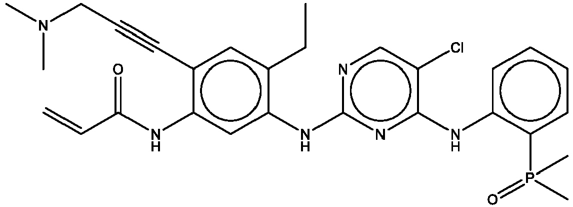

### N-(5-((5-chloro-4-((2-(dimethylphosphoryl)phenyl)amino)pyrimidin-2-yl)amino)-2-(3-(dimethylamino)prop-1-yn-1-yl)-4-ethylphenyl)acrylamide

### Example 153

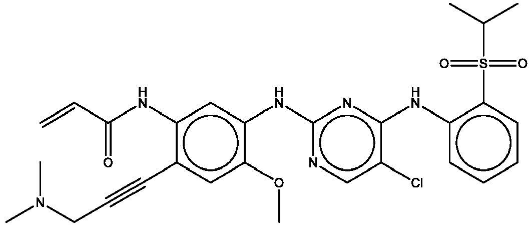

### N-(5-((5-chloro-4-((2-(isopropylsulfonyl)phenyl)amino)pyrimidin-2-yl)amino)-2-(3-(dimethylamino)prop-1-yn-1-yl)-4-methoxyphenyl)acrylamide

### Example 154

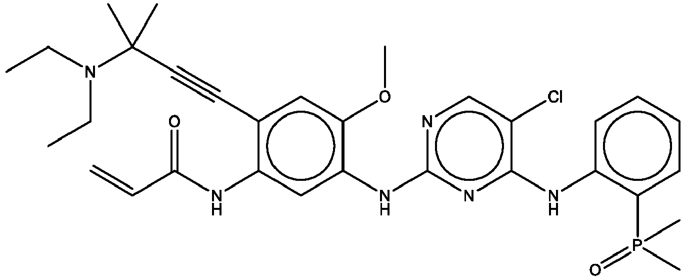

### N-(5-((5-chloro-4-((2-(dimethylphosphoryl)phenyl)amino)pyrimidin-2-yl)amino)-2-(3-(diethylamino)-3-methylbut-1-yn-1-yl)-4-methoxyphenyl)acrylamide

### Example 155

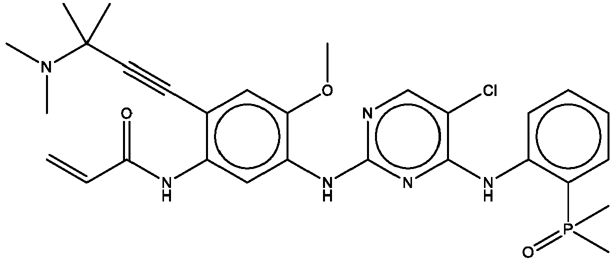

### N-(5-((5-chloro-4-((2-(dimethylphosphoryl)phenyl)amino)pyrimidin-2-yl)amino)-2-(3-(dimethylamino)-3-methylbut-1-yn-1-yl)-4-methoxyphenyl)acrylamide

### Example 156

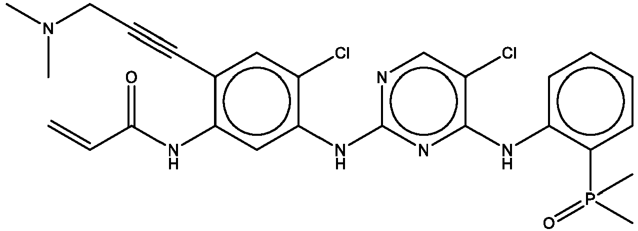

### N-(4-chloro-5-((5-chloro-4-((2-(dimethylphosphoryl)phenyl)amino)pyrimidin-2-yl)amino)-2-(3-(dimethylamino)prop-1-yn-1-yl)phenyl)acrylamide

### Example 157

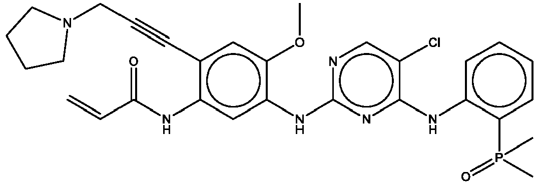

### N-(5-((5-chloro-4-((2-(dimethylphosphoryl)phenyl)amino)pyrimidin-2-yl)amino)-4-methoxy-2-(3-(pyrrolidin-1-yl)prop-1-yn-1-yl)phenyl)acrylamide

### Example 158

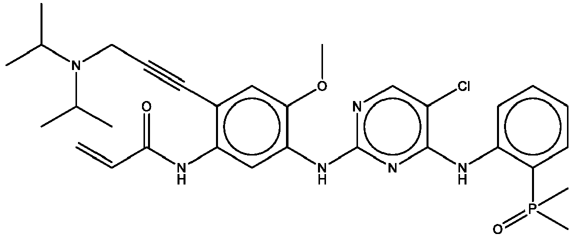

### N-(5-((5-chloro-4-((2-(dimethylphosphoryl)phenyl)amino)pyrimidin-2-yl)amino)-2-(3-(diisopropylamino)prop-1-yn-1-yl)-4-methoxyphenyl)acrylamide

### Example 159

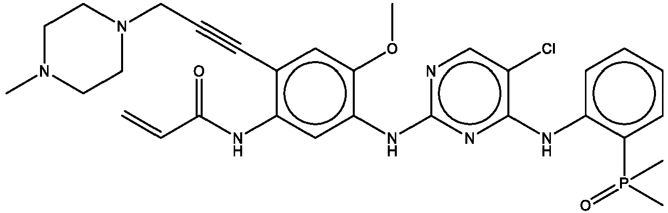

### N-(5-((5-chloro-4-((2-(dimethylphosphoryl)phenyl)amino)pyrimidin-2-yl)amino)-4-methoxy-2-(3-(4-methylpiperazin-1-yl)prop-1-yn-1-yl)phenyl)acrylamide

### Example 160

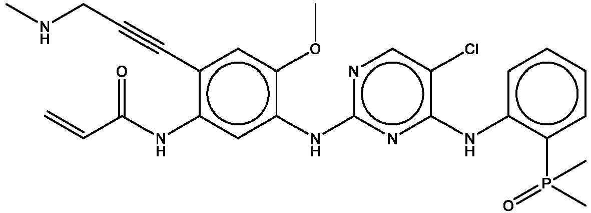

### N-(5-((5-chloro-4-((2-(dimethylphosphoryl)phenyl)amino)pyrimidin-2-yl)amino)-4-methoxy-2-(3-(methylamino)prop-1-yn-1-yl)phenyl)acrylamide

### Example 161

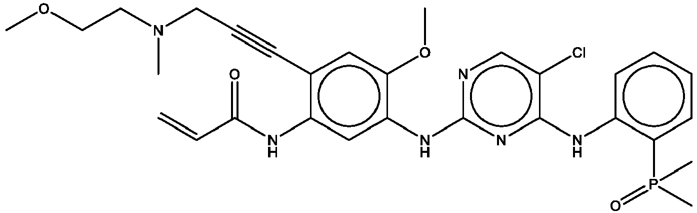

### N-(5-((5-chloro-4-((2-(dimethylphosphoryl)phenyl)amino)pyrimidin-2-yl)amino)-4-methoxy-2-(3-((2-methoxyethyl)(methyl)amino)prop-1-yn-1-yl)phenyl)acrylamide

### Example 162

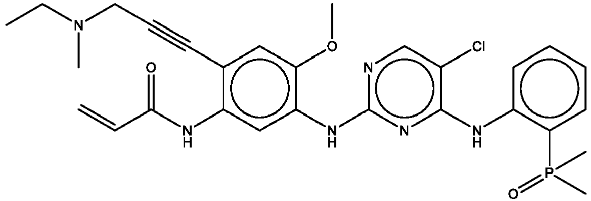

### N-(5-((5-chloro-4-((2-(dimethylphosphoryl)phenyl)amino)pyrimidin-2-yl)amino)-2-(3-(ethyl(methyl)amino)prop-1-yn-1-yl)-4-methoxyphenyl)acrylamide

### Example 163

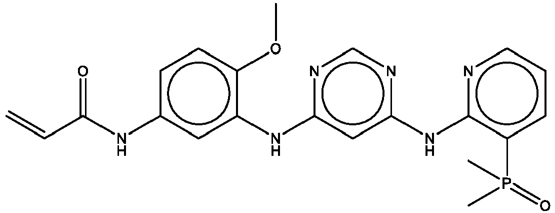

### N-(3-((6-((3-(dimethylphosphoryl)pyridin-2-yl)amino)pyrimidin-4-yl)amino)-4-methoxyphenyl)acrylamide

### Example 164

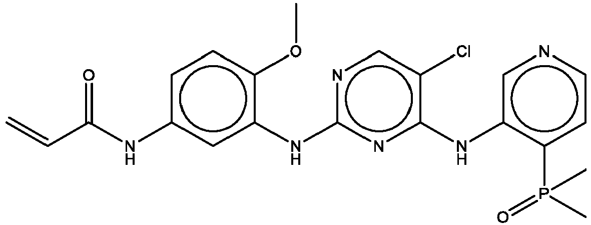

### N-(3-((5-chloro-4-((4-(dimethylphosphoryl)pyridin-3-yl)amino)pyrimidin-2-yl)amino)-4-methoxyphenyl)acrylamide

### Example 165

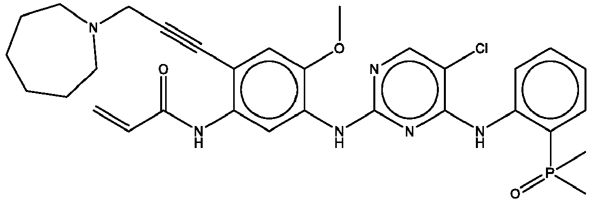

### N-(2-(3-(azepan-1-yl)prop-1-yn-1-yl)-5-((5-chloro-4-((2-(dimethylphosphoryl)phenyl)amino)pyrimidin-2-yl)amino)-4-methoxyphenyl)acrylamide

### Example 166

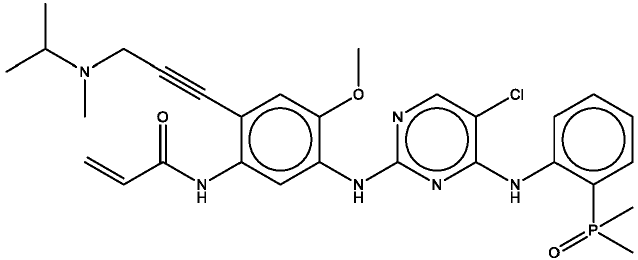

### N-(5-((5-chloro-4-((2-(dimethylphosphoryl)phenyl)amino)pyrimidin-2-yl)amino)-2-(3-(isopropyl(methyl)amino)prop-1-yn-1-yl)-4-methoxyphenyl)acrylamide

### Example 167

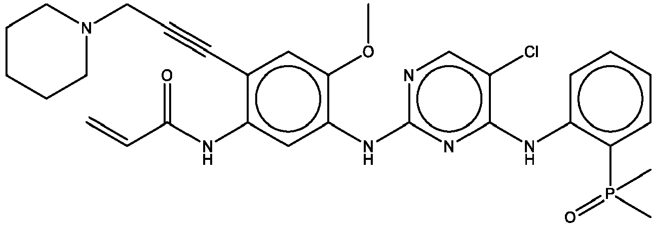

### N-(5-((5-chloro-4-((2-(dimethylphosphoryl)phenyl)amino)pyrimidin-2-yl)amino)-4-methoxy-2-(3-(piperidin-1-yl)prop-1-yn-1-yl)phenyl)acrylamide

### Example 168

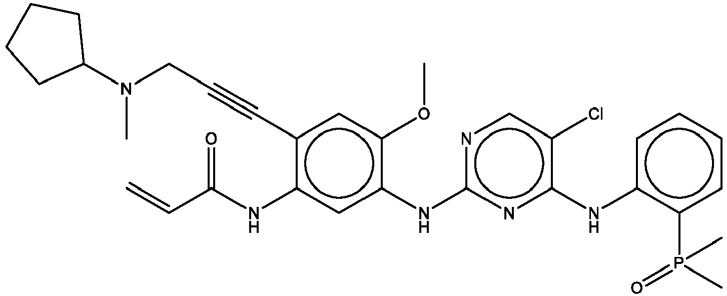

### N-(5-((5-chloro-4-((2-(dimethylphosphoryl)phenyl)amino)pyrimidin-2-yl)amino)-2-(3-(cyclopentyl(methyl)amino)prop-1-yn-1-yl)-4-methoxyphenyl)acrylamide

### Example 169

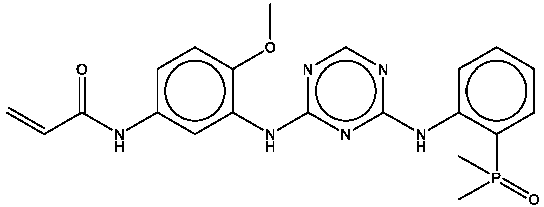

### N-(3-((4-((2-(di-ethylphosphoryl)phenyl)amino)-1,3,5-triazin-2-yl)amino)-4-methoxyphenyl)acrylamide

**Example 170**

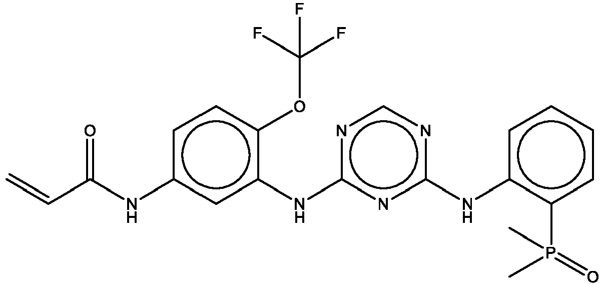

### N-(3-((4-((2-(dimethylphosphoryl)phenyl)amino)-1,3,5-triazin-2-yl)amino)-4-(trifluoromethoxy)phenyl)acrylamide

### Example 171

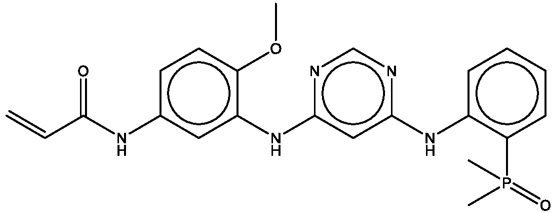

### N-(3-((6-((2-(dimethylphosphoryl)phenyl)amino)pyrimidin-4-yl)amino)-4-methoxyphenyl)acrylamide

### Example 172

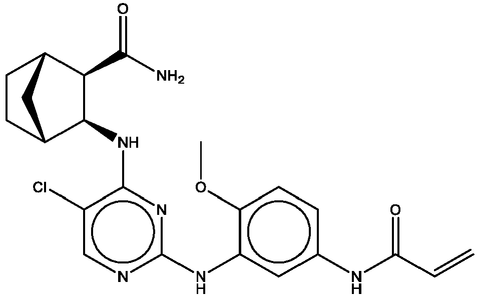

### (1S,2R,3S,4R)-3-((2-((5-acrylamido-2-methoxyphenyl)amino)-5-chloropyrimidin-4-yl)amino)bicyclo[2.2.1]heptane-2-carboxamide

### Example 173

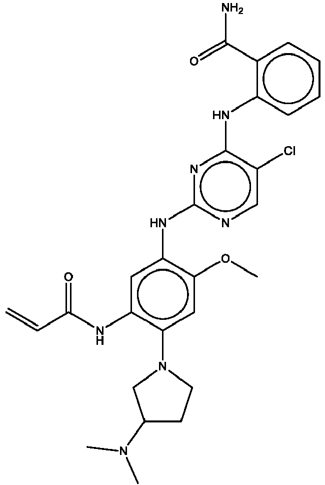

### 2-((2-((5-acrylamido-4-(3-(dimethylamino)pyrrolidin-1-yl)-2-methoxyphenyl)amino)-5-chloropyrimidin-4-yl)amino)benzamide

### Example 174

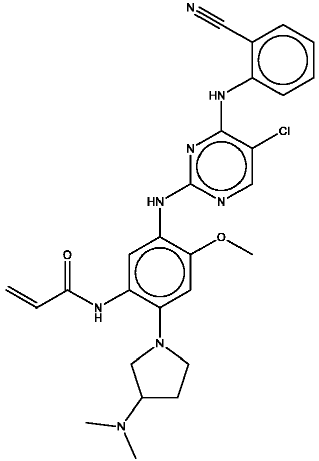

### N-(5-((5-chloro-4-((2-eyanophenyl)amino)pyrimidin-2-yl)amino)-2-(3-(dimethylamino)pyrrolidin-1-yl)-4-methoxyphenyl)acrylamide

### Example 175

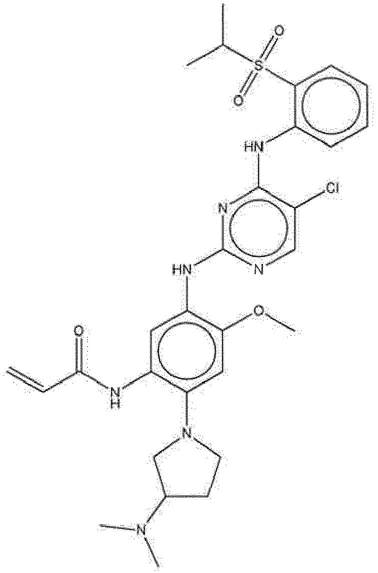

### N-(5-((5-chloro-4-((2-(isopropylsulfonyl)phenyl)amino)pyrimidin-2-yl)amino)-2-(3-(dimethylamino)pyrrolidin-1-yl)-4-methoxyphenyl)acrylamide

### Biological Assays.

Kinase inhibitory activity of the compounds was measured against human EGFR (native sequence) and against EGFR bearing the L858R mutation and the L858R/T790M double mutation (EGFR, L858R, and L858R/T790M, respectively in Table 1). Additional assays can be conducted with EGFR deletion mutants such as delE746 - A750 with or without the additional T790M mutation. Assay conditions included 10 pt curves with 3 µM top concentration (singlicates) and 10 µM ATP.

We also assessed the antiproliferative activity of compounds of the invention against BaF3 cells expressing the target EGFR mutations or control (i.e., cell lines expressing wildtype EGFR). Assays were conducted using MTT.

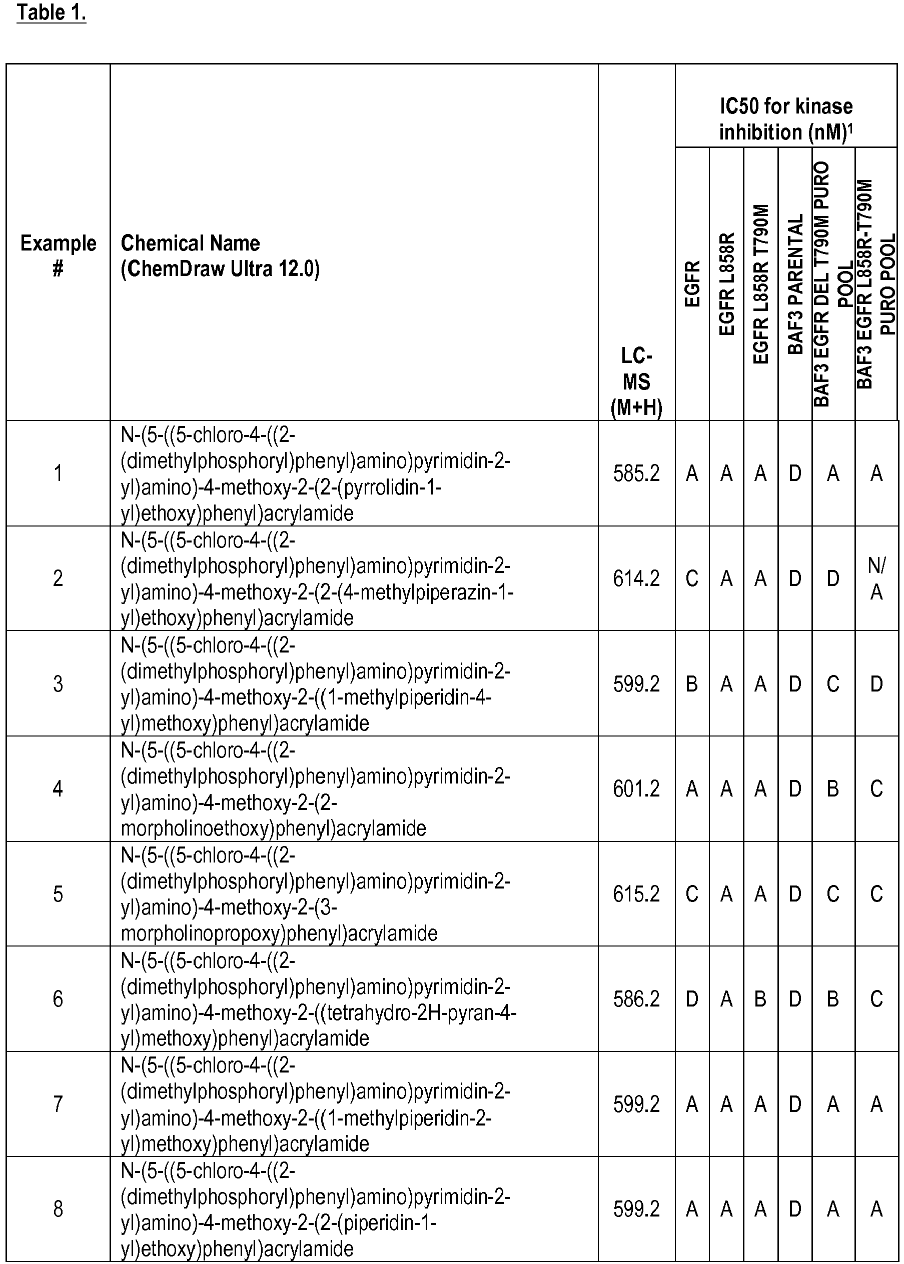

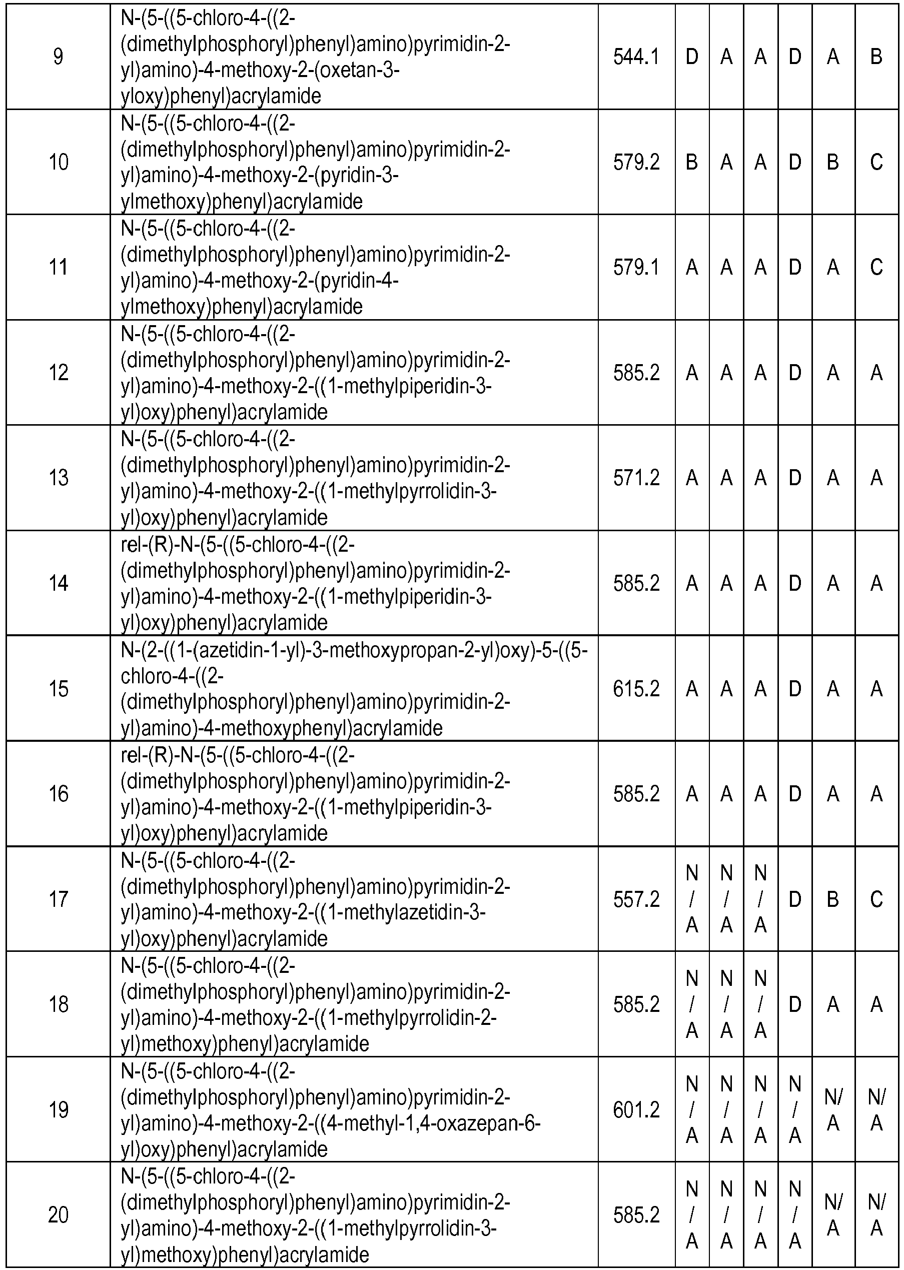

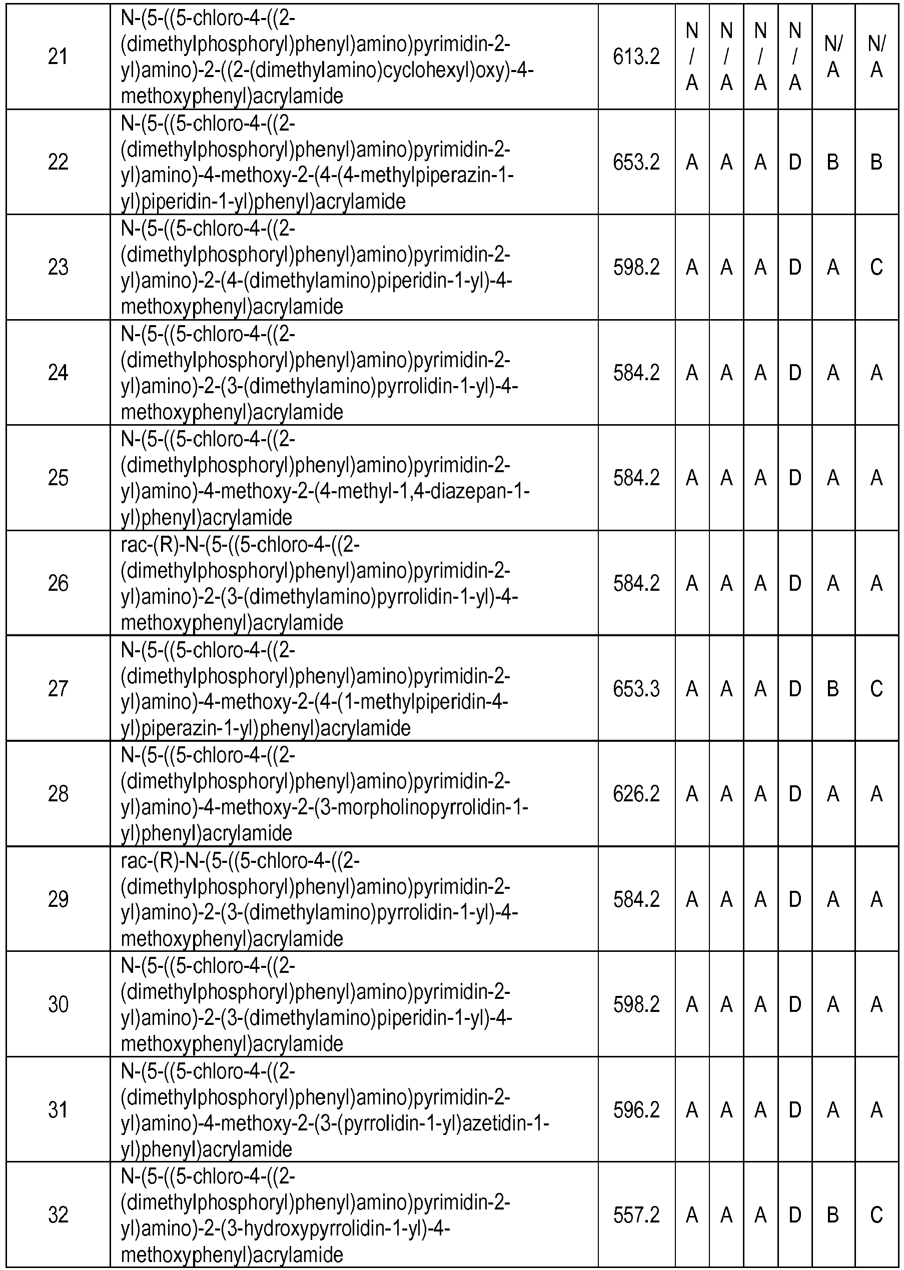

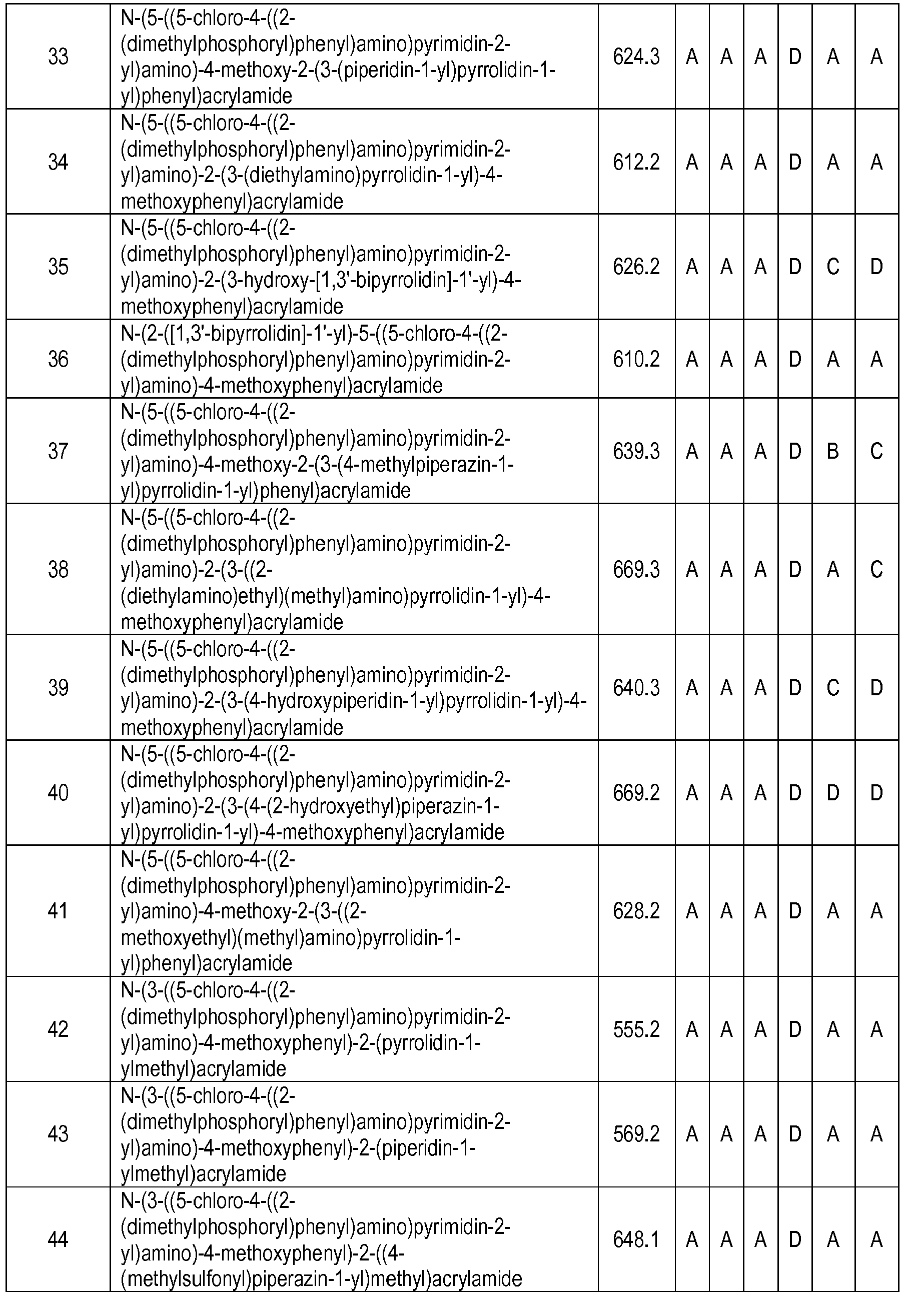

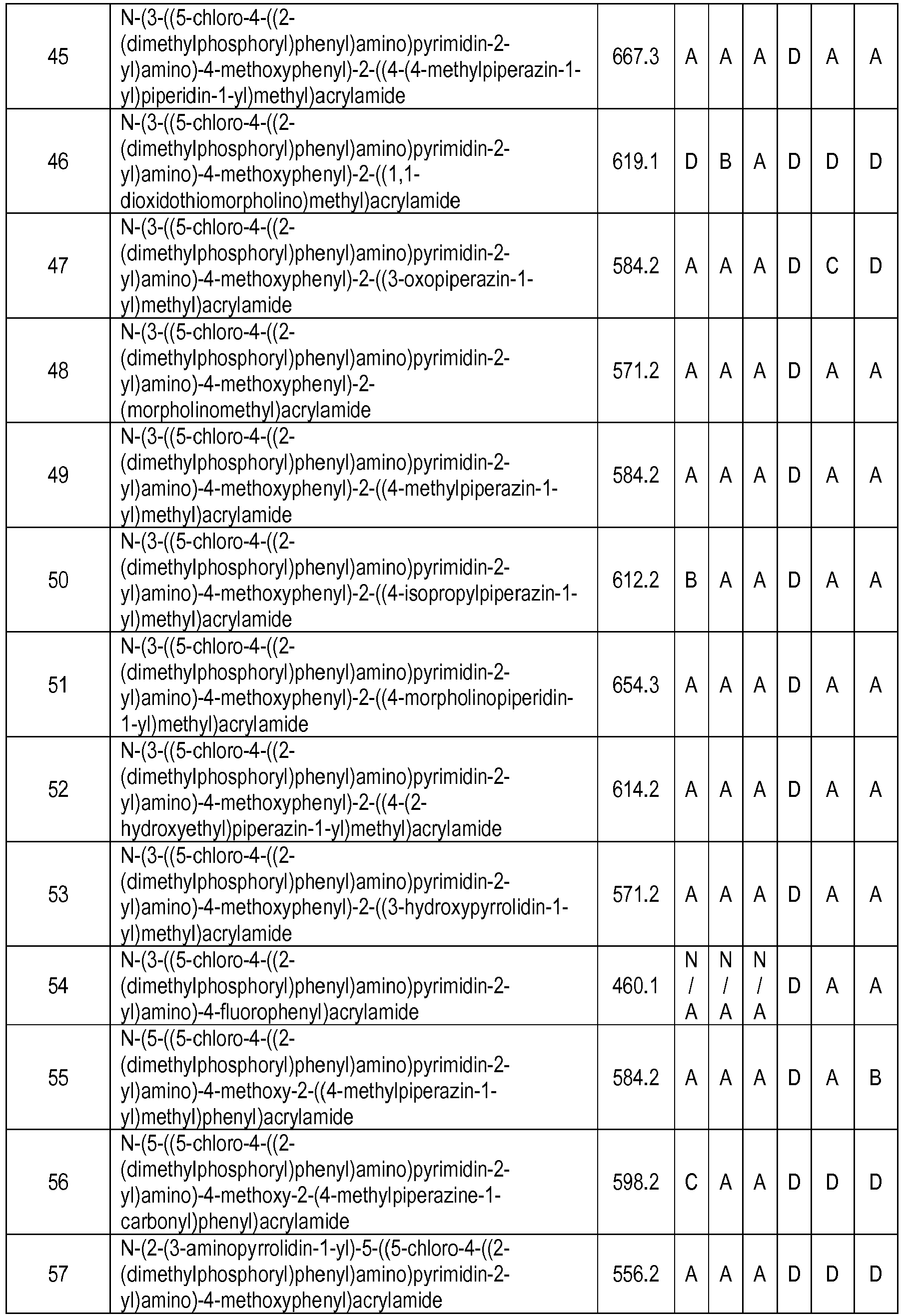

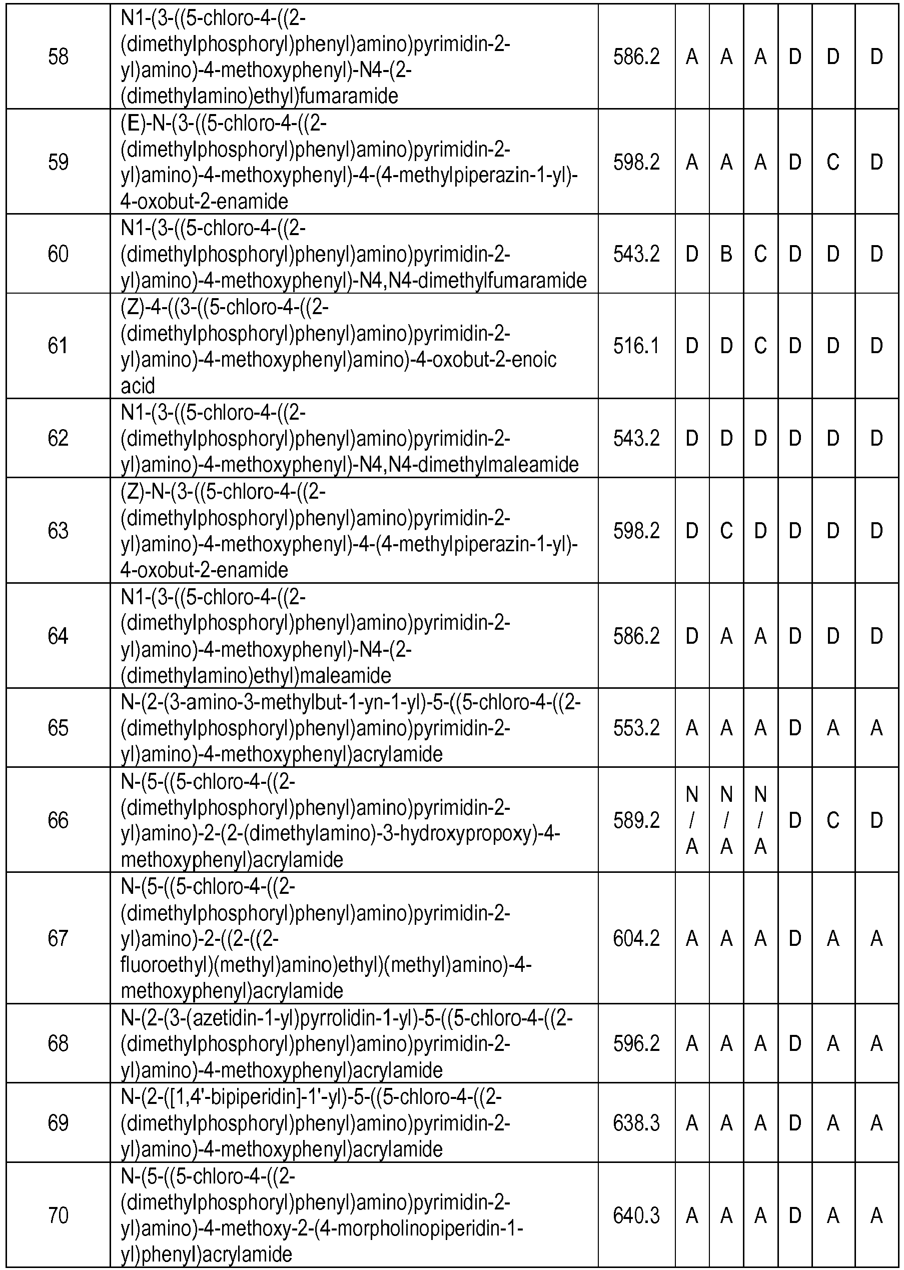

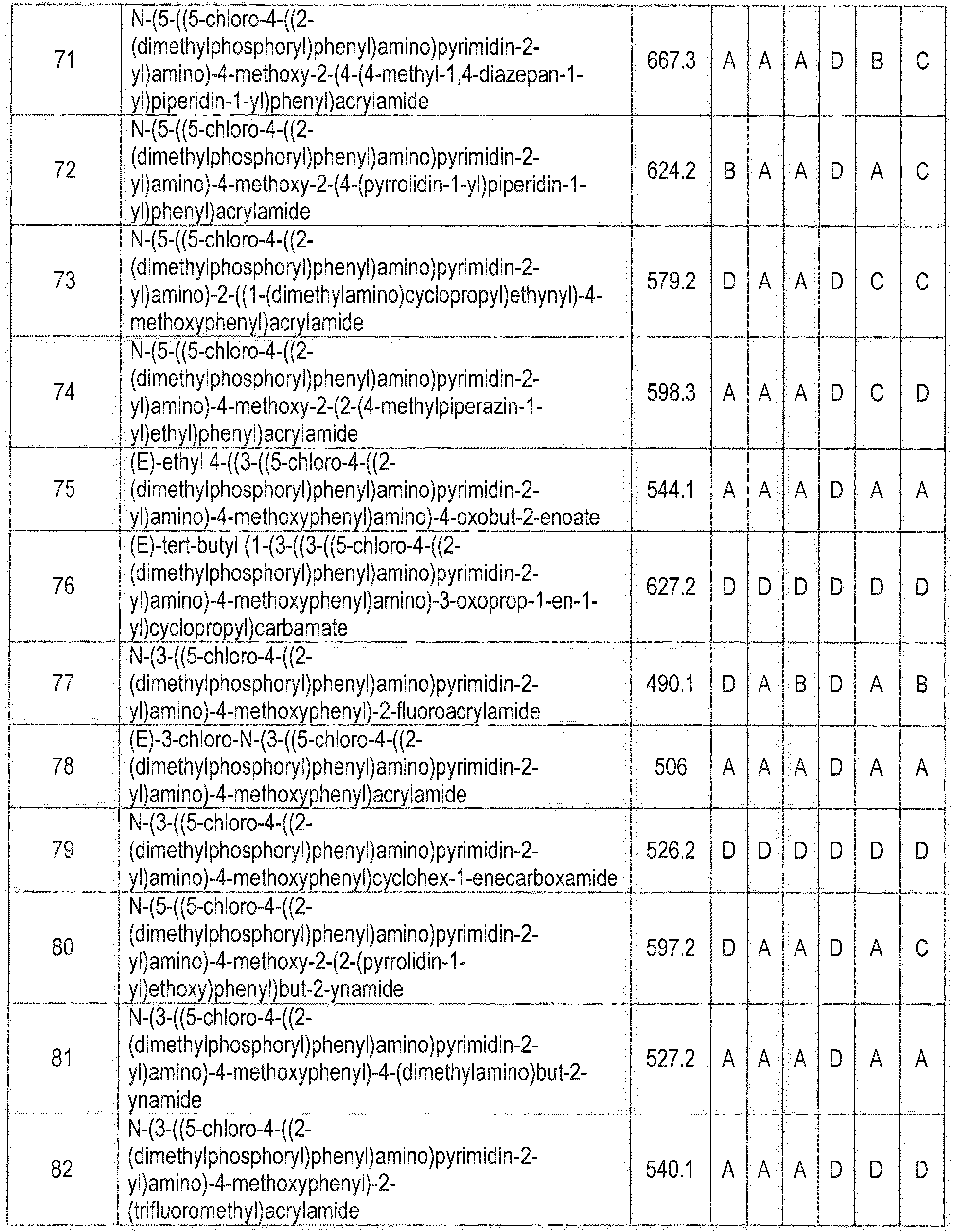

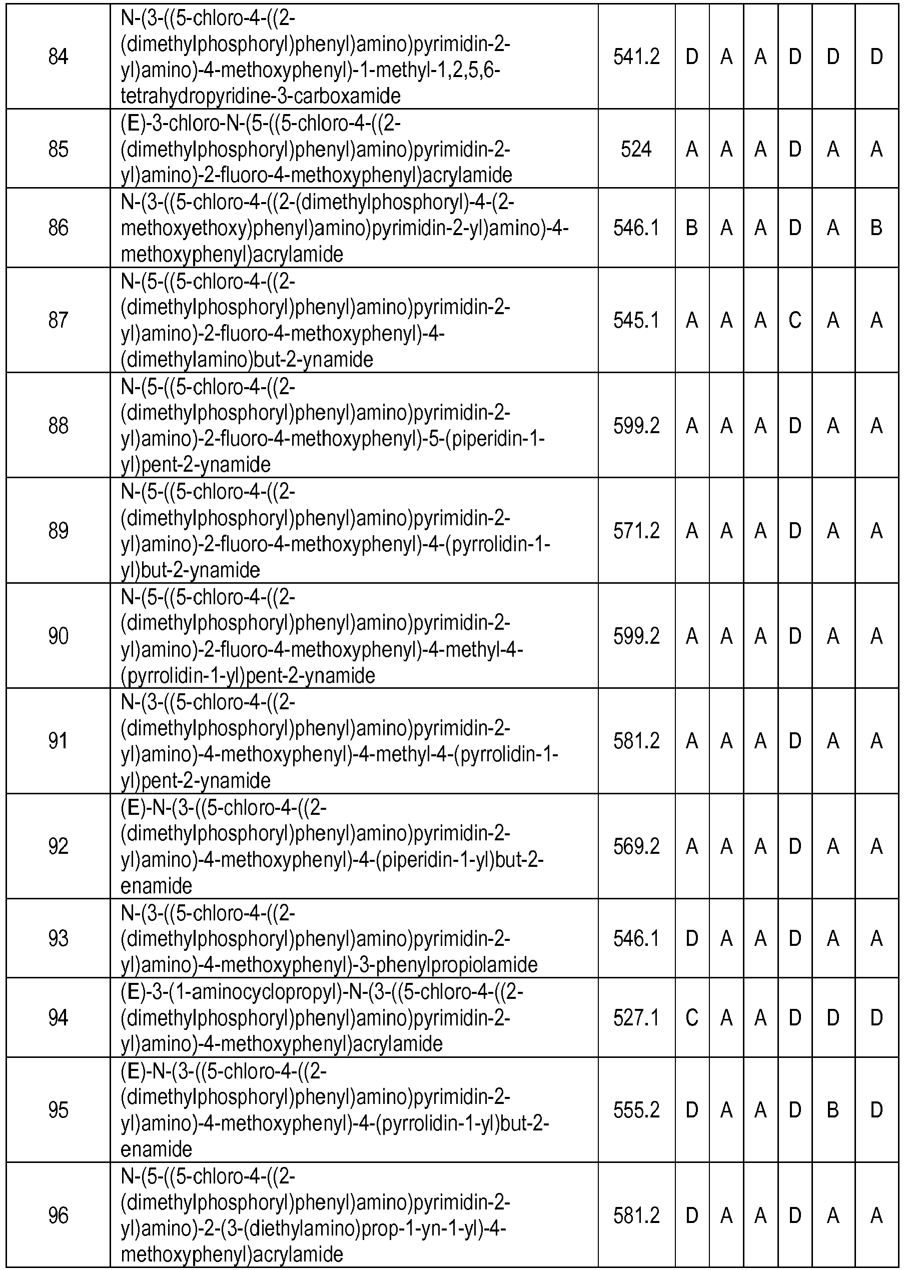

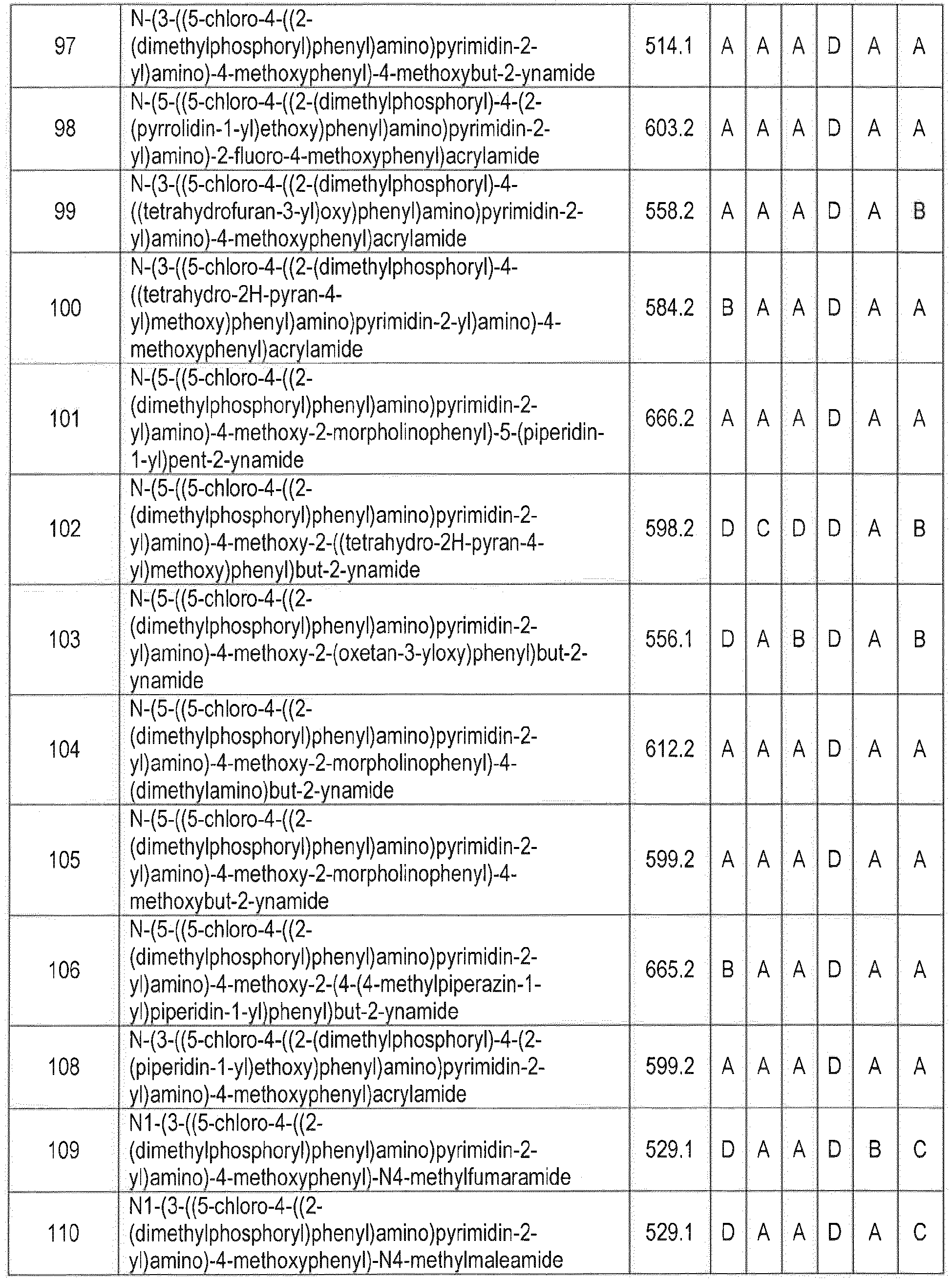

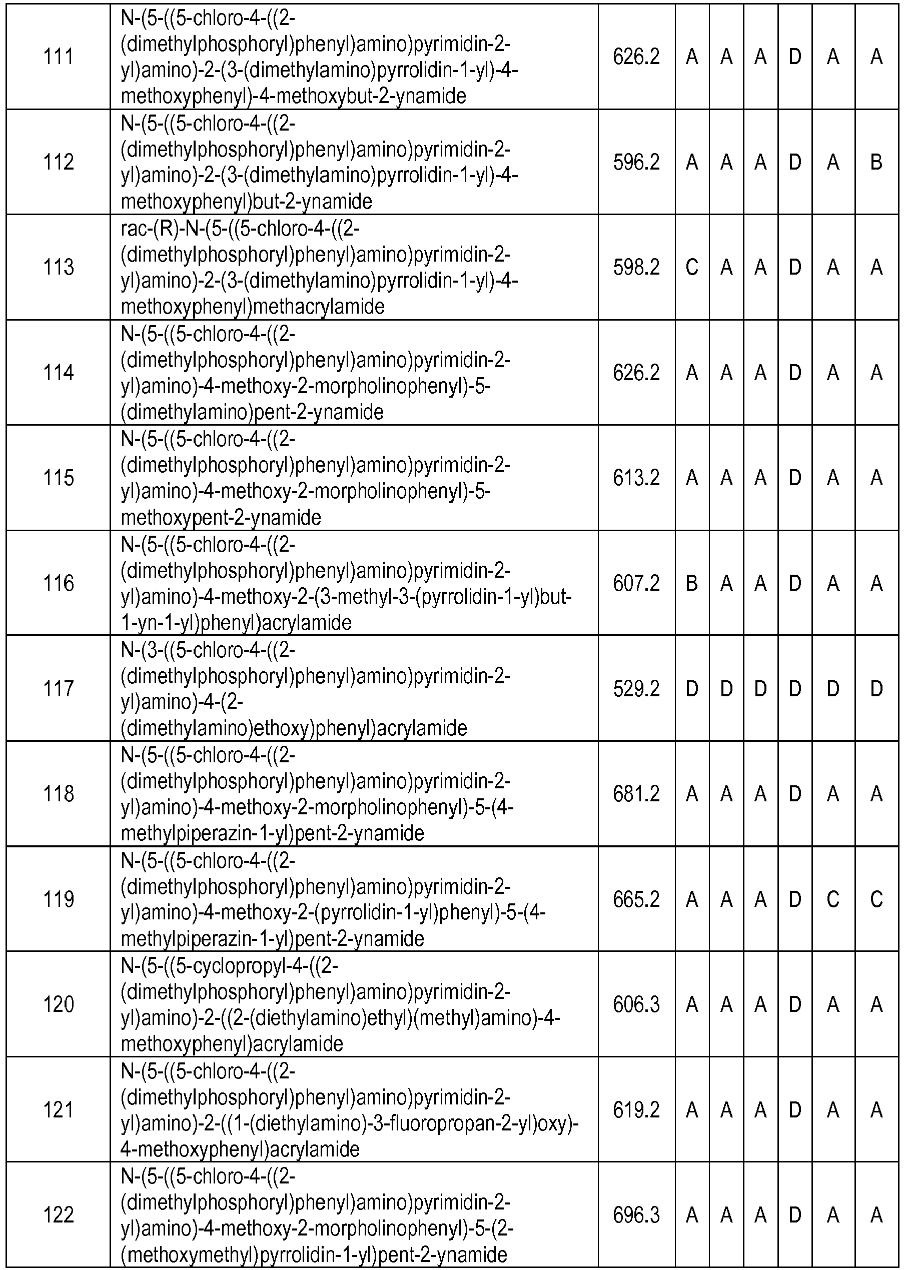

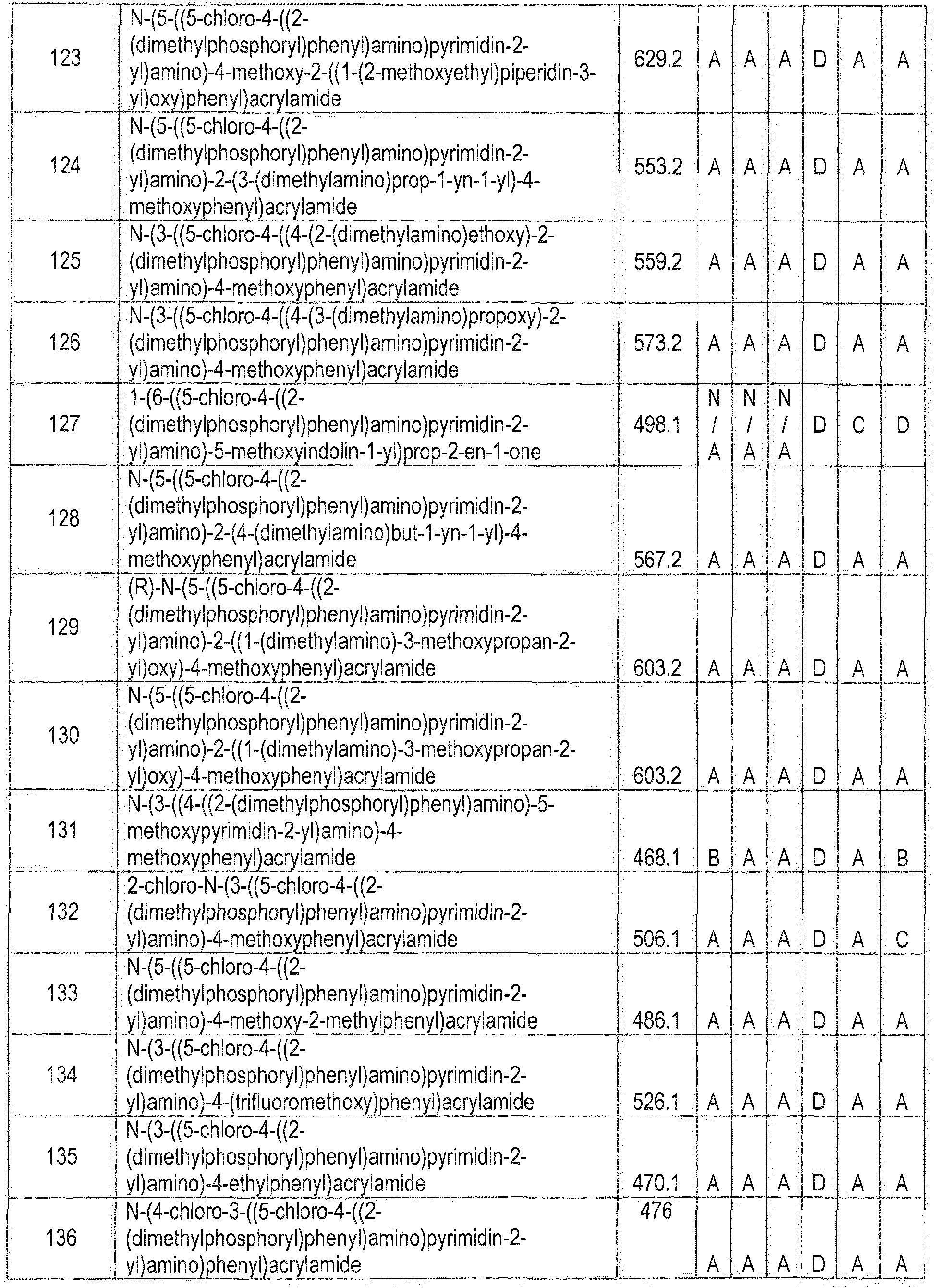

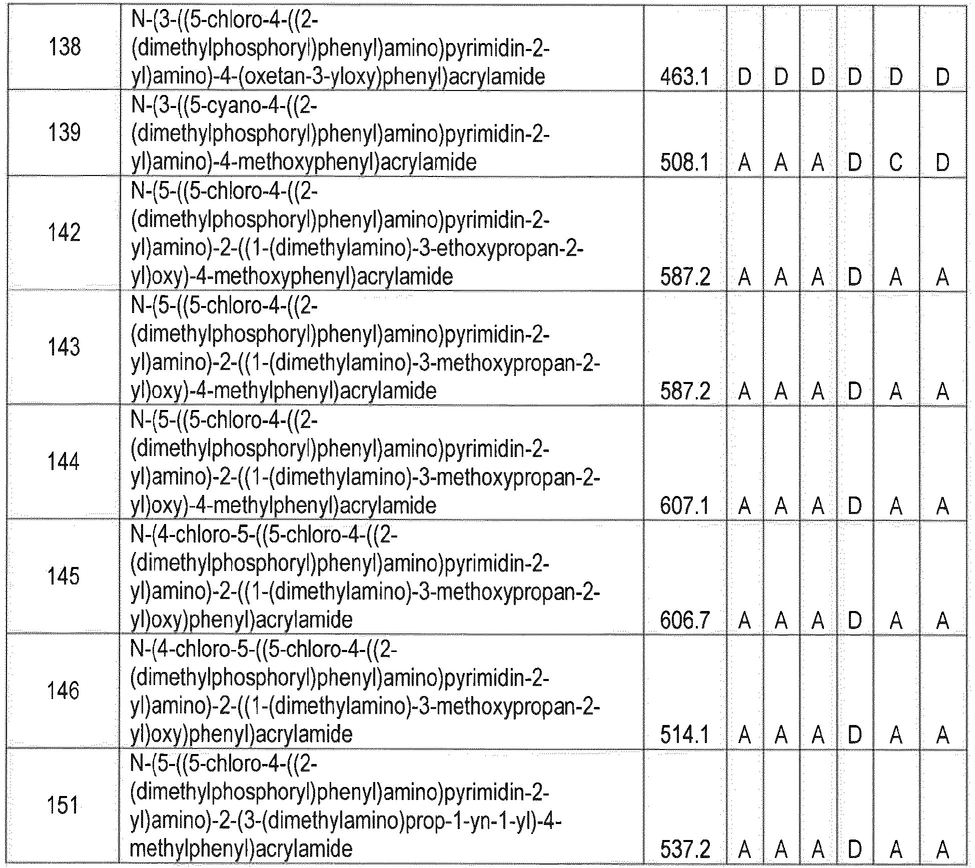

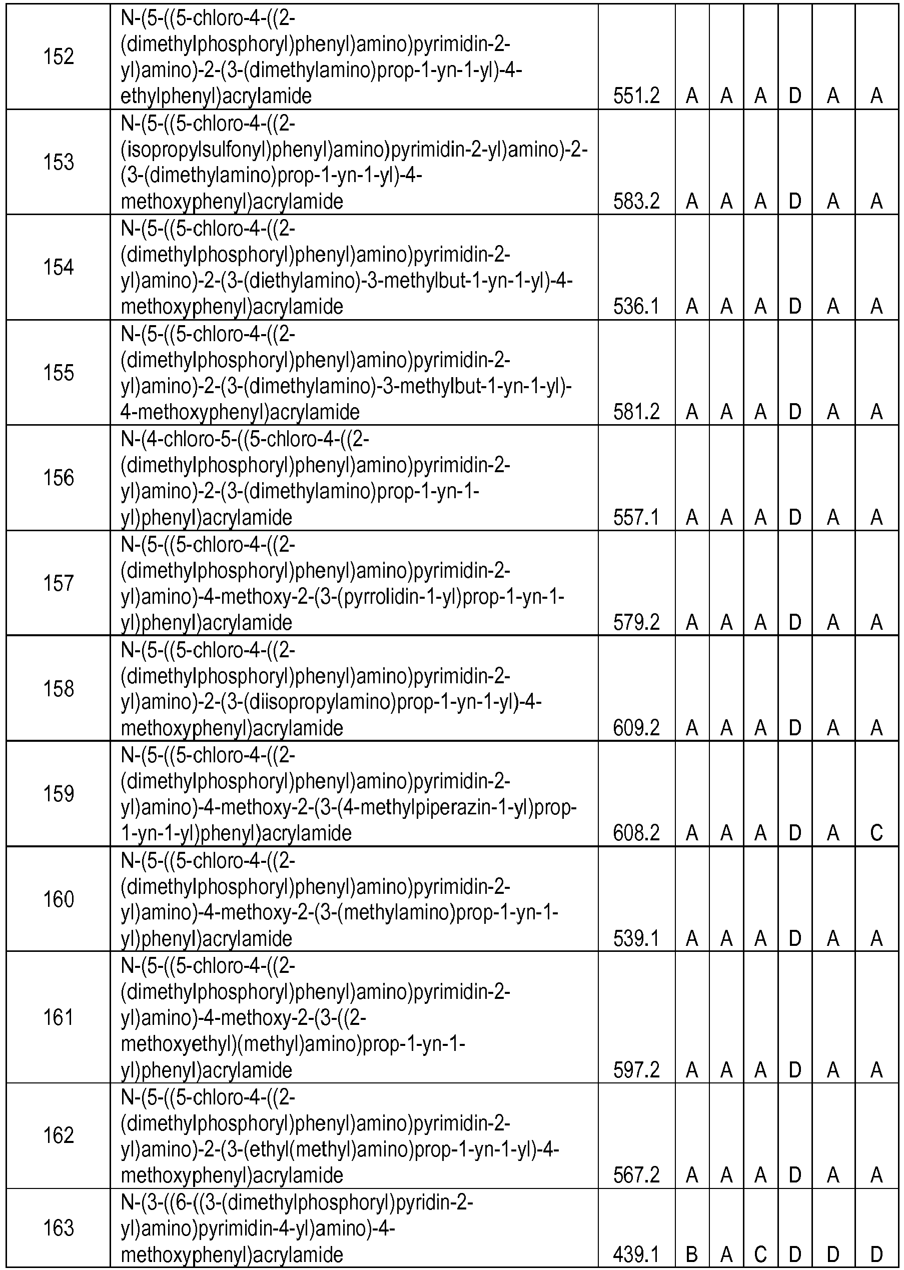

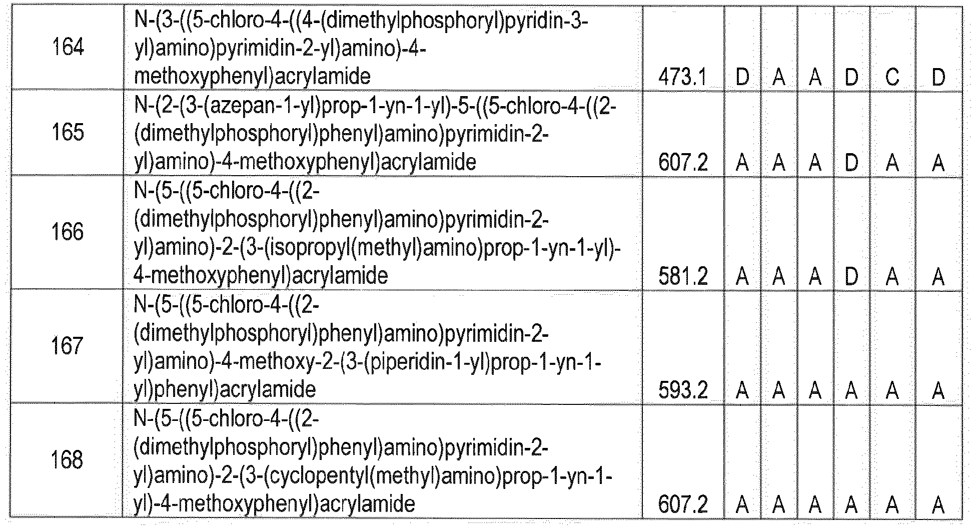

1. A = < 50 nM; B = 50 →100 nM; C = 101 nM → < 250 nM; D = ≥ 250 nM

## Claims

1. A compound selected from the group consisting of:
N-(5-((5-chloro-4-((2-(dimethylphosphoryl)phenyl)amino)pyrimidin-2-yl)amino)-4-methoxy-2-(2-(pyrrolidin-1-yl)ethoxy)phenyl)acrylamide;
N-(5-((5-chloro-4-((2-(dimethylphosphoryl)phenyl)amino)pyrimidin-2-yl)amino)-4-methoxy-2-(2-(4-methylpiperazin-1-yl)ethoxy)phenyl)acrylamide;
N-(5-((5-chloro-4-((2-(dimethylphosphoryl)phenyl)amino)pyrimidin-2-yl)amino)-4-methoxy-2-((1-methylpiperidin-4-yl)methoxy)phenyl)acrylamide;
N-(5-((5-chloro-4-((2-(dimethylphosphoryl)phenyl)amino)pyrimidin-2-yl)amino)-4-methoxy-2-(2-morpholinoethoxy)phenyl)acrylamide;
N-(5-((5-chloro-4-((2-(dimethylphosphoryl)phenyl)amino)pyrimidin-2-yl)amino)-4-methoxy-2-(3-morpholinopropoxy)phenyl)acrylamide;
N-(5-((5-chloro-4-((2-(dimethylphosphoryl)phenyl)amino)pyrimidin-2-yl)amino)-4-methoxy-2-((tetrahydro-2H-pyran-4-yl)methoxy)phenyl)acrylamide;
N-(5-((5-chloro-4-((2-(dimethylphosphoryl)phenyl)amino)pyrimidin-2-yl)amino)-4-methoxy-2-((1-methylpiperidin-2-yl)methoxy)phenyl)acrylamide;
N-(5-((5-chloro-4-((2-(dimethylphosphoryl)phenyl)amino)pyrimidin-2-yl)amino)-4-methoxy-2-(2-(piperidin-1-yl)ethoxy)phenyl)acrylamide;
N-(5-((5-chloro-4-((2-(dimethylphosphoryl)phenyl)amino)pyrimidin-2-yl)amino)-4-methoxy-2-(oxetan-3-yloxy)phenyl)acrylamide;
N-(5-((5-chloro-4-((2-(dimethylphosphoryl)phenyl)amino)pyrimidin-2-yl)amino)-4-methoxy-2-(pyridin-3-ylmethoxy)phenyl)acrylamide;
N-(5-((5-chloro-4-((2-(dimethylphosphoryl)phenyl)amino)pyrimidin-2-yl)amino)-4-methoxy-2-(pyridin-4-ylmethoxy)phenyl)acrylamide;
N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide;
N-(5-((5-chloro-4-((2-(dimethylphosphoryl)phenyl)amino)pyrimidin-2-yl)amino)-4-methoxy-2-((1-methylpyrrolidin-3-yl)oxy)phenyl)acrylamide;
rel-(R)-N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide;
N-(2-((1-(azetidin-1-yl)-3-methoxypropan-2-yl)oxy)-5-((5-chloro-4-((2-(dimethylphosphoryl)phenyl)amino)pyrimidin-2-yl)amino)-4-methoxyphenyl)acrylamide;
rel-(R)-N-(5-((5-chloro-4-((2-(dimethylphosphoryl)phenyl)amino)pyrimidin-2-yl)amino)-4-methoxy-2-((1-methylpiperidin-3-yl)oxy)phenyl)acrylamide;
N-(5-((5-chloro-4-((2-(dimethylphosphoryl)phenyl)amino)pyrimidin-2-yl)amino)-4-methoxy-2-((1-methylazetidin-3-yl)oxy)phenyl)acrylamide;
N-(5-((5-chloro-4-((2-(dimethylphosphoryl)phenyl)amino)pyrimidin-2-yl)amino)-4-methoxy-2-((1-methylpyrrolidin-2-yl)methoxy)phenyl)acrylamide;
N-(5-((5-chloro-4-((2-(dimethylphosphoryl)phenyl)amino)pyrimidin-2-yl)amino)-4-methoxy-2-((4-methyl-1,4-oxazepan-6-yl)oxy)phenyl)acrylamide;
N-(5-((5-chloro-4-((2-(dimethylphosphoryl)phenyl)amino)pyrimidin-2-yl)amino)-4-methoxy-2-((1-methylpyrrolidin-3-yl)methoxy)phenyl)acrylamide;
N-(5-((5-chloro-4-((2-(dimethylphosphoryl)phenyl)amino)pyrimidin-2-yl)amino)-2-((2-(dimethylamino)cyclohexyl)oxy)-4-methoxyphenyl)acrylamide;
N-(5-((5-chloro-4-((2-(dimethylphosphoryl)phenyl)amino)pyrimidin-2-yl)amino)-4-methoxy-2-(4-(4-methylpiperazin-1-yl)piperidin-1-yl)phenyl)acrylamide;
N-(5-((5-chloro-4-((2-(dimethylphosphoryl)phenyl)amino)pyrimidin-2-yl)amino)-2-(4-(dimethylamino)piperidin-1-yl)-4-methoxyphenyl)acrylamide;
N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide;
N-(5-((5-chloro-4-((2-(dimethylphosphoryl)phenyl)amino)pyrimidin-2-yl)amino)-4-methoxy-2-(4-methyl-1,4-diazepan-1-yl)phenyl)acrylamide;
rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide;
N-(5-((5-chloro-4-((2-(dimethylphosphoryl)phenyl)amino)pyrimidin-2-yl)amino)-4-methoxy-2-(4-(1-methylpiperidin-4-yl)piperazin-1-yl)phenyl)acrylamide;
N-(5-((5-chloro-4-((2-(dimethylphosphoryl)phenyl)amino)pyrimidin-2-yl)amino)-4-methoxy-2-(3-morpholinopyrrolidin-1-yl)phenyl)acrylamide;
rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)acrylamide;
N-(5-((5-chloro-4-((2-(dimethylphosphoryl)phenyl)amino)pyrimidin-2-yl)amino)-2-(3-(dimethylamino)piperidin-1-yl)-4-methoxyphenyl)acrylamide;
N-(5-((5-chloro-4-((2-(dimethylphosphoryl)phenyl)amino)pyrimidin-2-yl)amino)-4-methoxy-2-(3-(pyrrolidin-1-yl)azetidin-1-yl)phenyl)acrylamide;
N-(5-((5-chloro-4-((2-(dimethylphosphoryl)phenyl)amino)pyrimidin-2-yl)amino)-2-(3-hydroxypyrrolidin-1-yl)-4-methoxyphenyl)acrylamide;
N-(5-((5-chloro-4-((2-(dimethylphosphoryl)phenyl)amino)pyrimidin-2-yl)amino)-4-methoxy-2-(3-(piperidin-1-yl)pyrrolidin-1-yl)phenyl)acrylamide;
N-(5-((5-chloro-4-((2-(dimethylphosphoryl)phenyl)amino)pyrimidin-2-yl)amino)-2-(3-(diethylamino)pyrrolidin-1-yl)-4-methoxyphenyl)acrylamide;
N-(5-((5-chloro-4-((2-(dimethylphosphoryl)phenyl)amino)pyrimidin-2-yl)amino)-2-(3-hydroxy-[1,3'-bipyrrolidin]-1'-yl)-4-methoxyphenyl)acrylamide;
N-(2-([1,3'-bipyrrolidin]-1'-yl)-5-((5-chloro-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-4-methoxy-2-(3-(4-methylpiperazin-1-yl)pyrrolidin-1-yl)phenyl)acrylamide;
N-(5-((5-chloro-4-((2-(dimethylphosphoryl)phenyl)amino)pyrimidin-2-yl)amino)-2-(3-((2-(diethylamino)ethyl)(methyl)amino)pyrrolidin-1-yl)-4-methoxyphenyl)acrylamide;
N-(5-((5-chloro-4-((2-(dimethylphosphoryl)phenyl)amino)pyrimidin-2-yl)amino)-2-(3-(4-hydroxypiperidin-1-yl)pyrrolidin-1-yl)-4-methoxyphenyl)acrylamide;
N-(5-((5-chloro-4-((2-(dimethylphosphoryl)phenyl)amino)pyrimidin-2-yl)amino)-2-(3-(4-(2-hydroxyethyl)piperazin-1-yl)pyrrolidin-1-yl)-4-methoxyphenyl)acrylamide;
N-(5-((5-chloro-4-((2-(dimethylphosphoryl)phenyl)amino)pyrimidin-2-yl)amino)-4-methoxy-2-(3-((2-methoxyethyl)(methyl)amino)pyrrolidin-1-yl)phenyl)acrylamide;
N-(3-((5-chloro-4-((2-(dimethylphosphoryl)phenyl)amino)pyrimidin-2-yl)amino)-4-methoxyphenyl)-2-(pyrrolidin-1-ylmethyl)acrylamide;
N-(3-((5-chloro-4-((2-(dimethylphosphoryl)phenyl)amino)pyrimidin-2-yl)amino)-4-methoxyphenyl)-2-(piperidin-1-ylmethyl)acrylamide;
N-(3-((5-chloro-4-((2-(dimethylphosphoryl)phenyl)amino)pyrimidin-2-yl)amino)-4-methoxyphenyl)-2-((4-(methylsulfonyl)piperazin-1-yl)methyl)acrylamide;
N-(3-((5-chloro-4-((2-(dimethylphosphoryl)phenyl)amino)pyrimidin-2-yl)amino)-4-methoxyphenyl)-2-((4-(4-methylpiperazin-1-yl)piperidin-1-yl)methyl)acrylamide;
N-(3-((5-chloro-4-((2-(dimethylphosphoryl)phenyl)amino)pyrimidin-2-yl)amino)-4-methoxyphenyl)-2-((1,1-dioxidothiomorpholino)methyl)acrylamide;
N-(3-((5-chloro-4-((2-(dimethylphosphoryl)phenyl)amino)pyrimidin-2-yl)amino)-4-methoxyphenyl)-2-((3-oxopiperazin-1-yl)methyl)acrylamide;
N-(3-((5-chloro-4-((2-(dimethylphosphoryl)phenyl)amino)pyrimidin-2-yl)amino)-4-methoxyphenyl)-2-(morpholinomethyl)acrylamide;
N-(3-((5-chloro-4-((2-(dimethylphosphoryl)phenyl)amino)pyrimidin-2-yl)amino)-4-methoxyphenyl)-2-((4-methylpiperazin-1-yl)methyl)acrylamide;
N-(3-((5-chloro-4-((2-(dimethylphosphoryl)phenyl)amino)pyrimidin-2-yl)amino)-4-methoxyphenyl)-2-((4-isopropylpiperazin-1-yl)methyl)acrylamide;
N-(3-((5-chloro-4-((2-(dimethylphosphoryl)phenyl)amino)pyrimidin-2-yl)amino)-4-methoxyphenyl)-2-((4-morpholinopiperidin-1-yl)methyl)acrylamide;
N-(3-((5-chloro-4-((2-(dimethylphosphoryl)phenyl)amino)pyrimidin-2-yl)amino)-4-methoxyphenyl)-2-((4-(2-hydroxyethyl)piperazin-1-yl)methyl)acrylamide;
N-(3-((5-chloro-4-((2-(dimethylphosphoryl)phenyl)amino)pyrimidin-2-yl)amino)-4-methoxyphenyl)-2-((3-hydroxypyrrolidin-1-yl)methyl)acrylamide;
N-(3-((5-chloro-4-((2-(dimethylphosphoryl)phenyl)amino)pyrimidin-2-yl)amino)-4-fluorophenyl)acrylamide;
N-(5-((5-chloro-4-((2-(dimethylphosphoryl)phenyl)amino)pyrimidin-2-yl)amino)-4-methoxy-2-((4-methylpiperazin-1-yl)methyl)phenyl)acrylamide;
N-(5-((5-chloro-4-((2-(dimethylphosphoryl)phenyl)amino)pyrimidin-2-yl)amino)-4-methoxy-2-(4-methylpiperazine-1-carbonyl)phenyl)acrylamide;
N-(2-(3-aminopyrrolidin-1-yl)-5-((5-chloro-4-((2-(dimethylphosphoryl)phenyl)amino)pyrimidin-2-yl)amino)-4-methoxyphenyl)acrylamide;
N1-(3-((5-chloro-4-((2-(dimethylphosphoryl)phenyl)amino)pyrimidin-2-yl)amino)-4-methoxyphenyl)-N4-(2-(dimethylamino)ethyl)fumaramide;
(E)-N-(3-((5-chloro-4-((2-(dimethylphosphoryl)phenyl)amino)pyrimidin-2-yl)amino)-4-methoxyphenyl)-4-(4-methylpiperazin-1-yl)-4-oxobut-2-enamide;
N1-(3-((5-chloro-4-((2-(dimethylphosphoryl)phenyl)amino)pyrimidin-2-yl)amino)-4-methoxyphenyl)-N4,N4-dimethylfumaramide;
(Z)-4-((3-((5-chloro-4-((2-(dimethylphosphoryl)phenyl)amino)pyrimidin-2-yl)amino)-4-methoxyphenyl)amino)-4-oxobut-2-enoic acid;
N1-(3-((5-chloro-4-((2-(dimethylphosphoryl)phenyl)amino)pyrimidin-2-yl)amino)-4-methoxyphenyl)-N4,N4-dimethylmaleamide;
(Z)-N-(3-((5-chloro-4-((2-(dimethylphosphoryl)phenyl)amino)pyrimidin-2-yl)amino)-4-methoxyphenyl)-4-(4-methylpiperazin-1-yl)-4-oxobut-2-enamide;
N1-(3-((5-chloro-4-((2-(dimethylphosphoryl)phenyl)amino)pyrimidin-2-yl)amino)-4-methoxyphenyl)-N4-(2-(dimethylamino)ethyl)maleamide;
N-(2-(3-amino-3-methylbut-1-yn-1-yl)-5-((5-chloro-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-2-(2-(dimethylamino)-3-hydroxypropoxy)-4-methoxyphenyl)acrylamide;
N-(5-((5-chloro-4-((2-(dimethylphosphoryl)phenyl)amino)pyrimidin-2-yl)amino)-2-((2-((2-fluoroethyl)(methyl)amino)ethyl)(methyl)amino)-4-methoxyphenyl)acrylamide;
N-(2-(3-(azetidin-1-yl)pyrrolidin-1-yl)-5-((5-chloro-4-((2-(dimethylphosphoryl)phenyl)amino)pyrimidin-2-yl)amino)-4-methoxyphenyl)acrylamide;
N-(2-([1,4'-bipiperidin]-1'-yl)-5-((5-chloro-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-4-methoxy-2-(4-morpholinopiperidin-1-yl)phenyl)acrylamide;
N-(5-((5-chloro-4-((2-(dimethylphosphoryl)phenyl)amino)pyrimidin-2-yl)amino)-4-methoxy-2-(4-(4-methyl-1,4-diazepan-1-yl)piperidin-1-yl)phenyl)acrylamide;
N-(5-((5-chloro-4-((2-(dimethylphosphoryl)phenyl)amino)pyrimidin-2-yl)amino)-4-methoxy-2-(4-(pyrrolidin-1-yl)piperidin-1-yl)phenyl)acrylamide;
N-(5-((5-chloro-4-((2-(dimethylphosphoryl)phenyl)amino)pyrimidin-2-yl)amino)-2-((1-(dimethylamino)cyclopropyl)ethynyl)-4-methoxyphenyl)acrylamide;
N-(5-((5-chloro-4-((2-(dimethylphosphoryl)phenyl)amino)pyrimidin-2-yl)amino)-4-methoxy-2-(2-(4-methylpiperazin-1-yl)ethyl)phenyl)acrylamide;
(E)-ethyl4-((3-((5-chloro-4-((2-(dimethylphosphoryl)phenyl)amino)pyrimidin-2-yl)amino)-4-methoxyphenyl)amino)-4-oxobut-2-enoate;
(E)-tert-butyl(1-(3-((3-((5-chloro-4-((2-(dimethylphosphoryl)phenyl)amino)pyrimidin-2-yl)amino)-4-methoxyphenyl)amino)-3-oxoprop-1-en-1-yl)cyclopropyl)carbamate;
N-(3-((5-chloro-4-((2-(dimethylphosphoryl)phenyl)amino)pyrimidin-2-yl)amino)-4-methoxyphenyl)-2-fluoroacrylamide;
(E)-3-chloro-N-(3-((5-chloro-4-((2-(dimethylphosphoryl)phenyl)amino)pyrimidin-2-yl)amino)-4-methoxyphenyl)acrylamide;
N-(3-((5-chloro-4-((2-(dimethylphosphoryl)phenyl)amino)pyrimidin-2-yl)amino)-4-methoxyphenyl)cyclohex-1-enecarboxamide;
N-(5-((5-chloro-4-((2-(dimethylphosphoryl)phenyl)amino)pyrimidin-2-yl)amino)-4-methoxy-2-(2-(pyrrolidin-1-yl)ethoxy)phenyl)but-2-ynamide;
N-(3-((5-chloro-4-((2-(dimethylphosphoryl)phenyl)amino)pyrimidin-2-yl)amino)-4-methoxyphenyl)-4-(dimethylamino)but-2-ynamide;
N-(3-((5-chloro-4-((2-(dimethylphosphoryl)phenyl)amino)pyrimidin-2-yl)amino)-4-methoxyphenyl)-2-(trifluoromethyl)acrylamide;
N-(3-((5-chloro-4-((2-(dimethylphosphoryl)phenyl)amino)pyrimidin-2-yl)amino)-4-methoxyphenyl)-1-methyl-1,2,5,6-tetrahydropyridine-3-carboxamide;
(E)-3-chloro-N-(5-((5-chloro-4-((2-(dimethylphosphoryl)phenyl)amino)pyrimidin-2-yl)amino)-2-fluoro-4-methoxyphenyl)acrylamide;
N-(3-((5-chloro-4-((2-(dimethylphosphoryl)-4-(2-methoxyethoxy)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-2-fluoro-4-methoxyphenyl)-4-(dimethylamino)but-2-ynamide;
N-(5-((5-chloro-4-((2-(dimethylphosphoryl)phenyl)amino)pyrimidin-2-yl)amino)-2-fluoro-4-methoxyphenyl)-5-(piperidin-1-yl)pent-2-ynamide;
N-(5-((5-chloro-4-((2-(dimethylphosphoryl)phenyl)amino)pyrimidin-2-yl)amino)-2-fluoro-4-methoxyphenyl)-4-(pyrrolidin-1-yl)but-2-ynamide;
N-(5-((5-chloro-4-((2-(dimethylphosphoryl)phenyl)amino)pyrimidin-2-yl)amino)-2-fluoro-4-methoxyphenyl)-4-methyl-4-(pyrrolidin-1-yl)pent-2-ynamide;
N-(3-((5-chloro-4-((2-(dimethylphosphoryl)phenyl)amino)pyrimidin-2-yl)amino)-4-methoxyphenyl)-4-methyl-4-(pyrrolidin-1-yl)pent-2-ynamide;
(E)-N-(3-((5-chloro-4-((2-(dimethylphosphoryl)phenyl)amino)pyrimidin-2-yl)amino)-4-methoxyphenyl)-4-(piperidin-1-yl)but-2-enamide;
N-(3-((5-chloro-4-((2-(dimethylphosphoryl)phenyl)amino)pyrimidin-2-yl)amino)-4-methoxyphenyl)-3-phenylpropiolamide;
(E)-3-(1-aminocyclopropyl)-N-(3-((5-chloro-4-((2-(dimethylphosphoryl)phenyl)amino)pyrimidin-2-yl)amino)-4-methoxyphenyl)acrylamide;
(E)-N-(3-((5-chloro-4-((2-(dimethylphosphoryl)phenyl)amino)pyrimidin-2-yl)amino)-4-methoxyphenyl)-4-(pyrrolidin-1-yl)but-2-enamide;
N-(5-((5-chloro-4-((2-(dimethylphosphoryl)phenyl)amino)pyrimidin-2-yl)amino)-2-(3-(diethylamino)prop-1-yn-1-yl)-4-methoxyphenyl)acrylamide;
N-(3-((5-chloro-4-((2-(dimethylphosphoryl)phenyl)amino)pyrimidin-2-yl)amino)-4-methoxyphenyl)-4-methoxybut-2-ynamide;
N-(5-((5-chloro-4-((2-(dimethylphosphoryl)-4-(2-(pyrrolidin-1-yl)ethoxy)phenyl)amino)pyrimidin-2-yl)amino)-2-fluoro-4-methoxyphenyl)acrylamide;
N-(3-((5-chloro-4-((2-(dimethylphosphoryl)-4-((tetrahydrofuran-3-yl)oxy)phenyl)amino)pyrimidin-2-yl)amino)-4-methoxyphenyl)acrylamide;
N-(3-((5-chloro-4-((2-(dimethylphosphoryl)-4-((tetrahydro-2H-pyran-4-yl)methoxy)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-4-methoxy-2-morphollnophenyl)-5-(piperidin-1-yl)pent-2-ynamide;
N-(5-((5-chloro-4-((2-(dimethylphosphoryl)phenyl)amino)pyrimidin-2-yl)amino)-4-methoxy-2-((tetrahydro-2H-pyran-4-yl)methoxy)phenyl)but-2-ynamide;
N-(5-((5-chloro-4-((2-(dimethylphosphoryl)phenyl)amino)pyrimidin-2-yl)amino)-4-methoxy-2-(oxetan-3-yloxy)phenyl)but-2-ynamide;
N-(5-((5-chloro-4-((2-(dimethylphosphoryl)phenyl)amino)pyrimidin-2-yl)amino)-4-methoxy-2-morpholinophenyl)-4-(dimethylamino)but-2-ynamide;
N-(5-((5-chloro-4-((2-(dimethylphosphoryl)phenyl)amino)pyrimidin-2-yl)amino)-4-methoxy-2-morpholinophenyl)-4-methoxybut-2-ynamide;
N-(5-((5-chloro-4-((2-(dimethylphosphoryl)phenyl)amino)pyrimidin-2-yl)amino)-4-methoxy-2-(4-(4-methylpiperazin-1-yl)piperidin-1-yl)phenyl)but-2-ynamide;
N-(3-((5-chloro-4-((2-(dimethylphosphoryl)-4-(2-(piperidin-1-yl)ethoxy)phenyl)amino)pyrimidin-2-yl)amino)-4-methoxyphenyl)acrylamide;
N1-(3-((5-chloro-4-((2-(dimethylphosphoryl)phenyl)amino)pyrimidin-2-yl)amino)-4-methoxyphenyl)-N4-methylfumaramide;
N1-(3-((5-chloro-4-((2-(dimethylphosphoryl)phenyl)amino)pyrimidin-2-yl)amino)-4-methoxyphenyl)-N4-methylmaleamide;
N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)-4-methoxybut-2-ynamide;
N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)but-2-ynamide;
rac-(R)-N-(5-((5-chloro-4-((2-(dimethylphosphoryl)phenyl)amino)pyrimidin-2-yl)amino)-2-(3-(dimethylamino)pyrrolidin-1-yl)-4-methoxyphenyl)methacrylamide;
N-(5-((5-chloro-4-((2-(dimethylphosphoryl)phenyl)amino)pyrimidin-2-yl)amino)-4-methoxy-2-morpholinophenyl)-5-(dimethylamino)pent-2-ynamide;
N-(5-((5-chloro-4-((2-(dimethylphosphoryl)phenyl)amino)pyrimidin-2-yl)amino)-4-methoxy-2-morpholinophenyl)-5-methoxypent-2-ynamide;
N-(5-((5-chloro-4-((2-(dimethylphosphoryl)phenyl)amino)pyrimidin-2-yl)amino)-4-methoxy-2-(3-methyl-3-(pyrrolidin-1-yl)but-1-yn-1-yl)phenyl)acrylamide;
N-(3-((5-chloro-4-((2-(dimethylphosphoryl)phenyl)amino)pyrimidin-2-yl)amino)-4-(2-(dimethylamino)ethoxy)phenyl)acrylamide;
N-(5-((5-chloro-4-((2-(dimethylphosphoryl)phenyl)amino)pyrimidin-2-yl)amino)-4-methoxy-2-morpholinophenyl)-5-(4-methylpiperazin-1-yl)pent-2-ynamide;
N-(5-((5-chloro-4-((2-(dimethylphosphoryl)phenyl)amino)pyrimidin-2-yl)amino)-4-methoxy-2-(pyrrolidin-1-yl)phenyl)-5-(4-methylpiperazin-1-yl)pent-2-ynamide;
N-(5-((5-cyclopropyl-4-((2-(dimethylphosphoryl)phenyl)amino)pyrimidin-2-yl)amino)-2-((2-(diethylamino)ethyl)(methyl)amino)-4-methoxyphenyl)acrylamide;
N-(5-((5-chloro-4-((2-(dimethylphosphoryl)phenyl)amino)pyrimidin-2-yl)amino)-2-((1-(diethylamino)-3-fluoropropan-2-yl)oxy)-4-methoxyphenyl)acrylamide;
N-(5-((5-chloro-4-((2-(dimethylphosphoryl)phenyl)amino)pyrimidin-2-yl)amino)-4-methoxy-2-morpholinophenyl)-5-(2-(methoxymethyl)pyrrolidin-1-yl)pent-2-ynamide;
N-(5-((5-chloro-4-((2-(dimethylphosphoryl)phenyl)amino)pyrimidin-2-yl)amino)-4-methoxy-2-((1-(2-methoxyethyl)piperidin-3-yl)oxy)phenyl)acrylamide;
N-(5-((5-chloro-4-((2-(dimethylphosphoryl)phenyl)amino)pyrimidin-2-yl)amino)-2-(3-(dimethylamino)prop-1-yn-1-yl)-4-methoxyphenyl)acrylamide;
N-(3-((5-chloro-4-((4-(2-(dimethylamino)ethoxy)-2-(dimethylphosphoryl)phenyl)amino)pyrimidin-2-yl)amino)-4-methoxyphenyl)acrylamide;
N-(3-((5-chloro-4-((4-(3-(dimethylamino)propoxy)-2-(dimethylphosphoryl)phenyl)amino)pyrimidin-2-yl)amino)-4-methoxyphenyl)acrylamide;
1-(6-((5-chloro-4-((2-(dimethylphosphoryl)phenyl)amino)pyrimidin-2-yl)amino)-5-methoxyindolin-1-yl)prop-2-en-1-one;
N-(5-((5-chloro-4-((2-(dimethylphosphoryl)phenyl)amino)pyrimidin-2-yl)amino)-2-(4-(dimethylamino)but-1-yn-1-yl)-4-methoxyphenyl)acrylamide;
(R)-N-(5-((5-chloro-4-((2-(dimethylphosphoryl)phenyl)amino)pyrimidin-2-yl)amino)-2-((1-(dimethylamino)-3-methoxypropan-2-yl)oxy)-4-methoxyphenyl)acrylamide;
N-(5-((5-chloro-4-((2-(dimethylphosphoryl)phenyl)amino)pyrimidin-2-yl)amino)-2-((1-(dimethylamino)-3-methoxypropan-2-yl)oxy)-4-methoxyphenyl)acrylamide;
N-(3-((4-((2-(dimethylphosphoryl)phenyl)amino)-5-methoxypyrimidin-2-yl)amino)-4-methoxyphenyl)acrylamide;
2-chloro-N-(3-((5-chloro-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-4-methoxy-2-methylphenyl)acrylamide;
N-(3-((5-chloro-4-((2-(dimethylphosphoryl)phenyl)amino)pyrimidin-2-yl)amino)-4-(trifluoromethoxy)phenyl)acrylamide;
N-(3-((5-chloro-4-((2-(dimethylphosphoryl)phenyl)amino)pyrimidin-2-yl)amino)-4-ethylphenyl)acrylamide;
N-(4-chloro-3-((5-chloro-4-((2-(dimethylphosphoryl)phenyl)amino)pyrimidin-2-yl)amino)phenyl)acrylamide;
N-(3-((5-chloro-4-((2-(dimethylphosphoryl)phenyl)amino)pyrimidin-2-yl)amino)-4-(oxetan-3-yloxy)phenyl)acrylamide;
N-(3-((5-cyano-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-2-((1-(dimethylamino)-3-ethoxypropan-2-yl)oxy)-4-methoxyphenyl)acrylamide;
N-(5-((5-chloro-4-((2-(dimethylphosphoryl)phenyl)amino)pyrimidin-2-yl)amino)-2-((1-(dimethylamino)-3-methoxypropan-2-yl)oxy)-4-methylphenyl)acrylamide;
N-(5-((5-chloro-4-((2-(dimethylphosphoryl)phenyl)amino)pyrimidin-2-yl)amino)-2-((1-(dimethylamino)-3-methoxypropan-2-yl)oxy)-4-methylphenyl)acrylamide;
N-(4-chloro-5-((5-chloro-4-((2-(dimethylphosphoryl)phenyl)amino)pyrimidin-2-yl)amino)-2-((1-(dimethylamino)-3-methoxypropan-2-yl)oxy)phenyl)acrylamide;
N-(4-chloro-5-((5-chloro-4-((2-(dimethylphosphoryl)phenyl)amino)pyrimidin-2-yl)amino)-2-((1-(dimethylamino)-3-methoxypropan-2-yl)oxy)phenyl)acrylamide;
N-(5-((5-chloro-4-((2-(dimethylphosphoryl)phenyl)amino)pyrimidin-2-yl)amino)-2-(3-(dimethylamino)prop-1-yn-1-yl)-4-methylphenyl)acrylamide;
N-(5-((5-chloro-4-((2-(dimethylphosphoryl)phenyl)amino)pyrimidin-2-yl)amino)-2-(3-(dimethylamino)prop-1-yn-1-yl)-4-ethylphenyl)acrylamide;
N-(5-((5-chloro-4-((2-(isopropylsulfonyl)phenyl)amino)pyrimidin-2-yl)amino)-2-(3-(dimethylamino)prop-1-yn-1-yl)-4-methoxyphenyl)acrylamide;
N-(5-((5-chloro-4-((2-(dimethylphosphoryl)phenyl)amino)pyrimidin-2-yl)amino)-2-(3-(diethylamino)-3-methylbut-1-yn-1-yl)-4-methoxyphenyl)acrylamide;
N-(5-((5-chloro-4-((2-(dimethylphosphoryl)phenyl)amino)pyrimidin-2-yl)amino)-2-(3-(dimethylamino)-3-methylbut-1-yn-1-yl)-4-methoxyphenyl)acrylamide;
N-(4-chloro-5-((5-chloro-4-((2-(dimethylphosphoryl)phenyl)amino)pyrimidin-2-yl)amino)-2-(3-(dimethylamino)prop-1-yn-1-yl)phenyl)acrylamide;
N-(5-((5-chloro-4-((2-(dimethylphosphoryl)phenyl)amino)pyrimidin-2-yl)amino)-4-methoxy-2-(3-(pyrrolidin-1-yl)prop-1-yn-1-yl)phenyl)acrylamide;
N-(5-((5-chloro-4-((2-(dimethylphosphoryl)phenyl)amino)pyrimidin-2-yl)amino)-2-(3-(diisopropylamino)prop-1-yn-1-yl)-4-methoxyphenyl)acrylamide;
N-(5-((5-chloro-4-((2-(dimethylphosphoryl)phenyl)amino)pyrimidin-2-yl)amino)-4-methoxy-2-(3-(4-methylpiperazin-1-yl)prop-1-yn-1-yl)phenyl)acrylamide;
N-(5-((5-chloro-4-((2-(dimethylphosphoryl)phenyl)amino)pyrimidin-2-yl)amino)-4-methoxy-2-(3-(methylamino)prop-1-yn-1-yl)phenyl)acrylamide;
N-(5-((5-chloro-4-((2-(dimethylphosphoryl)phenyl)amino)pyrimidin-2-yl)amino)-4-methoxy-2-(3-((2-methoxyethyl)(methyl)amino)prop-1-yn-1-yl)phenyl)acrylamide;
N-(5-((5-chloro-4-((2-(dimethylphosphoryl)phenyl)amino)pyrimidin-2-yl)amino)-2-(3-(ethyl(methyl)amino)prop-1-yn-1-yl)-4-methoxyphenyl)acrylamide;
N-(3-((6-((3-(dimethylphosphoryl)pyridin-2-yl)amino)pyrimidin-4-yl)amino)-4-methoxyphenyl)acrylamide;
N-(3-((5-chloro-4-((4-(dimethylphosphoryl)pyridin-3-yl)amino)pyrimidin-2-yl)amino)-4-methoxyphenyl)acrylamlde;
N-(2-(3-(azepan-1-yl)prop-1-yn-1-yl)-5-((5-chloro-4-((2-(dimethylphosphoryl)phenyl)amino)pyrimidin-2-yl)amino)-4-methoxyphenyl)acrylamide;
N-(5-((5-chloro-4-((2-(dimethylphosphoryl)phenyl)amino)pyrimidin-2-yl)amino)-2-(3-(isopropyl(methyl)amino)prop-1-yn-1-yl)-4-methoxyphenyl)acrylamide;
N-(5-((5-chloro-4-((2-(dimethylphosphoryl)phenyl)amino)pyrimidin-2-yl)amino)-4-methoxy-2-(3-(piperidin-1-yl)prop-1-yn-1-yl)phenyl)acrylamide;
N-(5-((5-chloro-4-((2-(dimethylphosphoryl)phenyl)amino)pyrimidin-2-yl)amino)-2-(3-(cyclopentyl(methyl)amino)prop-1-yn-1-yl)-4-methoxyphenyl)acrylamide;
N-(3-((4-((2-(dimethylphosphoryl)phenyl)amino)-1,3,5-triazin-2-yl)amino)-4-methoxyphenyl)acrylamide;
N-(3-((4-((2-(dimethylphosphoryl)phenyl)amino)-1,3,5-triazin-2-yl)amino)-4-(trifluoromethoxy)phenyl)acrylamide;
N-(3-((6-((2-(dimethylphosphoryl)phenyl)amino)pyrimidin-4-yl)amino)-4-methoxyphenyl)acrylamide;
(1S,2R,3S,4R)-3-((2-((5-acrylamido-2-methoxyphenyl)amino)-5-chloropyrimidin-4-yl)amino)bicyclo[2.2.1]heptane-2-carboxamide;
2-((2-((5-acrylamido-4-(3-(dimethylamino)pyrrolidin-1-yl)-2-methoxyphenyl)amino)-5-chloropyrimidin-4-yl)amino)benzamide;
N-(5-((5-chloro-4-((2-cyanophenyl)amino)pyrimidin-2-yl)amino)-2-(3-(dimethylamino)pyrrolidin-1-yl)-4-methoxyphenyl)acrylamide; and
N-(5-((5-chloro-4-((2-(isopropylsulfonyl)phenyl)amino)pyrimidin-2-yl)amino)-2-(3-(dimethylamino)pyrrolidin-1-yl)-4-methoxyphenyl)acrylamide;
or a pharmaceutically acceptable salt thereof.

2. The compound of claim 1 or a pharmaceutically acceptable salt thereof, for use in the treatment of an EGFR-driven cancer in a subject.

3. The compound for use of claim 2 said subject having an EGFR-driven cancer **characterized by** the presence of a mutation in epidermal growth factor receptor kinase (EGFR).

4. The compound for use of claim 3, wherein said EGFR-driven cancer is **characterized by** the presence of one or more mutations selected from: (i) L858R, (ii) T790M, (iii) both L858R and T790M, (iv) delE746_A750, and (v) both delE746_A750 and T790M.

5. The compound for use of any one of claims 2-4, wherein said EGFR-driven cancer is a non-small cell lung cancer (NSCLS); glioblastoma; pancreatic cancer; head and neck cancer (e.g., squamous cell carcinoma); breast cancer; colorectal cancer; epithelial cancer; ovarian cancer; prostate cancer; or an adenocarcinoma.

6. The compound of claim 1 for use in the treatment of an EGFR-driven cancer refractory to a first kinase inhibitor selected from erlotinib, gefitinib, and pharmaceutically acceptable salts thereof, in a subject.

7. A pharmaceutical composition comprising a compound of claim 1 or a pharmaceutically acceptable salt thereof.

## Patentansprüche

1. Verbindung, ausgewählt aus der Gruppe bestehend aus:
N-(5-((5-Chlor-4-((2-(dimethylphosphoryl)phenyl)amino)pyrimidin-2-yl)amino)-4-methoxy-2-(2-(pyrrolidin-1-yl)ethoxy)phenyl)acrylamid;
N-(5-((5-Chlor-4-((2-(dimethylphosphoryl)phenyl)amino)pyrimidin-2-yl)amino)-4-methoxy-2-(2-(4-methylpiperazin-1-yl)ethoxy)phenyl)acrylamid;
N-(5-((5-Chlor-4-((2-(dimethylphosphoryl)phenyl)amino)pyrimidin-2-yl)amino)-4-methoxy-2-((1-methylpiperidin-4-yl)methoxy)phenyl)acrylamid;
N-(5-((5-Chlor-4-((2-(dimethylphosphoryl)phenyl)amino)pyrimidin-2-yl)amino)-4-methoxy-2-(2-morpholinoethoxy)phenyl)acrylamid;
N-(5-((5-Chlor-4-((2-(dimethylphosphoryl)phenyl)amino)pyrimidin-2-yl)amino)-4-methoxy-2-(3-morpholinopropoxy)phenyl)acrylamid;
N-(5-((5-Chlor-4-((2-(dimethylphosphoryl)phenyl)amino)pyrimidin-2-yl)amino)-4-methoxy-2-((tetrahydro-2H-pyran-4-yl)methoxy)phenyl)acrylamid;
N-(5-((5-Chlor-4-((2-(dimethylphosphoryl)phenyl)amino)pyrimidin-2-yl)amino)-4-methoxy-2-((1-methylpiperidin-2-yl)methoxy)phenyl)acrylamid;
N-(5-((5-Chlor-4-((2-(dimethylphosphoryl)phenyl)amino)pyrimidin-2-yl)amino)-4-methoxy-2-(2-(piperidin-1-yl)ethoxy)phenyl)acrylamid;
N-(5-((5-Chlor-4-((2-(dimethylphosphoryl)phenyl)amino)pyrimidin-2-yl)amino)-4-methoxy-2-(oxetan-3-yloxy)phenyl)acrylamid;
N-(5-((5-Chlor-4-((2-(dimethylphosphoryl)phenyl)amino)pyrimidin-2-yl)amino)-4-methoxy-2-(pyridin-3-ylmethoxy)phenyl)acrylamid;
N-(5-((5-Chlor-4-((2-(dimethylphosphoryl)phenyl)amino)pyrimidin-2-yl)amino)-4-methoxy-2-(pyridin-4-ylmethoxy)phenyl)acrylamid;
N-(5-((5-Chlor-4-((2-(dimethylphosphoryl)phenyl)amino)pyrimidin-2-yl)amino)-4-methoxy-2-((1-methylpiperidin-3-yl)oxy)phenyl)acrylamid;
N-(5-((5-Chlor-4-((2-(dimethylphosphoryl)phenyl)amino)pyrimidin-2-yl)amino)-4-methoxy-2-((1-methylpyrrolidin-3-yl)oxy)phenyl)acrylamid;
rel-(R)-N-(5-((5-Chlor-4-((2-(dimethylphosphoryl)phenyl)amino)pyrimidin-2-yl)amino)-4-methoxy-2-((1-methylpiperidin-3-yl)oxy)phenyl)acrylamid;
N-(2-((1-(Azetidin-1-yl)-3-methoxypropan-2-yl)oxy)-5-((5-chlor-4-((2-(dimethylphosphoryl)phenyl)amino)pyrimidin-2-yl)amino)-4-methoxyphenyl)acrylamid;
rel-(R)-N-(5-((5-chlor-4-((2-(dimethylphosphoryl)phenyl)amino)pyrimidin-2-yl)amino)-4-methoxy-2-((1-methylpiperidin-3-yl)oxy)phenyl)acrylamid;
N-(5-((5-Chlor-4-((2-(dimethylphosphoryl)phenyl)amino)pyrimidin-2-yl)amino)-4-methoxy-2-((1-methylazetidin-3-yl)oxy)phenyl)acrylamid;
N-(5-((5-Chlor-4-((2-(dimethylphosphoryl)phenyl)amino)pyrimidin-2-yl)amino)-4-methoxy-2-((1-methylpyrrolidin-2-yl)methoxy)phenyl)acrylamid;
N-(5-((5-Chlor-4-((2-(dimethylphosphoryl)phenyl)amino)pyrimidin-2-yl)amino)-4-methoxy-2-((4-methyl-1,4-oxazepan-6-yl)oxy)phenyl)acrylamid;
N-(5-((5-Chlor-4-((2-(dimethylphosphoryl)phenyl)amino)pyrimidin-2-yl)amino)-4-methoxy-2-((1-methylpyrrolidin-3-yl)methoxy)phenyl)acrylamid;
N-(5-((5-Chlor-4-((2-(dimethylphosphoryl)phenyl)amino)pyrimidin-2-yl)amino)-2-((2-(dimethylamino)cyclohexyl)oxy)-4-methoxyphenyl)acrylamid;
N-(5-((5-Chlor-4-((2-(dimethylphosphoryl)phenyl)amino)pyrimidin-2-yl)amino)-4-methoxy-2-(4-(4-methylpiperazin-1-yl)piperidin-1-yl)phenyl)acrylamid;
N-(5-((5-Chlor-4-((2-(dimethylphosphoryl)phenyl)amino)pyrimidin-2-yl)amino)-2-(4-(dimethylamino)piperidin-1-yl)-4-methoxyphenyl)acrylamid;
N-(5-((5-Chlor-4-((2-(dimethylphosphoryl)phenyl)amino)pyrimidin-2-yl)amino)-2-(3-(dimethylamino)pyrrolidin-1-yl)-4-methoxyphenyl)acrylamid;
N-(5-((5-Chlor-4-((2-(dimethylphosphoryl)phenyl)amino)pyrimidin-2-yl)amino)-4-methoxy-2-(4-methyl-1,4-diazepan-1-yl)phenyl)acrylamid;
rac-(R)-N-(5-((5-Chlor-4-((2-(dimethylphosphoryl)phenyl)amino)pyrimidin-2-yl)amino)-2-(3-(dimethylamino)pyrrolidin-1-yl)-4-methoxyphenyl)acrylamid;
N-(5-((5-Chlor-4-((2-(dimethylphosphoryl)phenyl)amino)pyrimidin-2-yl)amino)-4-methoxy-2-(4-(1-methylpiperidin-4-yl)piperazin-1-yl)phenyl)acrylamid;
N-(5-((5-Chlor-4-((2-(dimethylphosphoryl)phenyl)amino)pyrimidin-2-yl)amino)-4-methoxy-2-(3-morpholinopyrrolidin-1-yl)phenyl)acrylamid;
rac-(R)-N-(5-((5-Chlor-4-((2-(dimethylphosphoryl)phenyl)amino)pyrimidin-2-yl)amino)-2-(3-(dimethylamino)pyrrolidin-1-yl)-4-methoxyphenyl)acrylamid;
N-(5-((5-Chlor-4-((2-(dimethylphosphoryl)phenyl)amino)pyrimidin-2-yl)amino)-2-(3-(dimethylamino)piperidin-1-yl)-4-methoxyphenyl)acrylamid;
N-(5-((5-Chlor-4-((2-(dimethylphosphoryl)phenyl)amino)pyrimidin-2-yl)amino)-4-methoxy-2-(3-(pyrrolidin-1-yl)azetidin-1-yl)phenyl)acrylamid;
N-(5-((5-Chlor-4-((2-(dimethylphosphoryl)phenyl)amino)pyrimidin-2-yl)amino)-2-(3-hydroxypyrrolidin-1-yl)-4-methoxyphenyl)acrylamid;
N-(5-((5-Chlor-4-((2-(dimethylphosphoryl)phenyl)amino)pyrimidin-2-yl)amino)-4-methoxy-2-(3-(piperidin-1-yl)pyrrolidin-1-yl)phenyl)acrylamid;
N-(5-((5-Chlor-4-((2-(dimethylphosphoryl)phenyl)amino)pyrimidin-2-yl)amino)-2-(3-(diethylamino)pyrrolidin-1-yl)-4-methoxyphenyl)acrylamid;
N-(5-((5-Chlor-4-((2-(dimethylphosphoryl)phenyl)amino)pyrimidin-2-yl)amino)-2-(3-hydroxy-[1,3'-bipyrrolidin]-1'-yl)-4-methoxyphenyl)acrylamid;
N-(2-([1,3'-Bipyrrolidin]-1'-yl)-5-((5-chlor-4-((2-(dimethylphosphoryl)phenyl)amino)pyrimidin-2-yl)amino)-4-methoxyphenyl)acrylamid;
N-(5-((5-Chlor-4-((2-(dimethylphosphoryl)phenyl)amino)pyrimidin-2-yl)amino)-4-methoxy-2-(3-(4-methylpiperazin-1-yl)pyrrolidin-1-yl)phenyl)acrylamid;
N-(5-((5-Chlor-4-((2-(dimethylphosphoryl)phenyl)amino)pyrimidin-2-yl)amino)-2-(3-((2-
(diethylamino)ethyl)(methypamino)pyrrolidin-1-yl)-4-methoxyphenyl)acrylamid;
N-(5-((5-Chlor-4-((2-(dimethylphosphoryl)phenyl)amino)pyrimidin-2-yl)amino)-2-(3-(4-hydroxypiperidin-1-yl)pyrrolidin-1-yl)-4-methoxyphenyl)acrylamid;
N-(5-((5-Chlor-4-((2-(dimethylphosphoryl)phenyl)amino)pyrimidin-2-yl)amino)-2-(3-(4-(2-hydroxyethyl)piperazin-1-yl)pyrrolidin-1-yl)-4-methoxyphenyl)acrylamid;
N-(5-((5-Chlor-4-((2-(dimethylphosphoryl)phenyl)amino)pyrimidin-2-yl)amino)-4-methoxy-2-(3-((2-methoxyethyl)(methyl)amino)pyrrolidin-1-yl)phenyl)acrylamid;
N-(3-((5-Chlor-4-((2-(dimethylphosphoryl)phenyl)amino)pyrimidin-2-yl)amino)-4-methoxyphenyl)-2-(pyrrolidin-1-ylmethyl)acrylamid;
N-(3-((5-Chlor-4-((2-(dimethylphosphoryl)phenyl)amino)pyrimidin-2-yl)amino)-4-methoxyphenyl)-2-(piperidin-1-ylmethyl)acrylamid;
N-(3-((5-Chlor-4-((2-(dimethylphosphoryl)phenyl)amino)pyrimidin-2-yl)amino)-4-methoxyphenyl)-2-((4-(methylsulfonyl)piperazin-1-yl)methyl)acrylamid;
N-(3-((5-Chlor-4-((2-(dimethylphosphoryl)phenyl)amino)pyrimidin-2-yl)amino)-4-methoxyphenyl)-2-((4-(4-methylpiperazin-1-yl)piperidin-1-yl)methyl)acrylamid;
N-(3-((5-Chlor-4-((2-(dimethylphosphoryl)phenyl)amino)pyrimidin-2-yl)amino)-4-methoxyphenyl)-2-((1,1-dioxidothiomorpholino)methyl)acrylamid;
N-(3-((5-Chlor-4-((2-(dimethylphosphoryl)phenyl)amino)pyrimidin-2-yl)amino)-4-methoxyphenyl)-2-((3-oxopiperazin-1-yl)methyl)acrylamid;
N-(3-((5-Chlor-4-((2-(dimethylphosphoryl)phenyl)amino)pyrimidin-2-yl)amino)-4-methoxyphenyl)-2-(morpholinomethyl)acrylamid;
N-(3-((5-Chlor-4-((2-(dimethylphosphoryl)phenyl)amino)pyrimidin-2-yl)amino)-4-methoxyphenyl)-2-((4-methylpiperazin-1-yl)methyl)acrylamid;
N-(3-((5-Chlor-4-((2-(dimethylphosphoryl)phenyl)amino)pyrimidin-2-yl)amino)-4-methoxyphenyl)-2-((4-isopropylpiperazin-1-yl)methyl)acrylamid;
N-(3-((5-Chlor-4-((2-(dimethylphosphoryl)phenyl)amino)pyrimidin-2-yl)amino)-4-methoxyphenyl)-2-((4-morpholinopiperidin-1-yl)methyl)acrylamid;
N-(3-((5-Chlor-4-((2-(dimethylphosphoryl)phenyl)amino)pyrimidin-2-yl)amino)-4-methoxyphenyl)-2-((4-(2-hydroxyethyl)piperazin-1-yl)methyl)acrylamid;
N-(3-((5-Chlor-4-((2-(dimethylphosphoryl)phenyl)amino)pyrimidin-2-yl)amino)-4-methoxyphenyl)-2-((3-hydroxypyrrolidin-1-yl)methyl)acrylamid;
N-(3-((5-Chlor-4-((2-(dimethylphosphoryl)phenyl)amino)pyrimidin-2-yl)amino)-4-fluorphenyl)acrylamid;
N-(5-((5-Chlor-4-((2-(dimethylphosphoryl)phenyl)amino)pyrimidin-2-yl)amino)-4-methoxy-2-((4-methylpiperazin-1-yl)methyl)phenyl)acrylamid;
N-(5-((5-Chlor-4-((2-(dimethylphosphoryl)phenyl)amino)pyrimidin-2-yl)amino)-4-methoxy-2-(4-methylpiperazine-1-carbonyl)phenyl)acrylamid;
N-(2-(3-Aminopyrrolidin-1-yl)-5-((5-chlor-4-((2-(dimethylphosphoryl)phenyl)amino)pyrimidin-2-yl)amino)-4-methoxyphenyl) acrylamid;
N1-(3-((5-Chlor-4-((2-(dimethylphosphoryl)phenyl)amino)pyrimidin-2-yl)amino)-4-methoxyphenyl)-N4-(2-(dimethylamino)ethyl)fumaramid;
(E)-N-(3-((5-Chlor-4-((2-(dimethylphosphoryl)phenyl)amino)pyrimidin-2-yl)amino)-4-methoxyphenyl)-4-(4-methylpiperazin-1-yl)-4-oxobut-2-enamid;
N1-(3-((5-Chlor-4-((2-(dimethylphosphoryl)phenyl)amino)pyrimidin-2-yl)amino)-4-methoxyphenyl)-N4,N4-dimethylfumaramid;
(Z)-4-((3-((5-Chlor-4-((2-(dimethylphosphoryl)phenyl)amino)pyrimidin-2-yl)amino)-4-methoxyphenyl) amino)-4-oxobut-2-ensäure;
N1-(3-((5-Chlor-4-((2-(dimethylphosphoryl)phenyl)amino)pyrimidin-2-yl)amino)-4-methoxyphenyl)-N4,N4-dimethylmaleamid;
(Z)-N-(3-((5-Chlor-4-((2-(dimethylphosphoryl)phenyl)amino)pyrimidin-2-yl)amino)-4-methoxyphenyl)-4-(4-methylpiperazin-1-yl)-4-oxobut-2-enamid;
N1-(3-((5-Chlor-4-((2-(dimethylphosphoryl)phenyl)amino)pyrimidin-2-yl)amino)-4-methoxyphenyl)-N4-(2-(dimethylamino)ethyl)maleamid;
N-(2-(3-Amino-3-methylbut-1-in-1-yl)-5-((5-chlor-4-((2-(dimethylphosphoryl)phenyl)amino)pyrimidin-2-yl) amino)-4-methoxyphenyl) acrylamid;
N-(5-((5-Chlor-4-((2-(dimethylphosphoryl)phenyl)amino)pyrimidin-2-yl)amino)-2-(2-(dimethylamino)-3-hydroxypropoxy)-4-methoxyphenyl)acrylamid;
N-(5-((5-Chlor-4-((2-(dimethylphosphoryl)phenyl)amino)pyrimidin-2-yl)amino)-2-((2-((2-fluorethyl)(methyl)amino)ethyl)(methyl)amino)-4-methoxyphenyl)acrylamid;
N-(2-(3-(Azetidin-1-yl)pyrrolidin-1-yl)-5-((5-chlor-4-((2-(dimethylphosphoryl)phenyl)amino)pyrimidin-2-yl)amino)-4-methoxyphenyl) acrylamid;
N-(2-([1,4'-Bipiperidin]-1'-yl)-5-((5-chlor-4-((2-(dimethylphosphoryl)phenyl)amino)pyrimidin-2-yl)amino)-4-methoxyphenyl) acrylamid;
N-(5-((5-Chlor-4-((2-(dimethylphosphoryl)phenyl)amino)pyrimidin-2-yl)amino)-4-methoxy-2-(4-morpholinopiperidin-1-yl)phenyl)acrylamid;
N-(5-((5-Chlor-4-((2-(dimethylphosphoryl)phenyl)amino)pyrimidin-2-yl)amino)-4-methoxy-2-(4-(4-methyl-1,4-diazepan-1-yl)piperidin-1-yl)phenyl)acrylamid;
N-(5-((5-Chlor-4-((2-(dimethylphosphoryl)phenyl)amino)pyrimidin-2-yl)amino)-4-methoxy-2-(4-(pyrrolidin-1-yl)piperidin-1-yl)phenyl)acrylamid;
N-(5-((5-Chlor-4-((2-(dimethylphosphoryl)phenyl)amino)pyrimidin-2-yl)amino)-2-((1-(dimethylamino)cyclopropyl)ethinyl)-4-methoxyphenyl)acrylamid;
N-(5-((5-Chlor-4-((2-(dimethylphosphoryl)phenyl)amino)pyrimidin-2-yl)amino)-4-methoxy-2-(2-(4-methylpiperazin-1-yl)ethyl)phenyl)acrylamid;
(E)-4-((3-((5-Chlor-4-((2-(dimethylphosphoryl)phenyl)amino)pyrimidin-2-yl)amino)-4-methoxyphenyl)amino)-4-oxobut-2-ensäureethylester;
(E)-(1-(3-((3-((5-Chlor-4-((2-(dimethylphosphoryl)phenyl)amino)pyrimidin-2-yl)amino)-4-methoxyphenyl)amino)-3-oxoprop-1-en-1-yl)cyclopropyl)carbamidsäure-tert-butylester;
N-(3-((5-Chlor-4-((2-(dimethylphosphoryl)phenyl)amino)pyrimidin-2-yl)amino)-4-methoxyphenyl)-2-fluoracrylamid;
(E)-3-Chlor-N-(3-((5-chlor-4-((2-(dimethylphosphoryl)phenyl)amino)pyrimidin-2-yl)amino)-4-methoxyphenyl)acrylamid;
N-(3-((5-Chlor-4-((2-(dimethylphosphoryl)phenyl)amino)pyrimidin-2-yl)amino)-4-methoxyphenyl)cyclohex-1-encarboxamid;
N-(5-((5-Chlor-4-((2-(dimethylphosphoryl)phenyl)amino)pyrimidin-2-yl)amino)-4-methoxy-2-(2-(pyrrolidin-1-yl)ethoxy)phenyl)but-2-inamid;
N-(3-((5-Chlor-4-((2-(dimethylphosphoryl)phenyl)amino)pyrimidin-2-yl)amino)-4-methoxyphenyl)-4-(dimethylamino)but-2-inamid;
N-(3-((5-Chlor-4-((2-(dimethylphosphoryl)phenyl)amino)pyrimidin-2-yl)amino)-4-methoxyphenyl)-2-(trifluormethyl)acrylamid;
N-(3-((5-Chlor-4-((2-(dimethylphosphoryl)phenyl)amino)pyrimidin-2-yl)amino)-4-methoxyphenyl)-1-methyl-1,2,5,6-tetrahydropyridin-3-carboxamid;
(E)-3-Chlor-N-(5-((5-chlor-4-((2-(dimethylphosphoryl)phenyl)amino)pyrimidin-2-yl)amino)-2-fluor-4-methoxyphenyl) acrylamid;
N-(3-((5-Chlor-4-((2-(dimethylphosphoryl)-4-(2-methoxyethoxy)phenyl)amino)pyrimidin-2-yl) amino)-4-methoxyphenyl)acrylamid;
N-(5-((5-Chlor-4-((2-(dimethylphosphoryl)phenyl)amino)pyrimidin-2-yl)amino)-2-fluor-4-methoxyphenyl)-4-(dimethylamino)but-2-inamid;
N-(5-((5-Chlor-4-((2-(dimethylphosphoryl)phenyl)amino)pyrimidin-2-yl)amino)-2-fluor-4-methoxyphenyl)-5-(piperidin-1-yl)pent-2-inamid;
N-(5-((5-Chlor-4-((2-(dimethylphosphoryl)phenyl)amino)pyrimidin-2-yl)amino)-2-fluor-4-methoxyphenyl)-4-(pyrrolidin-1-yl)but-2-inamid;
N-(5-((5-Chlor-4-((2-(dimethylphosphoryl)phenyl)amino)pyrimidin-2-yl)amino)-2-fluor-4-methoxyphenyl)-4-methyl-4-(pyrrolidin-1-yl)pent-2-inamid;
N-(3-((5-Chlor-4-((2-(dimethylphosphoryl)phenyl)amino)pyrimidin-2-yl)amino)-4-methoxyphenyl)-4-methyl-4-(pyrrolidin-1-yl)pent-2-inamid;
(E)-N-(3-((5-Chlor-4-((2-(dimethylphosphoryl)phenyl)amino)pyrimidin-2-yl)amino)-4-methoxyphenyl)-4-(piperidin-1-yl)but-2-enamid;
N-(3-((5-Chlor-4-((2-(dimethylphosphoryl)phenyl)amino)pyrimidin-2-yl)amino)-4-methoxyphenyl)-3-phenylpropiolamid;
(E)-3-(1-Aminocyclopropyl)-N-(3-((5-chlor-4-((2-(dimethylphosphoryl)phenyl)amino)pyrimidin-2-yl)amino)-4-methoxyphenyl) acrylamid;
(E)-N-(3-((5-Chlor-4-((2-(dimethylphosphoryl)phenyl)amino)pyrimidin-2-yl)amino)-4-methoxyphenyl)-4-(pyrrolidin-1-yl)but-2-enamid;
N-(5-((5-Chlor-4-((2-(dimethylphosphoryl)phenyl)amino)pyrimidin-2-yl)amino)-2-(3-(diethylamino)prop-1-in-1-yl)-4-methoxyphenyl)acrylamid;
N-(3-((5-Chlor-4-((2-(dimethylphosphoryl)phenyl)amino)pyrimidin-2-yl)amino)-4-methoxyphenyl)-4-methoxybut-2-inamid;
N-(5-((5-Chlor-4-((2-(dimethylphosphoryl)-4-(2-(pyrrolidin-1-yl)ethoxy)phenyl)amino)pyrimidin-2-yl)amino)-2-fluor-4-methoxyphenyl) acrylamid;
N-(3-((5-Chlor-4-((2-(dimethylphosphoryl)-4-((tetrahydrofuran-3-yl)oxy)phenyl)amino)pyrimidin-2-yl)amino)-4-methoxyphenyl)acrylamid;
N-(3-((5-Chlor-4-((2-(dimethylphosphoryl)-4-((tetrahydro-2H-pyran-4-yl)methoxy)phenyl)amino)pyrimidin-2-yl)amino)-4-methoxyphenyl)acrylamid;
N-(5-((5-Chlor-4-((2-(dimethylphosphoryl)phenyl)amino)pyrimidin-2-yl)amino)-4-methoxy-2-morpholinophenyl)-5-(piperidin-1-yl)pent-2-inamid;
N-(5-((5-Chlor-4-((2-(dimethylphosphoryl)phenyl)amino)pyrimidin-2-yl)amino)-4-methoxy-2-((tetrahydro-2H-pyran-4-yl)methoxy)phenyl)but-2-inamid;
N-(5-((5-Chlor-4-((2-(dimethylphosphoryl)phenyl)amino)pyrimidin-2-yl)amino)-4-methoxy-2-(oxetan-3-yloxy)phenyl)but-2-inamid;
N-(5-((5-Chlor-4-((2-(dimethylphosphoryl)phenyl)amino)pyrimidin-2-yl)amino)-4-methoxy-2-morpholinophenyl)-4-(dimethylamino)but-2-inamid;
N-(5-((5-Chlor-4-((2-(dimethylphosphoryl)phenyl)amino)pyrimidin-2-yl)amino)-4-methoxy-2-morpholinophenyl)-4-methoxybut-2-inamid;
N-(5-((5-Chlor-4-((2-(dimethylphosphoryl)phenyl)amino)pyrimidin-2-yl)amino)-4-methoxy-2-(4-(4-methylpiperazin-1-yl)piperidin-1-yl)phenyl)but-2-inamid;
N-(3-((5-Chlor-4-((2-(dimethylphosphoryl)-4-(2-(piperidin-1yl)ethoxy)phenyl)amino)pyrimidin-2-yl)amino)-4-methoxyphenyl)acrylamid;
N1-(3-((5-Chlor-4-((2-(dimethylphosphoryl)phenyl)amino)pyrimidin-2-yl)amino)-4-methoxyphenyl)-N4-methylfumaramid;
N1-(3-((5-Chlor-4-((2-(dimethylphosphoryl)phenyl)amino)pyrimidin-2-yl)amino)-4-methoxyphenyl)-N4-methylmaleamid;
N-(5-((5-Chlor-4-((2-(dimethylphosphoryl)phenyl)amino)pyrimidin-2-yl)amino)-2-(3(dimethylamino)pyrrolidin-1-yl)-4-methoxyphenyl)-4-methoxybut-2-inamid;
N-(5-((5-Chlor-4-((2-(dimethylphosphoryl)phenyl)amino)pyrimidin-2-yl)amino)-2-(3-(dimethylamino)pyrrolidin-1-yl)-4-methoxyphenyl)but-2-inamid;
rac-(R)-N-(5-((5-Chlor-4-((2-(dimethylphosphoryl)phenyl)amino)pyrimidin-2-yl)amino)-2-(3-(dimethylamino)pyrrolidin-1-yl)-4-methoxyphenyl)methacrylamid;
N-(5-((5-Chlor-4-((2-(dimethylphosphoryl)phenyl)amino)pyrimidin-2-yl)amino)-4-methoxy-2-morpholinophenyl)-5-(dimethylamino)pent-2-inamid;
N-(5-((5-Chlor-4-((2-(dimethylphosphoryl)phenyl)amino)pyrimidin-2-yl)amino)-4-methoxy-2-morpholinophenyl)-5-methoxypent-2-inamid;
N-(5-((5-Chlor-4-((2-(dimethylphosphoryl)phenyl)amino)pyrimidin-2-yl)amino)-4-methoxy-2-(3-methyl-3-(pyrrolidin-1-yl)but-1-in-1-yl)phenyl)acrylamid;
N-(3-((5-Chlor-4-((2-(dimethylphosphoryl)phenyl)amino)pyrimidin-2-yl)amino)-4-(2-(dimethylamino)ethoxy)phenyl)acrylamid;
N-(5-((5-Chlor-4-((2-(dimethylphosphoryl)phenyl)amino)pyrimidin-2-yl)amino)-4-methoxy-2-morpholinophenyl)-5-(4-methylpiperazin-1-yl)pent-2-inamid;
N-(5-((5-Chlor-4-((2-(dimethylphosphoryl)phenyl)amino)pyrimidin-2-yl)amino)-4-methoxy-2-(pyrrolidin-1-yl)phenyl)-5-(4-methylpiperazin-1-yl)pent-2-inamid;
N-(5-((5-Cyclopropyl-4-((2-(dimethylphosphoryl)phenyl)amino)pyrimidin-2-yl)amino)-2-((2-(diethylamino)ethyl)(methyl)amino)-4-methoxyphenyl)acrylamid;
N-(5-((5-Chlor-4-((2-(dimethylphosphoryl)phenyl)amino)pyrimidin-2-yl)amino)-2-((1-(diethylamino)-3-fluorpropan-2-yl)oxy)-4-methoxyphenyl)acrylamid;
N-(5-((5-Chlor-4-((2-(dimethylphosphoryl)phenyl)amino)pyrimidin-2-yl)amino)-4-methoxy-2-morpholinophenyl)-5-(2-(methoxymethyl)pyrrolidin-1-yl)pent-2-inamid;
N-(5-((5-Chlor-4-((2-(dimethylphosphoryl)phenyl)amino)pyrimidin-2-yl)amino)-4-methoxy-2-((1-(2-methoxyethyl)piperidin-3-yl)oxy)phenyl)acrylamid;
N-(5-((5-Chlor-4-((2-(dimethylphosphoryl)phenyl)amino)pyrimidin-2-yl)amino)-2-(3-(dimethylamino)prop-1-in-1-yl)-4-methoxyphenyl)acrylamid;
N-(3-((5-Chlor-4-((4-(2-(dimethylamino)ethoxy)-2(dimethylphosphoryl)phenyl)amino)pyrimidin-2-yl)amino)-4-methoxyphenyl) acrylamid;
N-(3-((5-Chlor-4-((4-(3-(dimethylamino)propoxy)-2(dimethylphosphoryl)phenyl)amino)pyrimidin-2-yl)amino)-4-methoxyphenyl) acrylamid;
1-(6-((5-Chlor-4-((2-(dimethylphosphoryl)phenyl)amino)pyrimidin-2-yl)amino)-5-methoxyindolin-1-yl)prop-2-en-1-on;
N-(5-((5-Chlor-4-((2-(dimethylphosphoryl)phenyl)amino)pyrimidin-2-yl)amino)-2-(4-(dimethylamino)but-1-in-1-yl)-4-methoxyphenyl)acrylamid;
(R)-N-(5-((5-Chlor-4-((2-(dimethylphosphoryl)phenyl)amino)pyrimidin-2-yl)amino)-2-((1-(dimethylamino)-3-methoxypropan-2-yl)oxy)-4-methoxyphenyl) acrylamid;
N-(5-((5-Chlor-4-((2-(dimethylphosphoryl)phenyl)amino)pyrimidin-2-yl)amino)-2-((1-(dimethylamino)-3-methoxypropan-2-yl)oxy)-4-methoxyphenyl) acrylamid;
N-(3-((4-((2-(Dimethylphosphoryl)phenyl)amino)-5-methoxypyrimidin-2-yl)amino)-4-methoxyphenyl)acrylamid;
2-Chlor-N-(3-((5-chlor-4-((2-(dimethylphosphoryl)phenyl)amino)pyrimidin-2-yl)amino)-4-methoxyphenyl) acrylamid;
N-(5-((5-Chlor-4-((2-(dimethylphosphoryl)phenyl)amino)pyrimidin-2-yl)amino)-4-methoxy-2-methylphenyl) acrylamid;
N-(3-((5-Chlor-4-((2-(dimethylphosphoryl)phenyl)amino)pyrimidin-2-yl)amino)-4-(trifluormethoxy)phenyl)acrylamid;
N-(3-((5-Chlor-4-((2-(dimethylphosphoryl)phenyl)amino)pyrimidin-2-yl)amino)-4-ethylphenyl)acrylamid;
N-(4-Chlor-3-((5-chlor-4-((2-(dimethylphosphoryl)phenyl)amino)pyrimidin-2-yl)amino)phenyl)acrylamid;
N-(3-((5-Chlor-4-((2-(dimethylphosphoryl)phenyl)amino)pyrimidin-2-yl)amino)-4-(oxetan-3-yloxy)phenyl)acrylamid;
N-(3-((5-Cyano-4-((2-(dimethylphosphoryl)phenyl)amino)pyrimidin-2-yl)amino)-4-methoxyphenyl) acrylamid;
N-(5-((5-chlor-4-((2-(dimethylphosphoryl)phenyl)amino)pyrimidin-2-yl)amino)-2-((1-(dimethylamino)-3-ethoxypropan-2-yl)oxy)-4-methoxyphenyl) acrylamid;
N-(5-((5-Chlor-4-((2-(dimethylphosphoryl)phenyl)amino)pyrimidin-2-yl)amino)-2-((1-(dimethylamino)-3-methoxypropan-2-yl)oxy)-4-methylphenyl)acrylamid;
N-(5-((5-Chlor-4-((2-(dimethylphosphoryl)phenyl)amino)pyrimidin-2-yl)amino)-2-((1-(dimethylamino)-3-methoxypropan-2-yl)oxy)-4-methylphenyl)acrylamid;
N-(4-Chlor-5-((5-chlor-4-((2-(dimethylphosphoryl)phenyl)amino)pyrimidin-2-yl)amino)-2-((1-(dimethylamino)-3-methoxypropan-2-yl)oxy)phenyl)acrylamid;
N-(4-Chlor-5-((5-chlor-4-((2-(dimethylphosphoryl)phenyl)amino)pyrimidin-2-yl)amino)-2-((1-(dimethylamino)-3-methoxypropan-2-yl)oxy)phenyl)acrylamid;
N-(5-((5-Chlor-4-((2-(dimethylphosphoryl)phenyl)amino)pyrimidin-2-yl)amino)-2-(3-(dimethylamino)prop-1-in-1-yl)-4-methylphenyl)acrylamid;
N-(5-((5-Chlor-4-((2-(dimethylphosphoryl)phenyl)amino)pyrimidin-2-yl)amino)-2-(3-(dimethylamino)prop-1-in-1-yl)-4-ethylphenyl)acrylamid;
N-(5-((5-Chlor-4-((2-(isopropylsulfonyl)phenyl)amino)pyrimidin-2-yl)amino)-2-(3-(dimethylamino)prop-1-in-1-yl)-4-methoxyphenyl)acrylamid;
N-(5-((5-Chlor-4-((2-(dimethylphosphoryl)phenyl)amino)pyrimidin-2-yl)amino)-2-(3-(diethylamino)-3-methylbut-1-in-1-yl)-4-methoxyphenyl)acrylamid;
N-(5-((5-Chlor-4-((2-(dimethylphosphoryl)phenyl)amino)pyrimidin-2-yl)amino)-2-(3-(dimethylamino)-3-methylbut-1-in-1-yl)-4-methoxyphenyl)acrylamid;
N-(4-Chlor-5-((5-chlor-4-((2-(dimethylphosphoryl)phenyl)amino)pyrimidin-2-yl)amino)-2-(3-(dimethylamino)prop-1-in-1-yl)phenyl)acrylamid;
N-(5-((5-Chlor-4-((2-(dimethylphosphoryl)phenyl)amino)pyrimidin-2-yl)amino)-4-methoxy-2-(3-(pyrrolidin-1-yl)prop-1-in-l-yl)phenyl)acrylamid;
N-(5-((5-Chlor-4-((2-(dimethylphosphoryl)phenyl)amino)pyrimidin-2-yl)amino)-2-(3-(diisopropylamino)prop-1-in-1-yl)-4-methoxyphenyl)acrylamid;
N-(5-((5-Chlor-4-((2-(dimethylphosphoryl)phenyl)amino)pyrimidin-2-yl)amino)-4-methoxy-2-(3-(4-methylpiperazin-1-yl)prop-1-in-1-yl)phenyl)acrylamid;
N-(5-((5-Chlor-4-((2-(dimethylphosphoryl)phenyl)amino)pyrimidin-2-yl)amino)-4-methoxy-2-(3-(methylamino)prop-1-in-1-yl)phenyl)acrylamid;
N-(5-((5-Chlor-4-((2-(dimethylphosphoryl)phenyl)amino)pyrimidin-2-yl)amino)-4-methoxy-2-(3-((2-methoxyethyl)(methyl)amino)prop-1-in-1-yl)phenyl)acrylamid;
N-(5-((5-Chlor-4-((2-(dimethylphosphoryl)phenyl)amino)pyrimidin-2-yl)amino)-2-(3-(ethyl(methyl)amino)prop-1-in-1-yl)-4-methoxyphenyl)acrylamid;
N-(3-((6-((3-(Dimethylphosphoryl)pyridin-2-yl)amino)pyrimidin-4-yl)amino)-4-methoxyphenyl)acrylamid;
N-(3-((5-Chlor-4-((4-(dimethylphosphoryl)pyridin-3-yl)amino)pyrimidin-2-yl)amino)-4-methoxyphenyl)acrylamid;
N-(2-(3-(Azepan-1-yl)prop-1-in-1-yl)-5-((5-chlor-4-((2-(dimethylphosphoryl)phenyl)amino)pyrimidin-2-yl)amino)-4-methoxyphenyl)acrylamid;
N-(5-((5-Chlor-4-((2-(dimethylphosphoryl)phenyl)amino)pyrimidin-2-yl)amino)-2-(3-(isopropyl(methyl)amino)prop-1-in-1-yl)-4-methoxyphenyl)acrylamid;
N-(5-((5-Chlor-4-((2-(dimethylphosphoryl)phenyl)amino)pyrimidin-2-yl)amino)-4-methoxy-2-(3-(piperidin-1-yl)prop-1-in-1-yl)phenyl)acrylamid;
N-(5-((5-Chlor-4-((2-(dimethylphosphoryl)phenyl)amino)pyrimidin-2-yl)amino)-2-(3-(cyclopentyl(methyl)amino)prop-1-in-1-yl)-4-methoxyphenyl)acrylamid;
N-(3-((4-((2-(Dimethylphosphoryl)phenyl)amino)-1,3,5-triazin-2-yl)amino)-4-methoxyphenyl)acrylamid;
N-(3-((4-((2-(Dimethylphosphoryl)phenyl)amino)-1,3,5-triazin-2-yl)amino)-4-(trifluormethoxy)phenyl)acrylamid;
N-(3-((6-((2-(Dimethylphosphoryl)phenyl)amino)pyrimidin-4-yl)amino)-4-methoxyphenyl)acrylamid;
(1S,2R,3S,4R)-3-((2-((5-Acrylamido-2-methoxyphenyl)amino)-5-chlorpyrimidin-4-yl)amino)bicyclo[2.2.1]heptan-2-carboxamid;
2-((2-((5-Acrylamido-4-(3-(dimethylamino)pyrrolidin-1-yl)-2-methoxyphenyl)amino)-5-chlorpyrimidin-4-yl)amino)benzamid;
N-(5-((5-Chlor-4-((2-cyanophenyl)amino)pyrimidin-2-yl)amino)-2-(3-(dimethylamino)pyrrolidin-1-yl)-4-methoxyphenyl)acrylamid und
N-(5-((5-Chlor-4-((2-(isopropylsulfonyl)phenyl)amino)pyrimidin-2-yl)amino)-2-(3-(dimethylamino)pyrrolidin-1-yl)-4-methoxyphenyl)acrylamid;
oder ein pharmazeutisch unbedenkliches Salz davon.

2. Verbindung nach Anspruch 1 oder ein pharmazeutisch unbedenkliches Salz davon zur Verwendung bei der Behandlung einer EGFR-getriebenen Krebserkrankung bei einem Empfänger.

3. Verbindung zur Verwendung nach Anspruch 2, wobei der Empfänger mit einer EGFR-getriebenen Krebserkrankung durch das Vorliegen einer Mutation in EGFR (Epidermal Growth Factor Receptor Kinase) gekennzeichnet ist.

4. Verbindung zur Verwendung nach Anspruch 3, wobei die EGFR-getriebene Krebserkrankung durch das Vorliegen einer oder mehrerer Mutationen, die aus (i) L858R, (ii) T790M, (iii) sowohl L858R als auch T790M, (iv) delE764_A750 und (v) sowohl delE764_A750 als auch T790M ausgewählt sind, gekennzeichnet ist.

5. Verbindung zur Verwendung nach einem der Ansprüche 2 bis 4, wobei es sich bei der EGFR-getriebenen Krebserkrankung um nichtkleinzelligen Lungenkrebs (NSCLC); Glioblastom; Bauchspeicheldrüsenkrebs; Kopf- und Halskrebs (z. B. Plattenepithelkarzinom); Brustkrebs; Kolorektalkrebs; Epithelkrebs; Eierstockkrebs; Prostatakrebs oder ein Adenokarzinom handelt.

6. Verbindung nach Anspruch 1 zur Verwendung bei der Behandlung einer EGFR-getriebenen Krebserkrankung, die gegenüber einem aus Erlotinib, Gefitinib und pharmazeutisch unbedenklichen Salzen davon ausgewählten ersten Kinaseinhibitor refraktär ist, bei einem Empfänger.

7. Pharmazeutische Zusammensetzung, umfassend eine Verbindung nach Anspruch 1 oder ein pharmazeutisch unbedenkliches Salz davon.

## Revendications

1. Composé choisi dans le groupe constitué par :
N-(5-((5-chloro-4-((2-(diméthylphosphoryl)phényl)amino)pyrimidin-2-yl) amino)-4-méthoxy-2-(2-(pyrrolidin-1-yl)éthoxy)phényl)acrylamide ;
N-(5-((5-chloro-4-((2-(diméthylphosphoryl)phényl)amino)pyrimidin-2-yl) amino)-4-méthoxy-2-(2-(4-méthylpipérazin-1-yl)éthoxy)phényl)acrylamide ;
N-(5-((5-chloro-4-((2-(diméthylphosphoryl)phényl)amino)pyrimidin-2-yl) amino)-4-méthoxy-2-((1-méthylpipéridin-4-yl)méthoxy)phényl)acrylamide ;
N-(5-((5-chloro-4-((2-(diméthylphosphoryl)phényl)amino)pyrimidin-2-yl) amino)-4-méthoxy-2-(2-morpholinoéthoxy)phényl)acrylamide ;
N-(5-((5-chloro-4-((2-(diméthylphosphoryl)phényl)amino)pyrimidin-2-yl) amino)-4-méthoxy-2-(3-morpholinopropoxy)phényl)acrylamide ;
N-(5-((5-chloro-4-((2-(diméthylphosphoryl)phényl)amino)pyrimidin-2-yl) amino)-4-méthoxy-2-((tétrahydro-2H-pyran-4-yl)méthoxy)phényl)acrylamide ;
N-(5-((5-chloro-4-((2-(diméthylphosphoryl)phényl)amino)pyrimidin-2-yl) amino)-4-méthoxy-2-((1-méthylpipéridin-2-yl)méthoxy)phényl)acrylamide ;
N-(5-((5-chloro-4-((2-(diméthylphosphoryl)phényl)amino)pyrimidin-2-yl) amino)-4-méthoxy-2-(2-(pipéridin-1-yl)éthoxy)phényl)acrylamide ;
N-(5-((5-chloro-4-((2-(diméthylphosphoryl)phényl)amino)pyrimidin-2-yl) amino)-4-méthoxy-2-(oxétan-3-yloxy)phényl)acrylamide ;
N-(5-((5-chloro-4-((2-(diméthylphosphoryl)phényl)amino)pyrimidin-2-yl) amino)-4-méthoxy-2-(pyridin-3-ylméthoxy)phényl)acrylamide ;
N-(5-((5-chloro-4-((2-(diméthylphosphoryl)phényl)amino)pyrimidin-2-yl) amino)-4-méthoxy-2-(pyridin-4-ylméthoxy)phényl)acrylamide ;
N-(5-((5-chloro-4-((2-(diméthylphosphoryl)phényl)amino)pyrimidin-2-yl) amino)-4-méthoxy-2-((1-méthylpipéridin-3-yl)oxy)phényl)acrylamide ;
N-(5-((5-chloro-4-((2-(diméthylphosphoryl)phényl)amino)pyrimidin-2-yl) amino)-4-méthoxy-2-((1-méthylpyrrolidin-3-yl)oxy)phényl)acrylamide ;
rel-(R)-N-(5-((5-chloro-4-((2-(diméthylphosphoryl)phényl)amino)pyrimidin-2-yl) amino)-4-méthoxy-2-((1-méthylpipéridin-3-yl)oxy)phényl)acrylamide ;
N-(2-((1-(azétidin-1-yl)-3-méthoxypropan-2-yl)oxy)-5-((5-chloro-4-((2-(diméthylphosphoryl)phényl)amino)pyrimidin-2-yl) amino)-4-méthoxyphényl)acrylamide ;
rel-(R)-N-(5-((5-chloro-4-((2-(diméthylphosphoryl)phényl)amino)pyrimidin-2-yl) amino)-4-méthoxy-2-((1-méthylpipéridin-3-yl)oxy)phényl)acrylamide ;
N-(5-((5-chloro-4-((2-(diméthylphosphoryl)phényl)amino)pyrimidin-2-yl) amino)-4-méthoxy-2-((1-méthylazétidin-3-yl)oxy)phényl)acrylamide ;
N-(5-((5-chloro-4-((2-(diméthylphosphoryl)phényl)amino)pyrimidin-2-yl)-amino)-4-méthoxy-2-((l-méthylpyrrolidin-2-yl)méthoxy)phényl)acrylamide ;
N-(5-((5-chloro-4-((2-(diméthylphosphoryl)phényl)amino)pyrimidin-2-yl) amino)-4-méthoxy-2-((4-méthyl-1,4-oxazépan-6-yl)oxy)phényl)acrylamide ;
N-(5-((5-chloro-4-((2-(diméthylphosphoryl)phényl)amino)pyrimidin-2-yl) amino)-4-méthoxy-2-((1-méthylpyrrolidin-3-yl)méthoxy)phényl)acrylamide ;
N-(5-((5-chloro-4-((2-(diméthylphosphoryl)phényl)amino)pyrimidin-2-yl) amino)-2-((2-(diméthylamino)cyclohexyl)oxy)-4-méthoxyphényl)acrylamide ;
N-(5-((5-chloro-4-((2-(diméthylphosphoryl)phényl)amino)pyrimidin-2-yl) amino)-4-méthoxy-2-(4-(4-méthylpipérazin-1-yl)pipéridin-1-yl)phényl)acrylamide ;
N-(5-((5-chloro-4-((2-(diméthylphosphoryl)phényl)amino)pyrimidin-2-yl) amino)-2-(4-(diméthylamino)pipéridin-1-yl)-4-méthoxyphényl)acrylamide ;
N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)acrylamide ;
N-(5-((5-chloro-4-((2-(diméthylphosphoryl)phényl)amino)pyrimidin-2-yl) amino)-4-méthoxy-2-(4-méthyl-1,4-diazépan-1-yl)phényl)acrylamide ;
rac-(R)-N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)acrylamide ;
N-(5-((5-chloro-4-((2-(diméthylphosphoryl)phényl)amino)pyrimidin-2-yl) amino)-4-méthoxy-2-(4-(1-méthylpipéridin-4-yl)pipérazin-1-yl)phényl)acrylamide ;
N-(5-((5-chloro-4-((2-(diméthylphosphoryl)phényl)amino)pyrimidin-2-yl) amino)-4-méthoxy-2-(3-morpholinopyrrolidin-1-yl)phényl)acrylamide ;
rac-(R)-N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)acrylamide ;
N-(5-((5-chloro-4-((2-(diméthylphosphoryl)phényl)amino)pyrimidin-2-yl) amino)-2-(3-(diméthylamino)pipéridin-1-yl)-4-méthoxyphényl)acrylamide ;
N-(5-((5-chloro-4-((2-(diméthylphosphoryl)phényl)amino)pyrimidin-2-yl) amino)-4-méthoxy-2-(3-(pyrrolidin-1-yl)azétidin-1-yl)phényl)acrylamide ;
N-(5-((5-chloro-4-((2-(diméthylphosphoryl)phényl)amino)pyrimidin-2-yl) amino)-2-(3-hydroxypyrrolidin-1-yl)-4-méthoxyphényl)acrylamide ;
N-(5-((5-chloro-4-((2-(diméthylphosphoryl)phényl)amino)pyrimidin-2-yl) amino)-4-méthoxy-2-(3-(pipéridin-1-yl)pyrrolidin-1-yl)phényl)acrylamide ;
N-(5-((5-chloro-4-((2-(diméthylphosphoryl)phényl)amino)pyrimidin-2-yl) amino)-2-(3-(diéthylamino)pyrrolidin-1-yl)-4-méthoxyphényl)acrylamide ;
N-(5-((5-chloro-4-((2-(diméthylphosphoryl)phényl)amino)pyrimidin-2-yl) amino)-2-(3-hydroxy-[1,3'-bipyrrolidin]-1'-yl)-4-méthoxyphényl)acrylamide ;
N-(2-([1,3'-bipyrrolidin]-1'-yl)-5-((5-chloro-4-((2-(diméthylphosphoryl)phényl)amino)pyrimidin-2-yl) amino)-4-méthoxyphényl)acrylamide ;
N-(5-((5-chloro-4-((2-(diméthylphosphoryl)phényl)amino)pyrimidin-2-yl) amino)-4-méthoxy-2-(3-(4-méthylpipérazin-1-yl)pyrrolidin-1-yl)phényl)acrylamide ;
N-(5-((5-chloro-4-((2-(diméthylphosphoryl)phényl)amino)pyrimidin-2-yl) amino)-2-(3-((2-(diéthylamino)éthyl)(méthyl)amino)pyrrolidin-1-yl)-4-méthoxyphényl)acrylamide ;
N-(5-((5-chloro-4-((2-(diméthylphosphoryl)phényl)amino)pyrimidin-2-yl) amino)-2-(3-(4-hydroxypipéridin-1-yl)pyrrolidin-1-yl)-4-méthoxyphényl)acrylamide ;
N-(5-((5-chloro-4-((2-(diméthylphosphoryl)phényl)amino)pyrimidin-2-yl) amino)-2-(3-(4-(2-hydroxyéthyl)pipérazin-1-yl)pyrrolidin-1-yl)-4-méthoxyphényl)acrylamide ;
N-(5-((5-chloro-4-((2-(diméthylphosphoryl)phényl)amino)pyrimidin-2-yl) amino)-4-méthoxy-2-(3-((2-méthoxyéthyl)méthyl)amino)pyrrolidin-1-yl)phényl)acrylamide ;
N-(2-([1,3'-bipyrrolidin]-1'-yl)-5-((5-chloro-4-((2-(diméthylphosphoryl)phényl)amino)pyrimidin-2-yl) amino)-4-méthoxyphényl)acrylamide ;
N-(3-((5-chloro-4-((2-(diméthylphosphoryl)phényl)amino)pyrimidin-2-yl) amino)-4-méthoxyphényl)-2-(pyrrolidin-1-ylméthyl)acrylamide ;
N-(3-((5-chloro-4-((2-(diméthylphosphoryl)phényl)amino)pyrimidin-2-yl) amino)-4-méthoxyphényl)-2-(pipéridin-1-ylméthyl)acrylamide ;
N-(3-((5-chloro-4-((2-(diméthylphosphoryl)phényl)amino)pyrimidin-2-yl) amino)-4-méthoxyphényl)-2-((4-(méthylsulfonyl)pipérazin-1-yl)méthyl)acrylamide ;
N-(3-((5-chloro-4-((2-(diméthylphosphoryl)phényl)amino)pyrimidin-2-yl) amino)-4-méthoxyphényl)-2-((4-(4-méthylpipérazin-1-yl)pipéridin-1-yl)méthyl)acrylamide ;
N-(3-((5-chloro-4-((2-(diméthylphosphoryl)phényl)amino)pyrimidin-2-yl) amino)-4-méthoxyphényl)-2-((1,1-dioxothiomorpholino)méthyl)acrylamide ;
N-(3-((5-chloro-4-((2-(diméthylphosphoryl)phényl)amino)pyrimidin-2-yl) amino)-4-méthoxyphényl)-2-((3-oxopipérazin-1-yl)méthyl)acrylamide ;
N-(3-((5-chloro-4-((2-(diméthylphosphoryl)phényl)amino)pyrimidin-2-yl) amino)-4-méthoxyphényl)-2-(morpholinométhyl)acrylamide ;
N-(3-((5-chloro-4-((2-(diméthylphosphoryl)phényl)amino)pyrimidin-2-yl) amino)-4-méthoxyphényl)-2-((4-méthylpipérazin-1-yl)méthyl)acrylamide ;
N-(3-((5-chloro-4-((2-(diméthylphosphoryl)phényl)amino)pyrimidin-2-yl) amino)-4-méthoxyphényl)-2-((4-isopropylpipérazin-1-yl)méthyl)acrylamide ;
N-(3-((5-chloro-4-((2-(diméthylphosphoryl)phényl)amino)pyrimidin-2-yl) amino)-4-méthoxyphényl)-2-((4-morpholinopipéridin-1-yl)méthyl)acrylamide ;
N-(3-((5-chloro-4-((2-(diméthylphosphoryl)phényl)amino)pyrimidin-2-yl)amino)-4-méthoxyphényl)-2-((4-(2-hydroxyéthyl)pipérazin-1-yl)méthyl)acrylamide ;
N-(3-((5-chloro-4-((2-(diméthylphosphoryl)phényl)amino)pyrimidin-2-yl)amino)-4-méthoxyphényl)-2-((3-hydroxypyrrolidin-1-yl)méthyl)acrylamide ;
N-(3-((5-chloro-4-((2-(diméthylphosphoryl)phényl)amino)pyrimidin-2-yl)amino)-4-fluorophényl)acrylamide ;
N-(3-((5-chloro-4-((2-(diméthylphosphoryl)phényl)amino)pyrimidin-2-yl) amino)-4-méthoxyphényl)-2-((4-méthylpipérazin-1-yl)méthyl)phényl)acrylamide ;
N-(5-((5-chloro-4-( (2-(diméthylphosphoryl)phényl)amino)pyrimidin-2-yl)amino)-4-méthoxy-2-(4-méthylpipérazine-1-carbonyl)phényl)acrylamide ;
N-(2-(3-aminopyrrolidin-1-yl)-5-((5-chloro-4-((2-(diméthylphosphoryl)phényl)amino)pyrimidin-2-yl)amino)-4-méthoxyphényl)acrylamide ;
N1-(3-((5-chloro-4-((2-(diméthylphosphoryl)phényl)amino)pyrimidin-2-yl)amino)-4-méthoxyphényl)-N4-(2-(diméthylamino)éthyl)fumaramide ;
(E)-N-(3-((5-chloro-4-((2-(diméthylphosphoryl)phényl)amino)pyrimidin-2-yl)amino)-4-méthoxyphényl)-4-(4-méthylpipérazin-1-yl)-4-oxobut-2-énamide ;
N1-(3-((5-chloro-4-((2-(diméthylphosphoryl)phényl)amino)pyrimidin-2-yl)amino)-4-méthoxyphényl)-N4,N4-diméthylfumaramide ;
acide (Z)-4-((3-((5-chloro-4-((2-(diméthylphosphoryl)phényl)amino)pyrimidin-2-yl)amino)-4-méthoxyphényl)amino)-4-oxobut-2-énoïque ;
N1-(3-((5-chloro-4-((2-(diméthylphosphoryl)phényl)amino)pyrimidin-2-yl)amino)-4-méthoxyphényl)-N4,N4-diméthylmaléamide;
(Z)-N-(3-((5-chloro-4-((2-(diméthylphosphoryl)phényl)amino)pyrimidin-2-yl)amino) -4-méthoxyphényl)-4-(4-méthylpipérazin-1-yl)-4-oxobut-2-énamide ;
N1-(3-((5-chloro-4-((2-(diméthylphosphoryl)phényl)amino)pyrimidin-2-yl)amino)-4-méthoxyphényl)-N4-(2-(diméthylamino)éthyl)maléamide ;
N-(2-(3-amino-3-méthylbut-1-yn-1-yl)-5-((5-chloro-4-((2-(diméthylphosphoryl)phényl)amino)pyrimidin-2-yl)amino)-4-méthoxyphényl)acrylamide ;
N-(5-((5-chloro-4-((2-(diméthylphosphoryl)phényl)amino)pyrimidin-2-yl)amino)-2-(2-(diméthylamino)-3-hydroxypropoxy)-4-méthoxyphényl)acrylamide ;
N-(5-((5-chloro-4-((2-(diméthylphosphoryl)phényl)amino)pyrimidin-2-yl)amino)-2-(2-((2-fluoroéthyl)(méthyl)amino)éthyl)(méthyl)amino)-4-méthoxyphényl)acrylamide ;
N-(2-(3-(azétidin-1-yl)pyrrolidin-1-yl)-5-((5-chloro-4-((2-(diméthylphosphoryl)phényl)amino)pyrimidin-2-yl)amino)-4-méthoxyphényl)acrylamide ;
N-(2-([1,4'-bipipéridin]-1'-yl)-5-((5-chloro-4-((2-(diméthylphosphoryl)phényl)amino)pyrimidin-2-yl)amino)-4-méthoxyphényl)acrylamide ;
N-(5-((5-chloro-4-((2-(diméthylphosphoryl)phényl)amino)pyrimidin-2-yl)amino)-4-méthoxy-2-(4-morpholinopipéridin-1-yl)phényl)acrylamide ;
N-(5-((5-chloro-4-((2-(diméthylphosphoryl)phényl)amino)pyrimidin-2-yl)amino)-4-méthoxy-2-(4-(4-méthyl-1,4-diazépan-1-yl)pipéridin-1-yl)phényl)acrylamide ;
N-(5-((5-chloro-4-((2-(diméthylphosphoryl)phényl)amino)pyrimidin-2-yl)amino)-4-méthoxy-2-(4-(pyrrolidin-1-yl)pipéridin-1-yl)phényl)acrylamide ;
N-(5-((5-chloro-4-((2-(diméthylphosphoryl)phényl)amino)pyrimidin-2-yl)amino)-2-((1-(diméthylamino)cyclopropyl)éthynyl)-4-méthoxyphényl)acrylamide ;
N-(5-((5-chloro-4-((2-(diméthylphosphoryl)phényl)amino)pyrimidin-2-yl)amino)-4-méthoxy-2-(2-(4-méthylpipérazin-1-yl)éthyl)phényl)acrylamide ;
(E)-4-((3-((5-chloro-4-((2-(diméthylphosphoryl)phényl)amino)pyrimidin-2-yl)amino)-4-méthoxyphényl)amino)-4-oxobut-2-énoate d'éthyle ;
(E)-(1-(3-((3-((5-chloro-4-((2-(diméthylphosphoryl)phényl)amino)pyrimidin-2-yl)amino)-4-méthoxyphényl)amino)-3-oxoprop-1-én-1-yl)cyclopropyl)carbamate de tert-butyle ;
N-(3-((5-chloro-4-((2-(diméthylphosphoryl)phényl)amino)pyrimidin-2-yl)amino)-4-méthoxyphényl)-2-fluoroacrylamide ;
(E)-3-chloro-N-(3-((5-chloro-4-((2-(diméthylphosphoryl)phényl)amino)pyrimidin-2-yl)amino)-4-méthoxyphényl)acrylamide ;
N-(3-((5-chloro-4-((2-(diméthylphosphoryl)phényl)amino)pyrimidin-2-yl) amino)-4-méthoxyphényl)cyclohex-1-ènecarboxamide ;
N-(5-((5-chloro-4-((2-(diméthylphosphoryl)phényl)amino)pyrimidin-2-yl) amino)-4-méthoxy-2-(2-(pyrrolidin-1-yl)éthoxy)phényl)but-2-ynamide ;
N-(3-((5-chloro-4-((2-(diméthylphosphoryl)phényl)amino)pyrimidin-2-yl) amino)-4-méthoxyphényl)-4-(diméthylamino)but-2-ynamide ;
N-(3-((5-chloro-4-((2-(diméthylphényl)amino)pyrimidin-2-yl)amino)-4-méthoxyphényl)-2-(trifluorométhyl)acrylamide ;
N-(3-((5-chloro-4-((2-(diméthylphosphoryl)phényl)amino)pyrimidin-2-yl) amino)-4-méthoxyphényl)-1-méthyl-1,2,5,6-tétrahydropyridine-3-carboxamide ;
(E)-3-chloro-N-(5-((5-chloro-4-((2-(diméthylphophoryl)phényl)amino)pyrimidin-2-yl)amino)-2-fluoro-4-méthoxyphényl)acrylamide ;
N-(3-((5-chloro-4-((2-(diméthylphosphoryl)-4-(2-méthoxyéthoxy)phényl)amino)pyrimidin-2-yl)amino)-4-méthoxyphényl)acrylamide ;
N-(5-((5-chloro-4-((2-(diméthylphosphoryl)phényl)amino)pyrimidin-2-yl) amino)-2-fluoro-4-méthoxyphényl)-4-(diméthylamino)but-2-ynamide ;
N-(5-((5-chloro-4-((2-(diméthylphosphoryl)phényl)amino)pyrimidin-2-yl) amino)-2-fluoro-4-méthoxyphényl)-5-(pipéridin-1-yl)pent-2-ynamide ;
N-(5-((5-chloro-4-((2-(diméthylphosphoryl)phényl)amino)pyrimidin-2-yl) amino)-2-fluoro-4-méthoxyphényl)-4-(pyrrolidin-1-yl)but-2-ynamide ;
N-(5-((5-chloro-4-((2-(diméthylphosphoryl)phényl)amino)pyrimidin-2-yl) amino)-2-fluoro-4-méthoxyphényl)-4-méthyl-4-(pyrrolidin-1-yl)pent-2-ynamide ;
N-(3-((5-chloro-4-((2-(diméthylphosphoryl)phényl)amino)pyrimidin-2-yl) amino)-4-méthoxyphényl)-4-méthyl-4-(pyrrolidin-1-yl)pent-2-ynamide ;
(E)-N-(3-((5-chloro-4-((2-(diméthylphosphoryl)phényl)pyrimidin-2-yl)amino)-4-méthoxyphényl)-4-(pipéridin-1-yl)but-2-énamide ;
N-(3-((5-chloro-4-((2-(diméthylphosphoryl)phényl)amino)pyrimidin-2-yl) amino)-4-méthoxyphényl)-3-phénylpropiolamide ;
(E)-3-(1-aminocyclopropyl)-N-(3-((5-chloro-4-((2-(diméthylphosphoryl)phényl)amino)pyrimidin-2-yl) amino)-4-méthoxyphényl)acrylamide ;
(E)-N-(3-((5-chloro-4-((2-(diméthylphosphoryl)phényl)amino)pyrimidin-2-yl) amino)-4-méthoxyphényl)-4-(pyrrolidin-1-yl)but-2-énamide ;
N-(5-((5-chloro-4-((2-(diméthylphosphoryl)phényl)amino)pyrimidin-2-yl) amino)-2-(3-(diéthylamino)prop-1-yn-1-yl)-4-méthoxyphényl)acrylamide ;
N-(3-((5-chloro-4-((2-(diméthylphosphoryl)phényl)amino)pyrimidin-2-yl) amino)-4-méthoxyphényl)-4-méthoxybut-2-ynamide ;
N-(5-((5-chloro-4-((2-(diméthylphosphoryl)-4-(2-(pyrrolidin-1-yl)éthoxy)phényl)amino)pyrimidin-2-yl)amino)-2-fluoro-4-méthoxyphényl)acrylamide ;
N-(3-((5-chloro-4-((2-(diméthylphosphoryl)-4-((tétrahydrofuran-3-yl)oxy)phényl)amino)pyrimidin-2-yl)amino)-4-méthoxyphényl)acrylamide ;
N-(3-((5-chloro-4-((2-(diméthylphosphoryl)-4-((tétrahydro-2H-pyran-4-yl)méthoxy)phényl)amino)pyrimidin-2-yl)amino)-4-méthoxyphényl)acrylamide ;
N-(5-((5-chloro-4-((2-(diméthylphosphoryl)phényl)amino)pyrimidin-2-yl) amino)-4-méthoxy-2-morpholinophényl)-5-(pipéridin-1-yl)pent-2-ynamide ;
N-(5-((5-chloro-4-((2-(diméthylphosphoryl)phényl)amino)pyrimidin-2-yl) amino)-4-méthoxy-2-((tétrahydro-2H-pyran-4-yl)méthoxy)phényl)but-2-ynamide ;
N-(5-((5-chloro-4-((2-(diméthylphosphoryl)phényl)amino)pyrimidin-2-yl) amino)-4-méthoxy-2-(oxétan-3-yloxy)phényl)but-2-ynamide ;
N-(5-((5-chloro-4-((2-(diméthylphosphoryl)phényl)amino)pyrimidin-2-yl) amino)-4-méthoxy-2-morpholinophényl)-4-(diméthylamino)but-2-ynamide ;
N-(5-((5-chloro-4-((2-(diméthylphosphoryl)phényl)amino)pyrimidin-2-yl) amino)-4-méthoxy-2-morpholinophényl)-4-méthoxybut-2-ynamide ;
N-(5-((5-chloro-4-((2-(diméthylphosphoryl)phényl)amino)pyrimidin-2-yl) amino)-4-méthoxy-2-(4-(4-méthylpipérazin-1-yl)pipéridin-1-yl)phényl)but-2-ynamide ;
N-(3-((5-chloro-4-((2-(diméthylphosphoryl)-4-(2-(pipéridin-1-yl)éthoxy)phényl)amino)pyrimidin-2-yl)amino)-4-méthoxyphényl)acrylamide ;
N1-(3-((5-chloro-4-((2-(diméthylphosphoryl)phényl)amino)pyrimidin-2-yl) amino)-4-méthoxyphényl)-N4-méthylfumaramide ;
N1-(3-((5-chloro-4-((2-(diméthylphosphoryl)phényl)amino)pyrimidin-2-yl) amino)-4-méthoxyphényl)-N4-méthylmaléamide ;
N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)-4-méthoxybut-2-ynamide ;
N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)but-2-ynamide ;
rac-(R)-N-(5-((5-chloro-4-((2-(diméthylphosphoryl)phényl)amino)pyrimidin-2-yl) amino)-2-(3-(diméthylamino)pyrrolidin-1-yl)-4-méthoxyphényl)méthacrylamide ;
N-(5-((5-chloro-4-((2-(diméthylphosphoryl)phényl)amino)pyrimidin-2-yl) amino)-4-méthoxy-2-morpholinophényl)-5-(diméthylamino)pent-2-ynamide ;
N-(5-((5-chloro-4-((2-(diméthylphoshoryl)phényl) amino)pyrimidin-2-yl) amino)-4-méthoxy-2-morpholinophényl)-5-méthoxypent-2-ynamide ;
N-(5-((5-chloro-4-((2-(diméthylphosphoryl)phényl)amino)pyrimidin-2-yl) amino)-4-méthoxy-2-(3-méthyl-3-(pyrrolidin-1-yl)but-1-yn-1-yl)phényl)acrylamide ;
N-(3-((5-chloro-4-((2-(diméthylphosphoryl)phényl)amino)pyrimidin-2-yl) amino)-4-(2-(diméthylamino)éthoxy)phényl)acrylamide ;
N-(5-((5-chloro-4-((2-(diméthylphosphoryl)phényl)amino)pyrimidin-2-yl) amino)-4-méthoxy-2-morpholinophényl)-5-(4-méthylpipérazin-1-yl)pent-2-ynamide ;
N-(5-((5-chloro-4-((2-(diméthylphosphoryl)phényl)amino)pyrimidin-2-yl) amino)-4-méthoxy-2-(pyrrolidin-1-yl)phényl)-5-(4-méthylpipérazin-1-yl)pent-2-ynamide ;
N-(5-((5-cyclopropyl-4-((2-(diméthylphosphoryl)phényl)amino)pyrimidin-2-yl) amino)-2-((2-(diéthylamino)éthyl)(méthyl)amino)-4-méthoxyphényl)acrylamide ;
N-(5-((5-chloro-4-((2-(diméthylphosphoryl)phényl)amino)pyrimidin-2-yl) amino)-2-((1-(diéthylamino)-3-fluoropropan-2-yl)oxy)-4-méthoxyphényl)acrylamide;
N-(5-((5-chloro-4-((2-(diméthylphosphoryl)phényl)amino)pyrimidin-2-yl) amino)-4-méthoxy-2-morpholinophényl)-5-(2-(méthoxyméthyl)pyrrolidin-1-yl)pent-2-ynamide ;
N-(5-((5-chloro-4-((2-(diméthylphosphoryl)phényl)amino)pyrimidin-2-yl) amino)-4-méthoxy-2-((1-(2-méthoxyéthyl)pipéridin-3-yl)oxy)phényl)acrylamide ;
N-(5-((5-chloro-4-((2-(diméthylphosphoryl)phényl)amino)pyrimidin-2-yl) amino)-2-(3-(diméthylamino)prop-1-yn-1-yl)-4-méthoxyphényl)acrylamide ;
N-(3-((5-chloro-4-((4-(2-(diméthylamino)éthoxy)-2-(diméthylphosphoryl)phényl)amino)pyrimidin-2-yl) amino)-4-méthoxyphényl)acrylamide ;
N-(3-((5-chloro-4-((4-(3-(diméthylamino)propoxy)-2-(diméthylphosphoryl)phényl)amino)pyrimidin-2-yl) amino)-4-méthoxyphényl)acrylamide ;
1-(6-((5-chloro-4-((2-(diméthylphosphoryl)phényl)amino)pyrimidin-2-yl) amino)-5-méthoxyindolin-1-yl)prop-2-én-1-one ;
N-(5-((5-chloro-4-((2-(diméthylphosphoryl)phényl)amino)pyrimidin-2-yl) amino)-2-(4-(diméthylamino)but-1-yn-1-yl)-4-méthoxyphényl)acrylamide ;
(R)-N-(5-((5-chloro-4-((2-(diméthylphosphoryl)phényl)amino)pyrimidin-2-yl) amino)-2-((1-(diméthylamino)-3-méthoxypropan-2-yl)oxy)-4-méthoxyphényl)acrylamide ;
N-(5-((5-chloro-4-((2-(diméthylphosphoryl)phényl)amino)pyrimidin-2-yl) amino)-2-((1-(diméthylamino)-3-méthoxypropan-2-yl)oxy)-4-méthoxyphényl)acrylamide ;
N-(3-((4-((2-(diméthylphosphoryl)phényl)amino)-5-méthoxypyrimidin-2-yl)amino)-4-méthoxyphényl)acrylamide ;
2-chloro-N-(3-((5-chloro-4-((2-(diméthylphosphoryl)phényl)aminp)pyrimidin-2-yl)amino)-4-méthoxyphényl)acrylamide ;
N-(5-((5-chloro-4-((2-(diméthylphosphoryl)phényl)amino)pyrimidin-2-yl) amino)-4-méthoxy-2-méthylphényl)acrylamide ;
N-(3-((5-chloro-4-((2-(diméthylphosphoryl)phényl)amino)pyrimidin-2-yl) amino)-4-(trifluorométhoxy)phényl)acrylamide ;
N-(3-((5-chloro-4-((2-(diméthylphosphoryl)phényl)amino)pyrimidin-2-yl) amino)-4-éthylphényl)acrylamide ;
N-(4-chloro-3-((5-chloro-4-((2-(diméthylphosphoryl)phényl)amino)pyrimidin-2-yl)amino)phényl)acrylamide ;
N-(3-((5-chloro-4-((2-(diméthylphosphoryl)phényl)amino)pyrimidin-2-yl) amino)-4-(oxétan-3-yloxy)phényl)acrylamide ;
N-(3-((5-cyano-4-((2-(diméthylphosphoryl)phényl)amino)pyrimidin-2-yl) amino)-4-méthoxyphényl)acrylamide ;
N-(5-((5-chloro-4-((2-(diméthylphosphoryl)phényl)amino)pyrimidin-2-yl) amino)-2-((1-(diméthylamino)-3-éthoxypropan-2-yl)oxy)-4-méthoxyphényl)acrylamide ;
N-(5-((5-chloro-4-((2-(diméthylphosphoryl)phényl)amino)pyrimidin-2-yl) amino)-2-((1-(diméthylamino)-3-méthoxypropan-2-yl)oxy)-4-méthylphényl)acrylamide ;
N-(5-((5-chloro-4-((2-(diméthylphosphoryl)phényl)amino)pyrimidin-2-yl) amino)-2-((1-(diméthylamino)-3-méthoxypropan-2-yl)oxy)-4-méthylphényl)acrylamide ;
N-(4-chloro-5-((5-chloro-4-((2-(diméthylphosphoryl)phényl)amino)pyrimidin-2-yl) amino)-2-((1-(diméthylamino)-3-méthoxypropan-2-yl)oxy)phényl)acrylamide ;
N-(4-chloro-5-((5-chloro-4-((2-(diméthylphosphoryl)phényl)amino)pyrimidin-2-yl) amino)-2-((1-(diméthylamino)-3-méthoxypropan-2-yl)oxy)phényl)acrylamide ;
N-(5-((5-chloro-4-((2-(diméthylphosphoryl)phényl)amino)pyrimidin-2-yl) amino)-2-(3-(diméthylamino)prop-1-yn-1-yl)-4-méthylphényl)acrylamide ;
N-(5-((5-chloro-4-((2-(diméthylphosphoryl)phényl)amino)pyrimidin-2-yl) amino)-2-(3-(diméthylamino)prop-1-yn-1-yl)-4-éthylphényl)acrylamide ;
N-(5-((5-chloro-4-((2-(isopropylsulfonyl)phényl) amino)pyrimidin-2-yl) amino)-2-(3-(diméthylamino)prop-1-yn-1-yl)-4-méthoxyphényl)acrylamide ;
N-(5-((5-chloro-4-((2-(diméthylphosphoryl)phényl)amino)pyrimidin-2-yl) amino)-2-(3-(diéthylamino)-3-méthylbut-1-yn-1-yl)-4-méthoxyphényl)acrylamide ;
N-(5-((5-chloro-4-((2-(diméthylphosphoryl)phényl)amino)pyrimidin-2-yl) amino)-2-(3-(diméthylamino)-3-méthylbut-1-yn-1-yl)-4-méthoxyphényl)acrylamide ;
N-(4-chloro-5-((5-chloro-4-((2-(diméthylphosphoryl)phényl)amino)pyrimidin-2-yl) amino)-2-(3-(diméthylamino)prop-1-yn-1-yl)phényl)acrylamide ;
N-(5-((5-chloro-4-((2-(diméthylphosphoryl)phényl)amino)pyrimidin-2-yl) amino)-4-méthoxy-2-(3-(pyrrolidin-1-yl)prop-1-yn-1-yl)phényl)acrylamide ;
N-(5-((5-chloro-4-((2-(diméthylphosphoryl)phényl)amino)pyrimidin-2-yl) amino)-2-(3-(diisopropylamino)prop-1-yn-1-yl)-4-méthoxyphényl)acrylamide ;
N-(5-((5-chloro-4-((2-(diméthylphosphoryl)phényl)amino)pyrimidin-2-yl) amino)-4-méthoxy-2-(3-(4-méthylpipérazin-1-yl)prop-1-yn-1-yl)phényl)acrylamide ;
N-(5-((5-chloro-4-((2-(diméthylphosphoryl)phényl)amino)pyrimidin-2-yl) amino)-4-méthoxy-2-(3-(méthylamino)prop-1-yn-1-yl)phényl)acrylamide ;
N-(5-((5-chloro-4-((2-(diméthylphosphoryl)phényl)amino)pyrimidin-2-yl) amino)-4-méthoxy-2-(3-((2-méthoxyéthyl) (méthyl)amino)prop-1-yn-1-yl)phényl)acrylamide ;
N-(5-((5-chloro-4-((2-(diméthylphosphoryl)phényl)amino)pyrimidin-2-yl) amino)-2-(3-(éthyl(méthyl)amino)prop-1-yn-1-yl)-4-méthoxyphényl)acrylamide ;
N-(3-((6-((3-(diméthylphosphoryl)pyridin-2-yl)amino)pyrimidin-4-yl)amino)-4-méthoxyphényl)acrylamide ;
N-(3-((5-chloro-4-((4-(diméthylphosphoryl)pyridin-3-yl)amino)pyrimidin-2-yl) amino)-4-méthoxyphényl)acrylamide ;
N-(2-(3-(azépan-1-yl)prop-1-yn-1-yl)-5-((5-chloro-4-((2-(diméthylphosphoryl)phényl)amino)pyrimidin-2-yl)amino)-4-méthoxyphényl)acrylamide ;
N-(5-((5-chloro-4-((2-(diméthylphosphoryl)phényl)amino)pyrimidin-2-yl) amino)-2-(3-(isopropyl(méthyl)amino)prop-1-yn-1-yl)-4-méthoxyphényl)acrylamide ;
N-(5-((5-chloro-4-((2-(diméthylphosphoryl)phényl)amino)pyrimidin-2-yl)amino-4-méthoxy-2-(3-(pipéridin-1-yl)prop-1-yn-1-yl)phényl)acrylamide ;
N-(5-((5-chloro-4-((2-(diméthylphosphoryl)phényl)amino)pyrimidin-2-yl) amino)-2-(3-(cyclopentyl(méthyl)amino)prop-1-yn-1-yl)-4-méthoxyphényl)acrylamide ;
N-(3-((4-((2-(diméthylphosphoryl)phényl)amino)-1,3,5-triazin-2-yl)amino)-4-méthoxyphényl)acrylamide ;
N-(3-((4-((2-(diméthylphosphoryl)phényl)amino)-1,3,5-triazin-2-yl)amino)-4-(trifluorométhoxy)phényl)acrylamide ;
N-(3-((6-((2-(diméthylphosphoryl)phényl)amino)pyrimidin-4-yl)amino)-4-méthoxyphényl)acrylamide ;
(1S,2R,3S,4R)-3-((2-((5-acrylamido-2-méthoxyphényl)amino)-5-chloropyrimidin-4-yl)amino)bicyclo[2.2.1]heptane-2-carboxamide ;
2-((2-((5-acrylamido-4-(3-(diméthylamino)pyrrolidin-1-yl)-2-méthoxyphényl)amino)-5-chloropyrimidin-4-yl)amino)benzamide ;
N-(5-((5-chloro-4-((2-cyanophényl)amino)pyrimidin-2-yl)amino)-2-(3-(diméthylamino)pyrrolidin-1-yl)-4-méthoxyphényl)acrylamide ; et
N-(5-((5-chloro-4-((2-(isopropylsulfonyl)phényl) amino)pyrimidin-2-yl) amino)-2-(3-(diméthylamino)pyrrolidin-1-yl)-4-méthoxyphényl)acrylamide ;
ou sel pharmaceutiquement acceptable de celui-ci.

2. Composé selon la revendication 1, ou sel pharmaceutiquement acceptable de celui-ci, pour utilisation dans le traitement d'un cancer entraîné par EGFR chez un sujet.

3. Composé pour utilisation selon la revendication 2, ledit sujet souffrant d'un cancer entraîné par EGFR étant **caractérisé par** la présence d'une mutation de la récepteur du facteur de croissance épidermique (EGFR) kinase.

4. Composé pour utilisation selon la revendication 3, où ledit cancer entraîné par EGFR est **caractérisé par** la présence d'une ou de plusieurs mutations choisies parmi : (i) L858R, (ii) T790M, (iii) à la fois L858R et T790M, (iv) delE746_A750, et (v) à la fois delE746_A750 et T790M.

5. Composé pour utilisation selon l'une quelconque des revendications 2 à 4, où ledit cancer entraîné par EGFR est un cancer du poumon non à petites cellules (CPNPC) ; un glioblastome ; un cancer du pancréas ; un cancer de la tête et du cou (par exemple carcinome à cellules squameuses) ; un cancer du sein ; un cancer colorectal ; un cancer épithélial ; un cancer de l'ovaire ; un cancer de la prostate ; ou un adénocarcinome.

6. Composé selon la revendication 1 pour utilisation dans le traitement d'un cancer entraîné par EGFR réfractaire à un premier inhibiteur de kinase choisi parmi l'ériotinib, le géfitinib, et les sels pharmaceutiquement acceptables de ceux-ci chez un sujet.

7. Composition pharmaceutique comprenant un composé selon la revendication 1 ou l'un des sels pharmaceutiquement acceptables de celui-ci.
